# EUROPEAN PATENT APPLICATION

(11) **EP 2 083 273 A1**
(43) Date of publication of application: **29.07.2009**
(21) Application number: 08172455.1
(22) Date of filing: 21.04.2005
(51) Int. Cl.: G01N 33/574

(54) **Use of novel biomarkers for detection of testicular carcinoma in situ and derived cancers in human samples**

(30) Priority: 23.04.2004 DK 200400647; 30.12.2004 DK 200402032
(62) Divisional of application: 05731863.6
(71) Applicant: Rigshospitalet, Copenhagen University Hospital, 2100 Copenhagen Ø (DK); University of Copenhagen, 2200 Copenhagen N (DK); European Molecular Biology Laboratories Heidelberg, 69117 Heidelberg (DE)
(72) Inventor: Almstrup, Kristian, Vindinge (DK); Ansorge, Wilhelm, 1278 La Rippe (CH); Leffers, Henrik, 2200 Copenhagen N (DK); Høi-Hansen, Christina E., 2830 Virum (DK); Rajpert-De Meyts, Ewa, 2670 Greve (DK); Schwager, Christian, 67117 Limburgerhof (DE); Skakkerbæk, Niels Erik, 5320 Farum (DK); Wirkner, Ute, 74925 Epfenbach (DE); Blake, Jonathon, 68723 Offerheim (DE)
(74) Representative: Plougmann & Vingtoft A/S

(57) **Abstract**

The present invention relates to methods and kits for identification of testicular carcinoma in situ (CIS), gonadoblastoma (a CIS-like pre-cancerous lesion found in dysgenetic gonads) and CIS-derived cancers based on at least one of the biomarkers included in the invention. It also relates to diagnosis of a subject's status of the testicular carcinoma in situ and the derived cancers based on the measurement of a relative abundance of one of the biomarkers.

SEQUENCE LISTING

<110> Rigshospitalet
      Almstrup, Kristian
      Ansorge, Wilhelm
      Høi-Hansen, Christina E.
      Rajpert-De Meyts, Ewa
      Leffers, Henrik
      Skakkebæk, Niels Erik

<120> Panel of gene expression for diagnosis
  of testicular cancer

<130> 36243PC01

<150> DK-PA 200402032
<151> 2004-12-30

<150> DK-PA 200400647
<151> 2004-04-23

<160> 255

<170> FastSEQ for Windows Version 4.0

<210> 1
<211> 483
<212> DNA
<213> unknown

<220>
<223> >sr|IMAGp998N195554.2|cl|IMAGp998N195554|gb|BX1087
      31|oid|234551841 BX108731 NCI_CGAP_GC6 Homo
      sapiens cDNA clone IMAGp998N195554 ;
      IMAGE:2244690, mRNA sequence

<400> 1
tggagagctg cgggcaagct cagcctcgaa acacacacac ccacacgaga tgggcacgga 60
gtagtcttga aagacatttt aataaccttg gctgctaagg acaacattga tagaagccgt 120
ctctggctat agataagtag atctaatact agtttggata tctttagggt ttagaatcta 180
acctcaagaa taagaaatac aagtacgaat tggtgatgaa gatgtattcg tattgtttgg 240
gattgggagg ctttgcttat tttttaaaaa ctattgaggt aaagggttaa gctgtaacat 300
acttaattga tttcttaccg tttttggctc tgttttgcta tatcccctaa tttgttggtt 360
gtgctaatct ttgtagaaag aggtcttgta tttgctgcat cgtaatgaca tgagtactac 420
tttagttggt ttaagttcaa atgaatgaaa caaatatttt tcctttagtt gattttaccc 480
tga                                                               483

<210> 2
<211> 683
<212> DNA
<213> unknown

<220>
<223> >ImageID=147103 IMAGP971H0410_S1 CC   FromEXP of
      IMAGp971H0410_s1.exp April 26, 2002 13:06

<221> misc_feature
<222> 675
<223> n=a, t, c or g

<400> 2
caaatggtct aagattgaca gtagcacagt tttactctgt gaaggtaatg ttcaggacaa 60
atttcaagaa aactagaaaa ccattcttta cagctgaaat ctttccctaa ccattgttat 120
ttccactttt aagtcctcaa gagatgagaa aagggaggta aggcttcctt atacatttcc 180
tgcacaatga aacatttttc ctcctccagg caaagattca agcagaactg gcaaatatct 240
tatcttgctc ttctcaataa taataatgtt gttagataat aaagttctat agcaatttaa 300
ccctagaatc tttttgaaaa gtaattcttt aaagttgaga atcacagctg tctagcaagc 360
atttccttgg gcacttgaag ctgtttattc actttggtct ttcctcccag gggaatgaag 420
tgaggccagt ggccgggtct gctgagtccg cagcactcag actacgtgca cctctgcagc 480
aggtgcaggc ccagttgtca cccctccaga acatctcccc atggattctg gcggtattga 540
ctcttcagat tcagaggtag ggatcattct gacttattaa agagctatat aaccagttaa 600
ttccatctgt ttgatgcttg acatccctaa ctagacagat gagggttgaa gttagttttt 660
ggtggggttt ggganggtga aca 683

<210> 3
<211> 718
<212> DNA
<213> unknown

<220>
<223> >sr|IMAGp998F08222.1|cl|IMAGp998F08222|gb|BX091352
      |oid|234517071 BX091352 Soares placenta Nb2HP Homo
      sapiens cDNA clone IMAGp998F08222 ; IMAGE:146695,
      mRNA sequence.

<400> 3
atttttttaa aagactatcc ccttcatcac tgtgctttgt ccctttgtct aaagtcggtt 60
gttcatatat gtgggtctgt tccgtagact ttattcagtt ctattgatct tttttgatat 120
gatgccagta ccatactctg ttagtaacga tagtgtttta ataatacatc ttgaaattag 180
gaagtataga gttattttgc ctatagtaga tcctttattt tgcattttag aatcagctta 240
agagtttctg caaaaatacc tgcaggaatt ttgatggaga ttgcattgat tctctagatc 300
aatttgggga gaattttaac aatattgagt attttgacat atgagtgcaa tatgcctgtc 360
attcattcag gtctttaatt tctctcagga ttgatctttt taaaagtaaa ccctttattt 420
tgggataatt ttcatttttt tcccccttca tgtcacagtt tttcatttta tttttccccc 480
tccatgtcac agttttgatc atggaataat ttttcaagat ctgagagcta cagtgaaaag 540
gaaaaaaaga aagaaagaaa acatgatact atagtccctg atgattgatt ttttttaatc 600
ccaatcatct gtaattcttc ccagagataa gaaatggttg tagtatgtta tctcctagga 660
attttttttg gaattcaaac agataaataa aatatactca tagaaaaaaa aaaaaaaa 718

<210> 4
<211> 461
<212> DNA
<213> unknown

<220>
<223> >gi|855247|sr|IMAGp998A06220s1|cl|IMAGp998A06220|g
      b|R78966|R78966 yi83b03.r1 Homo sapiens cDNA clone
      145805 5'.

<221> misc_feature
<222> 359, 371, 383, 406, 428, 442, 452, 460
<223> n=a, t, c or g

<400> 4
agccaactga acctgctaat tgccagaatt aggcatattc ggctgatctg tcacaggaaa 60

tgctgggctg gagcaggcct cccatgtttt acaacctgcc tacataagac ataagcatct 120
aggagcagag aatccctcct cctttcaagc agctctttcc caacagaata aagggaagtc 180
actgagctgt gaatgcctgt tctattcaga tttacctctt agataagttg ttccagctac 240
cccgggaagg aggggtaaag gggatgagat gtttgaactg taacaccctg tacatgggag 300
gagacaacat gagttgttga tgggagcatc cttccattct tacctttttg ggcttttana 360
aaaactttta ntggcagtgc agnaaaatat ttgaaaattg tttttnacag ggtttttgaa 420
ggccattncc caggctgctg gnaaatcctg gnaaaattgn t 461

<210> 5
<211> 305
<212> DNA
<213> unknown

<220>
<223> >sr|IMAGp998D203167.2|cl|IMAGp998D203167|gb|BX1016
      98|oid|234537773 BX101698 NCI_CGAP_GC4 Homo
      sapiens cDNA clone IMAGp998D203167 ;
      IMAGE:1258339, mRNA sequence

<400> 5
aaccaaagct ttgtgtaaaa tcttgaattt atggggcggg agggtaggaa agcctgtacc 60
tgtctgtttt tttcctgatc cttttccctc attcctgaac tgcaggagac tgagcccctt 120
tgggctttgg tgaccccatc actggggtgt gtttatttga tggttgattt tgctgtactg 180
ggtacttcct ttcccatttt ctaatcattt tttaacacaa gctgactctt cccttccctt 240
ctcctttccc tgggaaaata caatgaataa ataaagactt attggtactc aaaaaaaaaa 300
aaaaa 305

<210> 6
<211> 628
<212> DNA
<213> unknown

<220>
<223> >sr|IMAGp998D054091.2|cl|IMAGp998D054091|gb|BX0925
      81|oid|234519530 BX092581 NCI_CGAP_GC3 Homo
      sapiens cDNA clone IMAGp998D054091 ;
      IMAGE:1613140, mRNA sequence

<221> misc_feature
<222> 600
<223> n=a, t, c or g

<400> 6
ataagatggg aactctcccg gcacgtagta catatcccgc cgtgggtgaa agttcccgaa 60
gacctgaaag atccagaggt gttccaggtc cagacgcggc tgctgaaagc cattttcggc 120
ccggacggat ctcgaatccc ttacatcgag caggtgagca aggccatgct cgagctgaag 180
gctctggagt cttcagacct caccgaggtc gtggtttacg gctcctattt gtacaagctc 240
cggaccaagt ggatgctcca gtccatggct gagtggcacc gccagcgcca ggagcgaggg 300
atgctcaaac ttgccgaagc catgaatgcc ctcgaactag gcccttggat gaagtgaacc 360
agtttccagc caatgcaatg aagccgggtt gcagagatta ggttgtggcc agagctagag 420
tgattcctta agcttgtttt aaaatctgct ccagcctaaa gagttaaggg aaaaccattt 480
gttcccttaa agagttaagg gaaaaccctt ggctctgagt cttgttgtga atatttcttt 540
gatgattgtt aataaaaagt gtttttcttt ttttcccatt tttaaaaata acaataaagn 600
tttaaataag ttgaaaaaaa aaaaaaaa 628

<210> 7
<211> 545
<212> DNA
<213> unknown

<220>
<223> >gi|1526341|gb|AA046430|sr|IMAGp998K241161.1|cl|IM
      AGp998K241161|AA046430 zk70f12.s1
      Soares_pregnant_uterus_NbHPU Homo sapiens cDNA
      clone IMAGE:488207 3' similar to PIR:A54560 A54560
      TPA-induced protein OTS-8 - mouse ;contains
      element PTR5 repetitive element ;, mRNA sequence

<221> misc_feature
<222> 414
<223> n=a, t, c or g

<400> 7
tttttcacat ggttacagca tgtaatatcg atccaatggt ccagtgttag ttacaaatca 60
gtttatacag ttttgctcca ttttctatga attttgtctt tcaaataatc ttttatagaa 120
tgtatattca aaaacatgtt tgaacagcaa atgtttagag gagccaagtc tggtgaacgg 180
tctttccttc ggttattttg gcaagcggag aggtcaccgt ggattctgag tgggccaggc 240
aagtgttcca cgggtcatct gctcccacga gctcagggac agggcacaga gtcagaaacg 300
gtcttttttt aagcacgttg gcagcagggc gtaacccttc agctctttag ggcgagtacc 360
ttcccgacat ttttcgcata accacaacga tgattccacc aatgaagccg atgntagtaa 420
gaccccaact atgattccaa ccagggtcac tgttgacaaa ccatctttct caactgttgt 480
ctgtgtgtct ccatccactt tctccgtgga gtgactggtg gccacgtttg aggctgcctc 540
gtgcc                                                             545

<210> 8
<211> 223
<212> DNA
<213> unknown

<220>
<223> >gi|4682406|gb|AI631076|sr|IMAGp998J095673.1|cl|IM
      AGp998J095673|AI631076 tz32c05.x1 NCI_CGAP_Ut2
      Homo sapiens cDNA clone IMAGE:2290280 3', mRNA
      sequence.

<400> 8
tttttttttt tttttttttt tttttttttt tttttttttt tttttttttt tttttattgg 60
gaaaggttta tttaaaggtt tgggggcccc ttttaccccc ccccaaaaag aaaaaaaaaa 120
attttttttt tgccgtccca aacagggaag tcccttcaaa gaggagctgg cgggtgggtt 180
caaaccccaa aagggggacc cctgccaggg ggtttttccc ggc                   223

<210> 9
<211> 491
<212> DNA
<213> unknown

<220>
<223> >sr|IMAGp998I175722.2|cl|IMAGp998I175722|gb|BX0978
      70|oid|234530113 BX097870 NCI_CGAP_GC6 Homo
      sapiens cDNA clone IMAGp998I175722 ;
      IMAGE:2309080, mRNA sequence

<400> 9
gcacccaggc agcctgcgcg gagaaatcgg atcggctagg acggcctgca gcccctgcgc 60
gcggggaaag ggaagaagtc ctcccctaca aagcgagttc acaaacttgg aagaagccac 120
tttcacagga tgcacagatc tcaacggaag gacacagaaa acatgaaaaa gcaaggaaat 180
ggggcgcctc caacggaacc cagtaattct ccagcagcag atccccatcc aaaagaaaca 240
gaagacattt tggagagaga attgaatata catattatac agttgtgggc ataatacaag 300

catcatttgg aaaagaacag agaaaaaaga tggaaggagt taccgtactg caggaaaaaa 360
ttatcaactc cagtgatatt attaaataca acctaacgac acttctgaag ctgaagaaat 420
cactggatga aatacaaagt aaactcaaaa gcttcagtga tagactagaa caagaagaag 480
aaagactctc a 491

<210> 10
<211> 296
<212> DNA
<213> unknown

<220>
<223> >gi|4682412|gb|AI631082|sr|IMAGp998J105673.1|cl|IM
      AGp998J105673|AI631082 tz32d05.x1 NCI_CGAP_Ut2
      Homo sapiens cDNA clone IMAGE:2290281 3', mRNA
      sequence.

<400> 10
tttttttttt tttttttttt tttttttttt tttttttttt tttttttttt tttttattgg 60
gaaaggttta tttaaagggt tggggtcccc ttttaacccc ccccaaaaag agaaaaaaaa 120
attttttttt tgccgttcca aacaggaaag tcctttcaaa gaggagctgg cgtgtgggtt 180
caaagcccaa gagggggacc cctgccaggg ggtttttccc ggcctgggcc accctgggta 240
ggagggccac ccaggccccg gtttcctggg accctggggg ttttggaggg agggag 296

<210> 11
<211> 550
<212> DNA
<213> unknown

<220>
<223> >gi|4283562|gb|AI436660|sr|IMAGp998N115245.1|cl|IM
      AGp998N115245|AI436660 th91c06.x1
      Soares_NSF_F8_9W_OT_PA_P_S1 Homo sapiens cDNA
      clone IMAGE:2126026 3' similar to TR:P70423 P70423
      CATIONIC AMINO ACID TRANSPORTER. ;, mRNA sequence.

<400> 11
ttttattttt ctactttctt ttaatatcat tttttaaagt tggtaagcag ctagacatca 60
tttagaagca gacgggttaa aatagacaag aaatagcaaa gacacatcct tcacatcgta 120
cagaactgta ttagtatcca ccaccaccat cacaggggag ggctagctgt cactggggtc 180
aggagtactc tccattattg tgcaggggac cagacagcat ttaggtgtga cgatgtcaaa 240
ctgagtggac atagagagtg ccgggatcaa ggtctacagt tttggctcta gacttgcgtg 300
agggttggtt actcttaatc tcttccaggc tgtgctggat cccatagccg aagtagatag 360
caaagccaat cagcatccag accccaaatc gggcccaggt accagctgtc atctgcatca 420
taaggtaaat attcacaaag atgctcatta gtgggaggag aggcaaagca ggcaccttaa 480
agtgaagggg agtggaactc tgtggctgtc ttcagatgac cacaatgatc ccaataatga 540
gcagcaggag 550

<210> 12
<211> 618
<212> DNA
<213> unknown

<220>
<223> >sr|IMAGp998J05529.1|cl|IMAGp998J05529|gb|BX091496
      |oid|234517359 BX091496 Soares fetal liver spleen
      1NFLS Homo sapiens cDNA clone IMAGp998J05529 ;
      IMAGE:245476, mRNA sequence.

<400> 12

agtcattgac ggtgaataca ggtctggaag tctgaggtcc aatataataa aaaatctagt 60
cttcaccttc tatcctaggg atagacatta ggaatatttg aggatgttgt tcttggtctt 120
cacgttcatg cattttccct ctatttttat aattggatca atgaatcaat aagcaaatat 180
attttaactg cataaagata aaaataggcg ttgtccaatt atggaaatat atggtgaaat 240
aagaaatact attcacattt attaaaaaca ctgaggtctt ctttctatta acaaataaat 300
gactcagagg tattactcgc ttgctgcagg atggcatata cagtaaaaat aataataacg 360
tatgtgaggt catttgggtc aggaccatgt tcatggttgt ttgttttctc aagaaaaaat 420
agtttcacac atacttacat gactaataga atgaaaggag aaatgtgtgg ataaatggac 480
agaatatttc catcttttta tcttttttgc tttgtactct cacaatgaac accctcaagt 540
tgacttaagc acccttccat ggtcatgagg tttctggttg cctctgtgtg ttagaagctt 600
ggagaccaat gccttctg 618

<210> 13
<211> 713
<212> DNA
<213> unknown

<220>
<223> >sr|IMAGp998A03577.1|cl|IMAGp998A03577|gb|BX102059
      |oid|234538498 BX102059 Soares melanocyte 2NbHM
      Homo sapiens cDNA clone IMAGp998A03577 ;
      IMAGE:263690, mRNA sequence

<400> 13
ttaaatccag tgtggaatag ctgccttagg accttgaccc acacttactt taagaaatcg 60
ttctagttct taaatttcaa tggtaaagcc agaggctggt gcttgaaaag tatttacagt 120
gctcaggcag cactttctgc agttacagat tctttactgc ccctttttat gtctgaactt 180
tacatactgg attactcgtg tcaataggag aaggaacagt gccttgattt aacacgtcct 240
tgtaatagtc gtggtccttc tcttctggga aaggagaaga aatagtgccc ctggagtccc 300
attgccttgg gaggcatatg gctcgtttca gtgattcctt gagaattctg gtaagaagtc 360
aaaaataagc atttaaataa aaagaatagt aaaaaccaca gtaaggaaag agcttctgat 420
ccatttagta aaataaaaca tacattttat aaggtagaaa tattaaatag aaccatttat 480
tactctaaat tctatcctta tttatctctc aaaataatag aacagtgcca cttttgataa 540
tttttctatt tgcacaccca aaatgcctgt tttaaaatat ataatagaga atgaagaaat 600
ttggggaatt tcagaccaac aatgtgttta tattcatgta agtctgattt tcaaatcagt 660
gtaaattgtc agtaatttcc ataatacata ttggcctagt tctgtatgct tac 713

<210> 14
<211> 335
<212> DNA
<213> unknown

<220>
<223> >gi|3756508|gb|AI203902|sr|IMAGp998H234408.1|cl|IM
      AGp998H234408|AI203902 qd71g12.x1
      Soares_testis_NHT Homo sapiens cDNA clone
      IMAGE:1734982 3' similar to contains L1.t2 L1
      repetitive element ;, mRNA sequence.

<400> 14
tttttttttt tttttaaaca taccctttat taacatctag gtaatatctg taatattcct 60
tgctcctcat ccccaagtac gctattagct gtccatcctt ctgggtagaa gtgtattttc 120
gttttacttg ttgatttttg gatgcatgct gggggaggaa agcatattgt ttgtagtcac 180
cctggcgtgc taaggtatat tattccccag taattctctc aaggtgggca tatgcaaaac 240
ataatctcta aattcttcaa tactaagaaa tacctttgtt ttacccctaa aatcaaatgc 300
cattttggct ggatatagga ttctaggatt aaagc 335

<210> 15
<211> 565

<212> DNA
<213> unknown

<220>
<223> >sr|IMAGp998G13226.1|cl|IMAGp998G13226|gb|BX093404
      |oid|234521175 BX093404 Soares placenta Nb2HP Homo
      sapiens cDNA clone IMAGp998G13226 ; IMAGE:148260,
      mRNA sequence

<400> 15
tttttttata taacagaggt tatcttggaa gagagatagc aaacaatttc tgtgtgtctt 60
tgtgaccttt aaattcaaaa caaaaaaaat aagccactct tttaaaagtg ctcagtctaa 120
aaaataaaca cgtagtctag aggcggcaag gcccgtgtgt gaagcctggc tcttcctcgg 180
ctaagctgtg tgacggtcgg caagttgctt accttcttga agctgcattc tcctcattct 240
taaaataaaa atggtaataa caggaccgcc tcattgagct tgtgtgaaga gtaacagaca 300
actgcagcca agtccccaga aggtgcctgg cacatagtga gggcttgata tgaggtagct 360
attatcagga catgattcct gtaatcttca cattttttta ctttgacaat caaaaagcca 420
atacagataa ttgaaaagac aagaaaagtg acatcagcag gctccttagc ccaaaattca 480
ccccctccct ctctcgaccc agagctccac ttgagaaggg cgggtggcag acacacctct 540
tatatttagg aatattcctg cggcc 565

<210> 16
<211> 580
<212> DNA
<213> unknown

<220>
<223> >sr|IMAGp998J23134.1|cl|IMAGp998J23134|gb|BX092461
      |oid|234519290 BX092461 Soares fetal liver spleen
      1NFLS Homo sapiens cDNA clone IMAGp998J23134 ;
      IMAGE:129142, mRNA sequence.

<221> misc_feature
<222> 17
<223> n=a, t, c or g

<400> 16
caactatttg ccccagnaca actgtttttc ccccttcctt ctatggattg accagccagt 60
tctgctttag atggtattgg atggagccct ggaatggctg aaaagtccaa actggcctga 120
cttgctcagc cagcaatgcg gtgcaggctc ctagctgggg ccttagttct cctgcacctg 180
cggctctcca tatggctgct tggacttcct catagcatgg cagatggatt acgagaagga 240
gcattcccaa ataaaggacc acataagcta gatctctgga gggctagcct cagaagtcac 300
ccagtgtcac atgggccaca ttttattggt tacagggcca gccagtttga aggggaagag 360
aaatatgtcg ctgtttatgc tgtgtccagt gctagcttgc tacctgctct cccagttcca 420
gtgctcaggg cagcactggc agaacagatg tacttactga gttaaaaaca gcaacatcca 480
agacaattgt taacttttaa aactgtctcc catcccagaa ggtataacta aaaaactaac 540
aataaaaata atagtaataa ataataaaaa aaaaaaaaaa 580

<210> 17
<211> 306
<212> DNA
<213> unknown

<220>
<223> >gi|4987550|gb|AI699650|sr|IMAGp998K225545.1|cl|IM
      AGp998K225545|AI699650 tt18f11.x1 NCI_CGAP_GC6
      Homo sapiens cDNA clone IMAGE:2241165 3', mRNA
      sequence.

<400> 17
gtcacgcacc gcacgttcgt ggggaacctg gcgctaaacc gacagattta acaaagggag 60
tgatgtgtga tcacatatgt gttcttaaaa gattgttatg catgtttcat gtagaataga 120
ttagggaaga tcagggaaga tacagggaga caatatactc ctgctgtagt agtcaagcca 180
gaaatcctgt tgcctggatc cagggttgtg gcagtagaaa aatagatcaa aagtagataa 240
aatttgcagt agtaattaac tgtattggag catttgaaaa ggatgattta aaaaaaaaaa 300
aaaaaa 306

<210> 18
<211> 443
<212> DNA
<213> unknown

<220>
<223> >gi|1525834|gb|AA045940|sr|IMAGp998N121162.2|cl|IM
      AGp998N121162|AA045940 zk75d06.r1
      Soares_pregnant_uterus_NbHPU Homo sapiens cDNA
      clone IMAGE:488651 5', mRNA sequence

<221> misc_feature
<222> 351
<223> n=a, t, c or g

<400> 18
ggaccagcat gccggtgggg tgcaggcgcc atttgtcttg gaagtcaatt tccttcagga 60
gacaaagcaa gtttggggtg gtagccttaa tgcccagcac cttggattga ttcccatggt 120
ctaagagggt ctttttgact aaggccagcc tgtggtgatc ccagcagctt tggagggcct 180
gcagccacac actgactgat acatggtatg ttacagggac ttgtcacttt ggatcaggct 240
gggcactaca gggcctgatg ggcaaacttg ggtaatcact gggaggaggg aaactgaggc 300
ccgaggcaga tacctgggtt tggggagggc tgggtttagc ccggttctct naactacata 360
cccccagctg ttaagccttc ctcttgaggg gttgaggtgg gcagtataca ggagccaagc 420
agcccagggt ggggtcgcag ccc 443

<210> 19
<211> 509
<212> DNA
<213> unknown

<220>
<223> >sr|IMAGp998H07676.3|cl|IMAGp998H07676|gb|BX100963
      |oid|234536302 BX100963 Soares_fetal_lung_NbHL19W
      Homo sapiens cDNA clone IMAGp998H07676 ;
      IMAGE:301878, mRNA sequence.

<400> 19
gaaccctggc ttggtggatt ttgctagatt tttctgattt ttaaactcct gaaaaatatc 60
ccagataact gtcatgaagc tggtaactat cttcctgctg gtgaccatca gcctttgtag 120
ttactctgct actgccttcc tcatcaacaa agtgcccctt cctgttgaca agttggcacc 180
tttacctctg gacaacattc ttccctttat ggatccatta aagcttcttc tgaaaactct 240
gggcatttct ttgagcacct tgtggagggg ctaaggaagt gtgtaaatga gctgggacca 300
gaggcttctg aagctgtgaa gaaactgctg gaggcgctat cacacttggt gtgacatcaa 360
gataaagagc ggaggtggat ggggatggaa gatgatgctc ctatcctccc tgcctgaaac 420
ctgttctacc aattatagat caaatgccct aaaatgtagt gacccgtgaa aaggacaaat 480
aaagcaatga atacaaaaaa aaaaaaaaa 509

<210> 20
<211> 775
<212> DNA
<213> unknown

<220>
<223> >sr|IMAGp998B171780.3|cl|IMAGp998B171780|gb|BX1084
      62|oid|234551303 BX108462 Soares ovary tumor NbHOT
      Homo sapiens cDNA clone IMAGp998B171780 ;
      IMAGE:725680, mRNA sequence

<400> 20
cctgattgtc atagacaaat cctacatgaa ccctggagac cagagtccag ctgattctaa 60
caaaaccctg gagaaaatgg agaaacacag gaaataaaat tggaacgaag aaaggttagg 120
agagtaggga aggaacagga ctgcaaaaat ccttctccac cgcacagact gggaacccct 180
cctggcctgg gggaagagtt tgttacctac cttactattt aaagagcctt cactggttct 240
gcatcacccg cccctggact tcttagttgt ttctctagcg ctgagctatc tcctaacttt 300
ggacctatta tcagaaggtg acaagtactg gctctttatt cattaagctt tttttttttg 360
aaccccattc tttccttctc tgaaagtggt gctataagtt ttagaatctt ttaaatacat 420
tccctgggcc aacagaccca cacacttagc cattgaaatg tcaaattgat gtgccctaga 480
tcaacagatc aacaatacct tttttttcag tgttaaggta atggttggtt tttgtgtccg 540
ctaaatattt accttgaaaa aaagaaaagt gtgtatctag cttcttcaga gatcaagtcc 600
tctggtagga ggcaaaggtt ctatctgctt agcaactagt taataagtgg tatctgacac 660
actctaaacc ccgtgttcaa acgggggcct tctggtttta ggaaacttgt agaaacgaag 720
cctgctgatt gatttttttc tccttttttt tttttttttt ttttaacttt ggaag      775

<210> 21
<211> 773
<212> DNA
<213> unknown

<220>
<223> >sr|IMAGp998A2488.1|cl|IMAGp998A2488|gb|BX098995|o
      id|234532364 BX098995 Soares fetal liver spleen
      1NFLS Homo sapiens cDNA clone IMAGp998A2488 ;
      IMAGE:111647, mRNA sequence

<221> misc_feature
<222> 20
<223> n=a, t, c or g

<400> 21
atgaatgcag aacctcaagn ctacctgtgt caatatcaat gtgtcaatga acctgggaaa 60
ttctcatgta tgtgccccca gggataccaa gtggtgagaa gtagaacatg tcaagatata 120
aatgagtgtg agaccacaaa tgaatgccgg gaggatgaaa tgtgttggaa ttatcatggc 180
ggcttccgtt gttatccacg aaatccttgt caagatccct acattctaac accagagaac 240
cgatgtgttt gcccagtctc aaatgccatg tgccgagaac tgccccagtc aatagtctac 300
aaatacatga gcatccgatc tgataggtct gtgccatcag acatcttcca gatacaggcc 360
acaactattt atgccaacac catcaatact tttcggatta aatctggaaa tgaaaatgga 420
gagttctacc tacgacaaac aagtcctgta agtgcaatgc ttgtgctcgt gaagtcatta 480
tcaggaccaa gagaacatat cgtggacctg gagatgctga cagtcagcag tatagggacc 540
ttccgcacaa gctctgtgtt aagattgaca ataatagtgg ggccattttc attttagtct 600
tttctaagag tcaaccacag gcatttaagt cagccaaaga atattgttac cttaaagcac 660
tattttattt atagatatat ctagtgcatc tacatctcta tactgtacac tcacccataa 720
ttcaaacaat tacaccatgg tataaagtgg gcatttaata tgtaaagatt caa        773

<210> 22
<211> 623
<212> DNA
<213> unknown

<220>
<223> >sr|IMAGp998H01154.1|cl|IMAGp998H01154|gb|BX102894

|oid|234540168 BX102894 Soares infant brain 1NIB
      Homo sapiens cDNA clone IMAGp998H01154 ;
      IMAGE:32759, mRNA sequence

<400> 22
tttttttttt ttttaaaggg aaagttaatt tttaatacaa atacaaaatt tatttccata 60
gaaagcaaaa tttataagtt aaatataaag attaaaatag tgtttgaaaa gctcactata 120
gtgctaacaa actaaatgaa aataaggcta ttttgcattt aaaactgaaa tacctcttaa 180
aataatttga tccccagtgt ttgctctttt tgaagtaacc aacttactct taaaaaggat 240
ggctgccaag atggaaagtc ttactgggtt ttcatgttaa cctattcttt ggacataact 300
atgaattttg tatacaatgc acttcatgaa aagttgtggc tcccccagat tgcccacaag 360
tgtgatcttg aagtcctaaa catttgtcca tgtaagcttc aaaacagcgt taactgagtt 420
attcaagtag cagtacttaa agatacaatt cttgaagcag tttcaatggt ttctgatcca 480
aataatcagt ttctgaacat tactacttca cataatagag tccatcttca gtttcttctc 540
actttctctt tcccttttgg gtttcctttt tgtggcctga ggccaccagt tctttgggta 600
ctatcaagat acttccatca tgg                                          623

<210> 23
<211> 531
<212> DNA
<213> unknown

<220>
<223> >sr|IMAGp998J02274.1|cl|IMAGp998J02274|gb|BX107144
      |oid|234548669 BX107144 Soares infant brain 1NIB
      Homo sapiens cDNA clone IMAGp998J02274 ;
      IMAGE:46959, mRNA sequence

<221> misc_feature
<222> 14, 430, 431, 434, 435, 521
<223> n=a, t, c or g

<400> 23
tttttttttt tttnctgatt ctattacttt tattaaatag tgggtttcca cacatggctt 60
tttaaataat ccaggcagga gaagagagga gggcacactt ggaactcccc tccccacaat 120
acgtgattat ttacatttta gtaattggac aatcccggct caggaggagg ttgcaagaat 180
ctgcaaaagt tggagggagc gccccaggag aacaaacagc aagccttatt tcccctagcc 240
catcccccaa aaaaccatcc atcccatcct agtgtctggt ggtgtccggt ggtgtccatc 300
ttccattcct tcccaaatta tggaagtaag gttcttctca ccagaataag agcacttggg 360
ataacagagt agggtcccct cacccaaaaa aaaaaaaaaa aaaaagaagc cttggggtaa 420
caacagggcn nttnncctcc cccagaataa agaatcctgg gctgaggcag ctaagcagct 480
tggcccaata tgggacccta ggctagggga aagggtcccc nttactaaaa t          531

<210> 24
<211> 742
<212> DNA
<213> unknown

<220>
<223> >sr|IMAGp998L10121.1|cl|IMAGp998L10121|gb|BX111578
      |oid|234557533 BX111578 Soares fetal liver spleen
      1NFLS Homo sapiens cDNA clone IMAGp998L10121 ;
      IMAGE:124569, mRNA sequence

<221> misc_feature
<222> 721
<223> n=a, t, c or g

<400> 24

acctggcctg aatctatgta ctttcatcag cgcggccagt aagtttgcac agctccagtg 60
tctgtggaag acgagagact caggaactgt gagtgcgctg acttggagcc tctcttccta 120
tacctgtgca acaagaataa tcacaacctt aatgaccacc aatgatttta caattcttct 180
acgttttgtg atcatgctgg ctttaaatat atgggtaaca gtgacagtac ttcgctaccg 240
gaagaccgct ataaaggctg aatgatggat acattattcc ttcacacagt ggattttgag 300
taactgaacc aaaggaaaaa gaagctcttt gctaaattaa ggtcttttat aaatttagta 360
aatcagttta taatctttaa agccaaaggt ttttttagac ttgaaagaaa gagccactta 420
aattcttgtt taaaaatacc aatttgcctc ctccttcctc acttcgttag gttatggtag 480
tgctcagaca tctgcagtgt tgagaaggcc agtcactgtt ggaagtcatc caagaagccc 540
attttgagcc ttactcttaa gttctctatg aagaactaca ttgatttgtt ggctttcaga 600
atcttttagg aaataaatcc tctccaggac aaaaatgaac atgaatggag tggcattttg 660
ttccaagtca gaggtgggca cctataataa atgactaggg ttcactttct gggactgatg 720
nttaattgta acacagatac aa 742

<210> 25
<211> 455
<212> DNA
<213> unknown

<220>
<223> >gi|4735457|gb|AI651478|sr|IMAGp998J075711.1|cl|IM
      AGp998J075711|AI651478 wb06c04.x1 NCI_CGAP_GC6
      Homo sapiens cDNA clone IMAGE:2304870 3', mRNA
      sequence.

<400> 25
tttttttttt attttttaca tatgtaatta taatgaactg aaagatatat atatatatca 60
aaaggaacaa aaaaatgact tcaaattcag actagaattg ctcaactatc aattgctact 120
gatcaatcat ctttttaata acatcaaata attaggtcta ataactacat acaactaaac 180
agctgtctaa aggagatgct gttaaagaac tgttccaggt caaaatgacc aagctgttaa 240
attatactca aagtaaaaaa caaacacaat aaaaaatatt taaaaaaaca aacaaacaca 300
atagtagata ctgaaataat tatcaatccc ctgcaagtag atggatgtac ttaaggagac 360
tcaaaattct cgggtgtggg tgggatacat tgggaaggct gttttaacac tggagccttg 420
catggatttc aggaggttcc tgaatctact gaaat 455

<210> 26
<211> 704
<212> DNA
<213> unknown

<220>
<223> >sr|IMAGp998N06536.1|cl|IMAGp998N06536|gb|BX103225
      |oid|234540830 BX103225 Soares fetal liver spleen
      1NFLS Homo sapiens cDNA clone IMAGp998N06536 ;
      IMAGE:248261, mRNA sequence

<400> 26
gtcttaaaca agccacggaa actgcgatat taaatgccaa ctacatggcc aagcgattag 60
aaacacacta cagaattctt ttcaggggtg caagaggtta tgtgggtcat gaatttattt 120
tggacacgag acccttcaaa aagtctgcaa atattgaggc tgtggatgtg gccaagagac 180
tccaggatta tggatttcac gcccctacca tgtcctggcc tgtggcaggg accctcatgg 240
tggagcccac tgagtcggag gacaaggcag agctggacag attctgtgat gccatgatca 300
gcattcggca ggaaattgct gacattgagg agggccgcat cgaccccagg gtcaatccgc 360
tgaagatgtc tccacactcc ctgacctgcg ttacatcttc ccactgggac cggccttatt 420
ccagagaggt ggcagcattc ccactcccct tcgtgaaacc agagaacaaa ttctggccaa 480
cgattgcccg gattgatgac atatatggag atcagcacct ggtttgtacc tgcccaccca 540
tggaagttta tgagtctcca ttttctgaac aaaagagggc gtcttcttag tcctctctcc 600
ctaagtttaa aggactgatt tgatgcctct ccccagagca tttgataagc aagaaagatt 660
tcatctccca ccccagcctc aagtaggagt tttatatact gggt 704

<210> 27
<211> 468
<212> DNA
<213> unknown

<220>
<223> >sr|IMAGp998M17826.1|cl|IMAGp998M17826|gb|BX108828
      |oid|234552035 BX108828 Soares_fetal_heart_NbHH19W
      Homo sapiens cDNA clone IMAGp998M17826 ;
      IMAGE:359608, mRNA sequence

<400> 27
tttttttttt acttagtctg tgtttggttc cccagtttta tgctgccttt ctactctgtt 60
cttgctgtct ccctctttac ctgagtcaac ggtactgagt cctgtctctc tctgatgttc 120
cccagtcttc cttggtgcat gttctagctc cacacactag tccttggagg aaggttgaga 180
ccaatgattt cctgttatga gtcatgagga aactgaatca cctagaagtg gaataatgtg 240
ctcagggtca ccatagccca ttagtggaag gaccaggact agacctttag tcttctgagg 300
tccagcccct taggctgtct gtcatcactg tacccaagtg atgtcactac caaggccaaa 360
tgatggtggg ctaaatttta attctcaaaa gtgtaggagg ctaatattgt cttctaagtt 420
ccaaaagaag atgtaataaa agtctgttac cttaaaaaaa aaaaaaaa 468

<210> 28
<211> 756
<212> DNA
<213> unknown

<220>
<223> >sr|IMAGp998P11794.1|cl|IMAGp998P11794|gb|BX093757
      |oid|234521882 BX093757 Soares_fetal_heart_NbHH19W
      Homo sapiens cDNA clone IMAGp998P11794 ;
      IMAGE:347386, mRNA sequence

<400> 28
aggcctagga agagggtcat tcttaagcca cacattagct gcactgcgtg gctgcagcca 60
aaacaaagaa ctgggtgttg agtattcatc aactaagaac caaaatccag ggcactcata 120
tgtgaaggat aagaacctca cttccttact cctccaaaaa gaagtgggga aagaaccatc 180
aaacctttcc tcctgactta ccaaaccagg aaaacagcag gagagggtgg ctcaggactt 240
agggacaggg tatagcttag atggtggaaa gcaaaggaga gcaggaagtt gtaaatcact 300
ggctaatgag aaaaggagac agctaactct aggatgaagc tgtgactagg ctggagttgc 360
ttccttgaag atgggactcc ttgggtatca agacctatgc cacatcacac tggggctagg 420
gaagtaggtg atgccagccc tcaagtctgt cttcagccag ggacttgaga agttatattg 480
ggcagtggct ccaatctgtg gaccagtatt tcagctttcc ctgaagatca ggcagggtgc 540
cattcattgt ctttctctcc tagccccctc aggaaagaag gactatattt gtactgtacc 600
ctaggggttc tggaagggaa aacatggaat caggattcta tagactgata ggccctatcc 660
acaaggccca tgactgggaa aaggtatggg agcagaagga gaattgggat ttttaagggt 720
gcagctacgc tcaccctaaa cttttggtgg cctggg 756

<210> 29
<211> 363
<212> DNA
<213> unknown

<220>
<223> >gi|772089|sr|IMAGp998O08148|cl|IMAGp998O08148|gb|
      R18479|R18479 yf95f04.r1 Homo sapiens cDNA clone
      30149 5' similar to gb:X14174 ALKALINE
      PHOSPHATASE, TISSUE-NONSPECIFIC ISOZYME PRECURSOR
      (HUMAN);.

<221> misc_feature
<222> 85, 346
<223> n=a, t, c or g

<400> 29
acgtgatcat ggggggtggc cggaaataca tgtaccccaa gaataaaact gatgtggagt 60
atgagagtga cgagaaagcc agggnaccga ggctggacgg cctggacctc gttgacacct 120
ggaagagctt caaaccgaga tacaagcact cccacttcat ctggaaccgc acggaactcc 180
tgacccttga cccccacaat gtggactacc tattgggtct cttcgagcca ggggacatgc 240
agtacgagct gaacaggaac aacgtgacgg acccgtcact cttccgagat ggtggtggtg 300
gccatccaga tcctgcggaa gaaccccaaa ggcttcttct ttgctngttg gaagggaggc 360
aga 363

<210> 30
<211> 449
<212> DNA
<213> unknown

<220>
<223> >gi|5367341|gb|AI801869|sr|IMAGp998C095623.1|cl|IM
      AGp998C095623|AI801869 tx28e05.x1 NCI_CGAP_Lu24
      Homo sapiens cDNA clone IMAGE:2270912 3', mRNA
      sequence

<400> 30
tttgttgttg atccattcat aagcatttat tgagggctga ctggatgccc agcactaggg 60
aaacggcaga tggcacaaag ggtggggtcc ccaaggacag ggcgatttca gctcttccct 120
tgccaacccc gggtcatccc cagggctcaa tcccaggctg gggggagggt ctgggagtgg 180
gggcagtcgt catagggtgg gtcttcctgg aaatccagcc gagcccgggt gcccttgccc 240
aggtgtaggc agcctcctag gtggacatgg agggagagca ggagggcgaa ggctcagact 300
tcccggcacc tctgaaactc ggtgtttctc tacgtctaac tctccttctt gctgcaacgc 360
atttgccggg gcgggcggcc tctgcattcc tgaactgggt cagaccagga gagaggattc 420
ctggatcctg gcccagtggc caatccgcc 449

<210> 31
<211> 525
<212> DNA
<213> unknown

<220>
<223> >gi|4969890|gb|AI692550|sr|IMAGp998O205798.1|cl|IM
      AGp998O205798|AI692550 wd73f10.x1 NCI_CGAP_Lu24
      Homo sapiens cDNA clone IMAGE:2337259 3', mRNA
      sequence

<400> 31
atgttaaaat tgcacaaaca tggtgctcta ccaggaagga ttcgaggtag ataggctcag 60
gccacacttt aaaaacaaac acacaaacaa caaaaaacgg gtattctagt catcttgggg 120
taaaagcggg taatgaacat tcctatcccc aacacatcaa ttgtattttt tctgtaaaac 180
tcagattttc ctcagtattt gtgtttttac attttatggt taatttaatg gaagatgaaa 240
gggcattgca aagttgttca acaacagtta cctcattgag tgtgtccagt agtgcaggaa 300
atgatgtctt atctaatgat ttgcttctct agaggagaaa ccgagtaaat gtgctccagc 360
aagatagact ttgtgttatt ctatctttta ttctgctaag cccaaagatt acatgttggt 420
gttcaaagtg tagcaaaaaa tgatgtatat ttataaatct atttatacca ctatatcata 480
tgtatatata tttataacca cttaaattgt gagccaagcc atgta 525

<210> 32
<211> 461

<212> DNA
<213> unknown

<220>
<223> >gi|788859|sr|IMAGp998A10192|cl|IMAGp998A10192|gb|
      R33016|R33016 yh70b05.s1 Homo sapiens cDNA clone
      135057 3' similar to SP:ZK783.2 CE00841 TYROSINE
      PHOSPHATASE ;._gi|1343426|gb|G23100|G23100 human
      STS WI-11994

<221> misc_feature
<222> 199, 217, 311, 390, 456
<223> n=a, t, c or g

<400> 32
ggacaatgct tttaatggca agtcatcaca gcaggtctgc ggaggtgcag ggcagcgctc 60
aggccttgct cagtttcttc ttgatgaagt agctcaccag ccgctgcggc ctctgctggt 120
actcgctgag cacattgcga gggctgctga tctggtcccc ttccaggcgg ttcaggaggg 180
tgacacacac cacggccgnt tggaggccct agggctngca catggcggca aacaccgagg 240
actccatctc gatattgcgg acgccggctg cataggctgc ctccagatac gcctgcttgt 300
ccttctccgt ntaggagcag agagccccat ccagacggcc ttgcccttca taggaagtcc 360
aagggtgcac atgggttttc cccaccactn ttggttgaac tcgttcagtt ttgcagaaca 420
cagcaacagt ttctgcacca gttttttttt aaaggncctt t                     461

<210> 33
<211> 500
<212> DNA
<213> unknown

<220>
<223> >sr|IMAGp998F20979.1|cl|IMAGp998F20979|gb|BX115560
      |oid|234565495 BX115560
      Soares_fetal_liver_spleen_1NFLS_S1 Homo sapiens
      cDNA clone IMAGp998F20979 ; IMAGE:418195, mRNA
      sequence

<400> 33
   taatcagtga tgatactttg gtgtatgttc aggactgaaa ccttcttatt gtgctaactt 60
   gatggtttct ttaaaaataa tagtcttgcc ataaattatg aataagactt gtttttttag 120
   ccatatactc agaagaacac tagagttctc caactggcta cgcttagttc ttgctttgcc 180
   aggtagtccc acgcttacta taatgttcaa tttagagttc taagtataaa atatttttac 240
   ttatttattt atttttgtga cagagtcttg cactgttgtc caggctggag tgcagtggtg 300
   tgatctcggc tcactgcagc ctccacttcc tgggttcaag tgatcctcac gcctcaacct 360
   cctgagtagc tgggattaca ggtgtgagcc atggtgcctg gctaattttt ttttcttttt 420
   ttcctttctt gagacggagt ctcccactgt cactccagtt tgggcaacag agtgagaccc 480
   tgtttaaaaa aaaaaaaaaa                                             500

<210> 34
<211> 565
<212> DNA
<213> unknown

<220>
<223> >gi|4736233|gb|AI652254|sr|IMAGp998B095717.1|cl|IM
      AGp998B095717|AI652254 wb28c05.x1 NCI_CGAP_GC6
      Homo sapiens cDNA clone IMAGE:2306984 3', mRNA
      sequence

<400> 34

cacagtatga atgtatttta ttttgtgaac cgggaaggac accaaccaaa acataattga 60
aacaaaagtt tcacagcaca atagttacct tagtacatgt gatacacaat ggactctatt 120
cttgtccaca ctgttccatt aaagaaaaat atgacacact tactgatttc acagtggggc 180
cttgaaagtg cccacaatag gctgtcaggt gagaggaaac acagaaggga ggtagcttac 240
tacagaatga tcatggccta ataacatctg ggtgattata tgggttgcac ctcagctgtc 300
tataaacttt agcgtcagca gatctgtcca tggctgtctc tgcttttcca gttggttggg 360
catatctccg gcctgagatg gtcacccgtc agaatccttt gagtatcttg ccagaattca 420
gcagtttttc tgtgcaccag gaggatgctg cagggaggct gagattctct gaagatacgt 480
gtattgctga aagtagactt atgattatgg aatcataatt tccttgtact tttatctaaa 540
attatacatg agcccgctga aggag 565

<210> 35
<211> 518
<212> DNA
<213> unknown

<220>
<223> >gi|4889149|gb|AI678967|sr|IMAGp998M125582.1|cl|IM
      AGp998M125582|AI678967 tu60b06.x1 NCI_CGAP_Gas4
      Homo sapiens cDNA clone IMAGE:2255411 3', mRNA
      sequence

<400> 35
tttttttttt ttttttctta caaatgggag ttttatttgc attctaaccc aagcacagat 60
cccacataca gacttctcgt ccgcactatg gtacagtaac aagtgcaaaa tatgctttat 120
ttcaggtaca aaaacatggc agtaggacaa ctttgagctc aacgcccacc tccccaggac 180
ctttcacacc cacctgaccc atccaagggc cacaccaccc cgacagatac tcccaccacc 240
tttagaaaag agtcacccaa tctggagaaa ggtgtggagg ttacatcttt agaaagaaat 300
cattttaaat acatgaacat tagagaacac agtaaccgtg cttccaccca gcacggggag 360
gctgcagaga ggcaccccaa acagtcccct tctcttctgt ggttaaccaa gcaagccccg 420
caaagctttt cccagcagag cacacccagc cagcaatgag gcctcaggac agagccagca 480
ctgtgacaaa agaacttgtc ccacctatct gagagcat 518

<210> 36
<211> 430
<212> DNA
<213> unknown

<220>
<223> >gi|3801639|gb|AI219436|sr|IMAGp998M154512.1|cl|IM
      AGp998M154512|AI219436 qh13a08.x1
      Soares_NFL_T_GBC_S1 Homo sapiens cDNA clone
      IMAGE:1844534 3' similar to contains Alu
      repetitive element;contains element MER33
      repetitive element ;, mRNA sequence

<400> 36
tttgagacaa gagtctgact ttattgccta gtggagtggc atgatctctg ctcactgcaa 60
cctctgtctc ccgggttcag gcaattctca tgcctcaacc tcctgagtag ctgggattac 120
agacacatgc caccacgccc gactataaaa ttgagttctt atagttcaat aaagttaatt 180
tattatctct ttatgctagt attatagaat tttggagcag gtagtatctt agattcccta 240
cattagcagt gttatataaa agagaaataa ttcaagtcac atatgtaatt taaaatattt 300
tagtagccat attaaaaaag caaaaagaaa caggtgaaat tacttttgat tgtacatttt 360
tttaacccag tatatcagaa atactgtgtc aacatgtaat caatgtaaaa ttattagaga 420
tagtttgcac 430

<210> 37
<211> 493
<212> DNA

<213> unknown

<220>
<223> >sr|IMAGp998M144547.2|cl|IMAGp998M144547|gb|BX1071
      17|oid|234548615 BX107117 Soares_NhHMPu_S1 Homo
      sapiens cDNA clone IMAGp998M144547 ;
      IMAGE:1857973, mRNA sequence

<400> 37
aggacatagg ctgctaatga acagccccat ctgatttaat cactgtgcaa tgaccgtgaa 60
ctgtacagtt acctccgtgt gtcacaggaa gacttttttg acttaagcac ccttccatgg 120
tcatgaggtt tctggttgcc tctgtgtgtt agaagcttgg agaccaatgc cttctgctgt 180
gatcgcgttc tccacagttg cctctgggta ggagcctgga cactaataga aaatggatgc 240
acttgagccg acctaagcat tcatggtcat gagatttgtg gttgccactg ttggttagga 300
gcatagagat cagtaatgga cacacctggg ctggcagaac atcttgcctt gatctggagc 360
cccatgtctg cccctctgta gaggtgcaca tgggccaatg atggatgcaa tgagctgatc 420
attttgctgt gatcaaggct atgttgcctc tgtgttaggc caatgtgagt aatggttctt 480
gtcttggttt agg                                                    493

<210> 38
<211> 294
<212> DNA
<213> unknown

<220>
<223> >gi|4312796|gb|AI459915|sr|IMAGp998L105312.1|cl|IM
      AGp998L105312|AI459915 ar81h05.x1 Barstead colon
      HPLRB7 Homo sapiens cDNA clone IMAGE:2151705 3',
      mRNA sequence

<400> 38
tttccagtac aaaaccaatt tattttacat tccatcgtgg gcgatgggag aagctaaata 60
ataatttagc atgcatacgt agacgtttct ttttatatat aaatacaata tacaaaaata 120
aagacagtac agttttgaga tttccagcag tttgaacgct cgtgacataa tccccccgac 180
cccagagacg taagcataaa cccttttttc tttttccaaa tactctgcag aatggcggct 240
ccagaggcgg tttcaagttt cataagtcag gtaacactgt gggtttccgc cttc       294

<210> 39
<211> 434
<212> DNA
<213> unknown

<220>
<223> >sr|IMAGp998D201008.1|cl|IMAGp998D201008|gb|BX1092
      26|oid|234552831 BX109226
      Soares_fetal_liver_spleen_1NFLS_S1 Homo sapiens
      cDNA clone IMAGp998D201008 ; IMAGE:429283, mRNA
      sequence

<400> 39
tgaagatgca gcccctggat ggtcccagac tctcaggaca tgcccagctc aggggcttcg 60
agccacaggc ctggcctcat atggcatgag ggggatctgg cataggagcc agactgctct 120
tagccccctg gctttgtgcc aggcctggag gagggcagtc ccccatgggg tgccgagcca 180
acgcctcagg aatcaggagg ccagcctggt accaaaagga gtacccaggg cctggtaccc 240
aggcccactc cagaatggcc tctggactca ccttgagaag ggggagctgc tgggcctaaa 300
gcccactcct gggggtctcc tgctgcttag gtccttttgg gacccccacc catccaggcc 360
ctttctttgc acacttcttc ccccacctct atgcatcttc cccccactgc ggtgttcggc 420
ctgaaggtgg tggg                                                   434

<210> 40
<211> 513
<212> DNA
<213> unknown

<220>
<223> >gi|5397100|gb|AI810534|sr|IMAGp998H065732.1|cl|IM
      AGp998H065732|AI810534 wb89h03.x1 NCI_CGAP_Pr28
      Homo sapiens cDNA clone IMAGE:2312885 3', mRNA
      sequence

<221> misc_feature
<222> 7
<223> n=a, t, c or g

<400> 40
ataatanttt aatttttttt tttttttttt taacttttcg ccttatttaa tactgggata 60
tggggtcagg gagtttgccc cacttggaga gatgacttcc cttggaaaac atggggtttc 120
cttctgaagg ggaatcctga ttccttcctt aatgttgggg ggatccaaaa gtgtggccag 180
atggaacatc tcattggcgg tttctttctt gcttgcttgg ggcttctctg tttggccttt 240
tccccattct cctttgtgtc cggcaggggg agaggcggct tcgtataagg gcctttttga 300
ggtttattat ttccttggcc atatacttga tgctcttcat tggcttgtct gggacctgcc 360
ttaggttctc cgaggcataa aagggccgga cagcccccga gttgggggaa ctctgaagct 420
tcttggtggc tggaaccttg gtcatcttaa aaatccttca ggttttagcc tgtgccccca 480
agacaaggat ttttccagaa tcttctactt cag 513

<210> 41
<211> 489
<212> DNA
<213> unknown

<220>
<223> >sr|IMAGp998H084295.2|cl|IMAGp998H084295|gb|BX1047
      34|oid|234543848 BX104734
      Soares_fetal_heart_NbHH19W Homo sapiens cDNA clone
      IMAGp998H084295 ; IMAGE:1691575, mRNA sequence

<400> 41
ctttgtgagt tcttttccct cctttaaagt tgggaaaact tgattcgtgg ggcaggagat 60
tgtttcttca ttcttctgac agcccccatc tgacgcgtaa ctgcccattt taaggaaact 120
cttggtgcta caaaaccctg accagacact tggcaaattt acctctttct tcaaaagaaa 180
aactttaaga aaatgagcca atgggcttca ttctcagtca tgcccggaga tcacccagga 240
gaaataatac aaacaccacc actgtccaga gagagtaaag aagcagaaag agaaagaatt 300
tgcaaccatg aggaatgttc ccacctcccg acgggacgtg catttggaaa acacagaatc 360
agccctcagg gtgcactcca gccacctcag tgctctaagc tcacagaagt gaaataatgt 420
ctgtgggttg gcaatggctt tgtgggatca tatgtcttgg ccaaagatgg gaaaacctat 480
gttgaagag 489

<210> 42
<211> 467
<212> DNA
<213> unknown

<220>
<223> >sr|IMAGp998F176102.2|cl|IMAGp998F176102|gb|BX1072
      49|oid|234548879 BX107249 NCI_CGAP_Ut1 Homo
      sapiens cDNA clone IMAGp998F176102 ;
      IMAGE:2453776, mRNA sequence

<400> 42
   tttttttttt ttttaagcaa gcacattata agaagcaata gatgtgccag ggcatatgaa 60
   catcagatga atggaagtta tcctgtgttt ttctttttaa cttaaattca tcttttttga 120
   attaaatctt aattgataca tcctgtatat gaaatttcac tttatggtat aaatcagagg 180
   cttcctatcc tataggaaag attcatgctt gtttttcaaa ttcagtacaa ttctcccagt 240
   gatttctagg aaagaataaa gaggagagga gaggtagaca tcagaatgta gagaggcaat 300
   actgtgtaaa aatgaaaaac aaaaaaaaaa actttatttc tattttccca ggctgcacaa 360
   atctggcttt gatgaagaaa ctcagaagat cctgatgtct gtggaagaaa aaattcccca 420
   tctgaccatt tcacatggac cttggattga cgaggaatac aagatca 467

<210> 43
<211> 275
<212> DNA
<213> unknown

<220>
<223> RZPD CloneID : IMAGp998K013431

<400> 43
tttttttttt tttttttttt tttttttttt tttttccaaa agccaaaaag tttatggcca 60
gaaaagcaaa cttcataaag accccttaaa gtacatcgaa taggacaagc aaaataaaca 120
gaaaactttg cccaaagaaa agatggccgc ggtcaggcac agtcaaatta ataccaaccc 180
aaacagggtc ctcgaagggt ataggggtta tacaacccac cctcggaaac taagggggcc 240
ccattccaaa atccttggtt ttgggttggg ggttt 275

<210> 44
<211> 359
<212> DNA
<213> unknown

<220>
<223> >sr|IMAGp998M23174.2|cl|IMAGp998M23174|gb|BX113547
|oid|234561471 BX113547 Soares infant brain 1NIB
Homo sapiens cDNA clone IMAGp998M23174 ;
IMAGE:41558, mRNA sequence

<221> misc_feature
<222> 39, 43, 93, 96, 101, 106, 125, 141, 156, 227, 248, 271,
276, 316, 323, 328, 334, 352
<223> n=a, t, c or g

<400> 44
tttttttttt ttttaagcag aaaaaagaga atttttaang gcncaacaaa aaagatttac 60
agcctacttt tctacagaaa tgatggaagt canaanacaa nggagngaca tcttcaggta 120
aagtnttaaa agaaaataag ngccaattta taattntata ccagcaaaaa tatgcttcaa 180
aaatgaaagt aaaataaaga tgtctactag gcaaacaaaa cagaganttt atcaccagca 240
aacctgtnct gaaagaaatg ctaaagaatt ntttanacac aaggaaaatg atcccagagg 300
gaaacatgaa attatncaaa gtnttganca ccancagaaa cggtaaatat gngaataaa 359

<210> 45
<211> 713
<212> DNA
<213> unknown

<220>
<223> >sr|IMAGp998C17793.3|cl|IMAGp998C17793|gb|BX089984
|oid|234514327 BX089984 Soares_fetal_heart_NbHH19W
Homo sapiens cDNA clone IMAGp998C17793 ;
IMAGE:346696, mRNA sequence

<221> misc_feature
<222> 696
<223> n=a, t, c or g

<400> 45
acatccgcca aatctatgac aaattcccgg agaaaaaggg tggactcaag gatctcttcg 60
aacggggacc ctccaatgcc ttttttcttg tgaagttctg ggcagacctc aacaccaaca 120
tcgaggatga aggcagctcc ttctatgggg tctccagcca gtatgagagc cccgagaaca 180
tgatcatcac ctgctccacg aaggtctgct ctttcggcaa gcaggtggtg gagaaagttg 240
agacagagta tgctcgctat gagaatggac actactctta ccgcatccac cggtccccgc 300
tctgtgagta catgatcaac ttcatccaca agctcaagca cctccctgag aagtacatga 360
tgaacagcgt gctggagaac ttcaccatcc tgcaggtggt caccaacaga gacacacagg 420
agaccttgct gtgcattgcc tatgtctttg aggtgtcagc cagtgagcac ggggctcagc 480
accacatcta caggctggtg aaagaatgag agactcgggg agcagggagg gggggaagag 540
acgtgtgtgc aggaaacggg gacgggggga aggggaccct gcaggggcag ccccctgaag 600
tgccaagaga gctgagagga gcagttgtga ctctacccag gaacaaactg tgcctgaacc 660
tgaggtgccc aaccccaaat aaacccaaga tgctgngtaa aaaaaaaaaa aaa 713

<210> 46
<211> 432
<212> DNA
<213> unknown

<220>
<223> >sr|IMAGp998O144918.2|cl|IMAGp998O144918|gb|BX1000
      64|oid|234534504 BX100064 NCI_CGAP_Lym12 Homo
      sapiens cDNA clone IMAGp998O144918 ;
      IMAGE:2000485, mRNA sequence

<400> 46
tttttttttt ttttgacagt gagcaataaa gtttaattaa ccagtactgt gaggtcacct 60
tcacggacta gcatcccttc cgtgagctga aggactgaat aagcagaagc acaggccaaa 120
cccatccctc catgctcccc actgtgcaga tgggctcact gaggctccgg tgtacattgc 180
ttgctcagca ctggatctat ttcgggccaa agtgtcagca gaactgatac ccacagggca 240
tccaagtcca catccagact gggctccctc taatgccatc tcttaaaccg aaccactgcc 300
tgccgatggt gagtttccaa tcacgagctg cactgggggt gctggaggcc tggctgggca 360
cacatcctgg tgggtatgaa ggggatttgg ggagtggcat ctagaagatc agcatgaggg 420
gcacagagga ga 432

<210> 47
<211> 710
<212> DNA
<213> unknown

<220>
<223> >sr|IMAGp998O021010.1|cl|IMAGp998O021010|gb|BX1155
      10|oid|234565395 BX115510
      Soares_fetal_liver_spleen_1NFLS_S1 Homo sapiens
      cDNA clone IMAGp998O021010 ; IMAGE:430297, mRNA
      sequence

<221> misc_feature
<222> 547, 591
<223> n=a, t, c or g

<400> 47
ctggtggcct tgctggatga cccaacaaat gcccatttca ttgcctggac gggccgggga 60
atggagttca agctcattga gcctgaggag gtcgccaggc tcttgggcat ccagaagaac 120

cggccagcca tgaattacga caagctgagc cgctcgctcc gatactatta tgagaaaggc 180
atcatgcaga aggtggctgg tgagcgttac gtgtacaagt ttgtgtgtga gcccgaggcc 240
ctcttctctt tggccttccc ggacaatcag cgtccagctc tcaaggctga gtttgaccgg 300
cctgtcagtg aggaggacac agtccctttg tcccacttgg atgagagccc cgcctacctc 360
ccagagctgg ctggccccgc ccagccattt ggccccaagg gtggctactc ttactagccc 420
ccagcggctg ttccccctgc cgcaggtggg tgctgccctg tgtacatata aatgaatctg 480
gtgttgggga aaccttcatc tgaaacccac agatgtctct ggggcagatc cccactgtcc 540
taccagntgc cctagcccag actctgagct gctcaccgga gtcattggga nggaaaagtg 600
gagaaatggc aagtctagag tctcagaaac tcccctgggg ggtttcacct gggccctgga 660
ggaattcagc tcagcttctt cctaggtcca agccccccac accttttccc 710

<210> 48
<211> 666
<212> DNA
<213> unknown

<220>
<223> >sr|IMAGp998E201114.1|cl|IMAGp998E201114|gb|BX1095
      81|oid|234553541 BX109581
      Soares_pregnant_uterus_NbHPU Homo sapiens cDNA
      clone IMAGp998E201114 ; IMAGE:470011, mRNA
      sequence

<400> 48
cttgggtgga tgggaaagtt aaatattggt ctcttcaagt tttttttttc ttttgctttg 60
ttaccacttg tcactgtctc catgttaaaa tgccaaaaat gatgtagttg ttgttgcttt 120
tttccctatt ttccacccca gtcgctcctt accgtgactc ctgcccttgg agggcatgta 180
gcagtgtctg tcctgccagt cccaaggccc tgtgggagga gactggcctg catctctcta 240
agacttagtc tgacgccacg cgcatctctt gttctgtgtt caatcagtag tccaggggag 300
aagcttctgc tacttcagag ctttgctaaa ctaacctaat ttgtccaaat caccccaaaa 360
ccaccatctc tgacgtaagc ttccatgcga cagcctgatc cgtttccctg gacaggtctc 420
tttcctggaa tgcagcccag gcacctgtgc tcctggcacc cttgaggtct ctcctttgag 480
ccgtggtcac cgagagggtt gaggacgcag cacccgaggt cccagccttt gcaggagcct 540
ccctgggctt agctggactt agatcttcgg tggcctcatg taaacgtggc agccagcctc 600
ttctagaacc ctagcccagg gactggagca ggaaagggac cttcaaagtg aagactgcct 660
tgtccc 666

<210> 49
<211> 1515
<212> DNA
<213> unknown

<220>
<223> Homo
      sapiens|chromosome:NCBI34:2:43429015:43430529:1

<400> 49
ggctttgcta atcacatcac aagaccttga gccagggcca aaggcgcgga gtggcgcggg 60
acgtcccggg cgggggagct gactctgagg acaggaggaa aggccgggag agccgtgcgg 120
gcttgaaggc gacttagcgc cacctttccg tctgctggtc gggccggcct tccccagccc 180
tctggggctg ggggtgcagc tgcctgccga ggctccccgc ccggcacagc ctgggcctgt 240
tgcgtcctcc cagcctcttc cctccccggc agtgggggcg ccgcgcccag cctttgctcg 300
atgagggctg caggatccgg ctgagaccaa cccgcggcgc aggtggggct gcacagacgc 360
tgatggtggg cgcgccaccc tctgttttcc ctcactcgcc agccccgcac cccaacagct 420
tgcgctggaa aatgcgcttt gccagcgggc gccccgcctt cgtccgggta cggaggggac 480
actcccatct agcgcccagt gaggctaaag ccgctgacag ccccactagg aacgatctgg 540
agtctggtct agggcacttc tccctccgcg ttcatacagg ttgccacaaa aagcttgggc 600
ctgaagctcg aaggccccgg acccgctggg gaaagaacta ggcctgccac tcagggattc 660
gagtccctgg agtgtatcct gcatttccca ctactgtact cccctcatgg cccagggagc 720

cagagtccag ggtaaggatc aggaatgctg ggctggagtt caggtcctgc cccagagaaa 780
tcatgtggtc ttaggcaaat cacatcatct ctttggtact tgggtttcct tgttaaacga 840
agataatatt cttgctttat tcatctctca cctttggcaa ggtgcaaatg atatctgtga 900
aggtgtttaa aagacaagca agaactaaac catctttatg tattaaacca aattgacaaa 960
agcgtaaggg tggagaaagc ctggcttctg aactgtggcc agatggacag agagccacag 1020
aatcatgagt gcagagctgt gggggcaccc aagtcctgga ccctaggact cacagccaag 1080
agcaggtgac ctcagaggga ccagtttcac ttttagcacc taccctacac tgaccccttg 1140
acatacctgg gcaactgtgg tctggtttgt tctttggtga gatgacccca aacatctcac 1200
caaactgtga aacagccact tccctaggca ctcctattag gttagtaaca agatttcaga 1260
tacagaacca gtggaaacat ctgtttgaaa caacgtccaa atctggggtg agtcccagtc 1320
aacagctgtg agaacaccca ctttgcagat taggattctg gagataacat gtggacatgt 1380
tacgggacat gcttgtgtac agagtacctg ctagtataaa atgtctaatg tagctgcttt 1440
tatttcattt taaatggaga aaaaaagaag tgctggccat ttccaatgag ctgggctttg 1500
ttgggccagt ttcat 1515

<210> 50
<211> 302
<212> DNA
<213> unknown

<220>
<223> >gi|4851404|gb|AI671673|sr|IMAGp998G195647.1|cl|IM
      AGp998G195647|AI671673 ty26e10.x1 NCI_CGAP_Ut3
      Homo sapiens cDNA clone IMAGE:2280234 3', mRNA
      sequence

<400> 50
tttttttttt tttttttttt tttttttttt tttttttttt tttttttttt tttttttttt 60
caaaaaaacc ggggaatttt tttaaaggcc ggggggcccc agaacccaag ggggttgggg 120
gttttaaagg acccgaaaaa aaaagggtaa aacccccgaa tttccccccg gcccccaaaa 180
tttggggggg gaaaaaaggg gttttggaaa aaaacccccc gccaaaccca accaaaaaaa 240
aaatcgggag ggggtttttt tcccccttgg ggtttaaaaa aacccccaaa attaggggcc 300
ca 302

<210> 51
<211> 538
<212> DNA
<213> unknown

<220>
<223> >sr|IMAGp998K06166.1|cl|IMAGp998K06166|gb|BX092114
      |oid|234518596 BX092114 Soares infant brain 1NIB
      Homo sapiens cDNA clone IMAGp998K06166 ;
      IMAGE:38228, mRNA sequence

<221> misc_feature
<222> 531
<223> n=a, t, c or g

<400> 51
tttttttttt ttttgattaa gaaagctaaa tttatattaa attatcataa agtcctaaaa 60
tactgaacat agtggttaaa taactccaga aagtccaatc tctccagtga gtaacgttaa 120
aaccattaca catgagcatg ggagaatcgc ttccattagt ttaggacaga gagattttgc 180
tttttacaga gtaaatcagt gctcaaatag atacttcctc aaatatgtcc tttctacatt 240
ctgaacagcc caagtgcaat aagatccttc cccctttcca atcaagaaaa tgccactttt 300
ctacttgctc ttcctcccca gacatgagtc taaggaccca aagtgctcac tcctttactg 360
cttgttaagt gtaatgtggg gaggctcaga actggggctg acgctactga gagcaaggct 420
aagggcctgg tatctctctc tagcagtctt agaactgact agaaaagaat tgatcttgtc 480
ctttttcact ttggtagctg atttctcaat accagtaacc acaggagaag nttcaact 538

<210> 52
<211> 680
<212> DNA
<213> unknown

<220>
<223> >sr|IMAGp998D03384.3|cl|IMAGp998D03384|gb|BX111866
      |oid|234558109 BX111866 Soares fetal liver spleen
      1NFLS Homo sapiens cDNA clone IMAGp998D03384 ;
      IMAGE:200018, mRNA sequence

<221> misc_feature
<222> 3, 13, 33
<223> n=a, t, c or g

<400> 52
ttnggcccta cgngccagta attcggcacg agnatttgcc ttggctcaga aggcctctgg 60
gggccatccc tgaccactag gcttcccact tagacctgct ccagcagcac cacccctcgc 120
cactgcctgg tcctttcctt cacccttgat tctgtcttct tttgtccttc tccaggtctt 180
ggcagcacct gtctcctttc accccagggc ctgagcctgg ccagcctaca atggggatgt 240
tgtgtttctg ttcaccttcg tttactatgc ctgtgtcttc tccaccacgc tggggtctgg 300
gaggaatgga cagacagagg atgagctcta cccagggcct gcaggacctg cctgtagccc 360
actctgctcg ccttagcact accactcctg ccaaggagga ttccatttgc agagcttctt 420
ccaggtgccc agctatacct gtgcctcggc ttttctcagc tggatgatgg tcttcagcct 480
ctttctgtcc cttctgtccc tcacagcact agtatttcat gttgcacacc cactcagctc 540
cgtgaacttg tgagaacaca gccgattcac ctgagcagga cctctgaaac cctggaccag 600
tggtctcaca tggtgctacg cctgcatgta aacacgcctg caaacgctgc ctgccggtaa 660
acacgcctgc aaacgctgcc 680

<210> 53
<211> 713
<212> DNA
<213> unknown

<220>
<223> >sr|IMAGp998E01223.1|cl|IMAGp998E01223|gb|BX103782
      |oid|234541945 BX103782 Soares placenta Nb2HP Homo
      sapiens cDNA clone IMAGp998E01223 ; IMAGE:147048,
      mRNA sequence

<400> 53
cacgtgtggt accagggcaa cgcgtcgctg gggggacacc taacgcacgt gctggaaggc 60
ccagacacca acaccacgat cattcagctg cagcccttgc aggagcccga gagctgggcg 120
cgcacgcaga gtggcctgca gtcctacctg ctccagttcc acggcctcgt gcgcctggtg 180
caccaggagc ggaccttggc ctttcctctg accatccgct gcttcctggg ctgtgagctg 240
cctcccgagg gctctagagc ccatgtcttc ttcgaagtgg ctgtgaatgg gagctccttt 300
gtgagtttcc ggccggagag agccttgtgg caggcagaca cccaggtcac ctccggagtg 360
gtcaccttca ccctgcagca gctcaatgcc tacaaccgca ctcggtatga actgcgggaa 420
ttcctggagg acacctgtgt gcagtatgtg cagaaacata tttcctcgga aaacacgaaa 480
gggagccaaa caagccgctc ctacacttcg ctggtcctgg gcgtcctggt gggcagtttc 540
atcattgctg gtgtggctgt aggcatcttc ctgtgcacag gtggacggcg atgttaatta 600
ctctccagcc ccctcagaag gggctggatt gatggaggct ggcaagggaa agtttcagct 660
cactgtgaag ccagactccc caactgaaac accagaaggt ttggagtgac agc 713

<210> 54
<211> 317
<212> DNA
<213> unknown

<220>
<223> >gi|4073985|gb|AI337058|sr|IMAGp998D054941.1|cl|IM
      AGp998D054941|AI337058 qx82g03.x1 NCI_CGAP_GC6
      Homo sapiens cDNA clone IMAGE:2009044 3', mRNA
      sequence.

<400> 54
aagtcttcat taagagagga aaagatgaaa actgccttta catgataaat tatatatgtc 60
aaaaatctag tatcaagttt aatggaacaa tgtcgcagcc tgatgggttc ttctttccca 120
ctgccctgaa aaaagactaa tgagaccagc aggagttgca acaaagaatg taataatcac 180
aggcctgctg agcaaggaga acagggagaa agttctcaaa cctgtctccc ccagaatttg 240
gaggctgggg ttttgtaaaa gtacttttta aaaagtactt tcccagcagg gttctgggaa 300
actggaacag atgattg                                                317

<210> 55
<211> 832
<212> DNA
<213> unknown

<220>
<223> >sr|IMAGp998A17115.1|cl|IMAGp998A17115|gb|BX097937
      |oid|234530247 BX097937 Soares fetal liver spleen
      1NFLS Homo sapiens cDNA clone IMAGp998A17115 ;
      IMAGE:122008, mRNA sequence

<400> 55
   acaaaggcta ttaacacata ggaaaaaatg tatttactaa gtgtcacatt tctctaagat 60
   gaaagatttt tactctagaa actgtgcgag cacaacacac acaatccttt ctaactttat 120
   ggacactaaa ctggagccaa tagaaaagac aaaaatgaaa gagacacagg gtgtatatct 180
   agaacgataa tgcttttgca gaaactaaag cctttttaag aaatgccagc tgctgtagac 240
   cccatgagaa aagatgtctt aatcatcctt atgaaaacag atgtaaacaa ctatatttca 300
   actaacttca tcttcactgc atagcctcag gctagtgagt ttgccaaaac caaagggggt 360
   gaatacttcc ccaagattct tcctgggagg atggaaacag tgcagcccag gtcccatggg 420
   ggcagctcca tcccagagca tttctgatag ttgaactgta atttctactc ttaagtgaga 480
   tatgaagcat tatccttttg ttcagttgcc ccgggctttt gaacagaaga gtaaatacag 540
   aattgaaaaa gataaacact caaccaaaca atgtgaaaac gggttctgta gtatttgtaa 600
   aaaggcccgg cccaggacca ctgtgagctg gaaaagggag aaaggcagtg ggaaaagagg 660
   tgagccgaag atcaattcga cagacagacg gtgtgtatgc ccctccctgt ttgacttcac 720
   acacactcat aactttccaa atgaaacccc acagtatagc gcatattttc gatatttttg 780
   tgaattccaa aagggaaatc acaggggctg gtcgaaatat tgggggaaca ct        832

<210> 56
<211> 455
<212> DNA
<213> unknown

<220>
<223> >gi|4735095|gb|AI651116|sr|IMAGp998K035709.1|cl|IM
      AGp998K035709|AI651116 wa97e02.x1 NCI_CGAP_GC6
      Homo sapiens cDNA clone IMAGE:2304122 3', mRNA
      sequence

<400> 56
   tttttttttt tttttttttt ttttctaagg gaaattattg ttatttcgct ctaacgatta 60
   cccaagaccc caaaataaaa actaacccag caggttttgt tcacattaat ggccagagta 120
   caacccatga atctttaaag ggagaaaaat gcttcaaatt ttatgccctg agagtttttc 180
   ttctatttgt tctagcctat cattgaagct tttgagggta ctttgtattt catccaatga 240
   tttcttcagt tccagaaata ctcttctgtt gaattttaaa accttttcct ggcaggaaat 300
   catctcatat atctccccac ctcttttcct catttcttta tgttggtgtt caaaagactc 360
   ttccatgttg atgaccttct ttacaatcag tccggccaat tctctgctca attctctgtt 420
   tgagctttcc tgaatttctt ttggatgggg atctg 455

<210> 57
<211> 480
<212> DNA
<213> unknown

<220>
<223> >sr|IMAGp998F13278.1|cl|IMAGp998F13278|gb|BX092118
      |oid|234518604 BX092118 Soares infant brain 1NIB
      Homo sapiens cDNA clone IMAGp998F13278 ;
      IMAGE:48538, mRNA sequence

<400> 57
tttttttttt ttttaaacga aaagcaaact tctttattgt acacagtaca gaatataacg 60
cagcttggca ggatgcatac ggccctgcgc aggggaaagt atttcaaatc agctggcagg 120
ttcaagcctt tctgcactgt agactttcca cactctggaa aagaagcaaa caaacaaacc 180
ccaaagaacc cccgaaaaaa acaaaaacca tccgggaggt gcatgagtcc aatggaaatg 240
caaccgtgat gccgctgtcc tatgcccagt gacagcacag gtcacgtaag ttacagcagg 300
ggaggggtag ctcaagctac agaggattat tgtcatattg ctaagacagc ataaatccat 360
tcaaaaaaaa aaaaaaaaaa atccaaacca gggtaagtaa agaaaggaaa accaaatcta 420
tacagcattt acaacaaata aatctctagc cagctggggg taaaatatgc atctatgtat 480

<210> 58
<211> 786
<212> DNA
<213> unknown

<220>
<223> >sr|IMAGp998B06202.4|cl|IMAGp998B06202|gb|BX092972
      |oid|234520310 BX092972 Soares placenta Nb2HP Homo
      sapiens cDNA clone IMAGp998B06202 ; IMAGE:138917,
      mRNA sequence

<221> misc_feature
<222> 784
<223> n=a, t, c or g

<400> 58
ctttgtctca gagccctctg ggctctctcc tctgagggag ggacctttct ttcctcacaa 60
gggacttttt tgttccatta tgccttgtta tgcaatgggc tctacagcac cctttcccac 120
aggtcagaaa tatttcccca agacacaggg aaatcggtcc tagcctgggg cctggggata 180
gcttggagtc ctggcccatg aacttgatcc ctgcccaggt gttttccgag gggcacttga 240
ggcccagtct tttctcaagg caggtgtaag acacctcaga gggagaactg tactgctgcc 300
tctttcccac ctgcctcatc tcaatccttg agcggcaagt ttgaagttct tctggaacca 360
tgcaaatctg tcctcctcat gcaattccaa ggagcttgct ggctctgcag ccacccttgg 420
gccccttcca gcctgccatg aatcagatat ctttcccaga atctgggcgt ttctgaagtt 480
ttggggagag ctgttgggac tcatccagtg ctccagaagg tggacttgct tctggtgggt 540
tttaaaggag cctccaggag atatgcttag ccaaccatga tggattttac cccagctgga 600
ctcggcagct ccaagtggaa tccacgtgca gcttctagtc tgggaaagtc acccaaccct 660
agcagttgtc atgtgggtaa cctcaggcac ctctaagcct gtcctggaag aaggaccagc 720
agcccctcca gaactctgcc caggacagca ggtgcctgct ggctctggtt tggaagttgg 780
gggnaa 786

<210> 59
<211> 476

<212> DNA
<213> unknown

<220>
<223> >sr|IMAGp998E1783.1|cl|IMAGp998E1783|gb|BX109827|o
      id|234554033 BX109827 Soares fetal liver spleen
      1NFLS Homo sapiens cDNA clone IMAGp998E1783 ;
      IMAGE:109816, mRNA sequence

<400> 59
ctttgccctg ggtaagctgc tgtagggtca gaagtaaccc tttctgtgtc agttgagaat 60
gagcctgtgt ggtagctgat gtcagaggac aaagctctct gcaagggctg gacacagagc 120
tgcagagtcc tgaacatccc tcctttcagg ctgcagaagg gagaggcaat gaagacagct 180
gctccggaag cagcatcagg gctcttggag gggactggtg gggactcagg ctgggtgcag 240
cctccaaaca gagaacggaa cttaggtgtg tctctacagc taggcccagc ctagcccagc 300
ccagaacaaa cacccttcag agcctaacca aagaacataa gctgcaaaat gtgcacccat 360
attttaagct gctttttcag gggataaata gtgtttgggg acattgaaat ggatgttctc 420
aggttgtatt tatttcggac aaataaacta gagaattgtg taaaaaaaaa aaaaaa     476

<210> 60
<211> 457
<212> DNA
<213> unknown

<220>
<223> >sr|IMAGp998L095608.2|cl|IMAGp998L095608|gb|BX1059
      35|oid|234546251 BX105935 NCI_CGAP_Ut3 Homo
      sapiens cDNA clone IMAGp998L095608 ;
      IMAGE:2265368, mRNA sequence

<400> 60
tttttttttt ttttaaaaat aatttacttt attacaaaga tagtaataca atttctagaa 60
aatacatgca tgtacaaaga ttgaaataaa aaaaacactc ataaatatcc agatttaaat 120
gtcagcatat accttcctga tctttttcta tcatactgta tctatcaaca tctaacctgc 180
ttttttaact ataagtcata cattttgcca tatcagtaca cttttttctg taacattatt 240
tttagagcat atcaaaccat agtttattta accaatcccc aattaattca taattagaat 300
atttcgtgtt tttccagtat aacagttcta taatctttga ttataacctt tgtgtaaatt 360
cctataagta ttgagtcaaa ggagacacac atcttttcag gcattacata taaaataaat 420
actaaactag actccaggaa agtggtatca attcaca                          457

<210> 61
<211> 517
<212> DNA
<213> unknown

<220>
<223> >gi|1274428|sr|IMAGp998E11660s1|cl|IMAGp998E11660|
      gb|W02449|W02449 za47a06.r1 Soares fetal liver
      spleen 1NFLS Homo sapiens cDNA clone 295666 5'

<221> misc_feature
<222> 481
<223> n=a, t, c or g

<400> 61
tctttagaga aaaatatttt taatgtagaa aagatgaata gctgtaatga aatacaaacc 60
gttcctatac acagtaaact tactaagaac tgctaatcct tctccaggtt cttagaatct 120
gggtacagca ttatagaaag taaccacgct atcgaaaaca cacacccaaa acccgatggg 180
tggcaattgt gatagattag gaagacaaaa cagattcaga agtagcagag aatactgcac 240

attttcttgg aaaatttctt aattgttcag tgctttctga caagtttccc cagcccaatt 300
tatctcttag ctattctctg ctacttagtc ctgtcttctg acaatcatca tacggggacg 360
atcatctgcc ttacagtgta acatgttttt cctaacacag ccgcatttta tctggcccta 420
agagaatagc aactaatggg ctttcaatga agtcaccatg atttaggaaa gtttaaaacc 480
ncaaattttt ccatggtggg taaaacttct aattacc 517

<210> 62
<211> 343
<212> DNA
<213> unknown

<220>
<223> >gi|2229312|gb|AA495991|sr|IMAGp998K051891.2|cl|IM
      AGp998K051891|AA495991 zw06e03.s1 Soares_NhHMPu_S1
      Homo sapiens cDNA clone IMAGE:768508 3', mRNA
      sequence

<400> 62
tttttttttt ttttttaaga aaaaatgtct attatttatt acacaataat tctgaccacc 60
aacaaccaac ggcgggggcg ggcaggagag aagaacatct tgcttctcaa caaactttcc 120
tcccttgctt taacattttt gaggattctt tcccaaacct attacacctg tattatgatg 180
gttacaaatt ttccaactct tccactcctt ccagtgcatt atttttagta tcttcattaa 240
acgggcaaaa aaaaagatcc cctacttgta ataacaaaac aatgttggaa actgtcatta 300
aatcaggata agtgaaaaac agatctgttc ccagactcgc agg 343

<210> 63
<211> 330
<212> DNA
<213> unknown

<220>
<223> >gi|1018091|sr|IMAGp998J14406|cl|IMAGp998J14406|gb
      |H63290|H63290 yr49d07.r1 Homo sapiens cDNA clone
      208621 5'

<221> misc_feature
<222> 198, 235, 252, 268, 282, 292
<223> n=a, t, c or g

<400> 63
atgttcaacc tcagtccctc aaggccatac ctggacccaa cggtgaagaa agacgatgag 60
gaggaggacc cgctggacca gctgatctcc cgctctggct gtgctgcctc ccactttgca 120
gtgcaggagt gcatggccca gcaccaggac tggcggcaat gcagccacag gtgcaggcgt 180
tcaaggattg catgagtnaa cagcaggcga ggcggcaaga ggagctgcag aggangcaag 240
aacaagccgg tntccaccac tgagaccnca aaccacctat cnccagtaga tnggcccttc 300
aagaccagca tcctagcaag attattagag 330

<210> 64
<211> 430
<212> DNA
<213> unknown

<220>
<223> >sr|IMAGp998B231782.3|cl|IMAGp998B231782|gb|BX0898
      18|oid|234513990 BX089818 Soares_testis_NHT Homo
      sapiens cDNA clone IMAGp998B231782 ; IMAGE:726454,
      mRNA sequence

<400> 64

ctcaatggcg gtctggccgt gaagaccaat gagatctctg tgatccagag tgggggggtt 60
cctaccctcc cggtttcctt gggggccacc tccgttgtga ataacgccac tgtctccaag 120
atggatggct cccagtcggg tatcagtgca gatgtggaaa aaccaagtgc tactgacggc 180
gttcccaaac accagtttcc tcacttcctg gaagaaaaca agattgcggt cagctaaggg 240
agaacttgcg tggaaggagc aatgcagaca cagtgaaatc tctagaatct gctttgtttt 300
gtaagaactc atctcctcct gttttctttt tcttactgat atgcaaatga tgtttactac 360
gttggttgtg accacaacct caggcaagtg ctacaatcac gattgttgct atgctgcttt 420
gcaaaaagtt 430

<210> 65
<211> 365
<212> DNA
<213> unknown

<220>
<223> >gi|3148939|gb|AA973759|sr|IMAGp998M024021.1|cl|IM
      AGp998M024021|AA973759 oq16b01.s1 NCI_CGAP_GC4
      Homo sapiens cDNA clone IMAGE:1586473 3' similar
      to contains element MSR1 repetitive element ;,
      mRNA sequence

<400> 65
tcactttgca attctttatt agaactcaag tttgtgtttg caaccatgca acagaaaggg 60
taaaacaaaa aatccacatc ctctccccaa ccccagcttc tgggcggaaa tacgggcttt 120
cactttaacc cctgactggc tctggctcca gggcctgaga tgccttcata atctagtaac 180
tgggtcccgg gcatcctcgc gaaaccgctg ggtcactgcc ttgctggcag cttcggggga 240
tcgctgggtc cccgacttgt tggcagcctg gagagactgc tgggtcccgg cctcctgcac 300
ctccaccgga gaaccctgtg tcccgacctc ctggacttcc accgaacgct gggtcccggc 360
ctccc 365

<210> 66
<211> 507
<212> DNA
<213> unknown

<220>
<223> >gi|5368831|gb|AI803437|sr|IMAGp998C105092.1|cl|IM
      AGp998C105092|AI803437 tc39f05.x1
      Soares_total_fetus_Nb2HF8_9w Homo sapiens cDNA
      clone IMAGE:2067009 3', mRNA sequence.

<400> 66
tttgtttata aatggttatt ttattcagag tggttgcttt ttaatgctgc tctcttaatt 60
tcctgttaaa tctgtttcat ctatagccat ccaatattag tatttatttt gttcatttaa 120
ctttaaagta ttcttaaatt aaatctatta caaatattct actgctggtg aagctaattc 180
agcataggca ctaccacaaa aatatggaaa acatgatcaa tgtattatag atgtaatctg 240
gcttcagaaa cacaatacta aaaactattt tagtaaatta agaaataagc attctagatt 300
gtgttataaa atccatttct aaatataccc ctttttaaac ctttatgctt ctttaccttg 360
gaattcttac cagcgtagct agattttgta ttgcaatggt aattatactt aagaatcatt 420
gtgtgttaaa atattcccca cgtacagatt atttttatca tcttttccct aaaataaaat 480
tctcagcatt taacctatgc atgttgg 507

<210> 67
<211> 638
<212> DNA
<213> unknown

<220>
<223> >sr|IMAGp998I19131.1|cl|IMAGp998I19131|gb|BX110791

|oid|234555961 BX110791 Soares fetal liver spleen
      1NFLS Homo sapiens cDNA clone IMAGp998I19131 ;
      IMAGE:127962, mRNA sequence

<400> 67
tgtggaagtt gttctagaag ttagatggac agtgggagca cattcgtgag tggggcttgg 60
gagagctcaa aagtggcact ttgattttag gctgggcagg cagaagtctc acagtaaaat 120
atatttcact agacgtccaa accaacactg cattgtagta acctcttctg aagtaactgg 180
ttaaattaat aactggtccc ataagtcagg gtcaagccac atttataacc cattcgggcc 240
ggccatgaag ggttctgact cgacattggt atttaaatgc tgttactttc ccaatgctta 300
gtagcacttg agactttatt tccatcgaca aaaaccaaga aggccacaga agtagcctaa 360
cagtcctccc tggtcatact cgtttcaaac aggtctgcct gtctagacat attcaggcag 420
gaggttgctt gctgctatct tgaaaaccac tggaagattc agcttcttgg ggacctcaga 480
gcccatattc atatggaagc ccacccatat aagcctcaca gagcaaatca cagcaatgtt 540
gaagttgtta ttttcaagtg cttatttcac gacattaaaa aaaatatttt ttcctggtgt 600
attaaaatta aaaataaaat cataactttt gatttaaa                         638

<210> 68
<211> 445
<212> DNA
<213> unknown

<220>
<223> >sr|IMAGp998F246080.2|cl|IMAGp998F246080|gb|BX1049
      07|oid|234544194 BX104907 NCI_CGAP_Gas4 Homo
      sapiens cDNA clone IMAGp998F246080 ;
      IMAGE:2445335, mRNA sequence

<400> 68
tttttttttt ttttaatgca caaaggtttt aataccttgg ctttaatgat ttttcaaggt 60
taagaaacaa attcaaattg gttggagctt caactcagta attacaatca caatgcatct 120
ctgaaaggcc ctgcatttgg aggcagagta atctgcaaag atgatagttt ttacatatgt 180
cctgttacct acaccaatat aattactaca ttatcttata aagacaaaca gttgcttcaa 240
actctttaaa aaatatatat ataatgagtt tcccaaagac tcgagtctat attcaaagat 300
gagtaaaaaa aaatccatta cttccctagg gtcactttct tcctttactc ctgcttaaat 360
gcaaaagctg atagtttctg atttgtagaa aaatctaaag gtttctgctt tttagacaaa 420
ttcaggttct cttttgcttt ttctt                                       445

<210> 69
<211> 464
<212> DNA
<213> unknown

<220>
<223> >gi|3483725|gb|AF086380|sr|IMAGp998B03791.1|cl|IMA
      Gp998B03791|AF086380 Homo sapiens full length
      insert cDNA clone ZD69C02

<400> 69
gtttggaaac atttaattta tacccctctc ctctgtctcc agaagcttct agctctgggg 60
tctggctacc gctaggaggc tgagcaagcc aggaagggaa ggagtctgtg tggtgtgtat 120
gtgcatgcag cctacaccca cacgtgtgta ccgggggtga atgtgtgtga gcatgtgtgt 180
gtgcatgtac cggggaatga aggtgaacat acacctctgt gtgtgcactg cagacacgcc 240
ccagtgtgtc cacatgtgtg tgcatgagtc catgtgtgcg cgtggggggg ctctaactgc 300
actttcggcc cttttgctct gggggtccca caaggcccag ggcagtgcct gctcccagaa 360
tctggtgctc tgaccaggcc aggtggggag gctttggctg gctgggcgtg taggacggtg 420
agagcacttc tgtcttaaag gttttttctg attgaaaaaa aaaa                   464

<210> 70

<211> 727
<212> DNA
<213> unknown

<220>
<223> >sr|IMAGp998N15372.1|cl|IMAGp998N15372|gb|BX105335
      |oid|234545051 BX105335 Soares fetal liver spleen
      1NFLS Homo sapiens cDNA clone IMAGp998N15372 ;
      IMAGE:195662, mRNA sequence

<221> misc_feature
<222> 725
<223> n=a, t, c or g

<400> 70
caatccttct gccttggccc cccaagtgct gggattgtgg gcatgagctg ctgtgcccag 60
cctccatgtt ttaatatcaa ctctcactcc tgaattcagt tgctttgccc aagataggag 120
ttctctgatg cagaaattat tgggctcttt tagggtaaga agtttgtgtc tttgtctggc 180
cacatcttga ctaggtattg tctactctga agacctttaa tggcttccct ctttcatctc 240
ctgagtatgt aacttgcaat gggcagctat ccagtgactt gttctgagta agtgtgttca 300
ttaatgttta tttagctctg aagcaagagt gatatactcc aggacttaga atagtgccta 360
aagtgctgca gccaaagaca gagcggaact atgaaaagtg ggcttggaga tggcaggaga 420
gcttgtcatt gagcctggca atttagcaaa ctgatgctga ggatgattga ggtgggtcta 480
cctcatctct gaaaattctg gaaggaatgg aggagtctca acatgtgttt ctgacacaag 540
atccgtggtt tgtactcaaa gcccagaatc cccaagtgcc tgcttttgat gatgtctaca 600
gaaaatgctg gctgagctga acacatttgc ccaattccag gtgtgcacag aaaaccgaga 660
atattcaaaa ttccaaattt ttttcttagg agcaagaaga aaatgtggcc ctaaaggggg 720
ttagntg                                                           727

<210> 71

<220>
<223> gi|2113210|gb|AA430036|sr|IMAGp998G201924.1|cl|IMA
      Gp998G201924|AA430036 zw65f10.s1 Soares_testis_NHT
      Homo sapiens cDNA clone IMAGE:781099 3', mRNA
      sequence

<400> 71
 000

<210> 72
<211> 428
<212> DNA
<213> unknown

<220>
<223> >gi|713479|sr|IMAGp998E1983|cl|IMAGp998E1983|gb|T8
      5127|T85127 yd31a10.r1 Homo sapiens cDNA clone
      109818 5'


<221> misc_feature
<222> 191, 306, 365, 390, 417
<223> n=a, t, c or g

<400> 72
taagcccaga atgaagctgc tgggtcatcg tagggcactt gcatgcttta tgatttaatg 60
actgcctagt tgtgcacaga ggcctgccca gtctgtttcc accagtatat ccagtaaagg 120
gtgctgtaca ccacctctgc ccacacttgg cctttcccac tctctggtgg ttgccagtcc 180

acaggcgtag nagctgcctt gctgttcttc aggcacatcc ctctgggcac ccacataaca 240
gaggtgtctc tgtgtgctct gcctgctgtt cattcccagc taattcgggg agggccgagg 300
gcaggnaaaa ttcgctttaa acccggggag ggcaggaggt ttggcaagtt gaggccgagg 360
atggncaacc atttgcggtt ttcagttttn ggggcaaaca ggaggttagg atttccnttt 420
ttcaaaaa 428

<210> 73
<211> 379
<212> DNA
<213> unknown

<220>
<223> >sr|IMAGp998C09200.1|cl|IMAGp998C09200|gb|BX105022
      |oid|234544425 BX105022 Soares placenta Nb2HP Homo
      sapiens cDNA clone IMAGp998C09200 ; IMAGE:138176,
      mRNA sequence

<221> misc_feature
<222> 335, 371
<223> n=a, t, c or g

<400> 73
tttaaatgca ctgaacgtga aagcatttct ctcaccaaat tagaaagttc acccaggaag 60
cttcataaag ataagagaca ggaaaataaa cataagacct ttttaccggt gaaaggtaac 120
acagaaaaat caaacatgct ggagtttaaa ttatgtccag atatcttact aaagaataca 180
aactctgtgg aagaacggaa ggatgtaaag cctcatccta ggaaggagca agcccctctg 240
caagtttcag gaataaaaag tacaaaagaa gactggttaa aatttgttgc tacaaagaaa 300
aggacacaga aagacagcca agagagagat aatgntaatt caagactctc gaagagaagc 360
ttcagtgcag ntggatttg 379

<210> 74
<211> 607
<212> DNA
<213> unknown

<220>
<223> >gi|4074023|gb|AI337096|sr|IMAGp998I204942.1|cl|IM
      AGp998I204942|AI337096 qx88b10.x1 NCI_CGAP_GC6
      Homo sapiens cDNA clone IMAGE:2009563 3', mRNA
      sequence

<400> 74
ccagttgtta caaatttatt ttctatagtg gacttcacat gtaatttttt ccaacaaagt 60
attcaattga atggaagcag gaaattgaaa atcactgata ttgaagatga tgtactggcg 120
gataaaggca cctgccctcc tgtaatattc agtagaaatt ttgagatctt gaaaacatat 180
gaaaaatgtg ggctatgtta tcactcctga cagtgatggc tttgatggag tgggatgact 240
ttggacccat ctcttggcca gtgtatctgt gccagggtca aaggagaagg cactgggttt 300
ggactcactc aatccaaatt gccttgctct gagattttat gtagatcagt gaaaacttga 360
gacttcctga agaccagaag gaggcttcca ttccatctga tctcacctag atctggagca 420
aaggcaacat gataggtaaa atttgcacat cagatggtca aataggaatc agataataaa 480
aatgtggtcc tcgctgtctg ttcagcatgg tgagtgtgtg cctgtgctcc ctgattcttc 540
ttggctgagg tgttgagctg atactggtga gtggcaagga gtcctttgcc ttctgagctt 600
tgagcac 607

<210> 75
<211> 495
<212> DNA
<213> unknown

<220>
<223> >gi|5439998|gb|AI820919|sr|IMAGp998H171818.2|cl|IM
      AGp998H171818|AI820919 zu39g09.y5 Soares ovary
      tumor NbHOT Homo sapiens cDNA clone IMAGE:740416
      5', mRNA sequence

<221> misc_feature
<222> 483
<223> n=a, t, c or g

<400> 75
actatcctga cgtgtgacag gtccctgatc cgccccagcc tgcaggccag aagcagtgtt 60
acacacttcc tggaggagct aaggaaatcc tcagactcct gcgtttttgt tgttgctgtt 120
gatgtttttt aaaaggtgtg ttggcatata atttatggta atttattttg tctgccactt 180
gaacagtttg ggggggtgag gtttcattta aaatttgttc agagatttgt ttcccatagt 240
tggattgtca aaaccctatt tccaagttca agttaactag ctttgaatgt gtcccaaaac 300
agcttcctcc atttcctgaa agtttattga tcaaagaaat gttgtcctgg gtgtgttttt 360
tcaatcttct aaaaaataaa atctggaatc ctgctttttt gctctactag tacctctgtc 420
acactagtct tatcaaaaac cagttcttaa gaatcaatgt aagtttatta gttaatgtaa 480
atntctcatc ctcga                                                  495

<210> 76
<211> 719
<212> DNA
<213> unknown

<220>
<223> >gi|4435381|gb|AI521246|sr|IMAGp998P175394.1|cl|IM
      AGp998P175394|AI521246 to66g09.x1 NCI_CGAP_Gas4
      Homo sapiens cDNA clone IMAGE:2183296 3' similar
      to gb:U02570 !!!! ALU CLASS C WARNING ENTRY !!!!
      (HUMAN);, mRNA sequence.

<400> 76
aacttattaa aaagagtttt atttagcact tggttctgtt ggctgggaaa ttcaggatca 60
ggcagctgta tctggtgggt tctcatgact gcctcatgct gcatcaaaac atcgtagaga 120
aacagaaggg gaccgagttt gtgcaaacaa aaagcgcaaa atagaagagg cagcattgtt 180
ttataacaac tcactctctt gggaactaac cattcccagg agaacccagt ctcagtgtca 240
agagaaagat gttaatccat cttagcaacc taattacctc ttaaaaatac catctcccaa 300
cactattaca ttggcaattg aactacaaca tgagttttgg aggggccaaa caacatgcaa 360
accatagcag caggtttaaa ggtaaaagga tgggagaaaa gtacacagaa agatattctg 420
gagaaaaggc acagagtgag ccaaggcaca aggtaaaatg tttgactggt tagagacgct 480
agattatttt gtgtggctaa gacaaaacaa agctggtggg gagtggcagg aggtaagtct 540
agaatggcac aatggggcca gactgcgaat gcaaacaagt atgtgatctt ttatttctaa 600
agaaatgtag tatgattagc acacacacac acaaaaatta ttggccgggc gcagtggcca 660
tgcctgttat ccagcacttt gggaggccga gagggtggac atgaggtcag agttcagac  719

<210> 77
<211> 420
<212> DNA
<213> unknown

<220>
<223> >gi|4834825|gb|AI670051|sr|IMAGp998F105821.1|cl|IM
      AGp998F105821|AI670051 we64d05.x1
      Soares_thymus_NHFTh Homo sapiens cDNA clone
      IMAGE:2345865 3', mRNA sequence

<400> 77
tttttttttt tttttttttt tttttttttt tttttttttt tttttttttt tttttttttt 60
tttttttttt ttggggtttt aaaaaaaaaa agggtttttc caggggggaa agggaggggt 120
aaacccgggc ccccaaatgg gggcccgttc ccccgggtaa aaaaaagccc ccggaaaaaa 180
gggtccccat tgggtttaaa ccccctaaaa aattttttgg cccccccccc ccccgggggc 240
cttttggaaa aaaaaatttg gctaatatgg caaaaaaccc caaccctttt tcccgccccc 300
tttaaaaaaa ccggccccta ccggggggcc ggcccccccc caaaaggggg gaaattccaa 360
atgggttggg taaacccccc cctttaaaaa aaggggaaac cggggccaaa aaaggaaaaa 420

<210> 78
<211> 708
<212> DNA
<213> unknown

<220>
<223> >sr|IMAGp998G01671.1|cl|IMAGp998G01671|gb|BX109981
      |oid|234554341 BX109981 Soares_fetal_lung_NbHL19W
      Homo sapiens cDNA clone IMAGp998G01671 ;
      IMAGE:299928, mRNA sequence

<221> misc_feature
<222> 654, 690, 707
<223> n=a, t, c or g

<400> 78
catctgcaat agtatggcta agacgctgca gacagccctg cagccccctg actggctgca 60
gggccactac ctggtggtgc ggtacgagga cctggtggga gaccccgtca agacactacg 120
gagagtgtac gattttgtgg gactgttggt gagccccgaa atggagcagt ttgccctgaa 180
catgaccagt ggctcgggct cctcctccaa gcctttcgtg gtatctgcac gcaatgccac 240
gcaggccgcc aatgcctggc ggaccgccct caccttccag cagatcaaac aggtggagga 300
gttttgctac cagcccatgg ccgtcctggg ctatgagcgg gtcaacagcc ctgaggaggt 360
caaagacctc agcaagaccc tgcttcggaa gccccgtctc taaaaggggt tcccaggaga 420
cctgattccc tgtggtgata cctataaaga ggatcgtagt gtgtttaaat aaacacagtc 480
cagactcaaa cggaggaagc ccacatattc tattatagat atataaataa tcacacacac 540
acttgctgtc aatgttttga gtcagtgcat ttcaaggaac agccacaaaa tacacacccc 600
taagaaaagg caagacttga acgttctgac caggtgcccc ctcttcttct ttgncttctc 660
ttggcctctt tctcctattt cttaccctgn cctccacctg cctttcnt 708

<210> 79
<211> 398
<212> DNA
<213> unknown

<220>
<223> >gi|4687900|gb|AI636570|sr|IMAGp998D125539.1|cl|IM
      AGp998D125539|AI636570 ts91h06.x1 NCI_CGAP_GC6
      Homo sapiens cDNA clone IMAGE:2238683 3', mRNA
      sequence

<400> 79
tttttttttt tttttttagg ggaagagaag caaggcccct cagagacacc tttattcttg 60
ctctttctcc tttcaagaca tggttgctcc ctaggcagtc acatttctct ggtgatggag 120
agaaaggagg gcagttggag attgagggga cagcatggga tagccgagac atgctgatag 180
ctggagggga ggctgcctgg gacctgaaga agcagtgatg tcccagctcc agtcatctga 240
actcccctaa taggcctctc catctctcca gacattagat cttgtagaga aagaaactgc 300
tagaatccaa atcttttcac acagtgaggc ccccaaacag cccagccttc acacacaagc 360
cagcctgctt ctcctgactg acttgatcaa tacctcaa 398

<210> 80
<211> 615
<212> DNA
<213> unknown

<220>
<223> >gi|1123701|sr|IMAGp998C22571|cl|IMAGp998C22571|gb
      |H99033|H99033 yx11f11.s1 Homo sapiens cDNA clone
      261453 3'

<221> misc_feature
<222> 1, 397, 434, 452, 490, 563
<223> n=a, t, c or g

<400> 80
ntttttaaag ttggtctctg ctttttatta tcatgtgctg acaagtctaa acaatgtcaa 60
tgctaaaata tttcttcttg ggcagaccat tcctaccttc tcggtaccac accgtaccac 120
aagtagctta cccattcctc tagtgataga catttgggtt gtttccagca tgggtttatt 180
agaaagagca ctgtagccgg gcatggtggt tcatgcctgt taagtgagct gaggtaaaat 240
atattaatac atgaaatcta tcattttaac catttttatg tgtacaattc agtgcattaa 300
gtacattcac aatgttatgc aaccaccacc actctccatt tccagaacat cttcatcatc 360
ccagagaatc tgtttgcagc ctcactccct attccancct tcccctagcc ctggcaacct 420
ctaccttctt ggcnctgaaa ttggcctatt cnagatgcct catctaaatg ggatcaaatt 480
ggccctttgn ggctggctaa ttcattaaca ggtttcaagg tcatccacgt ggagctggca 540
cagacttatg cagcccaaac canccctacg gatcaatccc cggagaaggg gcaggccagc 600
agatccctgg caccg                                                   615

<210> 81
<211> 759
<212> DNA
<213> unknown

<220>
<223> >sr|IMAGp998M24260.1|cl|IMAGp998M24260|gb|BX097294
      |oid|234528960 BX097294 Soares breast 3NbHBst Homo
      sapiens cDNA clone IMAGp998M24260 ; IMAGE:161471,
      mRNA sequence

<400> 81
tgcacaggat cccaagtcat gctgtagagc cggattccta gacccagggc tctgcactct 60
caaggctggc cccatgtgct caagggggtc taatgtttgg gctccaaact aaccatctcg 120
gagctgggct cctcatttac tgccaaaccc tcagcttatg tagctagaaa gggccctgga 180
gtgagaaagc ctggattttc aaattgatgc tcccctactg actagctgtg ccactctggg 240
caaatgctct tccttgagcc tgtttccaca cctgtaaagt ggggatgatg atcctatctc 300
actgcttttg tgaggattac aggaaagcac ctgtcctggc tctgtacctg gcacgtagta 360
ggtgctcagt tcatgctggt ttccttcctg cctttagtag ggacctgctc tgtgctcaca 420
cctcggctgc atgcaccctg ctgtgacgga ggctagtgtg gaagaggtcc tgtcctcagg 480
gaattaactg tcttattggg agacaacaac tgtcctcctt ggaacaccca agaaaccatg 540
caaagcagtg gacaacacag aacacgccct cctcctcgct gcctgcagct ccaatctgat 600
tctgcttggg aatgggcgga gcacgtgggc tgcttaactg ctgtatagga caagcccctt 660
acccctctct gggcccatga attcctggct tggtttatgt tctgatttga cacactgatt 720
ttaatcttcg aatcatgaca ctgagtgcag aggaggtgg                         759

<210> 82
<211> 751
<212> DNA
<213> unknown

<220>

<223> >sr|IMAGp998N11579.1|cl|IMAGp998N11579|gb|BX118177
      |oid|234570730 BX118177 Soares melanocyte 2NbHM
      Homo sapiens cDNA clone IMAGp998N11579 ;
      IMAGE:264778, mRNA sequence

<221> misc_feature
<222> 702, 745
<223> n=a, t, c or g

<400> 82
gtttctttgg attttacaga ggtctgatgc ctactatttt aggaatggct ccatatgcag 60
gtgtttcatt ttttactttt ggtaccttga agagtgttgg gctttcccat gctcctaccc 120
ttcttggcag accttcatca gacaatccta atgtcttagt tttcaaaact catgtaaact 180
tactttgtgg tggtgttgct ggagcaatag cgcagacaat atcctaccca tttgatgtga 240
ctcgtcggcg aatgcaatta ggaactgttc tgccggaatt tgaaaagtgc cttaccatgc 300
gggatactat gaagtatgtc tatggacacc atggaattcg aaaaggactc tatcgtggtt 360
tatctcttaa ttacattcgc tgtattccct ctcaagcagt ggcttttaca acatacgaac 420
ttatgaagca gttttttcac ctcaactaaa aaaaaattat ggttggtttt tcttaataca 480
ttctcagagg gagaaatgaa acattactat aattgtgggg ggaacattac ttgaatgggg 540
atatttaccc tgtcacaaga gccactggta ttttagtact tgattatttt ttctttagtc 600
acaaatcaga actgcttacc atactttttg atgccaaaca ttatacctta gaacattgaa 660
gaaaatattc ctaagctgat gctggctaaa ccgctttaaa gntttatttg gaagtagaaa 720
ctagctttaa aacggggttc aagangttgc c                                751

<210> 83
<211> 401
<212> DNA
<213> unknown

<220>
<223> >ImageID=36807 IMAGP971H10113_S1 CC   FromEXP of
      IMAGp971H10113_s1.exp April 26, 2002 13:08

<400> 83
agagaaaaat atttttaatg tagaaaagat gaatagctgt aatgaaatac aaaccgttcc 60
tatacacagt aaacttacta agaactgcta atccttctcc aggttcttag aatctgggta 120
cagcattata gaaagtaacc acgctatgaa aacacacacc caaaacccga tgggtggcaa 180
ttgtgataga ttaggaagac aaaacagatt cagaagtagc agagaatact gcacattttc 240
ttggaaaatt tcttaattgt tcagtgcttt ctgacaagtt tccccagccc aatttatctc 300
ttagctattc tctgctactt agtcctgtct tctgacaatc atcatatggg gacgatcatc 360
tgccttacag tgtaacatgt ttttcctaac acagccgcat t                     401

<210> 84
<211> 406
<212> DNA
<213> unknown

<220>
<223> >gi|900602|sr|IMAGp998N06288s1|cl|IMAGp998N06288|g
      b|H29692|H29692 ym61d03.r1 Homo sapiens cDNA clone
      52960 5'

<400> 84
tcagtccttc cccaggtggg ccaacccaca gggctgcttg agtgtcttca caatatggcg 60
ctcggcttcc ccccagagca agagattcaa gggcccaggg taaaagccaa cgtgttattt 120
ttatccctag cctcagaatt cacacgccgt tgcctccacc atgctctggt ttgatacagc 180
ccagctctga ttggaagggg ctggggctgc ccgtgctgac tcttcaaagg catcccatcc 240
tgcagatggt gttcacaggg agagtttgtg ggggccggca ctccctcatc tactggggct 300
cattctggaa gaaggtccag aagaattggg agaccccttg ccccttcacc caaactttgg 360

gaggttggca gggtgaacag caggccaagt tcaggttcca agacag                406

<210> 85
<211> 646
<212> DNA
<213> unknown

<220>
<223> >sr|IMAGp998M18194.1|cl|IMAGp998M18194|gb|BX114372
      |oid|234563121 BX114372 Soares placenta Nb2HP Homo
      sapiens cDNA clone IMAGp998M18194 ; IMAGE:136121,
      mRNA sequence

<221> misc_feature
<222> 8, 14
<223> n=a, t, c or g

<400> 85
ctttattnag tatnatatat gtggtcggca agaaatatta ataataaatg ttgaaaacac 60
tattcccatt aagaaataag tggatcaaag tgttaccctt aatgatcata tagacactga 120
taaataatag tcaactttat tactgtggtt caaaccaagt ggaggttgta tgttttgtct 180
agaaaatata taaactacaa aaagttaata aacacctaag tgaatgagta aattaaatgt 240
ggggagtaag attcaattaa gataaagtcc taattttctt ttaatcaaat gctctgcttt 300
gtactcctta gaatggatgc actggagctg atttaaacat tctttggtgg tcacaagatg 360
tatagctgcc tctgtatgtt tggcgcatgg aaaaaaatat gagtttttgt ggacatacac 420
tctttatttg aaaagtatga aatataatcc aagaaagcca gccatggctc agtgataaat 480
atggaattga tatgaaacat tatattgatt aatcaccttt gaggatgatt aataaaagag 540
agaaagtgat tctatataga gatgcaggac tatttcaggc tcattcatga tctaggtttt 600
tgtgcttcat ggttatttta ttatcatcaa taaaaaaaaa aaaaaa                646

<210> 86
<211> 437
<212> DNA
<213> unknown

<220>
<223> >sr|IMAGp998J155722.2|cl|IMAGp998J155722|gb|BX1121
      14|oid|234558605 BX112114 NCI_CGAP_GC6 Homo
      sapiens cDNA clone IMAGp998J155722 ;
      IMAGE:2309102, mRNA sequence

<400> 86
agtaacttca ttggcttcta ctcagtagat gccaatgttg accctcctcc cagtgtgacg 60
atcaaacatt tcttcagact ttggcaaatt tcatctgtgg ggggcaacat tgtccccagt 120
tgagaaccac tgaggtacaa catggactga agaggagcat gtgttaaaaa cgtgtgaatt 180
atttatcctg tagtatctta ctgatgttca gataaagaaa aagcaaaaca aaagacctaa 240
aactaggctc ttcccccacg gggtcaggtt catgtgtata gttgcattta attgtattac 300
gattctgggt tgttgatggg ctgatcatac tgtgttttct ccgaaaaaac aaagaattgt 360
tcatgagtac ttactaaatg aataagtgaa atgacaaatg aataaagaaa cacataaagg 420
agaaaaaaaa aaaaaaa                                                437

<210> 87
<211> 236
<212> DNA
<213> unknown

<220>
<223> >sr|IMAGp998B21790.1|cl|IMAGp998B21790|gb|BX114745
      |oid|234563867 BX114745 Soares_fetal_heart_NbHH19W

Homo sapiens cDNA clone IMAGp998B21790 ;
      IMAGE:345524, mRNA sequence

<221> misc_feature
<222> 232, 235
<223> n=a, t, c or g

<400> 87
agaagtatct gcacaattag aaaagtcctc agtaagcttt ttcttggagg gtacactttc 60
ttcactgtcc ctattcctag acctggggct tgagctgagg atgggacgat gtgcccaggg 120
agggacccac cagagcacaa gagaaggtgg ctacctgggg gtgtcccagg gactctgtca 180
gtgccttcag cccaccagca ggagcttgga gtttggggag tggggatgag tncgnc     236

<210> 88
<211> 506
<212> DNA
<213> unknown

<220>
<223> >sr|IMAGp998J09792.1|cl|IMAGp998J09792|gb|BX088778
      |oid|234511653 BX088778 Soares_fetal_heart_NbHH19W
      Homo sapiens cDNA clone IMAGp998J09792 ;
      IMAGE:346472, mRNA sequence

<400> 88
acccacaggg ctgcttgagt gtcttcacaa tatggcgctc ggcttccccc cagagcaaga 60
gattcaaggg cccagggtaa aagccaacgt gttattttta tccctagcct cagaattcac 120
acgccgttgc ctccaccatg ctctggtttg atacagccca gctctgattg gaaggggctg 180
gggctgcccg tgctgactct tcaaaggcat cccatcctgc agatggtgtt cacagggaga 240
gtttgtgggg gccggcactc cctcatctac tggggctcat tctggaagaa ggtccagaag 300
aattggagac ccctgcccct cacccaaact ttggaggtgg cagggtgaac agcaggccaa 360
gttcaggtcc caagacaggc caaggccagt gcggtttccc ttccactgcc tcagtttacc 420
tgtattcaga agacagtcta ggaagagttg agcagagttc cctctaaaag agtagggagc 480
tgataacagt ccccaagccc tcctct                                      506

<210> 89
<211> 371
<212> DNA
<213> unknown

<220>
<223> >sr|IMAGp998H175550.2|cl|IMAGp998H175550|gb|BX1148
      20|oid|234564017 BX114820 NCI_CGAP_GC6 Homo
      sapiens cDNA clone IMAGp998H175550 ;
      IMAGE:2243008, mRNA sequence

<400> 89
acagccaggt gggaaggact ccttggcaaa actccaacca gcctgtacac tgggaggaat 60
gtgcactggg atggagccat agaagtttgt gtcgtttgca gtggggagga gcctggtccc 120
tcctcttcct gtgaggaacc tggaattcaa tctgtgaggt tgttctggag atgttctggg 180
gagactgcat taaacacagc ttcgcaccat tgaataaact cagcaacaag ccaatgcata 240
aaagtaatct atgcttcagg tcacagaagc ttcaagggga aaaaaacaga atactctagg 300
gccattgttc acaaactcat ctgaaaacat cctggaaaaa ttttcccaaa cacatgaaaa 360
aaaaaaaaaa a                                                      371

<210> 90
<211> 784
<212> DNA
<213> unknown

<220>
<223> >sr|IMAGp998E24312.1|cl|IMAGp998E24312|gb|BX099422
      |oid|234533220 BX099422 Soares adult brain
      N2b5HB55Y Homo sapiens cDNA clone IMAGp998E24312 ;
      IMAGE:172415, mRNA sequence

<400> 90
cttctggagg ggtgtgggac tagagctggg tgtcacgtga acccttcctg gtagggtgac 60
ccccttcccc tggagggggt tttagagctg ccgcctctgc tccctctaac ctctttggag 120
gcagggttgg gggtattgtc attcaaggcc ttttttttgt ctgctccctc cccgaccctg 180
tgccctcttc tggaggtttc tcgtctggga gagtccctcc cagcagtccc tccacctcca 240
taaggacaca ctggacaaaa ctcccgcagc tcttcaggaa tgaccgatgc ctacctgtgg 300
ggttcagttg cccatagttt gaggccttct ctcctccctt accaccgctc tggatcatgt 360
tactagttcc gtcttttgtg tggccttggg ccagcttcct tgataccttg aagatgggct 420
tcttgtgagt ccccagggag aaagggacaa gagctaagat ttttgcatca gcccttctgg 480
cagaaggtgt ggtaggggcc atttgttttt tttagtggac ttgggatttg tggtgtaatc 540
atatcattaa tgatccaggg tgtgggaaaa atggaggtcc ttgaagtggc tgaatctcat 600
tgtatttaag acactgtcag ttgccagatg taggcttatt tttggagatg tctaggagag 660
gaaaaagcta ccaatcatac tcttgatatc cgtctggctg tgtgaggcac ccctacctca 720
tggggggtgt cttgggattg atgaactgtg gaacctgcct cctgcgctcc ccaaagctta 780
ttaa                                                              784

<210> 91
<211> 579
<212> DNA
<213> unknown

<220>
<223> >sr|IMAGp998J081153.1|cl|IMAGp998J081153|gb|BX1000
      23|oid|234534422 BX100023
      Soares_pregnant_uterus_NbHPU Homo sapiens cDNA
      clone IMAGp998J081153 ; IMAGE:485095, mRNA
      sequence

<221> misc_feature
<222> 19
<223> n=a, t, c or g

<400> 91
ttgctacagg tccctgagna tgcaatcaga ttaagcgtag caagcattgc caatgggaaa 60
gtcaaaataa tttatttttt ttccctttcc ccctacccca tccccagcca agaatttctt 120
ttcaagatat cgtcatcatt cttaaacaac attcttaacc cccagctggg gtccccattt 180
taatagatgt cattgcttca agtctaacgg cgccgggagg cctgtttgag ggaaaacatt 240
agtttgaaaa atccccgttc ccttcatcca ctgcccttgt tctccacgtg ggagtgtgct 300
tgtggcccct cagaaagata gtctgctggc tcctaggggt tggggtgggg gacacacctt 360
tttctcagga agaggtgatg gcaatgtaaa acatctaagc aaagttttaa atgaaaaaaa 420
ggaaacacat ttaaacatcc tgataatgga gggaaggggg gcacatttac acatagccca 480
gaacttgtag aattctgcat agtgaatgta tattgaatta gtctcctgcc ttatacattc 540
aggaggaata aatttccata atgtaaaaaa aaaaaaaaa                        579

<210> 92
<211> 628
<212> DNA
<213> unknown

<220>
<223> >sr|IMAGp998G18196.1|cl|IMAGp998G18196|gb|BX091497
      |oid|234517361 BX091497 Soares placenta Nb2HP Homo

sapiens cDNA clone IMAGp998G18196 ; IMAGE:136745,
      mRNA sequence

<400> 92
tatgttgaag tctttcagtg ttcagctgag gagatgaact ttgtgttaat ggggggcact 60
ttaaatcgaa atggcttatc cccaccgcca tgtaagttac catgtaagtt tttcttgggt 120
cttggcgcta ttctacgcta tatgctggta ggtgcttaag ctgctttcgt aactttctgt 180
gcccctggtt ctttctgagg caggtgaggt ggttatataa ggctctccca tctgtaatca 240
gtagtacctg gtaatcattt aagaccttgg ttgtggacac ccataattag agatgctaca 300
aatacctcct ttcaaattat attcctgctt ttccagtgat aagcacttga tttctgaagc 360
ttagtgtgct ccagataagc actgactctg ggaggcaacc tggctatata gataatttta 420
gtacgcattt aaaggcaaaa taaaagtatg gctggattct ttttatttta ttttttaaaa 480
cgtattaacc tttaagaagg gcagtttgat gcacatgttg catttaactt tttgagcatc 540
aagtattttt ttaacatttt tgagcatttc tgttgctctc caggatatct ggaatgagca 600
agtgagcaaa aacaaaaaaa aaaaaaaa 628

<210> 93
<211> 260
<212> DNA
<213> unknown

<220>
<223> >gi|2106587|gb|AA426115|sr|IMAGp998F241862.1|cl|IM
      AGp998F241862|AA426115 zv52d12.s1
      Soares_testis_NHT Homo sapiens cDNA clone
      IMAGE:757271 3', mRNA sequence

<400> 93
tttttttcca actggttaga gaggcaagtg atttattctc cggctcatgt ttctatatgg 60
aggcattttc caagactaac aatcctggct tctctctttt ggctttcctt gtcctccgtg 120
gggcaaagtt atctggaaag tctggaaggg accaggcaca cgcagctcag cctttgctgc 180
tctcccctct cttcacactt gtgttatctg gagggagatg gaggcaggtg tccgtagccg 240
ggtcctgagc ccctcgtccc 260

<210> 94
<211> 723
<212> DNA
<213> unknown

<220>
<223> >sr|IMAGp998B09229.1|cl|IMAGp998B09229|gb|BX112820
      |oid|234560017 BX112820 Soares placenta Nb2HP Homo
      sapiens cDNA clone IMAGp998B09229 ; IMAGE:149288,
      mRNA sequence

<400> 94
tatttcttgt acttggcctg ctccttttct tcgaggattt gttaattatc taaaagtcag 60
aaacatgtct gaaagtatgg cagctgctca tagaacaagg tatttcttct tattgtagag 120
actgcatcaa cccgacattc ctttcttata ccaatgtgaa atttccagat catctgtaaa 180
cctacaactt taatagaaga ctactaataa cagaagacaa attagtgaag aaaagactga 240
gtttcgaaat tgaatggcag ggtggttttt gcttacaagc catttctgtt cattctttaa 300
gtatctatat ttcatttgtt ttgcacatat gcatatgtgc ccatttaaga tatttgcata 360
tacttgatag aaaccataaa gttgtagcag ttaagtccag tcacatttgg ttaatcagtg 420
tttgatataa ttgaaagagt tgagtggata aacagtcttc cagcttgtaa atgccattga 480
cttctgacct gacatttagt ataataaaaa tgaaattctt aaccatgtca aatgatttag 540
tttctggctc ttagactcat ctggcagttc tacacatgaa acatcttttg ttatataagg 600
tgtattgaaa cctgcagtgc tgattattag aaaggatttg tcagattttt gaacatgata 660
tttacattat tatttaggaa aactcttcct gtaaataacc atgcataact tactttctgc 720
aat 723

<210> 95
<211> 450
<212> DNA
<213> unknown

<220>
<223> >gi|757038|sr|IMAGp998E11127|cl|IMAGp998E11127|gb|
      R06418|R06418 yf09a06.s1 Homo sapiens cDNA clone
      126322 3' similar to SP:I1BC_HUMAN P29466
      INTERLEUKIN-1 BETA CONVERTASE PRECURSOR

<221> misc_feature
<222> 7, 50, 61, 78, 156, 182, 189, 196, 249, 312, 316, 348, 378,
       415, 423, 436
<223> n=a, t, c or g

<400> 95
tttgttngtc ttcactttta ttgaaataca aaatgttaaa tatgcaagcn gtactaatga 60
nggtgctcct tgaagttnat taaggagggc tgggctgctt gtggcttcca ttttcaattg 120
ccaggaaaga ggtagaaata tcttgtcatg gacagncgtt ctatggtggg catttgagct 180
tnggccctnc gagttncaaa tgattgctgt accttccgaa atacttcctc taggtggcag 240
caccaagant atttctggga agcatgtgat gagtcgtgtg atgatgatag agcccattgt 300
gctgtctctc cngggncacg tgtgtgtggg cgttgaagga ggcagtanag ccaatgaagt 360
cccttctcca cgtggggnct ttgttaaaca gcatcttccc ctagggtgct cagancactt 420
ttnaagacgc cacttnccag ggtgctggga                                  450

<210> 96
<211> 725
<212> DNA
<213> unknown

<220>
<223> >sr|IMAGp998N02421.1|cl|IMAGp998N02421|gb|BX110042
      |oid|234554463 BX110042 Soares fetal liver spleen
      1NFLS Homo sapiens cDNA clone IMAGp998N02421 ;
      IMAGE:214465, mRNA sequence

<400> 96
tttgagtgga acaaagtgac ctcttgcatc cataacatct taagcgggca gaggtggatt 60
gagcactatg gagagattgt catcaagaac ctgcatgatg attcctgcta ctgcaaagtg 120
aattttataa aggcaaaata ctggagcact aatgcccatg agattgaagg cacagtgttt 180
gacaggagtg gaaaagcggt tcatcggctg tttgggaaat ggcatgaaag catctactgt 240
ggcggcggct cctcttctgc ctgtgtatgg agagcaaatc ctatgccgaa aggctacgag 300
caatactata gcttcacaca gtttgcgctg gaattaaatg aaatggatcc atcatcaaag 360
tctttattgc cacctactga cactcgattt aggccagacc agaggtttct agaggaaggg 420
aacttagaag aagctgaaat acaaaagcag aggattgaac aactgcagag agaaaggcgg 480
cgggtcttag aagaaaatca tgtggagcac cagcctcggt ttttcaggaa atccgacgat 540
gactcttggg tgagcaacgg cacctatttg gaacttagaa aagatcttgg tttttccaaa 600
ctggaccatc ctgtcttatg gtgaaaaagt aaagaagaaa gataacatta gtgtatttct 660
cctgtgcttg ccttctgaag tggcacaaac ctgtgtttat atatttaaaa gatactctag 720
gatga                                                             725

<210> 97
<211> 335
<212> DNA
<213> unknown

<220>

<223> >gi|5637283|gb|AI917428|sr|IMAGp998B145536.1|cl|IM
      AGp998B145536|AI917428 ts79d07.x1 NCI_CGAP_GC6
      Homo sapiens cDNA clone IMAGE:2237485 3' similar
      to contains element MER22 repetitive element ;,
      mRNA sequence

<400> 97
   tttttttttt tttttttttt tttttttttt tttttttttt tttttttttt tttttttttt 60
   tttttttttt ttaaaaaggg gccccttaaa ttttccttgg gaaaaaactt ttcaaaaatc 120
   cctttatttt tcccaattcc tttgggggga aaccgggggg ggggggggaa aaaaccaaaa 180
   accaattttt ttaaaccccc ggccccccaa aaaagccttt tggttcaaaa ccccaaaaaa 240
   aagtttttgg tctttttggg gccccccccg gggggggggg ggaacccaac cggccacccc 300
   gggggaaaat ttaaaaaacc cccccccggg gggaa 335

<210> 98
<211> 464
<212> DNA
<213> unknown

<220>
<223> >gi|3179587|gb|AA993042|sr|IMAGp998C144120.1|cl|IM
      AGp998C144120|AA993042 ot92f07.s1
      Soares_total_fetus_Nb2HF8_9w Homo sapiens cDNA
      clone IMAGE:1624261 3', mRNA sequence

<400> 98
tttcctttat tttattcact gacatctctc agacctagac cagtgcctgg catatcctag 60
gcatacatta tattgaatgc atgaatttaa acatttaata attttaattt catagttgtc 120
ttttaatatt tgagattttt accagatctt gcacattctg ttgctactag aaaagcccct 180
tttctatggg cttgaaggat agaaaaatct tagcctgact tcactaacca gtataaatcc 240
agtggcccca ggaaggctgg gaacatccta tgtgaccatc cttgattttg gaccctaaaa 300
cgaaagctca acttcacact catctggaaa catctaatat cttttcaaac acatgtattt 360
tgttccccca actagacatg aaacacattg acagaaattg cacaaagtat tcagcacagt 420
cctgaatact tcataaagac ataaaacctg gcttttgatg gact 464

<210> 99
<211> 232
<212> DNA
<213> unknown

<220>
<223> >gi|2583451|gb|AA651799|sr|IMAGp998M232973.1|cl|IM
      AGp998M232973|AA651799 ns19a12.s1 NCI_CGAP_GCB1
      Homo sapiens cDNA clone IMAGE:1184062 3' similar
      to contains Alu repetitive element;contains
      LTR5.b3 LTR5 repetitive element ;, mRNA sequence.

<400> 99
ctttgcacaa atttaggatc aattttgtta ggaaagcaag cattgaagtg taggccgatg 60
cgggaggatc acttggggtc aggaattcaa gatcagtgtg ggaaacatac caagagccca 120
tctatacaaa aaacattttt aattagctgg gcatggtggt gttcacctgt agacccaatt 180
acttggaaag ctgagacagg aggattactt gaaccatgat tgcaccactg ca 232

<210> 100
<211> 680
<212> DNA
<213> unknown

<220>

<223> >sr|IMAGp998D051943.3|cl|IMAGp998D051943|gb|BX1034
      10|oid|234541200 BX103410
      Soares_total_fetus_Nb2HF8_9w Homo sapiens cDNA
      clone IMAGp998D051943 ; IMAGE:788308, mRNA
      sequence

<221> misc_feature
<222> 38, 114, 638
<223> n=a, t, c or g

<400> 100
ataaggatgg atgaatttag acagataatt caaaggtnca tgttaaaaga actggatact 60
ttttgtcact ggaacgaact tgatcagttc atcaataagt ttttcctaaa cggngtcttt 120
atacatgatc agaagctctt cactgacttt gttaatgatg ttaaagatta tcttagaaac 180
atgaaggaat atgaagtaga taatgatgga gtatttgaga agttggatga atatatatat 240
agacacttct ttggtcacac tttttcccct ccatatggac ccaggtcggt ttacataaaa 300
ccgtgtcatt acagtagttt gtaacatttg tagattggat agcattttta tgatttgatg 360
agtttcttgt aaggttaccg tttctaagag ttgtgcttta tggccactga gagaattcag 420
aataaattga aagatggagt ctaaaaatta ttagctgtta caaatggaac atttcattat 480
aacgtgatca ctttgacttg agcaaatggt ttaattttta tcttaaaaat cagttaagaa 540
tatataaaat cctactttgg ccaagtttgt ttcttttcat tatagtttat atgaaaagat 600
caccttaagt gaaattattt tcctttaatc ttttatgnat ttattcactt ttggaagcta 660
ggaatgagca acacaaattt                                             680

<210> 101
<211> 402
<212> DNA
<213> unknown

<220>
<223> >gi|2538624|gb|AA626237|sr|IMAGp998M031831.1|cl|IM
      AGp998M031831|AA626237 zu93a02.s1
      Soares_testis_NHT Homo sapiens cDNA clone
      IMAGE:745514 3' similar to SW:POL_MLVMO P03355 POL
      POLYPROTEIN ;, mRNA sequence.

<400> 101
tttaaagtct tctcctggtt agcctcctag aggaggggct ctttctttcc ttctctttgt 60
ggcttagcca taagctagaa atttgggatc taaatgcagc agaaccttgc tgcccttagg 120
aactctctta tttgacacca ggtggttgga gtaggaagtg cacaaacaga ttgcttttgt 180
ttactactaa gccatctttc ctcttggctt atataaaggc ttaaatactg gagactttta 240
gagcagattt gagctttttt ctgacacttt atgtcctgcc tttcacagca ggtgcaggga 300
gtcttgggtt tctcctgggt gcagtagccc ttggctggag ggtttttttt tataccatgg 360
ccttgaccat tctccttatt ttatttattt attttttggg gc                    402

<210> 102
<211> 593
<212> DNA
<213> unknown

<220>
<223> >sr|IMAGp998K243513.1|cl|IMAGp998K243513|gb|BX0890
      19|oid|234512315 BX089019 Soares_testis_NHT Homo
      sapiens cDNA clone IMAGp998K243513 ;
      IMAGE:1391375, mRNA sequence

<400> 102
agaggatgtt aaagtctctc tttgataaac ttaaattatg aaatagccca gttgttcagg 60
gtcacttcta ttgactcaat ttgtgtgaga ctttgcactc actatctcgc cggccggact 120

gcatgcccag ggtcagtagt cagcagagct aaaggtgccc gaatttagca cagctggctg 180
tggtgcctga catagggcag taccggggga ttcttcagag gtcagccaag tcggtaacat 240
tctgtgaagc agattaaata cagtaaaatc aaacaaacgg aggtctttgg aagtgatgct 300
gggcccaggc agtgaaaatc aagaaaagag gagaaagacc aagacctgga tgtcctggac 360
gacctgaccc aaaaaggggc acaaagcagc agagcttcac caggtgactg ggtggcttct 420
ttgtcctcat taaatgtaca tctttgcatg aaaaggacct tgactggtct ggactttaac 480
aaagtaaaat atgaagaaac gtaaaattat gcttctgaaa aatatactga aataaaattt 540
tgtaaatagc taaaagaaaa ataaataaat ataataacaa aaaaaaaaaa aaa 593

<210> 103
<211> 671
<212> DNA
<213> unknown

<220>
<223> >gi|4511234|gb|AI560893|sr|IMAGp998B165468.1|cl|IM
      AGp998B165468|AI560893 tq41d08.x1 NCI_CGAP_Ut1
      Homo sapiens cDNA clone IMAGE:2211375 3' similar
      to gb:M20560 ANNEXIN III (HUMAN);, mRNA sequence.

<221> misc_feature
<222> 634
<223> n=a, t, c or g

<400> 103
ttaacaaaag caacaatttt tattatcttg ctttatattt aatggattag aactataaag 60
attcttaact ttgaaagcag aaatataagt tggatagtag ttgcagatct ttaataccat 120
tttcaatttc atttatgagc tgctacatta taaatgagat gctctaaaat aataatcgct 180
tttgttgttg ttgttataga acaatgaaaa ttcctgttag gaacacaagt tgctgtttat 240
atttgcttgt tctcttaaat agtatgagaa gaagtaaggt ggagctgttg gaaaagccca 300
tcgtggacct ttggagatta tcttcttggt tcagtcatct ccaccacaga tttttaagag 360
tgtgatttca tagtctccag aagtatccga tttaattgct gaatataggg aatagccata 420
atgcttcttg aactctgttc gaatgtccaa aaggtcaatt tctgatctgg acaccattat 480
tcggttcaga gtaaactcat cagttccaat acccttcaag gctcgatgca gtctttcggc 540
taaaaaaggc cggcgtgttc tcacacaatt aactatggcc cacagtagtc ttcaaaatgc 600
cagatatttc tctttatgct ggccacatgt cctnttggct gatattctga ttcatcaaat 660
gtagttttaa t 671

<210> 104
<211> 467
<212> DNA
<213> unknown

<220>
<223> >sr|IMAGp998G024171.2|cl|IMAGp998G024171|gb|BX0897
      65|oid|234513883 BX089765 Soares_testis_NHT Homo
      sapiens cDNA clone IMAGp998G024171 ;
      IMAGE:1643929, mRNA sequence

<400> 104
tatgccaggt actttgcttt ctttgtatta actcttattc ctcacaacaa ttctgaagta 60
ttactgtaac taccaacatg ttatagatga ggaaacaaag agaagttaag caacttgctc 120
cagtgacgca gctggtaagc agcagagctg ggattaaaac ccaggcattc tgattccacc 180
acctacacac ttagccattc cgccccactg agaatgaaaa tgtcaaaaaa tactaggcca 240
gtaagtgtta gcaaggcaag aaatttccac accaaaggtg agtatgttgt ttggaaagat 300
tgggatggaa atgagtaaag tatgtttctt tttaaaagtc ttttagtaag atttaaagct 360
ctatatgtaa gaagcagatg aaaataccaa gtgaattttt agatttgatg ttatgtaagg 420
ttttgttttg ttaattaaaa tttggtcatt ttaaaaaaaa aaaaaaa 467

<210> 105
<211> 510
<212> DNA
<213> unknown

<220>
<223> >sr|IMAGp998K15174.1|cl|IMAGp998K15174|gb|BX094991
      |oid|234524351 BX094991 Soares infant brain 1NIB
      Homo sapiens cDNA clone IMAGp998K15174 ;
      IMAGE:41539, mRNA sequence.

<400> 105
tttttttttt ttttatctct attagtgggt ttattgacat aactggacat aacaatgtat 60
aacatttatt catctcccac tgaaagactt cccagtgaaa ctaaagtaga acacagatcc 120
aggcattagg cattttcagt gatgttctat ggctgaaaag gtgggtggtg aggagaacca 180
gaggccttgt ccatacttgc aatgggactc aatattaccc tgctgggcaa aagaaatagc 240
taggagatgc aatagtgcta tgattgaaaa aagccaggag cacaaaaggt gccagatagc 300
tccctggttg aaaataagag caaacaacac tactgctctc atgtaccagt atgctctcaa 360
tctaaaataa gccctggcag caaactaaga atgacagtct atttcctgtg gtttattaag 420
ttttggcccc gcagacgaga ctgccagaaa agcagctgta gttttaagtg gtctggacat 480
ttactcattt gaacataaac aacgatcagc                                  510

<210> 106
<211> 268
<212> DNA
<213> unknown

<220>
<223> >sr|IMAGp998I04624.1|cl|IMAGp998I04624|gb|BX116135
      |oid|234566646 BX116135
      Soares_multiple_sclerosis_2NbHMSP Homo sapiens
      cDNA clone IMAGp998I04624 ; IMAGE:281931, mRNA
      sequence

<400> 106
tctgcagttc tcagcctggt cctttcttct gtcgagatca ggtctgcttc aaatacttct 60
gccggagctg ctggcactgg cggcacagca tggagggcct gcgccaccac agccccctga 120
tgcggaacca gaagaaccga gattccagct agaggagctg gccttgccca gtggcctgtg 180
gcgcccaaag ctggcaggtc aggcaagcag cctgtaccac cctgccactg gcgaccaggg 240
agctggcttc ccaaggacaa gggaaaat                                    268

<210> 107
<211> 828
<212> DNA
<213> unknown

<220>
<223> >sr|IMAGp998M24235.1|cl|IMAGp998M24235|gb|BX116782
      |oid|234567940 BX116782 Soares placenta Nb2HP Homo
      sapiens cDNA clone IMAGp998M24235 ; IMAGE:151871,
      mRNA sequence

<221> misc_feature
<222> 720
<223> n=a, t, c, or g

<400> 107
ttacccaggg tttttggagt ctctggatga tttttacatt cttagcagtg gattgatatt 60
gctgcagacc acaaacagtg tgtttaataa aaccctgcta aagcaggtaa tacccgagac 120

tctcctgtcc tggcaaagag tccgtgtggc caatatgatg gcagatagtg gcaagaggtg 180
ggcagacatc ttttcaaaat acaactctgg cacctataac aatcaataca tggttctgga 240
cctgaagaaa gtaaagctga accacagtct tgacaaaggc actctgtaca ttgtggagca 300
aattcctaca tatgtagaat attctgaaca aactgatgtt ctacggaaag gatattggcc 360
ctcctacaat gttcctttcc atgaaaaaat ctacaactgg agtggctatc cactgttagt 420
tcagaagctg ggcttggact actcttatga tttagctcca cgagccaaaa ttttccggcg 480
tgaccaaggg aaagtgactg atacggcatc catgaaatat atcatgcgat acaacaatta 540
taagaaggat ccttacagta gaggtgaccc ctgtaatacc atctgctgcc gtgaggacct 600
gaactcacct aacccaagtc ctggaggttg ttatgacaca aaggtggcag atatctacct 660
agcatctcag tacacatcct atgccataag tggtcccaca gtacaaggtg gcctccctgt 720
ntttcgctgg gaccgtttca acaaaactct acatcagggc atggcagagg tctacaactt 780
tgattttatt accatgaaac caattttgaa acttgatata aaatgaag 828

<210> 108
<211> 565
<212> DNA
<213> unknown

<220>
<223> >sr|IMAGp998F16280.1|cl|IMAGp998F16280|gb|BX102345
      |oid|234539070 BX102345 Soares infant brain 1NIB
      Homo sapiens cDNA clone IMAGp998F16280 ;
      IMAGE:49499, mRNA sequence

<400> 108
tttttttttt ttttacaaat aagttttacc ttttattgta ttccattata caattttaca 60
tagtaccatt attttaagca ataattaagc aaattgaact actgtttttg agctctactt 120
ttcagcatat agatttacct aaaaaagtac aaaccaagca cagtggtgca attttccagg 180
cactttcaag ttgcttattt acaaatatgt aagcaccctg ttggaacaca tgaaaatttt 240
ggtgtcgaga agaaacactc catgtatatc aaatctttaa attttaacat ttgcacagct 300
ccaaatattc ctgagaaagg tcagtctcat tgtcacgtac atctgcattt cctcacactt 360
cagcgtatct tggggaacca taacctcggt cacttgatgt accactaatt gcacaaaata 420
catgtaatgt gttgctcatc cagggagaat gcattgtgtc ttcttgtaat atcgacttaa 480
gtcttcaaag ctgctcatgg ataatgtcag ataattcttt taccatgtct ctgaaatttg 540
actttagccc ttcatggtac tcaac 565

<210> 109
<211> 453
<212> DNA
<213> unknown

<220>
<223> >gi|1290893|sr|IMAGp998B23677s1|cl|IMAGp998B23677|
      gb|W16493|W16493 zb15c12.r1 Soares fetal lung
      NbHL19W Homo sapiens cDNA clone 302134 5'

<221> misc_feature
<222> 304, 390, 412, 417, 450
<223> n=a, t, c, or g

<400> 109
gagaaactgt gtgtgagggg aagaggcctg tttcgctgtc gggtctctag ttcttgcacg 60
ctctttaaga gtctgcactg gaggaactcc tgccattacc agctcccttc ttgcagaagg 120
gagggggaaa catacattta ttcatgccag tctgttgcat gcaggctttt tggcttccta 180
ccttgcaaca aaataattgc accaactcct tagtgccgat tccgcccaca gagagtcctg 240
gagccacagt cttttttgct ttgcattgta ggagagggac taagtgctag agactatgtc 300
gctntcctga gctaccgaga gcgctcgtga actggaatca actgcttcag ggaaaaagaa 360
aaaaaaaaaa aaaaagactt gcctgggagn cgcgagaaac ttgcattgga anttcanaac 420
cagcattcga gaaactcctc tctactttan cac 453

<210> 110
<211> 490
<212> DNA
<213> unknown

<220>
<223> >sr|IMAGp998N204778.2|cl|IMAGp998N204778|gb|BX0924
      50|oid|234519268 BX092450 NCI_CGAP_GC4 Homo
      sapiens cDNA clone IMAGp998N204778 ;
      IMAGE:1946707, mRNA sequence

<400> 110
cctttggcct tttataaaaa ggtatttata attcgttgat aaaaaccaat atttgaagct 60
gttgattacc aacaaaaaag tttcacactc tttatagtgc ttcaggatac aactttttca 120
gggccttatt agagaaaaac tgattcacat gttattcttc taaagctaaa ttattaaaat 180
gtaggttgaa agattggatt gattgtgaat ctacactaaa gatatggttc caggcagacc 240
tccttagaga cctccaggca gacctcctta ctgtcttcag tgatgtttaa tttcattact 300
gttactgggt gccaagtgtc tttcatttgg aagtgaactt actccagtta ttgaatgttt 360
atcatatcat aatttgcatt tttccaacat tgaaggtatt ttaaaaagtc actaagagat 420
tcattctttt attattcaac tacaaaaaaa tagtgtaatg atcagaattt aaaatgtgtg 480
attctttttc                                                         490

<210> 111
<211> 493
<212> DNA
<213> unknown

<220>
<223> >ImageID=1309431 IMAGP971F12104_S1 CC   FromEXP of
      IMAGp971F12104_s1.exp April 26, 2002 12:57

<400> 111
agtttttttt tagaatttag agcattatga gactattgta gaaattttaa ctaccaggac 60
ctacaggatg gagagtgggg gaggggagaa gaccagaaga gaggcaggaa aacagatgcc 120
tttgctgatg tcctttgtag tataaaactt aacagcacca cagcacctaa tcattttctc 180
aggtgtggct ttgagaaata aacgttttat gttaaaaatg aaaatacttc acaatatgag 240
acagctggca tttgaatact tttcccagta tgtttgtaaa cattttggat tcatatttca 300
tattaaaatg gacatttagt aggattatta gatgaaaagg gtcccccagg ggatatctaa 360
tatcaaatta tctgcaggac atcttctcca gaaagcagac tgactgtaat atgaactaat 420
tttcccccct ttgagttgct tgcaagaatc tctgagatct gacactgatg ggctacagta 480
atcagcagtt ctt                                                     493

<210> 112
<211> 496
<212> DNA
<213> unknown

<220>
<223> >sr|IMAGp998I144113.2|cl|IMAGp998I144113|gb|BX0934
      51|oid|234521269 BX093451 Soares_testis_NHT Homo
      sapiens cDNA clone IMAGp998I144113 ;
      IMAGE:1621717, mRNA sequence

<221> misc_feature
<222> 22
<223> n=a, t, c, or g

<400> 112

cggctacata gctgtgacta cngggggtgc agtctctggg gaccaccact accgaggctg 60
ggtaccatga ttatcgcgga acacctgccc attaatcttg accctgttaa aacaataact 120
gcctatccag atatgccaca cttctgcctg ctgttggcaa ccctcctgga ctaggctgct 180
cttgttaatc acatggatgt tgctgattac tctgagtgca aaccttttca ctgttccaga 240
gaggagcctg acaaccacat tctccttctc aagatgtgaa aggcattgta cagaagaaga 300
tgagaatcac agtaaaccaa acaaaggaaa tgcagaagag atattttcag ctgaatattg 360
gaaagtaaga ttccctgcga gcaggatctc aggcaacgtc aatgaacaac atgaagcctg 420
acacattgta ggtgccccgt aaatattggt tgagtaactg aataaacgta tttagtaact 480
taaaaaaaaa aaaaaa 496

<210> 113
<211> 496
<212> DNA
<213> unknown

<220>
<223> >sr|IMAGp998I144113.2|cl|IMAGp998I144113|gb|BX0934
      51|oid|234521269 BX093451 Soares_testis_NHT Homo
      sapiens cDNA clone IMAGp998I144113 ;
      IMAGE:1621717, mRNA sequence

<221> misc_feature
<222> 22
<223> n=a, t, c, or g

<400> 113
cggctacata gctgtgacta cngggggtgc agtctctggg gaccaccact accgaggctg 60
ggtaccatga ttatcgcgga acacctgccc attaatcttg accctgttaa aacaataact 120
gcctatccag atatgccaca cttctgcctg ctgttggcaa ccctcctgga ctaggctgct 180
cttgttaatc acatggatgt tgctgattac tctgagtgca aaccttttca ctgttccaga 240
gaggagcctg acaaccacat tctccttctc aagatgtgaa aggcattgta cagaagaaga 300
tgagaatcac agtaaaccaa acaaaggaaa tgcagaagag atattttcag ctgaatattg 360
gaaagtaaga ttccctgcga gcaggatctc aggcaacgtc aatgaacaac atgaagcctg 420
acacattgta ggtgccccgt aaatattggt tgagtaactg aataaacgta tttagtaact 480
taaaaaaaaa aaaaaa 496

<210> 114
<211> 392
<212> DNA
<213> unknown

<220>
<223> >sr|IMAGp998N045486.2|cl|IMAGp998N045486|gb|BX0998
      82|oid|234534140 BX099882 NCI_CGAP_Ov23 Homo
      sapiens cDNA clone IMAGp998N045486 ;
      IMAGE:2218563, mRNA sequence

<400> 114
tttttttttt ttttatagct taaattttca actccaaaat tttatgattt tattattcta 60
gttagccaaa tatttttcag agtccacgga tgtctctgtg gacattccag aaaactaaga 120
gtctattaaa ctccaaatct tgatctaacc attttacact gtcaaattat ttattcctta 180
cagtttttga taataaaatg gtcgaggatt ttgagaacat gtgggcagca gattcctgcc 240
tctaattggg attcgtgatg ccagggcagg aaaatatcac ttcccagtcc ctgccagtga 300
aaactcaaaa aaagctgttc agctcatttc atcatgctgt cactaccctc tttctaaaaa 360
ctgcaaaact cttttcttgg aaatcattat cc 392

<210> 115
<211> 615
<212> DNA

<213> unknown

<220>
<223> >sr|IMAGp998I16328.1|cl|IMAGp998I16328|gb|BX104592
      |oid|234543564 BX104592 Soares adult brain
      N2b5HB55Y Homo sapiens cDNA clone IMAGp998I16328 ;
      IMAGE:178647, mRNA sequence

<221> misc_feature
<222> 580
<223> n=a, t, c, or g

<400> 115
tttttgtggg atcctgcagt aaaattgact tttttgtgtc tttgggagat ttaaattgcg 60
ctaacagtgt tgcgcaaaaa tgagttcatg ccatttaaca tattgtattt taattattaa 120
ctgtattaat ttactatgaa atggacatcc ttttaactaa aatggaattg aacattgcag 180
ttttcaaata tttttccttg ttgggtctgg aaaaggaatt ctactttgat ctgcatagaa 240
aattttgata caattttttg aaagttctta ggtgaaacat ttacccatta aaaaggaagc 300
agaaatactg agacatgaaa ggcattatca actaactcta gactctagaa cccattctag 360
catatctcac gtgcaatttt taaaaataag ttaataattc atctcatatc aacaaaagcc 420
tttgaaacat gggttttcac tagatatcac ctagtgctaa gataaaaacc aaaacaatat 480
cagaattaca tttatgctct aaatttgtag ttgtccattg ttgtgcttag taaatgtgtg 540
tcattaatgc tgtattctcc tagctattat ggaaacttgn ttaaataaag atatggatat 600
aaaaaaaaaa aaaaa                                                  615

<210> 116
<211> 508
<212> DNA
<213> unknown

<220>
<223> >sr|IMAGp998C14658.1|cl|IMAGp998C14658|gb|BX106304
      |oid|234546989 BX106304 Soares fetal liver spleen
      1NFLS Homo sapiens cDNA clone IMAGp998C14658 ;
      IMAGE:294853, mRNA sequence

<221> misc_feature
<222> 93
<223> n=a, t, c, or g

<400> 116
tctagtattt atatgaatct gtgagagata atttttttgg tctcactgca atgaaccaaa 60
agcggcctga gtttggtctt ttaattgtag ccntgtattg aaggcatctt tttgaccaac 120
tcttgttggt tctgtcttga accattgtta cgtcactgtg ctgtaattag tatagctaaa 180
tcttttcctt ccttgctcct cccccagccc accccgtctt cccttaacat tttttcaggg 240
ggggttggga gtggtttcat tttagtgtga gtggatgttt tgatagttgt aaggaaaaaa 300
tgcatttcag acacatttca cacatgagct attttcttac acagtatgtc ttattgttaa 360
taagaatgta atttcatgat acaagtattt aatacctttt ttaagtcagt aaatattcta 420
gccatcaata aattgcagta gagtattaag aggggacagg atgagtttac tcttaaaatt 480
aatcagtatc aaactaagtc tactgtgt                                    508

<210> 117
<211> 707
<212> DNA
<213> unknown

<220>
<223> >ImageID=1704376 IMAGP971I1573_S1 CC   FromEXP of
      IMAGp971I1573_s1.exp April 26, 2002 13:15

<400> 117
ccacctttaa attcctgcaa tttatgctgc aaccaacatc ttagtgtttg ctctctccgg 60
gaagaggcca ggtgggggag aacatacggt ccagagcctg agatcgcctg gctggggtgc 120
aggcaagcag gaacccagtc tagatagaat gcagtgttct ctaaagctat ggatggtttc 180
agtgaaatgt ctgtgtctga caatcgtcac gcaagagtac ttactgtctt cccttgtaca 240
tacattgtct gtatttagct tttcctagta ttgtgaggtt tcaaataggg gttccatgta 300
aatactcttt ctgctactga aatgccggcc cccttgagta tgcatcaccc ccacagatgg 360
ttttgccttt ctctatgcta acactttttg agccactgac gtgcagatcg gtggttagtt 420
tcttgggctt ttattttgca attttatata tatacacata ttatatatat atatatattt 480
atgggttggt tttgtatagt tttctgtttg aatctctgag caccaaagct gccctttgat 540
tttcacgaga actttccaaa gccacttcct gagccctcct gctgctttgg tgtctgacgg 600
tgatgctgcg gtggcatctc tgcccgtgaa tttctgtatg tgtgtgctgt tacctcacct 660
gcagacatga ccctagtaaa tctaacgtgc ttggcaaaaa aaaaaaa 707

<210> 118
<211> 390
<212> DNA
<213> unknown

<220>
<223> >ImageID=2328917 IMAGP971B0496_S1 CC FromEXP of
IMAGp971B0496_s1.exp April 26, 2002 12:37

<221> misc_feature
<222> 41, 285, 312, 346, 372
<223> n=a, t, c, or g

<400> 118
tgagttgtcc aatcagtttt caattgatac tgttggtagt natggagctg ttaaatgtaa 60
aggcctgaaa atggactatc aagttggtgt cactatagac ctgagcagtt ttaacattac 120
tagaattgtg acatttaccc ctttttatat gattaaaaac aaaagcaaat accatatatc 180
agtggctgaa gaaggaaatg ataaatggct ctctcttgat ttggagcagg tgggtagatg 240
aatttcaaaa atatacctct ttggattcta ttttctcctt gaagntagat acctaaagtc 300
accatgtaat gntttaactc tccccttact ccaattttgt tttagnttca tgaatcttta 360
ttgaatattt gngccctgct ctagatagat 390

<210> 119
<211> 385
<212> DNA
<213> unknown

<220>
<223> >sr|IMAGp998D01196.1|cl|IMAGp998D01196|gb|BX106181
|oid|234546743 BX106181 Soares placenta Nb2HP Homo
sapiens cDNA clone IMAGp998D01196 ; IMAGE:136656,
mRNA sequence

<221> misc_feature
<222> 5, 14, 324
<223> n=a, t, c, or g

<400> 119
tttgntttct tatnttgtat aatcaggtta atgatttatt ttttgactaa atgtgcaatt 60
tcttatcact agataacttt cagtatcagt ggtggttact tattacttaa atcagaggaa 120
ggattttata aagattaata aatttaattt taccaataaa tattcccata atttagaaaa 180
ggatgtcgac ttgctaattt cagaaataat tattcatttt taaaaagccc cttttaaagc 240
atctacttga agattggtat aattttcata aaatgtcttt ttttttagtg tcccaaagat 300
atcttagata aactattttg aagntcagat ttcagatgag gcaacatttt cttgagataa 360

ttacccaagc ttcatccatg ttgaa                                          385

<210> 120
<211> 501
<212> DNA
<213> unknown

<220>
<223> >sr|IMAGp998B2070.1|cl|IMAGp998B2070|gb|BX112318|o
      id|234559013 BX112318 Soares infant brain 1NIB
      Homo sapiens cDNA clone IMAGp998B2070 ;
      IMAGE:22170, mRNA sequence

<221> misc_feature
<222> 29
<223> n=a, t, c, or g

<400> 120
tttttttttt ttttaacatg tgtcaaagna ttttattaaa ctaattcaat ttgttaggga 60
aatggtaaga tgttacaact ggttaagagg agcattaaaa gtgcagacat atattcaggc 120
catcaagaat gaggaaatga atttgctaaa ttgatgcaac atcaatcaat agccagggcc 180
tgtcattcac agacaatcac tccaccacac gcacacatac attcctacgg tttgtccaca 240
acttccagag ctgaagagta gctcaaggac atcaaagggc agaggcccct aggaagggct 300
gagccacaat gaagcagtgc tccttcactc ccgatttcca tggctaccac cgtttttctc 360
tggcaaatca gagacgaatt ttaaagactg agtaaatcta taaaagcgat taatcacaga 420
agttcaatgt ctgataaatt attccaaaag tgttcctggg cactgagact caccaagcat 480
acattctgaa atcaaattct a                                              501

<210> 121
<211> 764
<212> DNA
<213> unknown

<220>
<223> >sr|IMAGp998I17258.1|cl|IMAGp998I17258|gb|BX109425
      |oid|234553229 BX109425 Soares breast 3NbHBst Homo
      sapiens cDNA clone IMAGp998I17258 ; IMAGE:160600,
      mRNA sequence

<221> misc_feature
<222> 647
<223> n=a, t, c, or g

<400> 121
gtggctgttg gattctgact gctgcacatt gtctcagagc cagtaaaact catcgttacc 60
aaatatggac aacagtagta gactggatac accccgacct taaacgtata gtaattgaat 120
acgtggatag aattattttc catgaaaact acaatgcagg cacttaccaa aatgacatcg 180
ctttgattga aatgaaaaaa gacggaaaca aaaaagattg tgagctgcct cgttccatcc 240
ctgcctgtgt cccctggtct ccttacctat tccaacctaa tgatacatgc atcgtttctg 300
gctggggacg agaaaaagat aacgaaagag tcttttcact tcagtggggt gaagttaaac 360
taataagcaa ctgctctaag ttttacggaa atcgtttcta tgaaaaagaa atggaatgtg 420
caggtacata tgatggttcc atcgatgcct gtaaagggga ctctggaggc cccttagtct 480
gtatggatgc caacaatgtg acttatgtct ggggtgttgt gagttggggg gaaaactgtg 540
gaaaaccaga gttcccaggt gtttacacca aagtggccaa ttattttgac tggattagct 600
accatgtagg aaggcctttt atttctcagt acaatgtata aaattgngat ctctctcttc 660
attctattct ttttctctca agagttccat ttaatggaaa taaaaacggt ataattaata 720
attctctagg ggggaaaaat gaagcaaatc tcattggata tttt                     764

<210> 122

<211> 457
<212> DNA
<213> unknown

<220>
<223> >gi|776508|sr|IMAGp998M04179|cl|IMAGp998M04179|gb|
      R21727|R21727 yh21b02.s1 Homo sapiens cDNA clone
      130347 3'

<221> misc_feature
<222> 19, 130, 326, 357, 372, 392, 435, 455
<223> n=a, t, c, or g

<400> 122
ttttttgtgg ttccaactnt ttttatactg aaatatgtct gagctgatcc aaaaaagcgt 60
tgggttgggg gagaaggaaa tgatggaagt ggggtgatct cagagctcca tgttagcaac 120
tctgggagtn tgggccggtc catctgtctg tctgcccacc ctggcaggac aatcaggcct 180
aggaggtctg gaggagctcg ccgagctggg cggaggaggc aggccagtcc catgctggag 240
gggaccccac ggccccttct acagggcagg agagtcctca gctgaagggc ttcaccagct 300
tcatggcagc atagttcggg aaggcntaga tgaagatggc cagggaacag cagcagnctg 360
aagaggatga tnaagaggat gatggcccac cngaaacgcc gccacaggat gaactttcat 420
ggtcttgtat tgggnaagta aaccacagga aggangt 457

<210> 123
<211> 376
<212> DNA
<213> unknown

<220>
<223> >gi|5631530|gb|AI911675|sr|IMAGp998K175764.1|cl|IM
      AGp998K175764|AI911675 wc71e09.x1 NCI_CGAP_Pan1
      Homo sapiens cDNA clone IMAGE:2324104 3' similar
      to contains element MER22 repetitive element ;,
      mRNA sequence

<221> misc_feature
<222> 374, 375
<223> n=a, t, c, or g

<400> 123
ttgacacaaa atatgttatt tattgtcaat ttctgcaaag acacatttaa gcaacaaaat 60
atacatttgt caacctttta gaatttgttt tgtacattaa caaatacaat gtgctaccat 120
agcaataaat taaatgtaca ttatttacat tacataggca gttgggatct acgaatcacc 180
tcctcactaa gaggaaagaa agcaaagtcg tcgtcacatg gagcatcact tccaagccac 240
agttcttagg ttcgaccaca caggggaatc tgcacactca cacgccagca gtccccgagc 300
tggagtgtcc aaggggaaca cccaaaacag gagagagctg cacgcatgct aaggagcagg 360
aagccggggg gggnng 376

<210> 124
<211> 501
<212> DNA
<213> unknown

<220>
<223> >sr|IMAGp998J11128.1|cl|IMAGp998J11128|gb|BX090520
      |oid|234515407 BX090520 Soares fetal liver spleen
      1NFLS Homo sapiens cDNA clone IMAGp998J11128 ;
      IMAGE:126826, mRNA sequence

<400> 124
gatacagtgt gacattttaa tacatttaca tattgtgtaa tgatcaaatt aggataatta 60
gcttatgtat cacttcaaaa acataatttc tttgtgatga gaacattcaa aatcttctag 120
tattttgaaa tacataatgc aatattgtta accacaatca ccctactgtg caatagaaca 180
ccagagttta ctcctccttt ccatctgtag cttcagaccc attgaccaat ctctcccctt 240
cgcccccacc accccccccc cgctgtcacc actctcctcc cctccccagc aagaggcttg 300
aatcttgaag gagcacatca gatagaaaga gggaaatcaa ctgggacatg gtttgttgga 360
agtggggtgg aatctggttt gtttacagtt tcagagtttg aggcatcaaa agtgtatgtc 420
attgcccaaa agtatttaac agctttctat gttttaggaa ttcactagaa ataaggcatg 480
ctttttaaaa aaaaaaaaaa a 501

<210> 125
<211> 293
<212> DNA
<213> unknown

<220>
<223> gi|4628611|gb|AI619485|sr|IMAGp998H195650.1|cl|IMA
      Gp998H195650|AI619485 ty38g10.x1 NCI_CGAP_Ut2 Homo
      sapiens cDNA clone IMAGE:2281410 3' similar to
      contains MSR1.t1 MSR1 repetitive element ;, mRNA
      sequence

<400> 125
tttttttttt tttttttttt tttttttttt tttttttttt tttttttttt tttttttttt 60
tttttgggga aaagggatta aaaaaccttt ttcccaaaaa ggaaggcccc cccccccccc 120
aaatcccttt gggacaagaa ccgacccccc ccggaaaccc caaccattgt ccccccctcc 180
cacccctggt ctttcccaag gaaaaaaggc cattcaaaaa tggggggggg gggttagagg 240
gggggggccc cttcccttta aatgggggaa aaaaaaaaaa gcccccgggt ggc 293

<210> 126
<211> 519
<212> DNA
<213> unknown

<220>
<223> gi|5111779|gb|AI743491|sr|IMAGp998P025858.1|cl|IMA
      Gp998P025858|AI743491 wf63h01.x2
      Soares_NFL_T_GBC_S1 Homo sapiens cDNA clone
      IMAGE:2360305 3', mRNA sequence

<400> 126
tttaaactcc acactgtaac caggagtctt ttcaaagcaa tatcctggtc ttgtctggca 60
ttcccttggt ctaccttgga ggcatttatc caggtgagaa ttgtggttaa tgaagctcca 120
taaaacccac acaaagggtc aaccctgcat tatttggtaa agtgatgggg ttaccataac 180
cagcagcaca aagcacagca cttagacctg tgccagagag cttaactgta caatgtgggc 240
caccctagcc atgataaaac aatgactgat gaagactaac tcgacaaaga attgtttgag 300
agtcacgcat tctacccact tacctactct gtattttatt ctggcgttaa actggactgt 360
cagaaacaaa atgctttggc ttcactaccc aacccaacta gtctcccaac acaaccccat 420
cccgctaaga aaactgctat cactgagtca tgtttttttg tcatgccctc accttccagc 480
aagggttgct gtaagaaaca gcctcgagaa tactaaggc 519

<210> 127
<211> 370
<212> DNA
<213> unknown

<220>
<223> >gi|846987|sr|IMAGp998M03208|cl|IMAGp998M03208|gb|

R72955|R72955 yi38a02.r1 Homo sapiens cDNA clone
      141482 5'.

<221> misc_feature
<222> 331, 360
<223> n=a, t, c, or g

<400> 127
cgagataaag ctttcaggta caagctgaag gtggggtgtc taacaactaa aaactatcac 60
taaatctcaa agagaaagtt cttgcaaaat atgtaaagtt cacaaggtgc agacattttc 120
cttctttagg cttttatcta aggaagggct atgaaactgg gcccatctgt atacaggctc 180
aaatttacgt ttttaaagga agaatctatg cagctgaggc ttattgcagg aaatacttca 240
tttgtatgta aatattattg taaataaata gggggctgta tatttttgaa gttgttgatc 300
tgcacttgaa ataggaagtc tctaggggtc ntgcctataa accataaatg tttccccggn 360
aattagttgg                                                        370

<210> 128
<211> 283
<212> DNA
<213> unknown

<220>
<223> >gi|3483811|gb|AF086466|sr|IMAGp998H07795.1|cl|IMA
      Gp998H07795|AF086466 Homo sapiens full length
      insert cDNA clone ZD86G04

<400> 128
caagactcca tctcaagaaa aaaaaaaaaa ttagctgggt gtggtggcac atgcctgtag 60
tcccagctac ttgggaggct gaggtgaaag gatcgcttgg gcctgggaag cggtgattgc 120
aacgctgcac atcagcctgg gtgaccaagc gggactctgt ctcagaacaa aacaaaacaa 180
aaatacatat tcccagatcc tggaatgtcc tgggaagcta tatgtgtgtc atgccctccc 240
agctgcacct gaacaatgag catagcaaac atcaaaaaaa aaa                   283

<210> 129
<211> 451
<212> DNA
<213> unknown

<220>
<223> >gi|2183927|gb|AA459020|sr|IMAGp998A112011.2|cl|IM
      AGp998A112011|AA459020 aa26a06.r1 NCI_CGAP_GCB1
      Homo sapiens cDNA clone IMAGE:814354 5', mRNA
      sequence

<400> 129
agaatccctg tgcaattctg taaaagaata ccaggttctc tggcatgtag aagtgaactt 60
gggcaggtgg aaatggttaa caaagaattc taggagtaga gaataaggtc tgaatcctct 120
taggacagtt gggagttaca aaaaattaac aaacagaaac attgagattg gtgctttaga 180
acttggagta aattaagaat aaagggaagt agacaccttt aaaaaacaaa gaaaaagaac 240
ttggtgagta aaaatagaaa agacaaatta tggagtagga aataaaaact caccagaaaa 300
gttgtctttt acctcctaag ggaaaagcta aaggaattag agttgttttg tctaaagaaa 360
ggcaagggaa gactcaattt gttgttgtca gttattaaca atttatataa agaatagtgt 420
actatttctt ttctgtctgc agagattgag c                                451

<210> 130
<211> 424
<212> DNA
<213> unknown

<220>
<223> >sr|IMAGp998A205772.2|cl|IMAGp998A205772|gb|BX0975
      89|oid|234529551 BX097589 NCI_CGAP_Co3 Homo
      sapiens cDNA clone IMAGp998A205772 ;
      IMAGE:2326939, mRNA sequence

<221> misc_feature
<222> 424
<223> n=a, t, c, or g

<400> 130
agtgaaatag agaaagcggg cctggcatcc ccagtaggag gaagacgctg ttggtaaaga 60
gccgctgaag gcagcaccca agaaacgaca attgctggac agcgacgagg aacaggaaga 120
agatgagggc aggaacagag caccagagtt gggagctcca ggaatccaaa agaagaaacg 180
ataccagatt gaggtgggtc gcagcaccag atggggagct ttatgacatg acaggcggga 240
gttttgtacc acttttagaa gataccagat gcctctgtcc tcattttgtt tcctatgatc 300
atcctctaga taccagatag aggcccattc ctgcatttga gatagggttg gaatatagta 360
ctctagatga tgaatgtgtt tgctttgtgt ttggtattaa acatgtaaga ttagaagttc 420
tcan                                                              424

<210> 131
<211> 472
<212> DNA
<213> unknown

<220>
<223> >sr|IMAGp998I09280.1|cl|IMAGp998I09280|gb|BX091207
      |oid|234516781 BX091207 Soares infant brain 1NIB
      Homo sapiens cDNA clone IMAGp998I09280 ;
      IMAGE:49203, mRNA sequence

<221> misc_feature
<222> 27, 186, 204, 238, 376
<223> n=a, t, c, or g

<400> 131
tttttttttt ttttaaaaat atataangtt ttattgtcaa aaatagacaa actttaattt 60
cctttaacag gaatattaat ttaacagcct tccataagcc atcaccattt tgtaagcata 120
acaggcaaga gagtcaaaga taactgttag tggaaaaagg acaacagttc tacatcgatg 180
cccaanaagc cttgcccagt cagnggtgac aactccagga cagcggcaga aacacagnga 240
acctttggag cttaacaata gacatgcaaa acaacattga tttatcttgg cccaattcta 300
taaagattgg ctttgtagta tctttccaag catttgaaga gtttagtttg ttagaacact 360
ggctaatttg aacagngaca ttttaggtca cttatagtat cagtaacagg atcacacctg 420
tttttcagta acaccaggat gaatacatct gtccctacgc cggtctccag ca         472

<210> 132
<211> 666
<212> DNA
<213> unknown

<220>
<223> >sr|IMAGp998J24653.1|cl|IMAGp998J24653|gb|BX098770
      |oid|234531914 BX098770 Soares fetal liver spleen
      1NFLS Homo sapiens cDNA clone IMAGp998J24653 ;
      IMAGE:293111, mRNA sequence

<400> 132
ttctttggat gtagacactt ttcaaagacc aatgagctgc tgcagaagtc tggcaagaag 60
cccattgact ggaaggagct gtgatcatca gctgaggggt ggcctttgag aagctgctgt 120

taacgtattt gccagttacg aagttccact gaaaattttc ctattaattc ttaagtactc 180
tgcataaggg ggaaaagctt ccagaaagca gccatgaacc aggctgtcca ggaatggcag 240
ctgtatccaa ccacaaacaa caaaggctac cctttgacca aatgtctttc tctgcaacat 300
ggcttcggcc taaaatatgc agaagacaga tgaggtcaaa tactcagttg gctctcttta 360
tctcccttgc ctttatggtg aaacagggga gatgtgcacc tttcaggcac agccctagtt 420
tggcgcctgc tgctccttgg ttttgcctgg ttagactttc agtgacagat gttggggtgt 480
ttttgcttag aaaggtcccc ttgtctcagc cttgcagggc aggcatgcca gtctctgcca 540
gttccactgc ccccttgatc tttgaaggag tcctcaggcc cctcgcagca taaggatgtt 600
ttgcaacttt ccagaatctg gcccagaaat tagggctcaa tttcctgatt gtagtagagg 660
ttaaga 666

<210> 133
<211> 430
<212> DNA
<213> unknown

<220>
<223> >gi|2103286|gb|AA424316|sr|IMAGp998N161887.1|cl|IM
      AGp998N161887|AA424316 zv90d08.r1 Soares_NhHMPu_S1
      Homo sapiens cDNA clone IMAGE:767055 5', mRNA
      sequence

<400> 133
caagaaaata agaaaggtag aaacaggagg acccaccgaa tttgagaggg cacccaggag 60
tgtctgagct gcattacctc tagaaacctc aaacaagtag aaacttgcct agacaataac 120
tggaaaaaca aatgcaatat acatgaactt ttttcaaagc attatgtgga tgtttacaat 180
ggtgggaaat tcagctgagt tcaccaatat aaattaaatc catgagtaac tttcctaata 240
ggcttttttt cctaatacca ccacctaaca gagaacacag gtgaatgcag atgttcactt 300
tagcagactt aatgtttcct atgagatttc actgtacagg tttgtctttc ttctttgcct 360
gagaaataaa aatgtcattt gccatattgc catctaaagg agaaaaactg catcagcaaa 420
gccattgtat 430

<210> 134
<211> 2698
<212> DNA
<213> unknown

<220>
<223> >TXNIP (HUGO ID) THIOREDOXIN INTERACTING PROTEIN;
      UPREGULATED BY 1,25-DIHYDROXYVITAMIN D-3. [Source:
      RefSeq (NM_006472)]

<400> 134
gcttagtgta accagcggcg tatatttttt aggcgccttt tcgaaaacct agtagttaat 60
attcatttgt ttaaatctta ttttattttt aagctcaaac tgcttaagaa taccttaatt 120
ccttaaagtg aaataatttt ttgcaaaggg gtttcctcga tttggagctt tttttttctt 180
ccaccgtcat ttctaactct taaaaccaac tcagttccat catggtgatg ttcaagaaga 240
tcaagtcttt tgaggtggtc tttaacgacc ctgaaaaggt gtacggcagt ggcgagaagg 300
tggctggccg ggtgatagtg gaggtgtgtg aagttactcg tgtcaaagcc gttaggatcc 360
tggcttgcgg agtggctaaa gtgctttgga tgcagggatc ccagcagtgc aaacagactt 420
cggagtacct gcgctatgaa gacacgcttc ttctggaaga ccagccaaca ggtgagaatg 480
agatggtgat catgagacct ggaaacaaat atgagtacaa gttcggcttt gagcttcctc 540
aggggcctct gggaacatcc ttcaaaggaa aatatgggtg tgtagactac tgggtgaagg 600
cttttcttga ccgcccgagc cagccaactc aagagacaaa gaaaaacttt gaagtagtgg 660
atctggtgga tgtcaatacc cctgatttaa tggcacctgt gtctgctaaa aaagaaaaga 720
aagtttcctg catgttcatt cctgatgggc gggtgtctgt ctctgctcga attgacagaa 780
aaggattctg tgaaggtgat gagatttcca tccatgctga ctttgagaat acatgttccc 840
gaattgtggt ccccaaagct gccattgtgg cccgccacac ttaccttgcc aatggccaga 900
ccaaggtgct gactcagaag ttgtcatcag tcagaggcaa tcatattatc tcagggacat 960

gcgcatcatg gcgtggcaag agccttcggg ttcagaagat caggccttct atcctgggct 1020
gcaacatcct tcgagttgaa tattccttac tgatctatgt tagcgttcct ggatccaaga 1080
aggtcatcct tgacctgccc ctggtaattg gcagcagatc aggtctaagc agcagaacat 1140
ccagcatggc cagccgaacc agctctgaga tgagttgggt agatctgaac atccctgata 1200
ccccagaagc tcctccctgc tatatggatg tcattcctga agatcaccga ttggagagcc 1260
caaccactcc tctgctagat gacatggatg gctctcaaga cagccctatc tttatgtatg 1320
cccctgagtt caagttcatg ccaccaccga cttatactga ggtggatccc tgcatcctca 1380
acaacaatgt gcagtgagca tgtggaagaa aagaagcagc tttacctact tgtttctttt 1440
tgtctctctt cctggacact cactttttca gagactcaac agtctctgca atggagtgtg 1500
ggtccacctt agcctctgac ttcctaatgt aggaggtggt cagcaggcaa tctcctgggc 1560
cttaaaggat gcggactcat cctcagccag cgcccatgtt gtgatacagg ggtgtttgtt 1620
ggatgggttt aaaaataact agaaaaactc aggcccatcc attttctcag atctccttga 1680
aaattgaggc cttttcgata gtttcgggtc aggtaaaaat ggcctcctgg cgtaagcttt 1740
tcaaggtttt ttggaggctt tttgtaaatt gtgataggaa ctttggacct tgaacttatg 1800
tatcatgtgg agaagagcca atttaacaaa ctaggaagat gaaaagggaa attgtggcca 1860
aaactttggg aaaaggaggt tcttaaaatc agtgtttccc ctttgtgcac ttgtagaaaa 1920
aaaagaaaaa ccttctagag ctgatttgat ggacaatgga gagagctttc cctgtgatta 1980
taaaaaagga agctagctgc tctacggtca tctttgctta agagtatact ttaacctggc 2040
ttttaaagca gtagtaactg ccccaccaaa ggtcttaaaa gccatttttg gagcctattg 2100
cactgtgttc tcctactgca aatattttca tatgggagga tggttttctc ttcatgtaag 2160
tccttggaat tgattctaag gtgatgttct tagcacttta attcctgtca aattttttgt 2220
tctccccttc tgccatctta aatgtaagct gaaactggtc tactgtgtct ctagggttaa 2280
gccaaaagac aaaaaaaatt ttactacttt tgagattgcc ccaatgtaca gaattatata 2340
attctaacgc ttaaatcatg tgaaagggtt gctgctgtca gccttgccca ctgtgacttc 2400
aaacccaagg aggaactctt gatcaagatg ccgaaccctg tgttcagaac ctccaaatac 2460
tgccatgaga aactagaggg caggtcttca taaaagccct ttgaaccccc ttcctgccct 2520
gtgttaggag atagggatat tggcccctca ctgcagctgc cagcacttgg tcagtcactc 2580
tcagccatag cactttgttc actgtcctgt gtcagagcac tgagctccac ccttttctga 2640
gagttattac agccagaaag tgtgggctga agatggttgg tttcatgttt ttgtatta 2698

<210> 135
<211> 4609
<212> DNA
<213> unknown

<220>
<223> CALD1 (HUGO ID) CALDESMON (CDM). [Source:
      SWISSPROT (Q05682)]

<400> 135
ttgcagaatc gcctaccaga ggaatgacga tgatgaagag gaggcagccc gggaacggcg 60
ccgccgagcc cgacaggaac ggctgcggca gaagcaggag gaagaatcct tgggacaggt 120
gaccgaccag gtggaggtga atgcccagaa cagtgtgcct gacgaggagg ccaagacaac 180
caccacaaac actcaagtgg aaggggatga tgaggccgca ttcctggagc gcctggctcg 240
gcgtgaggaa agacgccaaa aacgccttca ggaggctctg gagcggcaga aggagttcga 300
cccaacaata acagatgcaa gtctgtcgct cccaagcaga agaatgcaaa atgacacagc 360
agaaaatgaa actaccgaga aggaagaaaa aagtgaaagt cgccaagaaa gatacgagat 420
agaggaaaca gaaacagtca ccaagtccta ccagaagaat gattggaggg atgctgaaga 480
aaacaagaaa gaagacaagg aaaaggagga ggaggaagag gagaagccaa agcgagggag 540
cattggagaa aatcaggtag aggtgatggt ggaagagaaa acaactgaaa gccaggagga 600
aacagtggta atgtcattaa aaaatgggca gatcagttca gaagagccta aacaagagga 660
ggagagggaa caaggttcag atgagatttc ccatcatgaa aagatggaag aggaagacaa 720
ggaaagagct gaggcagaga gggcaaggtt ggaagcagaa gaaagagaaa gaattaaagc 780
cgagcaagac aaaaagatag cagatgaacg agcaagaatt gaagcagaag aaaaagcagc 840
tgcccaagaa agagaaagga gagaggcaga agagagggaa aggatgaggg aggaagagaa 900
aagggcagca gaggagaggc agaggataaa ggaggaagag aaaagggcag cagaggagag 960
gcagaggata aaggaggaag agaaaagggc agcagaggag aggcagagga taaaagagga 1020
agagaaaagg gcagcagagg agaggcaaag ggccagggca gaggaggaag agaaggctaa 1080
ggtagaagag cagaaacgta acaagcagct agaagagaaa aaacatgcca tgcaagagac 1140

aaagataaaa ggggaaaagg tagaacagaa aatagaaggg aaatgggtaa atgaaaagaa 1200
agcacaagaa gataaacttc agacagctgt cctaaagaaa cagggagaag agaagggaac 1260
taaagtgcaa gctaaaagag aaaagctcca agaagacaag cctaccttca aaaaagaaga 1320
gatcaaagat gaaaagatta aaaaggacaa agaacccaaa gaagaagtta agagcttcat 1380
ggatcgaaag aagggattta cagaagttaa gtcgcagaat ggagaattca tgacccacaa 1440
acttaaacat actgagaata ctttcagccg ccctggaggg agggccagcg tggacaccaa 1500
ggaggctgag ggcgcccccc aggtggaagc cggcaaaagg ctggaggagc ttcgtcgtcg 1560
tcgcggggag accgagagcg aagagttcga gaagctcaaa cagaagcagc aggaggcggc 1620
tttggagctg gaggaactca agaaaaagag ggaggagaga aggaaggtcc tggaggagga 1680
agagcagagg aggaagcagg aggaagccga tcgaaaactc agagaggagg aagagaagag 1740
gaggctaaag gaagagattg aaaggcgaag agcagaagct gctgagaaac gccagaagat 1800
gccagaagat ggcttgtcag atgacaagaa accattcaag tgtttcactc ctaaaggttc 1860
atctctcaag atagaagagc gagcagaatt tttgaataag tctgtgcaga aaagcagtgg 1920
tgtcaaatcg acccatcaag cagcaatagt ctccaagatt gacagcagac tggagcagta 1980
taccagtgca attgagggaa caaaaagcgc aaaacctaca aagccggcag cctcggatct 2040
tcctgttcct gctgaaggtg tacgcaacat caagagtatg tgggagaaag ggaatgtgtt 2100
ttcatccccc actgcagcag gcacaccaaa taaggaaact gctggcttga aggtaggggt 2160
ttctagccgc atcaatgaat ggctaactaa aaccccagat ggaaacaagt cacctgctcc 2220
caaaccttct gacttgagac caggagacgt atccagcaag cggaacctct gggaaaagca 2280
atctgtggat aaggtcactt cccccactaa ggtttgagac agttccagaa agaacccaag 2340
ctcaagacgc aggacgagct cagttgtaga gggctaattc gctctgtttt gtatttatgt 2400
tgatttacta aattgggttc attatctttt atttttcaat atcccagtaa acccatgtat 2460
attatcacta tatttaataa tcacagtcta gagatgttca tggtaaaagt actgcctttg 2520
cacaggagcc tgtttctaaa gaaacccatg ctgtgaaata gagacttttc tactgatcat 2580
cataactctg tatctgagca gtgataccaa ccacatctga agtcaacaga agatccaagt 2640
ttaaaattgc ctgcggaatg tgtgcagtat ctagaaaaat gaaccgtagt ttttgttttt 2700
ttaaatacag aagtcatgtt gtttctgcac tttataataa agcatggaag aaattatctt 2760
agtaggcaat tgtaacactt tttgaaagta acccatttca gatttgaaat actgcaataa 2820
tggttgtctt taaaaaaaaa aaagaaatgt actgttaagg tattactttt tttcatgctg 2880
atgattcata tctaaattac attattatgt tagctgacag tggtactgat tttttaggtt 2940
ggttgttttg tggatttctt tagtagtgat agtagcctga accacatttt agataactca 3000
attatgtatg tatgtgcata cacatataca aacacactaa tggtagaatg cttttttatg 3060
tgctagacta ttatatttag tagtatgtca ttgtaactag ccaatatcac agcttttgaa 3120
aaattaaaaa atcacactat attaatattt catatttgcc aaaagaaaca tggcagatag 3180
gtctcaatat gttttcaatg cctgatgacc tataagaaga aagtattgaa aagaagagag 3240
attagaactg ttagaaggag ttgaaatttt ctaaaagaca tagtatttag tttataatta 3300
aatgcattct tgaagtccag tgtgaatttt attaatgcta tcatctcgac caagctcaaa 3360
gcctacttat tagaaacaat gaagttcaca ataggtcata aggtctcttc cttttctaaa 3420
attgaaagac aagaaattta gtgccaatat tgtacagaca gaaattccat gtatgagtct 3480
caacaaagac tacctttggc taaatgtcta gaagcagaga agtaaagtga gcaaaatcca 3540
gtgttgagga gtcatgacag tactttgatc tttatatact ctgaagcatt tcttcaaact 3600
tttctacttt tatttgtcat tgatacctgt agtaagttga caatgtggtg aaatttcaaa 3660
attatatgta acttctacta gttttacttt ctcccccaag tcttttttaa ctcatgattt 3720
ttacacacac aatccagaac ttattatata gcctctaagt ctttattctt cacagtagat 3780
aatgaaagag tcctccagtg tcttggcaaa atgttctagt atagctggat acatacagtg 3840
gagttctata aactcatatc tcagtggact taaccaaaat tgtgttagtc tcaattccta 3900
ccacactgag ggagcctccc aaataactat tttcttatct gcagtattcc tccagaagag 3960
ctaaccaggg cagggctggc atgagaagtg acatctgcgt tacaaagtct atcttcctca 4020
taagtctgta aagagcaatt gaatcttcta gctttagcaa acctaagcca aaggaaggaa 4080
agccacgaag aatgcagaag tcaaaccctc atgacaaagt aggcacaagt ctacaataag 4140
ctaaatcaga atttacaaat acaagtgtcc caggtagcat tgactcccgt cattggagtg 4200
aaatggatca aagtttgaat taaggcctat ggtaaggtaa cattgctttg ttgtactttt 4260
gaacaagagc tcctcctgat cactattaca tatttttcta gaaaatctaa agttcagaag 4320
agaatgtatc actgctgact tttattccaa tatttggatg gagtaagttt tagggtagaa 4380
ttttgttcag tttggattta atcttttgaa aagtaaattc cttgtttact ggtttgacta 4440
taattctctg ttatctttac gaggtaaaac tgcaagctga ctagcatgtt ctgtgaatct 4500
gccattccta aaaattttat aaacacttga tacttttcac tgataatgga tcgctccaat 4560
aaacatatat tgtgaaaatg catccacaat aaatggaatt ccttcctgc 4609

<210> 136
<211> 1501
<212> DNA
<213> unknown

<220>
<223> >CD74 (HUGO ID) HLA CLASS II HISTOCOMPATIBILITY
      ANTIGEN, GAMMA CHAIN (HLA-DR ANTIGENS ASSOCIATED
      INVARIANT CHAIN) (IA ANTIGEN-ASSOCIATED INVARIANT
      CHAIN) (II) (P33) (CD74 ANTIGEN). [Source:
      SWISSPROT (P04233)]

<400> 136
cagggtccca gatgcacagg aggagaagca ggagctgtcg ggaagatcag aagccagtca 60
tggatgacca gcgcgacctt atctccaaca atgagcaact gcccatgctg ggccggcgcc 120
ctgggccccc ggagagcaag tgcagccgcg gagccctgta cacaggcttt tccatcctgg 180
tgactctgct cctcgctggc caggccacca ccgcctactt cctgtaccag cagcagggcc 240
ggctggacaa actgacagtc acctcccaga acctgcagct ggagaacctg cgcatgaagc 300
ttcccaagcc tcccaagcct gtgagcaaga tgcgcatggc caccccgctg ctgatgcagg 360
cgctgcccat gggagccctg ccccaggggc ccatgcagaa tgccaccaag tatggcaaca 420
tgacagagga ccatgtgatg cacctgctcc agaatgctga ccccctgaag gtgtacccgc 480
cactgaaggg gagcttcccg gagaacctga gacaccttaa gaacaccatg gagaccatag 540
actggaaggt ctttgagagc tggatgcacc attggctcct gtttgaaatg agcaggcact 600
ccttggagca aaagcccact gacgctccac cgaaagtact gaccaagtgc caggaagagg 660
tcagccacat ccctgctgtc cacccgggtt cattcaggcc caagtgcgac gagaacggca 720
actatctgcc actccagtgc tatgggagca tcggctactg ctggtgtgtc ttccccaacg 780
gcacggaggt ccccaacacc agaagccgcg ggcaccataa ctgcagtgag tcactggaac 840
tggaggaccc gtcttctggg ctgggtgtga ccaagcagga tctgggccca gtccccatgt 900
gagagcagca gaggcggtct tcaacatcct gccagcccca cacagctaca gctttcttgc 960
tcccttcagc ccccagcccc tcccccatct cccaccctgt acctcatccc atgagaccct 1020
ggtgcctggc tctttcgtca cccttggaca agacaaacca agtcggaaca gcagataaca 1080
atgcagcaag gccctgctgc ccaatctcca tctgtcaaca ggggcgtgag gtcccaggaa 1140
gtggccaaaa gctagacaga tccccgttcc tgacatcaca gcagcctcca acacaaggct 1200
ccaagaccta ggctcatgga cgagatggga aggcacaggg agaagggata accctacacc 1260
cagaccccag gctggacatg ctgactgtcc tctcccctcc agcctttggc cttggctttt 1320
ctagcctatt tacctgcagg ctgagccact ctcttccctt tccccagcat cactccccaa 1380
ggaagagcca atgttttcca cccataatcc tttctgccga cccctagttc cctctgctca 1440
gccaagcttg ttatcagctt tcagggccat ggttcacatt agaataaaag gtagtaatta 1500
g                                                                 1501

<210> 137
<211> 4009
<212> DNA
<213> unknown

<220>
<223> >C7 (HUGO ID) COMPLEMENT COMPONENT C7 PRECURSOR.
      [Source: SWISSPROT (P10643)]

<400> 137
cagggagagg cagagaggca ggcagcctgc tgggctcttc ctgctgttga aaacttaccc 60
ggcccttaca gaggaaatct tcctcctctc ttctgccctg aatgttttcc caaacatgaa 120
ggtgataagc ttattcattt tggtgggatt tataggagag ttccaaagtt tttcaagtgc 180
ctcctctcca gtcaactgcc agtgggactt ctatgcccct tggtcagaat gcaatggctg 240
taccaagact cagactcgca ggcggtcagt tgctgtgtat gggcagtatg gaggccagcc 300
ttgtgttgga aatgcttttg aaacacagtc ctgtgaacct acaagaggat gtccaacaga 360
ggagggatgt ggagagcgtt tcaggtgctt ttcaggtcag tgcatcagca aatcattggt 420
ttgcaatggg gattctgact gtgatgaaga cagtgctgat gaagacagat gtgaggactc 480
agaaaggaga ccttcctgtg atatcgataa acctcctcct aacatagaac ttactggaaa 540

tggttacaat gaactcactg gccagtttag gaacagagtc atcaatacca aaagttttgg 600
tggtcaatgt agaaaggtgt ttagtgggga tggaaaagat ttctacaggc tgagtggaaa 660
tgtcctgtcc tatacattcc aggtgaaaat aaataatgat tttaattatg aattttacaa 720
tagtacttgg tcttatgtaa aacatacgtc gacagaacac acatcatcta gtcggaagcg 780
ctcctttttt agatcttcat catcttcttc acgcagttat acttcacata ccaatgaaat 840
ccataaagga aagagttacc aactgctggt tgttgagaac actgttgaag tggctcagtt 900
cattaataac aatccagaat tttacaactt tgctgagcca ttctggaagg agctttccca 960
cctcccctct ctgtatgact acagtgccta ccgaagatta atcgaccagt acgggacaca 1020
ttatctgcaa tctgggtcgt taggaggaga atacagagtt ctattttatg tggactcaga 1080
aaaattaaaa caaaatgatt ttaattcagt cgaagaaaag aaatgtaaat cctcaggttg 1140
gcattttgtc gttaaatttt caagtcatgg atgcaaggaa ctggaaaacg ctttaaaagc 1200
tgcttcagga acccagaaca atgtattgcg aggagaaccg ttcatcagag ggggaggtgc 1260
aggcttcata tctggcctta gttacctaga gctggacaat cctgctggaa acaaaaggcg 1320
atattctgcc tgggcagaat ctgtgactaa tcttcctcaa gtcataaaac aaaagctgac 1380
acctttatat gagctggtaa aggaagtacc ttgtgcctct gtgaaaaaac tatacctgaa 1440
atgggctctt gaagagtatc tggatgaatt tgacccctgt cattgccggc cttgtcaaaa 1500
tggtggtttg gctactgttg aggggaccca ttgtctgtgc cattgcaaac cgtacacatt 1560
tggtgcggcg tgtgagcaag gagtcctcgt agggaatcaa gcaggagggg ttgatggagg 1620
ttggagttgc tggtcctctt ggagcccctg tgtccaaggg aagaaaacaa gaagccgtga 1680
atgcaataac ccacctccca gtgggggtgg gagatcctgc gttggagaaa cgacagaaag 1740
cacacaatgc gaagatgagg agctggagca cttgaggttg cttgaaccac attgctttcc 1800
tttgtctttg gttccaacag aattctgtcc atcacctcct gccttgaaag atggatttgt 1860
tcaagatgaa ggtacaatgt ttcctgtggg gaaaaatgta gtgtacactt gcaatgaagg 1920
atactctctt attggaaacc cagtggccag atgtggagaa gatttacggt ggcttgttgg 1980
ggaaatgcat tgtcagaaaa ttgcctgtgt tctacctgta ctgatggatg gcatacagag 2040
tcacccccaa aaacctttct acacagttgg tgagaaggtg actgtttcct gttcaggtgg 2100
catgtcctta gaaggtcctt cagcatttct ctgtggctcc agccttaagt ggagtcctga 2160
gatgaagaat gcccgctgtg tacaaaaaga aaatccgtta acacaggcag tgcctaaatg 2220
tcagcgctgg gagaaactgc agaattcaag atgtgtttgt aaaatgccct acgaatgtgg 2280
accttccttg gatgtatgtg ctcaagatga gagaagcaaa aggatactgc ctctgacagt 2340
ttgcaagatg catgttctcc actgtcaggg tagaaattac acccttactg gtagggacag 2400
ctgtactctg cctgcctcag ctgagaaagc ttgtggtgcc tgcccactgt ggggaaaatg 2460
tgatgctgag agcagcaaat gtgtctgccg agaagcatcg gagtgcgagg aagaagggtt 2520
tagcatttgt gtggaagtga acggcaagga gcagacgatg tctgagtgtg aggcgggcgc 2580
tctgagatgc agagggcaga gcatctctgt caccagcata aggccttgtg ctgcggaaac 2640
ccagtaggct cctggaggcc ctggtcagct tgcttggaat ccagcaggca gctggggctg 2700
agtgaaaaca tctgcacaac tgggcactgg acagcttttc cttcttctcc agtgtctacc 2760
ttcctcctca actcccagcc atctgtataa acacaatcct ttgttctccc aaatctgaat 2820
cgaattactc ttttgcctcc tttttaatgt cagtaaggat atgagccttt gcacaggctg 2880
gctgcgtgtt cttgaaatag gtgttacctt ctctgggcct tggtttttta aaatctgtaa 2940
aattagagga ttgcactaga gaaacttgaa tgctccattc aggcctatca ttttattaag 3000
tatgattgac acagcccatg ggccagaaca cactctacaa aatgactagg ataacagaaa 3060
gaacgtgatc tcctgattag agagggtggt tttcctcaat ggaaccaaat ataaagagga 3120
cttgaacaaa aatgacagat acaaactatt tctatcctga gtagtaatct cacacttcat 3180
cctatagagt caaccaccac agataggaat tccttattct ttttttaatt tttttaagac 3240
agagtctcac tttgttgccc aggctggagc gcagtggggt gatctcatct ccctgcaacc 3300
tccgcctcct gggttcaagc gattcttgtg cctcagcttc ccaagcagct gggattacag 3360
gtgcccgcca ccacgcccag ctaatttttg catttttagt agagatgggg tttcaccatg 3420
ttggccacgc tcgtctccaa ctcctgacct caggtaatcc gcctgccttg gcctcccaaa 3480
gtgctgggat tacagacatg aaccaccacg cctggctgga atacttactc ttgtcgggag 3540
attgaaccac taaaatgtta gagcagaatt cattatgctg tggtcacagg ggtgtcttgt 3600
ctgagaacaa atacaattca gtcttctctt tggggtttta gtatgtgtca aacataggac 3660
tggaagtttg cccctgttct tttttctttt gaaagaacat cagttcatgc ctgaggcatg 3720
agtgactgtg catttgagaa tagttttccc tattctgtgg atacagtccc agagttttca 3780
gggagtacac aggtagatta gtttgaagca ttgacctttt atttattcct tatttctctt 3840
tcatcaaaac aaaacagcag ctgtgggagg agaaatgaga gggcttaaat gaaatttaaa 3900
ataagctata ttatacaaat actatctctg tattgttctg accctggtaa atatatttca 3960
aaacttcaga tgacaaggat tagaacactc attaaagatg ctattcttc 4009

<210> 138
<211> 2240
<212> DNA
<213> unknown

<220>
<223> >CXCL16 (HUGO ID) SMALL INDUCIBLE CYTOKINE B16
      (TRANSMEMBRANE CHEMOKINE CXCL16) (SR- PSOX)
      (SCAVENGER RECEPTOR FOR PHOSPHATIDYLSERINE AND
      OXIDIZED LOW DENSITY LIPOPROTEIN). [Source:
      SWISSPROT (Q9H2A7)]

<400> 138
catgcagcgc ggctgggtcc cgcggcgccc ggatcgggga agtgaaagtg cctcggagga 60
ggagggccgg tccggcagtg cagccgcctc acaggtcggc ggacgggcca ggcgggcggc 120
ctcctgaacc gaaccgaatc ggctcctcgg gccgtcgtcc tcccgcccct cctcgcccgc 180
cgccggagtt ttctttcggt ttcttccaag attcctggcc ttccctcgac ggagccgggc 240
ccagtgcggg ggcgcagggc gcgggagctc cacctcctcg gctttccctg cgtccagagg 300
ctggcatggc gcgggccgag tactgagcgc acggtcgggg cacagcaggg ccggggggtg 360
cagctggctc gcgcctcctc tccggccgcc gtctcctccg gtccccggcg aaagccattg 420
agacaccagc tggacgtcac gcgccggagc atgtctggga gtcagagcga ggtggctcca 480
tccccgcaga gtccgcggag ccccgagatg ggacgggact tgcggcccgg gtcccgcgtg 540
ctcctgctcc tgcttctgct cctgctggtg tacctgactc agccaggcaa tggcaacgag 600
ggcagcgtca ctggaagttg ttattgtggt aaaagaattt cttccgactc cccgccatcg 660
gttcagttca tgaatcgtct ccggaaacac ctgagagctt accatcggtg tctatactac 720
acgaggttcc agctcctttc ctggagcgtg tgtgggggca acaaggaccc atgggttcag 780
gaattgatga gctgtcttga tctcaaagaa tgtggacatg cttactcggg gattgtggcc 840
caccagaagc atttacttcc taccagcccc ccaatttctc aggcctcaga gggggcatct 900
tcagatatcc acacccctgc ccagatgctc ctgtccacct tgcagtccac tcagcgcccc 960
accctcccag taggatcact gtcctcggac aaagagctca ctcgtcccaa tgaaaccacc 1020
attcacactg cgggccacag tctggcagct gggcctgagg ctggggagaa ccagaagcag 1080
ccggaaaaaa atgctggtcc cacagccagg acatcagcca cagtgccagt cctgtgcctc 1140
ctggccatca tcttcatcct caccgcagcc ctttcctatg tgctgtgcaa gaggaggagg 1200
gggcagtcac cgcagtcctc tccagatctg ccggttcatt atatacctgt ggcacctgac 1260
tctaatacct gagccaagaa tggaagcttg tgaggagacg gactctatgt tgcccaggct 1320
gttatggaac tcctgagtca agtgatcctc ccaccttggc ctctgaaggt gcgaggatta 1380
taggcgtcac ctaccacatc cagcctacac gtatttgtta atatctaaca taggactaac 1440
cagccactgc cctctcttag gcccctcatt taaaaacggt tatactataa aatctgcttt 1500
tcacactggg tgataataac ttggacaaat tctatgtgta ttttgttttg ttttgctttg 1560
ctttgttttg agacggagtc tcgctctgtc atccaggctg gagtgcagtg gcatgatctc 1620
ggctcactgc aacccccatc tcccaggttc aagcgattct cctgcctcct cctgagtagc 1680
tgggactaca ggtgctcacc accacacccg gctaattttt tgtattttta gtagagacgg 1740
ggtttcacca tgttgaccag gctggtctcg aactcctgac ctggtgatct gcccacccag 1800
gcctcccaaa gtgctgggat taaaggtgtg agccaccatg cctggcccta tgtgtgtttt 1860
ttaactacta aaaattattt ttgtaatgat tgagtcttct ttatggaaac aactggcctc 1920
agcccttgcg cccttactgt gattcctggc ttcatttttt gctgatggtt ccccctcgtc 1980
ccaaatctct ctcccagtac accagttgtt cctcccccac ctcagccctc tcctgcatcc 2040
tcctgtaccc gcagcgaagg cctgggcttt cccaccctcc ctccttagca ggtgccgtgc 2100
tgggacacca tacgggttgg tttcacctcc tcagtccctt gcctacccca gtgagagtct 2160
gatcttgttt ttattgttat tgcttttatt attattgctt ttattatcat taaaactcta 2220
gttcttgttt tgtctctccg                                              2240

<210> 139
<211> 4234
<212> DNA
<213> unknown

<220>
<223> CYBRD1 (HUGO ID) CYTOCHROME B REDUCTASE 1;

DUODENAL CYTOCHROME B. [Source: RefSeq
(NM_024843)]

<400> 139
ggccactacc cagagggctg ccgccgcctc tccaagttct tgtggccccc gcggtgcgga 60
gtatggggcg ctgatggcca tggagggcta ctggcgcttc ctggcgctgc tggggtcggc 120
actgctcgtc ggcttcctgt cggtgatctt cgccctcgtc tgggtcctcc actaccgaga 180
ggggcttggc tgggatggga gcgcactaga gtttaactgg cacccagtgc tcatggtcac 240
cggcttcgtc ttcatccagg gcatcgccat catcgtctac agactgccgt ggacctggaa 300
atgcagcaag ctcctgatga aatccatcca tgcagggtta aatgcagttg ctgccattct 360
tgcaattatc tctgtggtgg ccgtgtttga gaaccacaat gttaacaata tagccaatat 420
gtacagtctg cacagctggg ttggactgat agctgtcata tgctatttgt tacagcttct 480
ttcaggtttt tcagtctttc tgcttccatg ggctccgctt tctctccgag catttctcat 540
gcccatacat gtttattctg gaattgtcat ctttggaaca gtgattgcaa cagcacttat 600
gggattgaca gagaaactga ttttttccct gagagatcct gcatacagta cattcccgcc 660
agaaggtgtt ttcgtaaata cgcttggcct tctgatcctg gtgttcgggg ccctcatttt 720
ttggatagtc accagaccgc aatggaaacg tcctaaggag ccaaattcta ccattcttca 780
tccaaatgga ggcactgaac agggagcaag aggttccatg ccagcctact ctggcaacaa 840
catggacaaa tcagattcag agttaaacag tgaagtagca gcaaggaaaa gaaacttagc 900
tctggatgag gctgggcaga gatctaccat gtaaaatgtt gtagagatag agccatataa 960
cgtcacgttt caaaactagc tctacagttt tgcttctcct attagccata tgataattgg 1020
gctatgtagt atcaatattt actttaatca caaaggatgg tttcttgaaa taatttgtat 1080
tgattgaggc ctatgaactg acctgaattg gaaaggatgt gattaatata aataatagca 1140
gatataaatt gtggttatgt tacctttatc ttgttgagga ccacaacatt agcacggtgc 1200
cttgtgcaga atagatactc aatatgtgaa tatgtgtcta ctagtagtta attggataaa 1260
ctggcagcat ccctggcctg ttgtcatgca gtcatttcct gttaattctg ggagacaatg 1320
atttcacaac tagcgggaag cagtcctaaa agtttaaaat ccgataagga atatctggga 1380
cagggtttag atcatgactc tacacagata ccatgatgag agtatattaa agaaatttag 1440
gaaagcacct ggttcctttc tccccatgcc tgccttctgc tccctcccca gctggtttgg 1500
gctcaaattg tccctggaga ctagggttta tgttagggta ttgatagatt agagcaggtg 1560
gttgaagaga tcttctctgg tcagacttgg aagaatttcc aaaactgaag ttagccccaa 1620
gacttcccta gggttgatgt actttatgat ccagatgcta aacttcttag aatgaaaata 1680
tgcttcaaca cttaagtagc atacactgcc ctacaaacct cagagagcac ttttccccaa 1740
gttcttgttt ttatttttga aagtactcac acagcactta ctatgctcca aacactcctc 1800
taagcacttt acacatatta gctcattcag tccccagaca gacgggatga agtaggtatt 1860
gttactgttc ccattttaca ggtgagagat ttgaagcctg gggaggctag taactcaccc 1920
caaggtcaca cggctcatac atggtgggac tgagactcag atgcaggcag tctggcacct 1980
cagtctggat tctaaccatt tcactaagct atttttgtct tgtactactt tgacccaccc 2040
ctgaataaac ctcaattgct ggagtggggt gtagttatta aagggatgct tttacctttt 2100
tgctgtttgc tgtggcagat tccccagata accaaggaaa aggggccacc catacctgga 2160
aataggccat agggccccta ctactgccaa caagccatgg cctaccttga cacttgtttg 2220
atcttaaaat tgtgtcttgg taacaaaaga tttggacagg catatctgta gctttcaagt 2280
taattaattg caatattttt ttcttcagga ttttagctgc tgaacaactt tcagtttgga 2340
gctaaaagag acctgtctca tggtctgccc ttccctgggg caatagctag ggtctttcct 2400
gatttttatg gaattttagg ggatattttg agctttgggt tctcagtagt gaattgagac 2460
ttggaggtga cttttcatgt ttggagtatc atctctgtct gggatctggg ctgacaaatt 2520
aaaacctaga gtagtgctta tgctgaaatg atacttttca ttttttggtt gatttttttg 2580
ccttcccttc aattttaaac tgaagcattt taatatgggt agaaactcta caccaaatac 2640
actaaacatt ttggtgctta gtggatttct ttttaggtaa ctggtactta cttccaaaga 2700
ctgaatacaa gccacactcc atcatatccc ttaaacttca tgaaaaacca ttcaagatcc 2760
ccttgctgca acactgttct cttcttctct actaaattct atttccaaaa ttggtaatag 2820
agccagaagg atccccagta cccagccctc tgcctggcac aaagtggtag cacaattaaa 2880
ttcagtatgg gtggagcatg gtacagtctt ggtgccatag aaggagtagt tgcatagtca 2940
cacatcattt gataagttgg atgttccatt acatagagga acacaaaatt ccagggtttt 3000
tggaggaagg gattagatag tgactaagcc gccagaattg aggtggccat tcctttttgt 3060
ataggctaag aaacaggtta tcagtgaaaa gttaattatg gctttggcac tagaatagca 3120
ctgttgcaaa gtatttaagc accccccatc tcagcccttt attttatctt tcatgtgggc 3180
taatgtgagg ataatcttac agatattata ggaatttctt ttctatcttt atgaaaacaa 3240
cgtatataaa atatatctag aaaacctttg tttgagactc ttatttaatg ggcttttgat 3300

tctaatgata attgtacctt tatctttcaa aagctgatat ttcctaccta agcatctccc 3360
gagaaaaata tctcattaaa aagcccataa ataatagggg agaagaaagc cttaggtatc 3420
aattccaaaa cagtgattga aatttcccaa aataattatg gcttctgtca tctccagaga 3480
taatctggct tggtttaccc cataatctaa tttcagaaaa gaaagcttta ttttaacact 3540
catctgaatc aacattaaag ccttttctct caaagcgttt attgagaaac tcaaatgaat 3600
atactttttg aattactgtc atcaaaagtg tacggcttcc tgtgctgctt gtgtcaaatg 3660
gaacctgccc tctaaagcac tttctttcct ttacttgcgt ggtttcatgt aagctgtgct 3720
gtttagaaac aacatctcag actttacaaa gaaatgacaa agaaggcaat tgcacttttt 3780
aagggatatc gacaagcagt ttctgttttc taaaggacaa aatacagagt gtgtgtcatt 3840
tttaattaga ttctttcccc tgctgagttg gaaattccag tgcagcactg attgaccaca 3900
gttgccaatc taaaagcaca aagacagaag taaagcttta tgctaatttt atttcaatat 3960
gatagaaaat ttatcttggt atgtcctttt ttagataact ccagcaggaa actgtaactg 4020
ctatgtcttt aggaaaatgt agaagaaaga acattattgt tctttaattc ctacaaggta 4080
ctcgaaaacc ttaagtgaaa aagatttcta tctttttatc ttagcgcatt tatggaaaaa 4140
atattaacta tcctgaatat tttataattt tgtaggaaaa atatgcatct attttttctt 4200
gacttctttt atatagtaat aaaagttatt ttgg 4234

<210> 140
<211> 606
<212> DNA
<213> unknown

<220>
<223> >IFITM1 (HUGO ID) INTERFERON-INDUCED TRANSMEMBRANE
      PROTEIN 1 (INTERFERON-INDUCED PROTEIN 17)
      (INTERFERON-INDUCIBLE PROTEIN 9-27) (LEU-13
      ANTIGEN) (CD225 ANTIGEN). [Source: SWISSPROT
      (P13164)]

<400> 140
cccttcgctc cacgcagaaa accacacttc tcaaaccttc actcaacact tccttcccca 60
aagccagaag atgcacaagg aggaacatga ggtggctgtg ctggggccac cccccagcac 120
catccttcca aggtccaccg tgatcaacat ccacagcgag acctccgtgc ccgaccatgt 180
cgtctggtcc ctgttcaaca ccctcttctt gaactggtgc tgtctgggct tcatagcatt 240
cgcctactcc gtgaagtcta gggacaggaa gatggttggc gacgtgaccg gggcccaggc 300
ctatgcctcc accgccaagt gcctgaacat ctgggccctg attctgggca tcctcatgac 360
cattggattc atcctgttac tggtattcgg ctctgtgaca gtctaccata ttatgttaca 420
gataatacag gaaaaacggg gttactagta gccgcccata gcctgcaacc tttgcactcc 480
actgtgcaat gctggccctg cacgctgggg ctgttgcccc tgcccccttg gtcctgcccc 540
tagatacagc agtttatacc cacacacctg tctacagtgt cattcaataa agtgcacgtg 600
cttgtg 606

<210> 141
<211> 3423
<212> DNA
<213> unknown

<220>
<223> SALL4 (HUGO ID) SAL-LIKE PROTEIN 4 (ZINC FINGER
      PROTEIN SALL4). [Source: SWISSPROT (Q9UJQ4)]

<400> 141
aactccagga atttgtggcg gagagggcaa ataactgcgg ctctcccggc gccccgatgc 60
tcgcaccatg tcgaggcgca agcaggcgaa accccagcac atcaactcgg aggaggacca 120
gggcgagcag cagccgcagc agcagacccc ggagtttgca gatgcggccc cagcggcgcc 180
cgcggcgggg gagctgggtg ctccagtgaa ccacccaggg aatgacgagg tggcgagtga 240
ggatgaagcc acagtaaagc ggcttcgtcg ggaggagacg cacgtctgtg agaaatgctg 300
tgcggagttc ttcagcatct ctgagttcct ggaacataag aaaaattgca ctaaaaatcc 360
acctgtcctc atcatgaatg acagcgaggg gcctgtgcct tcagaagact tctccggagc 420

tgtactgagc caccagccca ccagtcccgg cagtaaggac tgtcacaggg agaatggcgg 480
cagctcagag gacatgaagg agaagccgga tgcggagtct gtggtgtacc taaagacaga 540
gacagccctg ccacccaccc cccaggacat aagctattta gccaaaggca aagtggccaa 600
cactaatgtg accttgcagg cactacgggg caccaaggtg gcggtgaatc agcggagcgc 660
ggatgcactc cctgcccccg tgcctggtgc caacagcatc ccgtgggtcc tcgagcagat 720
cttgtgtctg cagcagcagc agctacagca gatccagctc accgagcaga tccgcatcca 780
ggtgaacatg tgggcctccc acgccctcca ctcaagcggg gcaggggccg acactctgaa 840
gaccttgggc agccacatgt ctcagcaggt ttctgcagct gtggctttgc tcagccagaa 900
agctggaagc caaggtctgt ctctggatgc cttgaaacaa gccaagctac ctcacgccaa 960
catcccttct gccaccagct ccctgtcccc agggctggca cccttcactc tgaagccgga 1020
tgggacccgg gtgctcccga acgtcatgtc ccgcctcccg agcgctttgc ttcctcaggc 1080
cccgggctcg gtgctcttcc agagcccttt ctccactgtg gcgctagaca catccaagaa 1140
agggaagggg aagccaccga acatctccgc ggtggatgtc aaacccaaag acgaggcggc 1200
cctctacaag cacaagtgta agtactgtag caaggttttt gggactgata gctccttgca 1260
gatccacctc cgctcccaca ctggagagag acccttcgtg tgctctgtct gtggtcatcg 1320
cttcaccacc aagggcaacc tcaaggtgca ctttcaccga catccccagg tgaaggcaaa 1380
cccccagctg tttgccgagt tccaggacaa agtggcggcc ggcaatggca tcccctatgc 1440
actctctgta cctgacccca tagatgaacc gagtctttct ttagacagca aacctgtcct 1500
tgtaaccacc tctgtagggc tacctcagaa tctttcttcg gggactaatc ccaaggacct 1560
cacgggtggc tccttgcccg gtgacctgca gcctgggcct tctccagaaa gtgagggtgg 1620
acccacactc cctggggtgg gaccaaacta taattcccca agggctggtg gcttccaagg 1680
gagtgggacc cctgagccag ggtcagagac cctgaaattg cagcagttgg tggagaacat 1740
tgacaaggcc accactgatc ccaacgaatg tctcatttgc caccgagtct taagctgtca 1800
gagctccctc aagatgcatt atcgcaccca caccggggag agaccgttcc agtgtaagat 1860
ctgtggccga gccttttcta ccaaaggtaa cctgaagaca caccttgggg ttcaccgaac 1920
caacacatcc attaagacgc agcattcgtg ccccatctgc cagaagaagt tcactaatgc 1980
cgtgatgctg cagcaacata ttcggatgca catgggcggt cagattccca acacgcccct 2040
gccagagaat ccctgtgact ttacgggttc tgagccaatg accgtgggtg agaacggcag 2100
caccggcgct atctgccatg atgatgtcat cgaaagcatc gatgtagagg aagtcagctc 2160
ccaggaggct cccagcagct cctccaaggt ccccacgcct cttcccagca tccactcggc 2220
atcacccacg ctagggtttg ccatgatggc ttccttagat gccccaggga aagtgggtcc 2280
tgcccctttt aacctgcagc gccagggcag cagagaaaac ggttccgtgg agagcgatgg 2340
cttgaccaac gactcatcct cgctgatggg agaccaggag tatcagagcc gaagcccaga 2400
tatcctggaa accacatcct tccaggcact ctccccggcc aatagtcaag ccgaaagcat 2460
caagtcaaag tctcccgatg ctgggagcaa agcagagagc tccgagaaca gccgcactga 2520
gatggaaggt cggagcagtc tcccttccac gtttatccga gccccgccga cctatgtcaa 2580
ggttgaagtt cctggcacat ttgtgggacc ctcgacattg tccccaggga tgaccccttt 2640
gttagcagcc cagccacgcc gacaggccaa gcaacatggc tgcacacggt gtgggaagaa 2700
cttctcgtct gctagcgctc ttcagatcca cgagcggact cacactggag agaagccttt 2760
tgtgtgcaac atttgtgggc gagcttttac caccaaaggc aacttaaagg ttcactacat 2820
gacacacggg gcgaacaata actcagcccg ccgtggaagg aagttggcca tcgagaacac 2880
catggctctg ttaggtacgg acggaaaaag agtctcagaa atctttccca aggaaatcct 2940
ggccccttca gtgaatgtgg accctgttgt gtggaaccag tacaccagca tgctcaatgg 3000
cggtctggcc gtgaagacca atgagatctc tgtgatccag agtggggggg ttcctaccct 3060
cccggtttcc ttgggggcca cctccgttgt gaataacgcc actgtctcca agatggatgg 3120
ctcccagtcg ggtatcagtg cagatgtgga aaaaccaagt gctactgacg gcgttcccaa 3180
acaccagttt cctcacttcc tggaagaaaa caagattgcg gtcagctaag ggagaacttg 3240
cgtggaagga gcaatgcaga cacagtgaaa tctctagaat ctgctttgtt ttgtaagaac 3300
tcatctcctc ctgttttctt tttcttactg atatgcaaat gatgtttact acgttggttg 3360
tgaccacaac ctcaggcaag tgctacaatc acgattgttg ctatgctgct ttgcaaaaag 3420
ttg 3423

<210> 142
<211> 2412
<212> DNA
<213> unknown

<220>
<223> TRA1 (HUGO ID) ENDOPLASMIN PRECURSOR (94 KDA

GLUCOSE-REGULATED PROTEIN) (GRP94) (GP96 HOMOLOG)
      (TUMOR REJECTION ANTIGEN 1). [Source: SWISSPROT
      (P14625)]

<400> 142
atgagggccc tgtgggtgct gggcctctgc tgcgtcctgc tgaccttcgg gtcggtcaga 60
gctgacgatg aagttgatgt ggatggtaca gtagaagagg atctgggtaa aagtagagaa 120
ggatcaagga cggatgatga agtagtacag agagaggaag aagctattca gttggatgga 180
ttaaatgcat cacaaataag agaacttaga gagaagtcgg aaaagtttgc cttccaagcc 240
gaagttaaca gaatgatgaa acttatcatc aattcattgt ataaaaataa agagattttc 300
ctgagagaac tgatttcaaa tgcttctgat gctttagata agataaggct aatatcactg 360
actgatgaaa atgctctttc tggaaatgag gaactaacag tcaaaattaa gtgtgataag 420
gagaagaacc tgctgcatgt cacagacacc ggtgtaggaa tgaccagaga agagttggtt 480
aaaaaccttg gtaccatagc caaatctggg acaagcgagt ttttaaacaa aatgactgaa 540
gcacaggaag atggccagtc aacttctgaa ttgattggcc agtttggtgt cggtttctat 600
tccgccttcc ttgtagcaga taaggttatt gtcacttcaa aacacaacaa cgatacccag 660
cacatctggg agtctgactc caatgaattt tctgtaattg ctgacccaag aggaaacact 720
ctaggacggg gaacgacaat tacccttgtc ttaaaagaag aagcatctga ttaccttgaa 780
ttggatacaa ttaaaaatct cgtcaaaaaa tattcacagt tcataaactt tcctatttat 840
gtatggagca gcaagactga aactgttgag gagcccatgg aggaagaaga agcagccaaa 900
gaagagaaag aagaatctga tgatgaagct gcagtagagg aagaagaaga agaaaagaaa 960
ccaaagacta aaaaagttga aaaaactgtc tgggactggg aacttatgaa tgatatcaaa 1020
ccaatatggc agagaccatc aaaagaagta gaagaagatg aatacaaagc tttctacaaa 1080
tcattttcaa aggaaagtga tgaccccatg gcttatattc actttactgc tgaaggggaa 1140
gttaccttca aatcaatttt atttgtaccc acatctgctc cacgtggtct gtttgacgaa 1200
tatggatcta aaaagagcga ttacattaag ctctatgtgc gccgtgtatt catcacagac 1260
gacttccatg atatgatgcc taaatacctc aattttgtca agggtgtggt ggactcagat 1320
gatctcccct tgaatgtttc ccgcgagact cttcagcaac ataaactgct taaggtgatt 1380
aggaagaagc ttgttcgtaa aacgctggac atgatcaaga agattgctga tgataaatac 1440
aatgatactt tttggaaaga atttggtacc aacatcaagc ttggtgtgat tgaagaccac 1500
tcgaatcgaa cacgtcttgc taaacttctt aggttccagt cttctcatca tccaactgac 1560
attactagcc tagaccagta tgtggaaaga atgaaggaaa aacaagacaa aatctacttc 1620
atggctgggt ccagcagaaa agaggctgaa tcttctccat ttgttgagcg acttctgaaa 1680
aagggctatg aagttattta cctcacagaa cctgtggatg aatactgtat tcaggccctt 1740
cccgaatttg atgggaagag gttccagaat gttgccaagg aaggagtgaa gttcgatgaa 1800
agtgagaaaa ctaaggagag tcgtgaagca gttgagaaag aatttgagcc tctgctgaat 1860
tggatgaaag ataaagccct taaggacaag attgaaaagg ctgtggtgtc tcagcgcctg 1920
acagaatctc cgtgtgcttt ggtggccagc cagtacggat ggtctggcaa catggagaga 1980
atcatgaaag cacaagcgta ccaaacgggc aaggacatct ctacaaatta ctatgcgagt 2040
cagaagaaaa catttgaaat taatcccaga cacccgctga tcagagacat gcttcgacga 2100
attaaggaag atgaagatga taaaacagtt ttggatcttg ctgtggtttt gtttgaaaca 2160
gcaacgcttc ggtcagggta tcttttacca gacactaaag catatggaga tagaatagaa 2220
agaatgcttc gcctcagttt gaacattgac cctgatgcaa aggtggaaga agagcccgaa 2280
gaagaacctg aagagacagc agaagacaca acagaagaca cagagcaaga cgaagatgaa 2340
gaaatggatg tgggaacaga tgaagaagaa gaaacagcaa aggaatctac agctgaaaaa 2400
gatgaattgt aa 2412

<210> 143
<211> 4757
<212> DNA
<213> unknown

<220>
<223> >COL1A2 (HUGO ID) ALPHA 2 TYPE I COLLAGEN;
      COLLAGEN I, ALPHA-2 POLYPEPTIDE; COLLAGEN OF SKIN,
      TENDON AND BONE, ALPHA-2 CHAIN. [Source: RefSeq
      (NM_000089)]

<400> 143

atgctcagct ttgtggatac gcggactttg ttgctgcttg cagtaacctt atgcctagca 60
acatgccaat gtaagtgcct tcagcttgtt tgggggagac tgggtagaga gggtccacca 120
ggccccccag gcagagatgg tgaagatggt cccacaggcc ctcctggtcc acctggtcct 180
cctggccccc ctggtctcgg tgggaacttt gctgctcagt atgatggaaa aggagttgga 240
cttggccctg gaccaatggg cttaatggga cctagaggcc cacctggtgc agctggagcc 300
ccaggccctc aaggtttcca aggacctgct ggtgagcctg gtgaacctgg tcaaactggt 360
cctgcaggtg ctcgtggtcc agctggccct cctggcaagg ctggtgaaga tggtcaccct 420
ggaaaacccg gacgacctgg tgagagagga gttgttggac cacagggtgc tcgtggtttc 480
cctggaactc ctggacttcc tggcttcaaa ggcattaggg gacacaatgg tctggatgga 540
ttgaagggac agcccggtgc tcctggtgtg aagggtgaac ctggtgcccc tggtgaaaat 600
ggaactccag gtcaaacagg agcccgtggg cttcctggtg agagaggacg tgttggtgcc 660
cctggcccag ctggtgcccg tggcagtgat ggaagtgtgg gtcccgtggg tcctgctggt 720
cccattgggt ctgctggccc tccaggcttc ccaggtgccc ctggccccaa gggtgaaatt 780
ggagctgttg gtaacgctgg tcctgctggt cccgccggtc cccgtggtga agtgggtctt 840
ccaggcctct ccggccccgt tggacctcct ggtaatcctg gagcaaacgg ccttactggt 900
gccaagggtg ctgctggcct tcccggcgtt gctggggctc ccggcctccc tggaccccgc 960
ggtattcctg gccctgttgg tgctgccggt gctactggtg ccagaggact tgttggtgag 1020
cctggtccag ctggctccaa aggagagagc ggtaacaagg gtgagcccgg ctctgctggg 1080
ccccaaggtc ctcctggtcc cagtggtgaa gaaggaaaga gaggccctaa tggggaagct 1140
ggatctgccg gccctccagg acctcctggg ctgagaggta gtcctggttc tcgtggtctt 1200
cctggagctg atggcagagc tggcgtcatg ggccctcctg gtagtcgtgg tgcaagtggc 1260
cctgctggag tccgaggacc taatggagat gctggtcgcc ctggggagcc tggtctcatg 1320
ggacccagag gtcttcctgg ttcccctgga aatatcggcc ccgctggaaa agaaggtcct 1380
gtcggcctcc ctggcatcga cggcaggcct ggcccaattg gcccagctgg agcaagagga 1440
gagcctggca acattggatt ccctggaccc aaaggcccca ctggtgatcc tggcaaaaac 1500
ggtgataaag gtcatgctgg tcttgctggt gctcggggtg ctccaggtcc tgatggaaac 1560
aatggtgctc agggacctcc tggaccacag ggtgttcaag gtggaaaagg tgaacagggt 1620
ccccctggtc ctccaggctt ccagggtctg cctggcccct caggtcccgc tggtgaagtt 1680
ggcaaaccag gagaaagggg tctccatggt gagtttggtc tccctggtcc tgctggtcca 1740
agaggggaac gcggtccccc aggtgagagt ggtgctgccg gtcctactgg tcctattgga 1800
agccgaggtc cttctggacc cccagggcct gatggaaaca agggtgaacc tggtgtggtt 1860
ggtgctgtgg gcactgctgg tccatctggt cctagtggac tcccaggaga gaggggtgct 1920
gctggcatac ctggaggcaa gggagaaaag ggtgaacctg gtctcagagg tgaaattggt 1980
aaccctggca gagatggtgc tcgtggcgaa gctggggctg ctggtcctgc tggtcctgct 2040
ggtcctcggg gaagccctgg tgaacgtggt gaggtcggtc ctgctggccc caatggattt 2100
gctggtcctg ctggtgctgc tggtcaacct ggtgctaaag gagaaagagg agccaaaggg 2160
cctaagggtg aaaacggtgt tgttggtccc acaggccccg ttggagctgc tggcccagct 2220
ggtatttctg gccctcctgg tccccctggt cctgctggga aagaagggct tcgtggtcct 2280
cgtggtgacc aaggtccagt tggccgaact ggagaagtag gtgcagttgg tccccctggc 2340
ttcgctggtg agaagggtcc ctctggagag gctggtactg ctggacctcc tggcactcca 2400
ggtcctcagg gtcttcttgg tgctcctggt attctgggtc tccctggctc gagaggtgaa 2460
cgtggtctac caggtgttgc tggtgctgtg ggtgaacctg gtcctcttgg cattgccggc 2520
cctcctgggg cccgtggtcc tcctggtgct gtgggtagtc ctggagtcaa cggtgctcct 2580
ggtgaagctg gtcgtgatgg caaccctggg aacgatggtc ccccaggtcg cgatggtcaa 2640
cccggacaca agggagagcg cggttaccct ggcaatattg gtcccgttgg tgctgcaggt 2700
gcacctggtc ctcatggccc cgtgggtcct gctggcaaac atggaaaccg tggtgaaact 2760
ggtccttctg gtcctgttgg tcctgctggt gctgttggcc caagaggtcc tagtggccca 2820
caaggcattc gtggcgataa gggagagccc ggtgaaaagg ggcccagagg tcttcctggc 2880
ttaaagggac acaatggatt gcaaggtctg cctggtatcg ctggtcacca tggtgatcaa 2940
ggtgctcctg gctccgtggg tcctgctggt cctaggggcc ctgctggtcc ttctggccct 3000
gctggaaaag atggtcgcac tggacatcct ggtacagttg gacctgctgg cattcgaggc 3060
cctcagggtc accaaggccc tgctggcccc cctggtcccc ctggccctcc tggacctcca 3120
ggtgtaagcg gtggtggtta tgactttggt tacgatggag acttctacag ggctgaccag 3180
cctcgctcag caccttctct cagacccaag gactatgaag ttgatgctac tctgaagtct 3240
ctcaacaacc agattgagac ccttcttact cctgaaggct ctagaaagaa cccagctcgc 3300
acatgccgtg acttgagact cagccaccca gagtggagca gtggttacta ctggattgac 3360
cctaaccaag gatgcactat ggatgctatc aaagtatact gtgatttctc tactggcgaa 3420
acctgtatcc gggcccaacc tgaaaacatc ccagccaaga actggtatag gagctccaag 3480
gacaagaaac acgtctggct aggagaaact atcaatgctg gcagccagtt tgaatataat 3540

gtagaaggag tgacttccaa ggaaatggct acccaacttg ccttcatgcg cctgctggcc 3600
aactatgcct ctcagaacat cacctaccac tgcaagaaca gcattgcata catggatgag 3660
gagactggca acctgaaaaa ggctgtcatt ctacagggct ctaatgatgt tgaacttgtt 3720
gctgagggca acagcaggtt cacttacact gttcttgtag atggctgctc taaaaagaca 3780
aatgaatggg gaaagacaat cattgaatac aaaacaaata agccatcacg cctgcccttc 3840
cttgatattg cacctttgga catcggtggt gctgaccagg aattctttgt ggacattggc 3900
ccagtctgtt tcaaataaat gaactcaatc taaattaaaa aagaaagaaa tttgaaaaaa 3960
ctttctcttt gccatttctt cttcttcttt tttaactgaa agctgaatcc ttccatttct 4020
tctgcacatc tacttgctta aattgtgggc aaaagagaaa aagaaggatt gatcagagca 4080
ttgtgcaata cagtttcatt aactccttcc cccgctcccc caaaaatttg aatttttttt 4140
tcaacactct tacacctgtt atggaaaatg tcaacctttg taagaaaacc aaaataaaaa 4200
ttgaaaaata aaaaccataa acatttgcac cacttgtggc ttttgaatat cttccacaga 4260
gggaagttta aaacccaaac ttccaaaggt ttaaactacc tcaaaacact ttcccatgag 4320
tgtgatccac attgttaggt gctgacctag acagagatga actgaggtcc ttgttttgtt 4380
ttgttcataa tacaaaggtg ctaattaata gtatttcaga tacttgaaga atgttgatgg 4440
tgctagaaga atttgagaag aaatactcct gtattgagtt gtatcgtgtg gtgtattttt 4500
taaaaaattt gatttagcat tcatattttc catcttattc ccaattaaaa gtatgcagat 4560
tatttgccca aatcttcttc agattcagca tttgttcttt gccagtctca ttttcatctt 4620
cttccatggt tccacagaag ctttgtttct tgggcaagca gaaaaattaa attgtaccta 4680
ttttgtatat gtgagatgtt taaataaatt gtgaaaaaaa tgaaataaag catgtttggt 4740
tttccaaaag aacatat 4757

<210> 144
<211> 1934
<212> DNA
<213> unknown

<220>
<223> HDAC3 (HUGO ID) HISTONE DEACETYLASE 3 (HD3)
      (RPD3-2). [Source: SWISSPROT (O15379)]

<400> 144
gcggcggccg cgggcggcgg gcggcggagg tgcggggcct gctcccgccg gcaccatggc 60
caagaccgtg gcctatttct acgaccccga cgtgggcaac ttccactacg gagctggaca 120
ccctatgaag ccccatcgcc tggcattgac ccatagcctg gtcctgcatt acggtctcta 180
taagaagatg atcgtcttca agccatacca ggcctcccaa catgacatgt gccgcttcca 240
ctccgaggac tacattgact tcctgcagag agtcagcccc accaatatgc aaggcttcac 300
caagagtctt aatgccttca acgtaggcga tgactgccca gtgtttcccg ggctctttga 360
gttctgctcg cgttacacag gcgcatctct gcaaggagca acccagctga acaacaagat 420
ctgtgatatt gccattaact gggctggtgg tctgcaccat gccaagaagt ttgaggcctc 480
tggcttctgc tatgtcaacg acattgtgat tggcatcctg gagctgctca agtaccaccc 540
tcgggtgctc tacattgaca ttgacatcca ccatggtgac ggggttcaag aagctttcta 600
cctcactgac cgggtcatga cggtgtcctt ccacaaatac ggaaattact tcttccctgg 660
cacaggtgac atgtatgaag tcggggcaga gagtggccgc tactactgtc tgaacgtgcc 720
cctgcgggat ggcattgatg accagagtta caagcacctt ttccagccgg ttatcaacca 780
ggtagtggac ttctaccaac ccacgtgcat tgtgctccag tgtggagctg actctctggg 840
ctgtgatcga ttgggctgct ttaacctcag catccgaggg catggggaat gcgttgaata 900
tgtcaagagc ttcaatatcc ctctactcgt gctgggtggt ggtggttata ctgtccgaaa 960
tgttgcccgc tgctggacat atgagacatc gctgctggta gaagaggcca ttagtgagga 1020
gcttccctat agtgaatact tcgagtactt tgccccagac ttcacacttc atccagatgt 1080
cagcacccgc atcgagaatc agaactcacg ccagtatctg gaccagatcc gccagacaat 1140
ctttgaaaac ctgaagatgc tgaaccatgc acctagtgtc cagattcatg acgtgcctgc 1200
agacctcctg acctatgaca ggactgatga ggctgatgca gaggagaggg gtcctgagga 1260
gaactatagc aggccagagg cacccaatga gttctatgat ggagaccatg acaatgacaa 1320
ggaaagcgat gtggagattt aagagtggct tgggatgctg tgtcccaagg aatttctttt 1380
cacctcttgg ttgggctgga gggaaaagga gtggctccta gagtcctggg ggtcacccca 1440
gggcttttgc tgactctggg aaagagtctg gagaccacat ttggttctcg aaccatctac 1500
ctgcttttcc tctctctccc aaggcctgac aatggtacct attagggatg agatacagac 1560
aaggatagct atctgggaca ttattggcag tgggccctgg aggccagtcc ctagcccccc 1620

ttgcccctta tttcttccct gcttccctcg aacccagaga tttttgaggg atgaacgggt 1680
agacaaggac tgagattgcc tctgacttcc tcctcccctg ggttctgact tcttcctccc 1740
cttgcttcca gggaagatga agagagagag atttggaagg ggctctggct ccctaacacc 1800
tgaatcccag atgatgggaa gtatgttttc aagtgtgggg aggatatgaa aatgttctgt 1860
tctcactttt ggctttatgt ccattttacc actgttttta tccaataaac taagtcggta 1920
ttttttgtac cttt 1934

<210> 145
<211> 1235
<212> DNA
<213> unknown

<220>
<223> >HLA-DRA (HUGO ID) HLA CLASS II HISTOCOMPATIBILITY
      ANTIGEN, DR ALPHA CHAIN PRECURSOR. [Source:
      SWISSPROT (P01903)]

<400> 145
attcttgtct gttctgcctc actcccgagc tctactgact cccaacagag cgcccaagaa 60
gaaaatggcc ataagtggag tccctgtgct aggatttttc atcatagctg tgctgatgag 120
cgctcaggaa tcatgggcta tcaaagaaga acatgtgatc atccaggccg agttctatct 180
gaatcctgac caatcaggcg agtttatgtt tgactttgat ggtgatgaga ttttccatgt 240
ggatatggca aagaaggaga cggtctggcg gcttgaagaa tttggacgat ttgccagctt 300
tgaggctcaa ggtgcattgg ccaacatagc tgtggacaaa gccaacctgg aaatcatgac 360
aaagcgctcc aactatactc cgatcaccaa tgtacctcca gaggtaactg tgctcacgaa 420
cagccctgtg gaactgagag agcccaacgt cctcatctgt ttcatagaca agttcacccc 480
accagtggtc aatgtcacgt ggcttcgaaa tggaaaacct gtcaccacag gagtgtcaga 540
gacagtcttc ctgcccaggg aagaccacct tttccgcaag ttccactatc tccccttcct 600
gccctcaact gaggacgttt acgactgcag ggtggagcac tggggcttgg atgagcctct 660
tctcaagcac tgggagtttg atgctccaag ccctctccca gagactacag agaacgtggt 720
gtgtgccctg ggcctgactg tgggtctggt gggcatcatt attgggacca tcttcatcat 780
caagggattg cgcaaaagca atgcagcaga acgcaggggg cctctgtaag gcacatggag 840
gtgatggtgt ttcttagaga gaagatcact gaagaaactt ctgctttaat ggctttacaa 900
agctggcaat attacaatcc ttgacctcag tgaaagcagt catcttcagc attttccagc 960
cctatagcca ccccaagagt ggttatgcct cctcgattgc tccatactct aacatctagc 1020
tggcttccct gtctattgcc ttttcctgta tctattttcc tctatttcct atcattttat 1080
tatcaccatg caatgcctct ggaataaaac atacaggagt ctgtctctgc tatggaatgc 1140
cccatggggc atctcttgtg tacttattgt ttaaggtttc ctcaaactgt gatttttctg 1200
aacacaataa actattttga agatcttggg tggaa 1235

<210> 146
<211> 5918
<212> DNA
<213> unknown

<220>
<223> >OPA1 (HUGO ID) DYNAMIN-LIKE 120 KDA PROTEIN,
      MITOCHONDRIAL PRECURSOR (OPTIC ATROPHY 1 GENE
      PROTEIN). [Source: SWISSPROT (O60313)]

<400> 146
cacgggggct cccgcgtggc cgtctcggcg cctgcgtgac ctccccgccg gcgggatgtg 60
gcgactacgt cgggccgctg tggcctgtga ggtctgccag tctttagtga aacacagctc 120
tggaataaaa ggaagtttac cactacaaaa actacatctg gtttcacgaa gcatttatca 180
ttcacatcat cctaccttaa agcttcaacg accccaatta aggacatcct ttcagcagtt 240
ctcttctctg acaaaccttc ctttacgtaa actgaaattc tctccaatta aatatggcta 300
ccagcctcgc aggaattttt ggccagcaag attagctacg agactcttaa aacttcgcta 360
tctcatacta ggatcggctg ttgggggtgg ctacacagcc aaaaagactt ttgatcagtg 420
gaaagatatg ataccggacc ttagtgaata taaatggatt gtgcctgaca ttgtgtggga 480

aattgatgag tatatcgatt ttgagaaaat tagaaaagcc cttcctagtt cagaagacct 540
tgtaaagtta gcaccagact ttgacaagat tgttgaaagc cttagcttat tgaaggactt 600
ttttacctca ggtcacaaat tggttagtga agtcatagga gcttctgacc tacttctctt 660
gttaggttct ccggaagaaa cggcgtttag agcaacagat cgtggatctg aaagtgacaa 720
gcattttaga aaggtgtcag acaaagagaa aattgaccaa cttcaggaag aacttctgca 780
cactcagttg aagtatcaga gaatcttgga acgattagaa aaggagaaca aagaattgag 840
aaaattagta ttgcagaaag atgacaaagg cattcatcat agaaagctta agaaatcttt 900
gattgacatg tattctgaag ttcttgatgt tctctctgat tatgatgcca gttataatac 960
gcaagatcat ctgccacggg ttgttgtggt tggagatcag agtgctggaa agactagtgt 1020
gttggaaatg attgcccaag ctcgaatatt cccaagagga tctggggaga tgatgacacg 1080
ttctccagtt aaggtgactc tgagtgaagg tcctcaccat gtggccctat ttaaagatag 1140
ttctcgggag tttgatctta ccaaagaaga agatcttgca gcattaagac atgaaataga 1200
acttcgaatg aggaaaaatg tgaaagaagg ctgtaccgtt agccctgaga ccatatcctt 1260
aaatgtaaaa ggccctggac tacagaggat ggtgcttgtt gacttaccag gtgtgattaa 1320
tactgtgaca tcaggcatgg ctcctgacac aaaggaaact attttcagta tcagcaaagc 1380
ttacatgcag aatcctaatg ccatcatact gtgtattcaa gatggatctg tggatgctga 1440
acgcagtatt gttacagact tggtcagtca aatggaccct catggaagga gaaccatatt 1500
cgttttgacc aaagtagacc tggcagagaa aaatgtagcc agtccaagca ggattcagca 1560
gataattgaa ggaaagctct tcccaatgaa agctttaggt tattttgctg ttgtaacagg 1620
aaaagggaac agctctgaaa gcattgaagc tataagagaa tatgaagaag agttttttca 1680
gaattcaaag ctcctaaaga caagcatgct aaaggcacac caagtgacta caagaaattt 1740
aagccttgca gtatcagact gcttttggaa aatggtacga gagtctgttg aacaacaggc 1800
tgatagtttc aaagcaacac gttttaacct tgaaactgaa tggaagaata actatcctcg 1860
cctgcgggaa cttgaccgga atgaactatt tgaaaaagct aaaaatgaaa tccttgatga 1920
agttatcagt ctgagccagg ttacaccaaa acattgggag gaaatccttc aacaatcttt 1980
gtgggaaaga gtatcaactc atgtgattga aaacatctac cttccagctg cgcagaccat 2040
gaattcagga acttttaaca ccacagtgga tatcaagctt aaacagtgga ctgataaaca 2100
acttcctaat aaagcagtag aggttgcttg ggagacccta caagaagaat tttcccgctt 2160
tatgacagaa ccgaaaggga aagagcatga tgacatattt gataaactta aagaggctgt 2220
taaggaagaa agtattaaac gacacaagtg gaatgacttt gcggaggaca gcttgagggt 2280
tattcaacac aatgctttgg aagaccgatc catatctgat aaacagcaat gggatgcagc 2340
tatttatttt atggaagagg ctctgcaggc tcgtctcaag gatactgaaa atgcaattga 2400
aaacatggtg ggtccagact ggaaaaagag gtggttatac tggaagaatc ggacccaaga 2460
acagtgtgtt cacaatgaaa ccaagaatga attggagaag atgttgaaat gtaatgagga 2520
gcacccagct tatcttgcaa gtgatgaaat aaccacagtc cggaagaacc ttgaatcccg 2580
aggagtagaa gtagatccaa gcttgattaa ggatacttgg catcaagttt atagaagaca 2640
ttttttaaaa acagctctaa accattgtaa cctttgtcga agaggttttt attactacca 2700
aaggcatttt gtagattctg agttggaatg caatgatgtg gtcttgtttt ggcgtataca 2760
gcgcatgctt gctatcaccg caaatacttt aaggcaacaa cttacaaata ctgaagttag 2820
gcgattagag aaaaatgtta aagaggtatt ggaagatttt gctgaagatg gtgagaagaa 2880
gattaaattg cttactggta aacgcgttca actggcggaa gacctcaaga aagttagaga 2940
aattcaagaa aaacttgatg ctttcattga agctcttcat caggagaaat aaattaaaat 3000
cgtactcata atcagctctg catacatctg aagaacaaaa acatcaacgt cttttgtcca 3060
gcctcttttt cttctgctgt tccacctttc taaacataca ataaagtcat gggataaaaa 3120
taatcgatgt atgttacggg cgctttaacc atcagctgcc tctcgaatgg aagaacagtg 3180
gtaatggatt aacatcctat tttgttgtac taaagtgaca aatcggaata atataattgg 3240
tatggccatt aggttcagtc cttgaagata agaaacttgt tctctgtttg ttgtcttatt 3300
tgtggtggca ctcgtttaat ggattaactg aggttgctca atgttcagtt tcttttccag 3360
aaatacaatg ctaggtgttt tgaaataaaa cttatatagc aattgtttaa agttatcaat 3420
tgtatataaa atcacagtag cctgctaaat cattgtatgt gtctgtagta ttctattccc 3480
agaaactatt tgaccatgat aattcagttt atattcacca catgaaagaa aaatgggtaa 3540
cagaagaacc cttaaaacag gttaatttgg attgtaacgt tcagtgaaag aaatttcaac 3600
ccttcatagc cagcgaagaa atttgccttg gaagccaagt cagtaccagc ttacctattt 3660
gattcagttg ctgttttctc actctctata tccatttgaa attgatttat tttagatgtt 3720
gtatacttac gttaggcttt ctgttaatag tggtttttct cctgttgaca gagccaccgg 3780
attatgacac aggatgagga agattaagga taatcaattg actaatttca tttagaatat 3840
tatcaaacat ttcaactagg tatcagaaaa aggctttctt tcataagact attttaaata 3900
gaaattattt caacaattaa agtaatgttg accatccccc tctcagctga ataaagaaaa 3960
atttagttca atttattgca atttaattac aatactacct tcacaacatt ttcatgtgtt 4020

ttaaataaat attttttaat tggctaaagg acattcaagc aaagaaatgc tttctttact 4080
taaaatgtct atctcatttg ctgccttttc actaagcctt tactttgtta ataaaagtgt 4140
ccattgtgtg atgtttttga ttttacagtt tgctaaatct tattttcttg gagttgcttt 4200
ttggtaacag ccccattgct actccccatt ttattgtttt acatcaatgc atgcttcgtt 4260
gtgatccctc aagatgtaac acttggtatg ctcggttgag gatatgaaaa aatacttccg 4320
aaaccaggaa ttcaatgtat gtttgtttta tactgtttga taagaaaagt aggtccagcc 4380
ttaagcagca cagatgcgct ggtagatgca tagtcaggaa ctttttttat ttcttttagg 4440
tctagggaca ggagtgaata gaaagggagg agagctctat tatgttctat acacagatta 4500
ggagatgacc ttactgggta cacccctcta accagtgctt acaggttaat gcatgttaat 4560
gaatattttt gcagttgtaa agcataacaa ttacaactac acatctattt ctaaagaata 4620
aaacaggacc atatttattt acttctgtca actatagaaa gaaagacctt cagctgtatt 4680
tccacagatt tctcccaagg aaaaggctaa tattagtcac tactgttatc acatcccttt 4740
gtataagttt taaaaagaga tggagggaga tcttcatttc tttgaggaga tcagtattgt 4800
aacgtatgtg aatagatgat aacaattaat attactaaaa gtcccacatg agagtcctga 4860
cgccctctcc atgccccaca gtaatgtggc ttctttcatg ggtttttttt tcttcttttt 4920
agctgatctc atcctaagca tgctttattt ttccttgaaa gctaggtatt tatcaactgc 4980
agatgttatt gaaagaaaat aaaattcagt ctcaagagta aaccctgtgt cttgtgtctg 5040
tagttcaaaa gtcagaaatg attctaattt aaacaaaaag atactaaata tacagaagtt 5100
aaattcgaac tagccacaga atcatttgtt tttatgtcag aatttgcaaa gagtggagtg 5160
gacaaagctc tgtatggaag actgaacaac tgtaaataga tgatatccaa acttaatttg 5220
gctaggactt caattttaaa aatcagtgta cctaggcagt gcacagcacg aaataagtgg 5280
cccttgcagc ttccccgttt aacccactgt gctatagttg cgggtggaac agtcaacctt 5340
tctagtagtt tatgatattg ccctctttgt attcccattt tctacagttt tttccgcaga 5400
cttctttctg caaattattc agcctccaaa tgcaaatgaa tgatataaaa ataagtaggg 5460
aacatggcag agagtggtgc ttcccagcct cacaatgtgg gaatttgaca taggatgaga 5520
gtcagagtat aggtttaaaa gataaaatct ttagttaata attttgtatt tatttattct 5580
agatgtatgt atctgaggaa agaaatctgg tatttttgct ttccaataaa ggggatcaaa 5640
gtaatggttt ttctctcagt tctctaagct ggtctatgtt atagctctag cagtatggaa 5700
atgtgcttta aaatatgctt accttttgaa tgatcatggc tatatgttgt tgagatattt 5760
gaaacttacc ttgttttcac ttgtgcactg tgaatgaact ttgtattatt tttttaaaac 5820
cttcacatta cgtgtagata ttattgcaac ttatattttg cctgagcttg atcaaaggtc 5880
atttgtgtag atgagtaatt aaaaaatatt taaatcac 5918

<210> 147
<211> 16396
<212> DNA
<213> unknown

<220>
<223> BPAG1 (HUGO ID) BULLOUS PEMPHIGOID ANTIGEN 1
      ISOFORMS 1/2/3/4/5/8 (230 KDA BULLOUS PEMPHIGOID
      ANTIGEN) (BPA) (HEMIDESMOSOMAL PLAQUE PROTEIN)
      (DYSTONIA MUSCULORUM PROTEIN) (FRAGMENT). [Source:
      SWISSPROT (Q03001)]

<400> 147
cagctgccac ttttcaccgt tagaagtaga gctttttcca gacctcctac cttttagtct 60
actttgaaag gtgaaagaaa gaacatcgtt tcaggaataa aaatgcacag tagtagttat 120
agttaccgta gcagtgattc tgtgtttagt aacactacca gcactcgaac cagtcttgat 180
tcaaatgaaa atcttctctt ggttcattgt ggtccaacac tgatcaactc ttgcattagc 240
ttcggcagtg aatcctttga tggacacagg ttagaaatgt tgcaacagat tgccaacaga 300
gttcagaggg acagtgtcat ctgtgaagac aaactgattc ttgctggaaa tgctcttcag 360
tctgattcta aaagattaga atcaggagtg cagtttcaga atgaagcaga aattgctggg 420
tatatacttg aatgtgagaa ccttttacgc cagcatgtaa ttgatgtaca gattcttatt 480
gatggaaaat actaccaggc agatcaattg gtacagaggg ttgcaaaact gcgtgacgaa 540
attatggcct taaggaacga atgttcttct gtgtacagca aaggacgcat actgacaaca 600
gaacagacaa agctcatgat atcaggaatc actcaaagtt taaactcagg atttgcacag 660

accttacacc ctagtctgac ctcagggctg acccagagtt taacaccttc cctaacctct 720
tctagtatga cttctggcct gtcatcaggg atgacttccc gcctgactcc atctgtcact 780
ccagcttata cacctggttt cccatcagga ttagttccaa atttcagttc aggagtagag 840
ccaaattcat tgcaaacttt gaagttgatg cagatccgaa aaccccttct aaagtcttct 900
ttgctggatc aaaatttaac agaagaagaa atcaatatga aatttgttca ggatcttttg 960
aattgggttg atgagatgca ggtacaactg gaccgcactg agtggggctc agatttgcca 1020
agtgttgaaa gccatttaga aaatcataaa aatgttcata gagctattga agaatttgaa 1080
tctagtctca aagaagctaa aatcagtgag attcaaatga cagcacctct taaactgact 1140
tatgcagaaa agttgcacag attagagagt cagtatgcaa aactcttgaa tacatccagg 1200
aatcaagaac ggcaccttga tacactccat aattttgtaa gtcgtgcgac taatgaactt 1260
atttggttga atgaaaaaga agaggaggaa gttgcttatg actggagtga gagaaacacc 1320
aacatagcta ggaaaaaaga ttatcatgct gaattaatga gagaacttga tcaaaaggaa 1380
gaaaatatta aatcagttca ggagatagca gagcagctac ttctagaaaa tcatccagcc 1440
cggttaacta ttgaggccta cagagcggca atgcagacgc agtggagctg gatcttacag 1500
ctctgccagt gtgtggagca gcacataaag gagaacacag cgtatttcga gtttttcaat 1560
gatgccaaag aagctactga ttacttaagg aatctaaaag atgccattca gcggaagtac 1620
agctgtgata gatcaagcag cattcacaag ctagaagacc ttgttcagga atcaatggaa 1680
gagaaagaag aacttctgca gtacaaaagc actatagcaa acctaatggg aaaagcaaaa 1740
acaataattc aactgaagcc aaggaattct gactgtccac tcaaaacttc tattccgatc 1800
aaagctatct gtgactacag acaaattgag ataaccattt acaaagacga tgaatgtgtt 1860
ttggcgaata actctcatcg tgctaaatgg aaggtcatta gtcctactgg gaatgaggct 1920
atggtcccat ctgtgtgctt caccgttcct ccaccaaaca aagaagcggt ggaccttgcc 1980
aacagaattg agcaacagta tcagaatgtc ctgactcttt ggcatgagtc tcacataaac 2040
atgaagagtg tagtatcctg gcattatctc atcaatgaaa ttgatagaat tcgagctagc 2100
aatgtggctt caataaagac aatgctacct ggtgaacatc agcaagttct aagtaatcta 2160
caatctcgtt ttgaagattt tctggaagat agccaggaat cccaagtctt ttcaggctca 2220
gatataacac aactggaaaa ggaggttaat gtatgtaagc agtattatca agaacttctt 2280
aaatctgcag aaagagagga gcaagaggaa tcagtttata atctctacat ctctgaagtt 2340
cgaaacatta gacttcggtt agagaactgt gaagatcggc tgattagaca gattcgaact 2400
cccctggaaa gagatgattt gcatgaaagt gtgttcagaa tcacagaaca ggagaaacta 2460
aagaaagagc tggaacgact taaagatgat ttgggaacaa tcacaaataa gtgtgaggag 2520
tttttcagtc aagcagcagc ctcttcatca gtccctaccc tacgatcaga gcttaatgtg 2580
gtccttcaga acatgaacca agtctattct atgtcttcca cttacataga taagttgaaa 2640
actgttaact tggtgttaaa aaacactcaa gctgcagaag ccctcgtaaa actctatgaa 2700
actaaactgt gtgaagaaga agcagttata gctgacaaga ataatattga gaatctaata 2760
agtactttaa agcaatggag atctgaagta gatgaaaaga gacaggtatt ccatgcctta 2820
gaggatgagt tgcagaaagc taaagccatc agtgatgaaa tgtttaaaac gtataaagaa 2880
cgggaccttg attttgactg gcacaaagaa aaagcagatc aattagttga aaggtggcaa 2940
aatgttcatg tgcagattga caacaggtta cgggacttag agggcattgg caaatcactg 3000
aagtactaca gagacactta ccatccttta gatgattgga tccagcaggt tgaaactact 3060
cagagaaaga ttcaggaaaa tcagcctgaa aatagtaaaa ccctagccac acagttgaat 3120
caacagaaga tgctggtgtc cgaaatagaa atgaaacaga gcaaaatgga cgagtgtcaa 3180
aaatatgcag aacagtactc agctacagtg aaggactatg aattacaaac aatgacctac 3240
cgggccatgg tagattcaca acaaaaatct ccagtgaaac gccgaagaat gcagagttca 3300
gcagatctca ttattcaaga gttcatggac ctaaggactc gatatactgc cctggtcact 3360
ctcatgacac aatatattaa atttgctggt gattcattga agaggctgga agaggaggag 3420
aagtcactgg aagaagaaaa gaaagaacat gttgaaaaag ccaaagaatt gcaaaaatgg 3480
gtatcaaata tcagcaagac attgaaagat gcagagaagg ctggaaaacc tcccttctct 3540
aagcaaaaga tttccagtga ggaaatatca accaagaagg aacaattatc ggaagcactt 3600
caaactatac agcttttttt ggcaaaacat ggagacaaaa tgacagatga agaaagaaat 3660
gaattggaaa aacaagtgaa aactcttcag gaaagttata atttattatt cagtgaatct 3720
ctgaaacagc tacaagaatc acaaacctca ggagatgtaa aggtagagga gaagattgta 3780
gcagagcgtc agcaggagta taaagagaaa ctccaaggga tatgtgatct tcttacccaa 3840
actgaaaacc gcctaattgg tcaccaagaa gccttcatga ttggagatgg cacagttgag 3900
ttaaagaaat atcagtccaa gcaagaggaa ttacagaaag atatgcaagg aagtgcacag 3960
gcattggctg aagtagtgaa aaacacagag aacttcttaa aagagaatgg tgaaaagctg 4020
tcacaggaag ataaggcttt gattgaacag aaacttaatg aagctaagat aaagtgtgaa 4080
cagcttaatt taaaagcaga acagtctaaa aaggagctgg ataaagttgt gacaacagca 4140
atcaaggaag aaactgaaaa ggtagcagca gtgaagcagc tggaagagag caaaaccaaa 4200

atagaaaacc ttttggactg gttgtcaaat gttgacaaag actcagaaag ggcagggaca 4260
aaacacaaac aggtaatcga acagaacggg acccattttc aagaaggtga tggcaagtca 4320
gcaattggag aagaggatga agttaatggt aacctgttgg agactgatgt tgatgggcaa 4380
gttggaacca ctcaggagaa tctgaatcag caatatcaga aagttaaggc ccaacacgag 4440
aagatcatct ctcagcacca agcagtgatc atagccactc agtctgcaca agtgttgctt 4500
gagaaacagg gtcagtatct ctctcctgaa gaaaaagaga aactgcaaaa gaacatgaag 4560
gaactgaaag tgcattatga aactgcactg gctgagtcag agaagaaaat gaagttaact 4620
cactctctgc aggaagaatt agaaaagttt gatgctgact ataccgagtt tgagcactgg 4680
ctacagcagt cagaacaaga acttgaaaac ctggaggcag gtgcagatga catcaatggt 4740
ttaatgacca aattgaagag gcagaagagt ttctcagaag acgttatttc tcacaaaggt 4800
gacttgagat acatcacaat ttctggaaac agagtgttgg aagctgccaa atcttgcagc 4860
aagagagacg gtggcaaggt tgatacttct gcaacccaca gagaagtgca gcgcaagttg 4920
gatcatgcta ctgatcggtt caggtctctc tactctaagt gcaatgttct tggaaataat 4980
cttaaagatc tggtggataa atatcaacac tatgaagatg cttcatgtgg acttcttgct 5040
ggactccagg cctgtgaggc cacagcgagc aaacacttat ctgaacctat tgcggtggac 5100
cccaaaaatc ttcaaaggca attagaagag accaaggctt tgcaaggaca gatatcaagt 5160
cagcaggttg ctgtagagaa actgaagaaa acggctgaag tgcttttaga tgccagggga 5220
tctttacttc cagccaagaa tgatatccag aaaacacttg atgacattgt tgggagatat 5280
gaggatctgt ccaagtctgt taatgaacga aatgaaaaac tccagataac cttgacccgt 5340
tcactgagtg tgcaggatgg cttggatgaa atgctggact ggatgggaaa tgtggaaagt 5400
tctctgaaag aacaaggtca agtgccttta aactctactg ctcttcagga tattatcagt 5460
aaaaacatta tgttggaaca ggatattgca ggtcgtcaga gcagtataaa tgccatgaat 5520
gaaaaagtga agaaatttat ggaaacaaca gatccatcca ctgcatcctc actgcaagcc 5580
aaaatgaaag acttgtctgc tcggttttca gaagcaagtc ataaacacaa agaaactctt 5640
gccaaaatgg aggagctaaa aaccaaagta gaactgtttg agaacctctc agaaaagctc 5700
cagacatttt tagaaacaaa aactcaggct cttactgaag tagatgtgcc aggaaaagat 5760
gttactgaat tgtctcagta tatgcaggaa tcaacttctg aatttttaga acacaagaaa 5820
catcttgaag ttttgcatag tctcttgaag gagatatcca gccatggctt accaagtgat 5880
aaggctttgg tccttgaaaa aacaaataat ttgtcaaaga aattcaagga aatggaggat 5940
accatcaaag aaaaaaaaga agctgtaact tcttgtcaag aacagttgga tgctttccaa 6000
gttcttgtta aatcattgaa gtcatggata aaagaaacaa caaaaaaagt tcccattgta 6060
cagccttctt ttggtgcaga ggatttagga aaatctttgg aagacactaa gaaattacaa 6120
gaaaagtgga gtttaaaaac accagagatt cagaaagtaa acaacagtgg gatctcacta 6180
tgtaacttaa taagtgctgt gaccaccccc gcaaaggcaa tagcagcagt taaatcaggt 6240
ggagcagtat taaatggtga aggaacagcc acaaatactg aggaattttg ggcaaataaa 6300
ggtttaacat ccattaaaaa ggacatgact gacataagtc atggttatga agatcttggc 6360
ctcttactca aggacaaaat agcggaactg aacactaaac tctccaaatt gcaaaaggct 6420
caggaagaat caagtgcaat gatgcagtgg ttacagaaaa tgaacaaaac tgcaacaaaa 6480
tggcagcaga cacctgcacc tacagatact gaagctgtga agactcaagt tgagcagaat 6540
aagtcgtttg aggcagaact gaagcagaat gtaaacaaag tacaggagtt gaaagacaaa 6600
ttgacagagc tgttggagga gaacccagat actcctgagg cccccagatg gaaacagatg 6660
ttgacagaaa tagattctaa gtggcaagaa ctcaatcaat taacaattga tagacaacaa 6720
aaactggaag aatcctccaa taatctaacc cagttccaga ctgtagaggc ccaattgaaa 6780
cagtggcttg tggaaaaaga acttatggtc agtgttcttg ggcccttgtc aattgaccca 6840
aatatgctaa acacacaaag gcagcaggtg cagattttgc tgcaagaatt cgccactcgg 6900
aaacctcaat atgaacagct gacagcagct ggtcagggca ttctgagcag gcctggagaa 6960
gacccttctt tacgtgggat tgtgaaagag caactggcag ctgtgaccca aaaatgggat 7020
agcctaacag ggcaattgag tgacagatgt gactggattg accaagccat tgttaaaagc 7080
acacagtatc aaagcctgct gagaagcctt tctgataaac tgagtgactt ggataataaa 7140
ctcagcagca gtctggctgt gagcacgcac cctgatgcta tgaaccaaca gttggaaaca 7200
gcccaaaaaa tgaagcagga gatacagcag gaaaagaagc agataaaagt ggcccaggca 7260
ctctgtgagg atttgtcagc actggttaaa gaagagtact tgaaagcaga acttagtagg 7320
caactagaag gcatcttaaa atcatttaag gatgttgaac agaaagcaga gaatcatgtc 7380
cagcaccttc agtcggcctg tgcaagctct catcaatttc agcaaatgtc tagagatttt 7440
caggcttggc tggatacaaa gaaagaagag caaaacaaat ctcatccaat atctgccaaa 7500
ctcgatgtct tggagtcatt aattaaagat cataaagact ttagtaaaac tttgaccgct 7560
cagtctcata tgtatgaaaa aaccattgca gaaggtgaaa atctgttatt aaaaacacaa 7620
gggtctgaga aggcagcctt acagttacag cttaatacaa ttaaaaccaa ttgggataca 7680
tttaataagc aggtgaaaga aagagaaaac aagttaaaag agtcattgga aaaagccctt 7740

aagtataaag agcaagtaga gactctctgg ccatggatag acaaatgcca aaacaacctg 7800
gaggaaataa aattttgctt ggatcctgct gaaggagaga attctattgc caagttaaag 7860
tctctgcaga aggaaatgga ccaacacttt ggtatggtag aattactgaa caacacagcc 7920
aatagcttgc tcagtgtctg tgagatagat aaagaagttg ttacagatga gaataagtca 7980
ctgatccaga aggtggacat ggtcactgaa caacttcaca gtaagaaatt ctgtctggag 8040
aacatgactc agaagtttaa agaatttcaa gaagtttcca aagaatctaa aaggcagctt 8100
cagtgtgcaa aggagcagct agatatccat gattcgctgg gatcccaggc ttacagtaac 8160
aaatacctga ccatgttgca aactcagcag aaatcacttc aggccttgaa gcatcaggta 8220
gatttggcta aaagacttgc acaggacctt gtggtagagg cctcagactc aaagggaacc 8280
tctgatgttt tattacaagt ggaaaccata gctcaagagc atagtacact aagtcagcag 8340
gttgatgaaa agtgttcttt cttagaaacc aagcttcagg gcattgggca tttccagaat 8400
accattcgag aaatgttttc tcagttcgca gagtttgatg atgaactgga tagcatggct 8460
ccagtgggga gagatgcaga aacattgcaa aagcaaaagg aaactataaa agcctttcta 8520
aagaaactag aagccctcat ggcaagcaat gacaatgcca ataaaacctg caagatgatg 8580
ttagccacag aagaaacctc tcctgacctt gttggaatca aaagggactt ggaggcctta 8640
agcaaacaat gcaacaagtt actggaccga gcccaagcca gagaagagca ggttgaaggg 8700
acaattaagc gccttgaaga attttacagc aaattgaaag aattttctat tctgctccag 8760
aaagccgaag aacatgaaga gtcacaaggt cctgttggta tggaaacgga gacaattaat 8820
cagcagctta acatgttcaa ggtattccag aaagaagaga ttgaaccctt gcaaggtaaa 8880
cagcaagatg taaactggtt aggtcaaggc cttattcaga gtgctgccaa aagcactagc 8940
actcagggct tggagcatga cctggatgat gtcaatgcac ggtggaagac tctcaataag 9000
aaggtggctc agcgagcagc ccagctgcag gaggccttgc tgcactgtgg gaggttccag 9060
gatgccctgg agtccctgct cagctggatg gtggacactg aggagcttgt ggccaatcag 9120
aagcccccgt cggctgagtt caaagtggta aaggcccaga tacaagaaca aaagcttctc 9180
cagagattgt tggatgaccg aaaatctacg gtggaggtaa tcaaacgaga aggagaaaaa 9240
attgctacaa cagcagagcc cgcagataaa gtgaagattt tgaaacagct cagtctcttg 9300
gatagcagat gggaggcatt gcttaataaa gctgaaacaa ggaatcgtca gttggaaggt 9360
atctcggtgg tagcacagca atttcatgaa accttagaac cactgaacga gtggcttaca 9420
accatagaaa agaggctggt gaattgtgaa cccataggaa cccaagcatc taaacttgag 9480
gaacaaattg cacagcacaa agccttagaa gatgacatca tcaatcacaa taaacattta 9540
caccaggctg ttagcattgg ccagtcctta aaggttttga gctccaggga ggataaagat 9600
atggtgcaga gtaaattaga cttctctcaa gtatggtaca ttgagattca agagaaaagt 9660
cacagcaggt ctgagctcct ccagcaggcc ttatgtaatg ctaagatttt tggggaagat 9720
gaagttgaac tgatgaactg gctgaatgaa gtgcatgaca aactgagcaa gctctcagtc 9780
caggattaca gcactgaggg gctatggaag cagcagtctg aacttcgggt tctgcaagag 9840
gacatcttac tcaggaaaca aaatgtagat caggctttac taaatggttt agaactactt 9900
aaacaaacca caggtgatga agttttaata attcaagata aattggaagc cattaaagca 9960
aggtacaaag acattactaa actgagcact gatgtggcca agactctgga acaggcgctg 10020
cagcttgcaa ggcggctgca ctccacacac gaagagctgt gtacctggct ggacaaagtg 10080
gaggtggaat tactttcata tgaaactcag gttctgaaag gagaagaagc aagtcaagca 10140
caaatgagac caaaggaact gaaaaaggaa gctaagaaca acaaagcctt actggactcc 10200
cttaatgaag tgagcagtgc tttgctggaa ctggtaccat ggagggcaag agaaggactt 10260
gagaaaatgg tagctgagga caatgagcgc taccgattag tgagcgacac catcactcag 10320
aaggtggagg agatcgatgc agccattctg cgatcacagc agtttgacca agcagctgat 10380
gctgagttat cctggattac tgaaacagaa aaaaaattga tgtctctggg tgacatcagg 10440
cttgagcaag accagacttc tgctcagctt caagttcaaa agacattcac catggagatt 10500
ttgagacaca aggatattat tgatgacctt gttaaatctg ggcataaaat catgaccgca 10560
tgcagtgaag aggaaaagca atcaatgaag aaaaaactgg acaaggtact gaagaactat 10620
gataccatct gccagattaa ttcagaaagg tatctgcagc tggaacgggc acagtccctg 10680
gttaaccaat tctgggaaac atatgaagaa ctttggccat ggctgacaga aacacaatca 10740
atcatctctc agcttcccgc cccagccctt gaatatgaaa ctctaaggca gcagcaggaa 10800
gaacatcggc aactgcgtga gttgatagct gaacacaagc ctcatataga taagatgaac 10860
aaaactgggc cacagttact ggaattgagc cctggggaag gcttttctat ccaagagaag 10920
tatgtggcag ccgacaccct ttacagtcaa attaaagaag atgtcaaaaa gcgtgctgtg 10980
gcactggatg aagccatttc tcaatcaact cagttccatg acaagataga tcagatcctt 11040
gagagcctgg aacgcatcgt ggaacgtctg aggcagccac cctctatctc tgcagaggtt 11100
gagaagatca aggaacagat cagtgaaaat aagaatgtgt cagtagacat ggaaaagcta 11160
cagccgttgt atgaaactct taaacagagg ggagaggaaa tgattgctag atctgggggg 11220
actgataaag acatatctgc caaagctgtt caggataagc ttgaccaaat ggttttcatt 11280

tgggagaaca tacacacact ggtggaagag agggaagcca aactactgga tgtgatggag 11340
ctagcagaaa agttctggtg tgatcacatg tcattgatag ttaccattaa agatactcaa 11400
gatttcatcc gggacctgga agatcctgga attgatcctt cagtagtaaa acaacagcaa 11460
gaagcagcag agaccataag ggaagaaata gatggactac aggaggagct ggatatagtt 11520
attaacctag gttctgaact cattgcggca tgtggggagc ctgataaacc cattgtcaag 11580
aagagtatag atgagttaaa ttcagcatgg gattctctaa ataaagcttg gaaagaccgg 11640
attgacaaac ttgaggaggc aatgcaggct gccgttcagt accaggatgg actgcaggcg 11700
gtatttgact gggtagatat tgcaggtggt aaattagctt caatgtctcc aattggaaca 11760
gatctcgaaa ctgtcaagca gcagattgaa gagctaaagc aatttaagtc tgaggcctat 11820
caacagcaga tagaaatgga aagactgaat catcaagcag agcttttgct aaagaaagta 11880
acagaagaga gtgacaaaca cactgttcaa gacccattaa tggaactgaa attgatatgg 11940
gatagcctgg aggagagaat catcaacaga cagcataaac tggagggtgc tctattagcc 12000
ttgggtcagt tccaacatgc cctggatgag ctcctggcat ggctgacaca caccgagggc 12060
ttgctaagtg agcagaaacc tgttggagga gaccctaaag ccattgaaat tgaacttgcc 12120
aagcatcatg tgctccaaaa tgatgtatta gcccatcagt ccacagtgga agccgttaat 12180
aaagcaggaa atgatctaat tgaatcaagt gcaggagaag aagcaagcaa ccttcagaac 12240
aagctagagg ttttaaatca acgctggcaa aatgttttgg aaaaaacaga acaaaggaag 12300
cagcagctgg atggtgcctt gcgccaggcc aaagggttcc atggcgaaat tgaggatttg 12360
cagcagtggc tgactgacac ggagcgtcat ctgttggcat ctaaaccgct gggaggttta 12420
ccggaaacag ccaaggagca gcttaatgtc catatggaag tctgtgctgc ctttgaagct 12480
aaagaagaaa catataagag tctgatgcag aaaggccagc agatgcttgc aagatgccca 12540
aaatctgcag agacaaatat tgaccaagac ataaataact tgaaagaaaa atgggaatcg 12600
gtggaaacca aactcaatga aaggaaaact aaactggaag aggctctcaa cttggcaatg 12660
gagttccaca attctctcca agacttcatc aactggctta ctcaggctga acagacccta 12720
aatgtagctt ctcggccaag tctcatcttg gacacagtct tatttcaaat tgacgaacac 12780
aaggtttttg ccaatgaagt aaattctcat cgtgagcaga taatagagct ggacaaaact 12840
ggaacccacc taaaatattt tagtcagaaa caagatgttg ttctaatcaa gaatctactt 12900
atcagtgtac aaagtcgatg ggaaaaagtg gttcaacggt tggtagagag aggaagatct 12960
ttggatgatg caaggaagag agccaagcag ttccatgaag cttggagtaa acttatggag 13020
tggctagaag agtcagaaaa gtctttggat tctgaactgg aaatcgcaaa tgatccagac 13080
aaaataaaaa cacaacttgc acaacataag gagtttcaga aatcactcgg agccaagcat 13140
tctgtctacg acaccaccaa caggactgga cgttctctga aggagaaaac ctccctggct 13200
gatgacaacc tgaaactgga tgacatgctg agtgaactca gagacaaatg ggataccata 13260
tgtggaaaat ctgtggaaag acaaaacaaa ttggaggaag ccctgttatt ttctggacaa 13320
ttcacagatg ccctacaggc tctcattgat tggttatata gagttgaacc ccagctggca 13380
gaagaccagc ctgttcatgg agacattgat ttggtgatga atctgatcga taatcacaag 13440
gccttccaaa aagagttggg gaagaggacc agcagtgtgc aggccctgaa gcgctcagcc 13500
cgagaactca tagaaggcag tcgggatgac tcctcctggg tcaaggtcca gatgcaggaa 13560
ttaagcacac gctgggagac cgtgtgtgca ctttctatat caaagcaaac acggttagaa 13620
gcagccctgc gtcaggcaga ggaattccac tcggtggtac atgccctctt ggagtggctg 13680
gctgaggcgg agcaaaccct gcgtttccat ggtgtcctcc cagatgatga ggatgctctc 13740
cggactctca ttgatcagca taaagaattc atgaagaaac tggaagaaaa gagagctgaa 13800
ctaaataaag ccaccactat gggcgacacc gttttggcta tctgccaccc cgactccatc 13860
actaccatta agcactggat aacaatcatc cgggcgaggt ttgaggaggt gctggcctgg 13920
gcaaagcaac atcagcagag attagcaagt gctctggctg ggcttattgc caaacaggaa 13980
ttgttggaag ctttgctggc ttggttgcaa tgggctgaaa ctacacttac tgataaggat 14040
aaagaagtca tcccccagga gatcgaagag gtgaaagcac tcattgcaga acaccagacc 14100
ttcatggagg aaatgaccag aaaacagcct gatgttgata aagtaacgaa gacctataag 14160
aggagagctg ctgatccttc ctcattacaa tcccatattc cagtcttgga taaggacgga 14220
gcaggaagaa aacgctttcc agcatcaagc ttgtatccct ctgggtcaca gacacaaatt 14280
gaaaccaaaa atcctagggt aaacttactg gtgagcaaat ggcagcaagt ctggctcctg 14340
gcgttggaaa gaaggaggaa actcaatgat gccttggaca gactagagga gctgagggaa 14400
tttgctaact ttgattttga tatctggcgc aaaaaataca tgcgatggat gaatcacaag 14460
aaatctcgag tgatggactt cttcaggaga attgataaag accaggatgg gaaaataacg 14520
cggcaggaat ttattgatgg aattctttcc tcaaagtttc caaccagtcg cttggagatg 14580
agcgcagttg cagacatctt tgacagagat ggcgatggat atattgacta ctatgaattt 14640
gtagcagccc ttcacccaaa taaagatgca tataaaccta tcacagatgc cgacaaaatc 14700
gaagatgagg tgacaaggca ggtagctaag tgtaaatgtg caaagcgatt tcaagttgag 14760
cagattggtg ataataaata cagggtaaag gttctttctt tcttcactga acagtttgga 14820

gactcccagc aactgcgact ggtccggatc ctgcggagta ctgtgatggt tcgtgttgga 14880
ggtggatgga tggcacttga tgagttctta gtgaaaaatg atccttgcag ggccaaagga 14940
aggacaaaca tggaactgcg tgagaagttc attttagcag atggtgccag ccagggtatg 15000
gctgctttcc gaccccgagg ccgaagatcc cggccatcat cacgaggcgc ttcacccaac 15060
agatccactt ctgtgtccag tcaggctgcg caggcggcct ccccacaggt ccctgccacc 15120
accacaccca agattctcca tcctttaaca cgcaattatg gtaaaccatg gttgacaaac 15180
agcaaaatgt caactccttg taaagcagca gagtgctcag actttcccgt gccatctgca 15240
gagggaacgc caatacaagg aagcaagctt cgacttccag gatatttatc agggaaaggc 15300
ttccactctg gggaggacag tggcttgata acaactgcag ctgccagagt ccgaacacag 15360
tttgctgatt ccaagaagac tcccagccga ccaggaagtc gagctggaag caaagctggc 15420
agcagggcca gcagccgccg aggcagtgat gcatcagact ttgacatttc agaaatccag 15480
tccgtgtgct cagatgtgga aactgtcccc cagacacaca gacctacacc ccgagcaggt 15540
tctcggccat ccacagcgaa gccttcaaaa atccccacgc cccagaggaa atcacctgcc 15600
agcaaattgg acaagtcctc aaagagatag tgcaattggt tctaccaagg cccttccttg 15660
agcatttatt atttaagttt gaacgatgta aaatatggtg tagaaattct tgtgaaatat 15720
tgcaagaggc gagtttaaaa ttctgcagat ggccttattt gtgtatttgt ctttttattt 15780
tatctgtata attttttttg tcagatattc tggggttaaa gtcacatcat atgtgaggag 15840
gaaaagttta acatgaacta acatttctgc actgtaacgt gccgggcaca cactaaactc 15900
agttactgta cctacaggta agtctacatc ctctctgaca gccacagcac tacatcaatc 15960
cctgacgtta gggatacctc atgacatttt cctgttttta tggaaactct gagaagctga 16020
atgatacatg caggggatat tttttgagat gatttaaatg taaaccaaaa gatggaagac 16080
aaaaagacaa acacacccac acgcagtctt tgcagtatct gacagagaac tcacaggaag 16140
ttacttcaag cacttgccag tactatgata ttcaagtacc ttgcagcatt tctctgccat 16200
tgctttcaat gaggccagag gcatcctgga tattagacct attatactgt aagaatataa 16260
gtataaagtg cgttcatata catgtgaggt tttcttttgc ttgagtggac agtagcacct 16320
gtatcattga actcattttg tatcagagca attttgcttg cagaaagcta tgaaataaaa 16380
cacgtccctt aactgc 16396

<210> 148
<211> 1751
<212> DNA
<213> unknown

<220>
<223> >DCN (HUGO ID) DECORIN PRECURSOR (BONE
      PROTEOGLYCAN II) (PG-S2) (PG40). [Source:
      SWISSPROT (P07585)]

<400> 148
tttaactgtg ctatggagta gaagcaggag gttttcaacc tagtcacaga gcagcaccta 60
ccccctcctc ctttccacac ctgcaaactc ttttacttgg gctgaatatt tagtgtaatt 120
acatctcagc tttgagggct cctgtggcaa attcccggat taaaaggttc cctggttgtg 180
aaaatacatg agataaatca tgaaggccac tatcatcctc cttctgcttg cacaagtttc 240
ctgggctgga ccgtttcaac agagaggctt atttgacttt atgctagaag atgaggcttc 300
tgggataggc ccagaagttc ctgatgaccg cgacttcgag ccctccctag gcccagtgtg 360
ccccttccgc tgtcaatgcc atcttcgagt ggtccagtgt tctgatttgg gtctggacaa 420
agtgccaaag gatcttcccc ctgacacaac tctgctagac ctgcaaaaca acaaaataac 480
cgaaatcaaa gatggagact ttaagaacct gaagaacctt cacgcattga ttcttgtcaa 540
caataaaatt agcaaagtta gtcctggagc atttacacct ttggtgaagt tggaacgact 600
ttatctgtcc aagaatcagc tgaaggaatt gccagaaaaa atgcccaaaa ctcttcagga 660
gctgcgtgcc catgagaatg agatcaccaa agtgcgaaaa gttactttca atggactgaa 720
ccagatgatt gtcatagaac tgggcaccaa tccgctgaag agctcaggaa ttgaaaatgg 780
ggctttccag ggaatgaaga agctctccta catccgcatt gctgatacca atatcaccag 840
cattcctcaa ggtcttcctc cttcccttac ggaattacat cttgatggca acaaaatcag 900
cagagttgat gcagctagcc tgaaaggact gaataatttg gctaagttgg gattgagttt 960
caacagcatc tctgctgttg acaatggctc tctggccaac acgcctcatc tgagggagct 1020
tcacttggac aacaacaagc ttaccagagt acctggtggg ctggcagagc ataagtacat 1080

ccaggttgtc taccttcata acaacaatat ctctgtagtt ggatcaagtg acttctgccc 1140
acctggacac aacaccaaaa aggcttctta ttcgggtgtg agtcttttca gcaacccggt 1200
ccagtactgg gagatacagc catccacctt cagatgtgtc tacgtgcgct ctgccattca 1260
actcggaaac tataagtaat tctcaagaaa gccctcattt ttataacctg gcaaaatctt 1320
gttaatgtca ttgctaaaaa ataaataaaa gctagatact ggaaacctaa ctgcaatgtg 1380
gatgttttac ccacatgact tattatgcat aaagccaaat ttccagttta agtaattgcc 1440
tacaataaaa agaaattttg cctgccattt tcagaatcat cttttgaagc tttctgttga 1500
tgttaactga gctactagag atattcttat ttcactaaat gtaaaatttg gagtaaatat 1560
atatgtcaat atttagtaaa gcttttcttt tttaatttcc aggaaaaaat aaaaagagta 1620
tgagtcttct gtaattcatt gagcagttag ctcatttgag ataaagtcaa atgccaaaca 1680
ctagctctgt attaatcccc atcattactg gtaaagcctc atttgaatgt gtgaattcaa 1740
tacaggctat g 1751

<210> 149
<211> 870
<212> DNA
<213> unknown

<220>
<223> >CD47 (HUGO ID) LEUKOCYTE SURFACE ANTIGEN CD47
      PRECURSOR (ANTIGENIC SURFACE DETERMINANT PROTEIN
      OA3) (INTEGRIN ASSOCIATED PROTEIN) (IAP) (MER6).
      [Source: SWISSPROT (Q08722)]

<400> 149
ggatcagctc agctactatt taataaaaca aaatctgtag aattcacgtt ttgtaatgac 60
actgtcgtca ttccatgctt tgttactaat atggaggcac aaaacactac tgaagtatac 120
gtaaagtgga aatttaaagg aagagatatt tacacctttg atggagctct aaacaagtcc 180
actgtcccca ctgactttag tagtgcaaaa attgaagtct cacaattact aaaaggagat 240
gcctctttga agatggataa gagtgatgct gtctcacaca caggaaacta cacttgtgaa 300
gtaacagaat taaccagaga aggtgaaacg atcatcgagc taaaatatcg tgttgtttca 360
tggttttctc caaatgaaaa tattcttatt gttattttcc caatttttgc tatactcctg 420
ttctggggac agtttggtat taaaacactt aaatatagat ccggtggtat ggatgagaaa 480
acaattgctt tacttgttgc tggactagtg atcactgtca ttgtcattgt tggagccatt 540
cttttcgtcc caggtgaata ttcattaaag aatgctactg gccttggttt aattgtgact 600
tctacaggga tattaatatt acttcactac tatgtgttta gtacagcgat tggattaacc 660
tccttcgtca ttgccatatt ggttattcag gtgatagcct atatcctcgc tgtggttgga 720
ctgagtctct gtattgcggc gtgtatacca atgcatggcc ctcttctgat ttcaggtttg 780
agtatcttag ctctagcaca attacttgga ctagtttata tgaaatttgt ggcttccaat 840
cagaagacta tacaacctcc tagggtaagt 870

<210> 150
<211> 941
<212> DNA
<213> unknown

<220>
<223> beta2-microglobulin

<400> 150
tcctgaagct gacagcattc gggccgagat gtctcgctcc gtggccttag ctgtgctcgc 60
gctactctct ctttctggcc tggaggctat ccagcgtact ccaaagattc aggtttactc 120
acgtcatcca gcagagaatg gaaagtcaaa tttcctgaat tgctatgtgt ctgggtttca 180
tccatccgac attgaagttg acttactgaa gaatggagag agaattgaaa aagtggagca 240
ttcagacttg tctttcagca aggactggtc tttctatctc ttgtactaca ctgaattcac 300
ccccactgaa aaagatgagt atgcctgccg tgtgaaccat gtgactttgt cacagcccaa 360
gatagttaag tgggatcgag acatgtaagc agcatcatgg agggtttgaa gatgccgcat 420
ttggattgga tgaattccaa attctgcttg cttgcttttt aatattgata tgcttataca 480
cttacacttt atgcacaaaa tgtagggtta taataatgtt aacatggaca tgatcttctt 540

tataattcta ctttgagtgc tgtctccatg tttgatgtat ctgagcaggt tgctccacag 600
gtagctctag gagggctggc aacttagagg tggggagcag agaattctct tatccaacat 660
caacatcttg gtcagatttg aactcttcaa tctcttgcac tcaaagcttg ttaagatagt 720
taagcgtgca taagttaact tccaatttac atactctgct tagaatttgg gggaaaattt 780
agaaatataa ttgacaggat tattggaaat ttgttataat gaatgaaaca ttttgtcata 840
taagattcat atttacttct tatacatttg ataaagtaag gcatggttgt ggttaatctg 900
gtttattttt gttccacaag ttaaataaat cataaaactt g 941

<210> 151
<211> 484
<212> DNA
<213> unknown

<220>
<223> >RPL27 (HUGO ID) 60S RIBOSOMAL PROTEIN L27.
      [Source: SWISSPROT (P08526)]

<400> 151
tccttctttc ctttttgctg gtagggccgg gtggttgctg cagaaatggg caagttcatg 60
aaacctggga aggtggtgct tgtcctggct ggacgctact ccggacgcaa agctgtcatc 120
gtgaagaaca ttgatgatgg cacctcagat cgcccctaca gccatgctct ggtggctgga 180
attgaccgct acccccgcaa agtgacagct gccatgggca agaagaagat cgccaagaga 240
tcaaagataa aatcttttgt gaaagtgtat aactacaatc acctaatgcc cacaaggtac 300
tctgtggata tccccttgga caaaactgtc gtcaataagg atgtcttcag agatcctgct 360
cttaaacgca aggcccgacg ggaggccaag gtcaagtttg aagagagata caagacaggc 420
aagaacaagt ggttcttcca gaaactgcgg ttttagatgc tttgttttga tcattaaaaa 480
ttat 484

<210> 152
<211> 605
<212> DNA
<213> unknown

<220>
<223> RPS16 (HUGO ID) 40S RIBOSOMAL PROTEIN S16.
      [Source: SWISSPROT (P17008)]

<400> 152
aaggcgcctg cgcagaccct gaaaagcggc cagggtggcc cctagctttc cttttccggt 60
tgcggcgccg cgcggtgagg ttgtctagtc cacgctcgga gccatgccgt ccaagggccc 120
gctgcagtct gtgcaggtct tcggacgcaa gaagacagcg acagctgtgg cgcactgcaa 180
acgcggcaat ggtctcatca aggtgaacgg gcggcccctg gagatgattg agccgcgcac 240
gctacagtac aagctgctgg agccagttct gcttctcggc aaggagcgat ttgctggtgt 300
agacatccgt gtccgtgtaa agggtggtgg tcacgtggcc cagatttatg ctatccgtca 360
gtccatctcc aaagccctgg tggcctatta ccagaaatat gtggatgagg cttccaagaa 420
ggagatcaaa gacatcctca tccagtatga ccggaccctg ctggtagctg accctcgtcg 480
ctgcgagtcc aaaaagtttg gaggccctgg tgcccgcgct cgctaccaga aatcctaccg 540
ataagcccat cgtgactcaa aactcacttg tataataaac agtttttgag ggattttaaa 600
gtttc 605

<210> 153
<211> 2054
<212> DNA
<213> unknown

<220>
<223> NM_018479 (RefSeq ID) UNCHARACTERIZED HYPOTHALAMUS
      PROTEIN HCDASE. [Source: RefSeq (NM_018479)]

<400> 153
   agtcaccacc gtggcctgcg acgaaatggc gaaaagtctt ttgaagacag cctctctgtc 60
   tggaaggaca aaattgctac atcaaacagg attgtcactt tatagtacat cccatggatt 120
   ttatgaggaa gaagtgaaaa aaacacttca gcagtttcct ggtggatcca ttgaccttca 180
   gaaggaagac aatggcattg gcattcttac tctgaacaat ccaagtagaa tgaatgcctt 240
   ttcaggtgtt atgatgctac aacttctgga aaaagtaatt gaattggaaa attggacaga 300
   ggggaaaggc ctcattgtcc gtggggcaaa aaatactttc tcttcaggat ctgatctgaa 360
   tgctgtgaaa tcactaggaa ctccagaggc aattttttct tcaatataca gacttccttt 420
   aataagtgtt gcgctggttc aaggttgggc attgggtgga ggagcagaat ttactacagc 480
   atgtgatttc aggttaatga ctccagagag taagatcaga ttcgtccaca aagagatggg 540
   cataatacca agctggggtg gcaccacccg gctagttgaa ataatcggaa gtagacaagc 600
   tctcaaagtg ttgagtgggg cccttaaact ggattcaaaa aatgctctaa acataggaat 660
   ggttgaagag gtcttgcagt cttcagatga aactaaatct ctagaagagg cacaagaatg 720
   gctaaagcaa ttcatccaag ggccaccgga agtaattaga gctttgaaaa aatctgtttg 780
   ttcaggcaga gagctatatt tggaggaagc attacagaac gaaagagatc ttttaggaac 840
   agtttggggt gggcctgcaa atttagaggc tattgctaag aaaggaaaat ttaataaata 900
   attggttttt cgtgtggatg tactccaagt aaagctccag tgactaatat gtataaatgt 960
   taaatgatat taaatatgaa catcagaatt actttgaagg ctactattaa tatgcagact 1020
   tacttttaat catttgaata tctgaactca tttacctcat ttcttgccaa ttactcactt 1080
   gggtatttac tgcgtaatct ggaacattta gctaaaatat acacttttgg cttaaaaatt 1140
   attgctgtca attccaataa taattcttag cttataacca aagagcagtg tttaaaagga 1200
   gagcttctat acaaaaccta ttcctggcgt tacttttcat acaatttttg ttctgtttta 1260
   cctggaaata atttaccaaa ataactgagt gttgctgcta aagaacaaaa gtggggaggt 1320
   atcagggaac aagaaaacaa gaaagggtat gatcaatcat tttcttctgc tccaaacagc 1380
   tggagtaaaa ttcatgggaa atggcccttc atttaaaaaa agatgtacct cactacccac 1440
   tacaaatttg gaactttgtt cttttcaata attagttttc tattgtaaat tacctactaa 1500
   acagtggtag ccatgacatg gaaagtcaac tgattctaca attggacatt catttgtgtg 1560
   ccctggaatt tccaactagt aataaacaac tactgttgat gtagttttaa accacttgaa 1620
   gggactcatg aagcatcctg caacataaat ttgcattttt acatcagatt tctttttttt 1680
   cctgaaaaac aactaacctt ctaacaacta tctttcaaaa gtaaatgtaa taaaaatgca 1740
   caacataaaa tgtttatgat cccagcaata cactttttaa aaaatgtgaa agtcaaagaa 1800
   ttaagttcta gttctgactc atcacaagag gtcaaaagta tttgctactg taacattcaa 1860
   ttcacatttg agaatcatgg taaaaataac ttgcatttgc cttaccatca tgatcctact 1920
   gttgagttag gaaaatatgg ttagacagac tcacattact ttttttcaga ggtaaactct 1980
   agattactgt gtcaacccaa tactatttgg ccatagatgt aaaaactacc aaataaaagt 2040
   ggattttgtg gtct 2054

<210> 154
<211> 170
<212> DNA
<213> unknown

<220>
<223> >OIC1 AJ617288

<400> 154
gtaaagcgta tttggttatt cctttgtgtt ctcaattttc tggacagtaa gatgtgcaag 60
aagtgtttgt attctcatgg ggcatgtgga acaggtaact atacccccaa caactcccac 120
cccatctctt catcttgcac acacaacacc aatcaccatc atgtctttcc 170

<210> 155
<211> 2037
<212> DNA
<213> unknown

<220>
<223> >Chr 5 clone (EST) ENSESTT00000023592

<400> 155

cactaggagg gaggatgcta cgatttcaga cctcagctat taaatagtaa atctggaaaa 60
ctagtggata aatttgccat ctccattcta tgttattaag accaagatag tgtactggtt 120
ttcttttaaa tcacattcca ttatgtcatt taaaatgtca caaaacccac acgaagcatc 180
tcatttacac taagatgctt atttagctaa caccactaat gataaatgct gttacaaata 240
tgcatagtca gatgatagta accacataca gaaaaaaaat ttgaacaagt atttatttct 300
taaaatttac ttaagggatt agagctaata tataatagaa catttaatat aacatttgga 360
gttatgtcaa cataaaaata gctgtggtta caattagcac atgcaattca ctgcaaaggt 420
aaaaatacat gctatactct agacaagcct tccaaatgaa gttagagtag atggggtaaa 480
acagcaagtg aacatgaaag gattgcactt agaagaaagt gggacatagc taggatataa 540
aagaaacata cctaatgcta gtcagtcact gcattgtcct actagcaaat tgcacattta 600
tttttagagt atattcaata cacatacata ttgagactag agaattttca aatatctacc 660
tttgaaatat ccctttgttt ctaacacatc acattatggt attaatgtaa cagcacttaa 720
aacctgtagt tatcattcaa agcttgtgtt caagagataa aagaacatca ttcagacaaa 780
gcctgtgttc aagaaataaa aaaaacatct atcactatcg gccttaaatg catctcatca 840
cacgacccat caactttaat gaaatgggta atgaagttaa actaaccagt ttgtacaaac 900
tattgactga gaaaaagccc ttaaaaattt aggaacaaat gagcaacaga agagctcacc 960
ctgagctgcc accttttaat ttttaatgca atgtatatga gaatatcatg ctaatgcaca 1020
tactagtagc atgtatgatg gaaaaaaata ggttaatgca aaagataata cgcttggctt 1080
tgtaaagttt tgttttgact tacatcatct gaattctgtt tatcgagtct gaaaaggaac 1140
tgctgccaag gaccagtcca aagaagaatt aggctgctaa tcctaaaaca cttgccattt 1200
tatgactcca tattctttta atttacagaa aagtacaaat tttttagaaa gtaatcttat 1260
cagcacaata taatctaaca atacctgtaa tagaaagcta tcccagtaaa ttttttttga 1320
acaattgaac ttttgtctta tgcagcttat aactttgtta ttttattcat ctcaacaaaa 1380
taaaacttta ttgaaacaaa agtgtatggt ttaggaatat gctcttaata acaactgagc 1440
aaaattagaa tgatgtggac tataacagaa tgaaggaaga tctctcctgc ttgatgtact 1500
tggtcaatat atgttagtaa acagaaatat tttagggaat tctatgatta ttctacttct 1560
acatttacat gtcatggttt taactagtta aatgggtaga aaaaagacat ctttaataat 1620
tgatttctat ttctcaaact agatgctcta ctttaaatat gagctttaat gaatgttttg 1680
taacatttta aagttgtaac atttctaagt atactccact aacttcacaa taagagctag 1740
ggtcactgga tgacttcatg tggtaaaatc ccttatcaag gaataaggct gaaatttaaa 1800
tgaacaatta ggtagagaag aggtatgaac acagaacatg tgaatgaaat ttgctttttt 1860
tccttgctgc tgttcccgag tgccgactga tccatagtaa aaaaggacaa gattaaaatt 1920
ttaggtttta atggtacaaa gtgtgaagtt ttatagtttt ctaataatta ttcagtatta 1980
tccctttcag agatgagggt aggataccac agacatcagt aactgacaag ttataat 2037

<210> 156
<211> 228
<212> DNA
<213> unknown

<220>
<223> >Chromosome 16 clone (EST)    No ESTs or genes

<400> 156
cattctgtta gtgcagagct acaaatctta tttgttacat agctcaaaga catcttccac 60
aggaagaaaa atacctgcca ttagtaaatt cttaaccggt atgaacctat gcttctattt 120
taaagaggtt agtgcacata tgccttctaa atatctcata aaaaaccacc gtagcacatt 180
taaaatcctg gtattattac ttaataccac cctacaacac aaaaatca 228

<210> 157
<211> 166
<212> DNA
<213> unknown

<220>
<223> >Chromosome-1 clone    hypothetical protein XP_211586

<400> 157
gacatgtctc actgtttgta tttctctctc ctgtcaaagt gaatgatata ttcttgaaaa 60

cccccaattt cttgaaatgg gtgttctgtt cattcatggg caggggtcag gattgaagtt 120
catatatgaa acaactgggg atgtagatag gaaagaatgt ctgctg 166

<210> 158
<211> 536
<212> DNA
<213> unknown

<220>
<223> >gi|5446595|gb|AI825924|sr|IMAGp998F065401.1|cl|IM
      AGp998F065401|AI825924 to92d03.x1 NCI_CGAP_Gas4
      Homo sapiens cDNA clone IMAGE:2185733 3', mRNA
      sequence.

<221> misc_feature
<222> 495
<223> n=a, t, c, or g

<400> 158
cacttttttc attttttttt attaatcaaa tgatacaaaa caaccatcat tctgtcaatg 60
cccaagcacc cagctggtcc tctccccaca tgtcacactc tcctcagcct ctcccccaac 120
cctgctctcc ctcctcccct gccctagccc agggacagag tctaggagga gcctggggca 180
gagctggagg caggaagaga gcactggaca gacagctatg gtttggattg gggaagagat 240
taggaagtag gttcttaaag accctttttt agtaccagat atccagccat attcccagct 300
ccattattca aatcatttcc catagcccag ctcctctctg ttctccccct actaccaatt 360
ctttggctct tacacaattt ttatccctca aatattcatc cctggcccaa ccagtcccct 420
gagcctccct ctggtggaga ctcctccacc catgagctcc ccagagcatc caagacagag 480
tgcacagaga cctgnggaag gaagctgaac tttgcagaga tgaggacagg tgcagg 536

<210> 159
<211> 699
<212> DNA
<213> unknown

<220>
<223> >gi|4534249|gb|AI570875|sr|IMAGp998J065499.1|cl|IM
      AGp998J065499|AI570875 tr68d03.x1 NCI_CGAP_Pan1
      Homo sapiens cDNA clone IMAGE:2223461 3' similar
      to gb:M35252 TUMOR-ASSOCIATED ANTIGEN CO-029
      (HUMAN);, mRNA sequence

<221> misc_feature
<222> 526, 538, 549, 631, 632, 637, 652, 654, 661
<223> n=a, t, c, or g

<400> 159
ttcaccaacg taaacagtta cttttatttt gagtaaaaat acacattcat atcattttcc 60
cttatatccc tcaaatctta aatgtgttca atatgtctag aagatatctg tggtctagct 120
agccgagaca ttttaaaaag acagctgctc ctgacttata tagcacttac atatttaaat 180
ttacaaagcc aaagcaacat tttaaagggg tttgactgac gatagcttga tgcatccaca 240
gattcatttg ttcccgatct ggcaatacag gaccatagaa aacaccaaac ccagtatctc 300
aataactgcc agtccaaatg ctattccaat aactataatc aaattttttg ccaagaagtc 360
ttttatgaaa gaaatacagg tctctttgta aacttgtttt ccattatagc tttggcatgg 420
tctctgctta tctagacagg cacataattc aggataggtt gaaaattatt tcccaatcag 480
cagctccatt gaccaaaccg cagcatttaa actcttcttg aaacancatt atggctcncc 540
tggaatgtnt tcacttttcc ctgtggcgct caaaaaagcc ttgtgttcat atgagagtca 600
tttccacaat cgatcagact tagattgaaa nncagtncta tggtatcctg cncncccttc 660
ncggggagca gatagaggca agcctttgaa aacacaaca 699

<210> 160
<211> 717
<212> DNA
<213> unknown

<220>
<223> >sr|IMAGp998A05535.2|cl|IMAGp998A05535|gb|BX114906
      |oid|234564189 BX114906 Soares fetal liver spleen
      1NFLS Homo sapiens cDNA clone IMAGp998A05535 ;
      IMAGE:247564, mRNA sequence

<221> misc_feature
<222> 696, 702
<223> n=a, t, c, or g

<400> 160
ctttatcttg agtttcatgt cttagggttg gagaagggac caagcctccc ctgaaagctg 60
aggggtttat ggtcaagaag aaagagtaac tatggccacc tttaggtcac cagccaataa 120
tactgcttct ctatcctgtt ctgctgtgac atttgtccca gcaggtgttc cagtctgtcc 180
tacagtagtg agaccacctg atatggtgag gaaggcaggt aattgatctt actccatcat 240
atcatcacat ctatttccac cccccgccct tggagacaag gtctcactcc atcatttagg 300
ccggaatgca gtggtgcaat catagctcat tgtagtctca aacacctggg cccaagtgat 360
cctcactgta gtctcccaag tagctaggac tacaggcaca tgccaccaca cctggctaat 420
taaaaaaaat tttttttgca cagagatggg ggtcttgata tgttgcccag gctggtctgg 480
aactcctggc cacaagtgat cctcccacct cagcctccca aagcactggg attactgttg 540
tgagccacca tgaccagccc catcacttct tttgtaggtt tgtatttgtt tttatttttg 600
acaaagactt ttatttcacg gtgacttctt cacaagtttc ctcagatgct agtctgaaca 660
aaggagagca gggaactaga aatgtagact ttctgntttt tncagctatg gcatcac 717

<210> 161
<211> 600
<212> DNA
<213> unknown

<220>
<223> >gi|1382358|sr|IMAGp998B19790|cl|IMAGp998B19790|gb
      |W72422|W72422 zd65c10.s1 Soares fetal heart
      NbHH19W Homo sapiens cDNA clone 345522 3' similar
      to SW:SC13_YEAST Q04491 PROTEIN TRANSPORT PROTEIN
      SEC13. [1]

<221> misc_feature
<222> 530
<223> n=a, t, c, or g

<400> 161
tataagcaat atgactttat ttagttcctt tggaaacaaa accccccaaa taatgcctga 60
acccaaaggt acataaaaat gacccaaaat agatttgaac atcacttgta gtttcttcct 120
cgtaacatga gtgctttcag ctggacagta gattacaaag catctccgat cacgttaagg 180
cagatgatca atctgtggct gcatctgtaa ctcctcctgg gaaaataatc ctgttggagt 240
tgggggctct tcccagttgt ctggttagtt ggcccaggaa ggggaagtcc tggagctggc 300
gggtggggag ccaggcccca cctgtcttgt cactgctccg ttctgctggc cctctgtcac 360
ttgatgctga tacggagccc tggcccttgt tgacatcact gatgcacacc cactgcccat 420
caactgactc cttccacagg gtcaccttat tgtctccacc agagacagcc aggatgttgg 480
ctgtgatgga caagctcaca tgccacacca catcgttgaa cttgtgcaan atttagggga 540
ccacgtattg cttgaggcat catcacaggt ccaaatgaac acacgaacat cctgggagca 600

<210> 162

<211> 202
<212> DNA
<213> unknown

<220>
<223> >gi|4648553|gb|AI623622|sr|IMAGp998M105514.1|cl|IM
      AGp998M105514|AI623622 ts22b05.x1 NCI_CGAP_Pan1
      Homo sapiens cDNA clone IMAGE:2229297 3', mRNA
      sequence.

<400> 162
tttttttttt tttttttttt tttttttttt tttttttttt tttttttttt tttttttttt 60
tttttggaaa aaaagggctt tgtttggtcc ctttttaacc ccccgaattt ggttttcccc 120
cattttggag gttttttttt ttcccccccc cccccccccg gttttaaacc gggggcgccc 180
cccctttttt tttttttttt gg                                          202

<210> 163
<211> 407
<212> DNA
<213> unknown

<220>
<223> >gi|4984008|gb|AI696108|sr|IMAGp998O095541.1|cl|IM
      AGp998O095541|AI696108 tt03e05.x1 NCI_CGAP_GC6
      Homo sapiens cDNA clone IMAGE:2239712 3' similar
      to contains L1.t3 L1 repetitive element ;, mRNA
      sequence

<400> 163
gagacataac aatatttaat gtgtatgtgc ctgacaacag agtataaaaa tatgtgaggc 60
aaaacccata gaaatatgag gagaaataaa tgcatacagt atcataattg acttcaacac 120
tccaacagaa atggacagat ccagtaggca gaaaatctgt atgtgcatag ctaaaattaa 180
ccataccatc aatcaaatag atataatgga catctactga ctacttcatc caacaacagc 240
agaatacata tttttctcaa gctcacatga aacattcaac aaaatagact gtattttggg 300
ccttcagaat caccttaaac acattttaaa cataaaaatt acataatgtc tgggtgtctg 360
accacaatgg acttgaacta gaaatcaata acagaaaaat agctgag               407

<210> 164
<211> 380
<212> DNA
<213> unknown

<220>
<223> >gi|2139170|gb|AA434256|sr|IMAGp998D121896.1|cl|IM
      AGp998D121896|AA434256 zw24h06.s1 Soares ovary
      tumor NbHOT Homo sapiens cDNA clone IMAGE:770267
      3', mRNA sequence

<400> 164
cgaatgaatt gatcagtctt tattgatgat tattacattt ttcatttgcc tggaacttac 60
cacaccctaa agtattctgg ctctccatat tttctgttca taagaatatg cagagcagtt 120
gattatcttc tgggtccttt agccctttag ctctcctgag ggtagcagag gactccctgg 180
ttttcctttg gatggaagtt tcctgacttt ctacttctgg aaccagggcc agtgtccttg 240
acatgggctt cctccaggat gaaatcggac aactgcccaa tgacagcaag taaagaaagt 300
atactcgcct ataatcccag cactttggga ggccaaggcg ggtggatcac gaggtcagct 360
ggataacata ctggctaacg                                             380

<210> 165
<211> 479

<212> DNA
<213> unknown

<220>
<223> >sr|IMAGp998H235725.2|cl|IMAGp998H235725|gb|BX1120
      57|oid|234558491 BX112057 NCI_CGAP_GC6 Homo
      sapiens cDNA clone IMAGp998H235725 ;
      IMAGE:2310214, mRNA sequence

<400> 165
   ccgatcgcct cggaagcccc gtagaccatc acggacgccg agcttccagt aactcacagt 60
   gaaagcttgt ttaatgaagc tagcttcaat gctgtgagct gctctataga cgcccatgta 120
   ggaggaaact caaggggccc ccagccacta gccattgagg aactgaggcc ctcaatccaa 180
   caacccacaa ggaactgaat cctgccaaca accactgagt gagcctggaa gcggatcctg 240
   cccagctgaa actcgagaag attgtagctc tgctgccctg accactattc taactgcacc 300
   cttgagagag accctcagcc agctaatctg tacctggatt cctaacctgg agaaactgaa 360
   atagcaacgc atgttgatgt aagtgctaag tttgggagta atttgttatg taacaataaa 420
   taactaataa tacttattat ttaggcaata aaaccataga aaataaaaaa aaaaaaaaa  479

<210> 166
<211> 716
<212> DNA
<213> unknown

<220>
<223> >sr|IMAGp998P03372.1|cl|IMAGp998P03372|gb|BX118749
      |oid|234571874 BX118749 Soares fetal liver spleen
      1NFLS Homo sapiens cDNA clone IMAGp998P03372 ;
      IMAGE:195698, mRNA sequence

<400> 166
ttttttttta gagttttaga ggaatataat gtatgaaaga aaattccaat aacataatgt 60
acaaaaatgt tgtcacatac agaagagcaa aaatctacgt attggggact attgctgtgg 120
gaagaagggc ctgatcttca tactcatctt cctcattgag taccatgacc ccttccaatt 180
catccatact acaccatcat ctgtgccatg ctttgccatg tcccaggtgt actgtccacc 240
ccagtagtat ctgccgtttg gattggctgc atgacatcta ttataccacc atccaccacc 300
gtcttcttta gaacactgtt ttctgggatc tgatgttaac ctgaaagaaa atgtgtttgg 360
cgttattaga aacagaacta cggtggtcaa gttacgtttt gcagattacc cataaaagac 420
tgctacactc gtgtgcacag tagtctttcc tttgggtgct atacatagag tcacactaat 480
acaccaaaga cctcctgtgc cctatagcaa gaaagagaga taactgggcc tttgtggcca 540
atccatctag ggactgtgtt tgttacttta tactggaata atagctttta caacccttag 600
tatcagagaa ccctggaaat gaaaatgaaa gttttcacac acactttaga atatagagat 660
ataggtattt atctaaatgc tcattattca gaaagtaact gtgctcaaat tctagt     716

<210> 167
<211> 747
<212> DNA
<213> unknown

<220>
<223> >sr|IMAGp998B071870.3|cl|IMAGp998B071870|gb|BX0956
      83|oid|234525737 BX095683
      Soares_total_fetus_Nb2HF8_9w Homo sapiens cDNA
      clone IMAGp998B071870 ; IMAGE:760230, mRNA
      sequence

<221> misc_feature
<222> 737
<223> n=a, t, c, or g

<400> 167
ccgcatccta gccgccgact cacacaaggc aggtgggtga ggaaatccag agttgccatg 60
gagaaaattc cagtgtcagc attcttgctc cttgtggccc tctcctacac tctggccaga 120
gataccacag tcaaacctgg agccaaaaag gacacaaagg actctcgacc caaactgccc 180
cagaccctct ccagaggttg gggtgaccaa ctcatctgga ctcagacata tgaagaagct 240
ctatataaat ccaagacaag caacaaaccc ttgatgatta ttcatcactt ggatgagtgc 300
ccacacagtc aagctttaaa gaaagtgttt gctgaaaata aagaaatcca gaaattggca 360
gagcagtttg tcctcctcaa tctggtttat gaaacaactg acaaacacct ttctcctgat 420
ggccagtatg tccccaggat tatgtttgtt gacccatctc tgacagttag agccgatatc 480
actggaagat attcaaatcg tctctatgct tacgaacctg cagatacagc tctgttgctt 540
gacaacatga agaaagctct caagttgctg aagactgaat tgtaaagaaa aaaaatctcc 600
aagcccttct gtctgtcagg ccttgagact tgaaaccaga agaagtgtga gaagactggc 660
tagtgtggaa gcatagtgaa cacactgatt aggttatggt ttaatgttac aacaactatt 720
ttttaagaaa aacaagnttt agaaatt 747

<210> 168
<211> 435
<212> DNA
<213> unknown

<220>
<223> >gi|1425401|sr|IMAGp998D08822|cl|IMAGp998D08822|gb
      |W95494|W95494 ze02h04.s1 Soares fetal heart
      NbHH19W Homo sapiens cDNA clone 357847 3'

<221> misc_feature
<222> 387, 432
<223> n=a, t, c, or g

<400> 168
taatatgcaa accatttatt gaactattta ctattttaaa atatcattct taaaagaggc 60
cacaaaaatt ataacattca gaaattttag gtaaactcca aaagtaagct tttttttttt 120
ttggtagtca acttgtacca agtttagcag caagaggata cttccttaga gactttcagt 180
ggacttaaac tcagtttccg ctggtgctat gtaaagcatc cacgatggtt ttattgtact 240
ctgcaatctg cttggtcaca tttttcttaa ttggctggta atcactctct tgactcttgg 300
ttgctatgaa ttctttcatc gcaaaattat tttggctcaa ggtgttgcca ctttctctcc 360
aaatttgtaa gctgagaatg gtggtcncat tttcttggca attgtgtttt tagtggcctc 420
atactctatg tnttt 435

<210> 169
<211> 410
<212> DNA
<213> unknown

<220>
<223> >gi|1423675|sr|IMAGp998I08822|cl|IMAGp998I08822|gb
      |W94545|W94545 ze04b04.s1 Soares fetal heart
      NbHH19W Homo sapiens cDNA clone 357967 3'

<400> 169
cccaacgttt tcaagtttta tttttaaaat atattacaac atcacattaa aaacagtaga 60
ggaaaacaag caaacatgat agaattactt ttttccaact caatagtact tagctttacc 120
tagaattttc atacagttga tgctaacttc ttctgaaccc tttctgacta gtattttacc 180
ttatgtattc tttatagtat ttccatataa tctttaccga gacctattca gagcataaca 240
tagacagggc agcacaagca actgacacct ttgttggtac tacagtgttt tttcaaccag 300
tttatgagag aatgaaaaga atgaaagtga aaattttact taacttaggg ttatgtagct 360
ctaggtaatc tcttcagaac tcccctggct tcaccttctc aagttaatca 410

<210> 170
<211> 132
<212> DNA
<213> unknown

<220>
<223> >ImageID=357848 IMAGP971C0935_S1 CC   FromEXP of
      IMAGp971C0935_s1.exp April 26, 2002 12:43

<221> misc_feature
<222> 15, 33, 73, 107, 123, 127
<223> n=a, t, c, or g

<400> 170
caagcatcac cctgngaggt tcctgaggga ttnctcataa atgagggctg cacattgcct 60
gttctgcttc gangtattca ataccgctca gtattttaaa tgaagcngat tctaagattt 120
ggntggngat ca                                                     132

<210> 171
<211> 390
<212> DNA
<213> unknown

<220>
<223> >sr|IMAGp998K045001.2|cl|IMAGp998K045001|gb|BX1091
      64|oid|234552707 BX109164 Barstead prostate BPH
      HPLRB4 1 Homo sapiens cDNA clone IMAGp998K045001 ;
      IMAGE:2032251, mRNA sequence.

<400> 171
atccacccag tctctcaaga ccaggaacga gaaaaaagaa gtatcagtga cagcgatgaa 60
ttagcttcag ggttttttgt gttcccttac ccatatccat ttcgcccact tccaccaatt 120
ccatttccaa gatttccatg gtttagacgt aattttccta ttccaatacc tgaatctgcc 180
cctacaactc cccttcctag cgaaaagtaa acaagaagga aaagtcacga taaacctggt 240
cacctgaaat tgaaattgag ccacttcctt gaagaatcaa aattcctgtt aataaaagaa 300
aaacaaatgt aattgaaata gcacacagca ttctctagtc aatatcttta gtgatcttct 360
ttaataaaca tgaaagcaaa aaaaaaaaaa                                  390

<210> 172
<211> 322
<212> DNA
<213> unknown

<220>
<223> >gi|5366932|gb|AI801460|sr|IMAGp998K125400.1|cl|IM
      AGp998K125400|AI801460 to89f06.x1 NCI_CGAP_Gas4
      Homo sapiens cDNA clone IMAGE:2185475 3' similar
      to contains element MER22 repetitive element ;,
      mRNA sequence

<221> misc_feature
<222> 278, 279
<223> n=a, t, c, or g

<400> 172
tttttttttt tttttttttt tttttttttt tttttttttt tttttttttt tttttttttt 60
tttttttttt tttttttttt tttttttttt ttttaaaaaa aaaaggggcc ccctgggggg 120
gaaacaaatt tggttccccc cccggccccc tgaaacccaa tttttaaaac tgggtttttt 180
taaaaaaaag ggggggggcc caaaaaccca accccaaaag ggggggtgcc ttttgccccc 240

cccccccccg gtttttcccc ctttttttgg gttaaaannc aagggccccc aaaaaaaagg 300
gggggggga tttttttttt tt 322

<210> 173
<211> 460
<212> DNA
<213> unknown

<220>
<223> >gi|2069924|gb|AA410759|sr|IMAGp998K151776.2|cl|IM
      AGp998K151776|AA410759 zt35e08.r1 Soares ovary
      tumor NbHOT Homo sapiens cDNA clone IMAGE:724358
      5' similar to gb:Y00503 KERATIN, TYPE I
      CYTOSKELETAL 19 (HUMAN);, mRNA sequence

<400> 173
cccttggacc ataaattttt attggcaggt caggagaaga gccgggggta agggtccctt 60
ccttcccatc cctctaccca gaagacaccc tccaaaggac agcagaagcc ccagagcctg 120
ctgcctcaga ggaccttgga ggcagacaaa ttgttgtagt gatcttcctg tccctcgagc 180
aggctgcggt aggtggcaat ctcctgctcc agccgcgact tgatgtccat gagccgctgg 240
tactcctgat tctgccgctc actatcagct cgcacatcgc ccagctgggc ttcaataccg 300
ctgatcagcg cctggatatg cgccacgtgg gctccaaagc gcgcctccgt ttctgccagt 360
gtgtcttcca aggcagcttt catgctcagc tgtgactgca gctcaatctc aagaccctga 420
agggtgcgcc gcaggtcagt aacctcggac ctgctcatct 460

<210> 174
<211> 797
<212> DNA
<213> unknown

<220>
<223> >gi|5054911|gb|AI733798|sr|IMAGp998I164581.2|cl|IM
      AGp998I164581|AI733798 qk35b08.x5 NCI_CGAP_Co8
      Homo sapiens cDNA clone IMAGE:1870935 3' similar
      to gb:X02761_cds1 FIBRONECTIN PRECURSOR (HUMAN);,
      mRNA sequence

<221> misc_feature
<222> 621, 665
<223> n=a, t, c, or g

<400> 174
tttttttttt ggggaatgga aatcttttat taaaacagtt gtctttccac agtagtaaag 60
ctttggcaca tacagtataa aaaataatca cccaccataa ttataccaaa ttcctcttat 120
caactgcata ctaagtgttt tcaatacaat tttttccgta taaaaatact gggaaaaatt 180
gataaataac aggtaagaga aagatatttc taggcaatta ctaggatcat ttggaaaaag 240
tgagtactgt ggatatttaa aatatcacag taacaagatc atgcttgttc ctacagtatt 300
gcgggccaga cacttaagtg aaagcagaag tgtttgggtg actttcctac ttaaaatttt 360
ggtcatatca tttcaaaaca tttgcatctt ggttggctgc atatgctttc ctattgatcc 420
caaaccaaat cttagaatca cttcatttaa aatactgagc ggtattgaat acttcgaagc 480
agaacaggca atgtgcagcc ctcatttatg agaaaaccct caggaaactc ccagggtgat 540
gcttggagaa gctgtgagtg agctgaagct ggagaacttc ctccagagca aagggcttaa 600
gaaagaaaga agaactccta ngctggtctg ctaccattca ctcagtttaa aatgatcctg 660
caganagaca atcttgtcct tttggaattg gaaagagatt acctctcgga atcctctctg 720
tcagcctgac atctaaaggc atgaagcact caatttggca ataaaacaaa tagggttggt 780
ctctgatgga tctctga 797

<210> 175
<211> 408

<212> DNA
<213> unknown

<220>
<223> >sr|IMAGp998F044825.2|cl|IMAGp998F044825|gb|BX1062
      84|oid|234546949 BX106284 NCI_CGAP_Ov23 Homo
      sapiens cDNA clone IMAGp998F044825 ;
      IMAGE:1964547, mRNA sequence

<221> misc_feature
<222> 81, 254, 338, 378, 403
<223> n=a, t, c, or g

<400> 175
tttttttttt ttttaaacat cagattttct atttttatta aaaactcaca aatttattca 60
acatgttttc tttcatacag ngaatggtct aatatgcact ggaggtcaca caagcttagg 120
tttattagaa caataaaaga catatgagaa atttaatata taaagaaaaa gtagcagctg 180
ttgactgcat atttgaccat aaaatttaaa tattttggac ttttatttta aagacacaaa 240
aataaaacct gtgngggtct atataagtca tattaacaat tccatgaatg ttcaacagga 300
caaaaaaatt agcaaagatg ttttttttta aatcttgnaa cacttttttt tttttttttt 360
tttaacactt tctcaggntg ctggggccag gcccctttac agnatttg 408

<210> 176
<211> 293
<212> DNA
<213> unknown

<220>
<223> >gi|5593910|gb|AI888746|sr|IMAGp998L206082.1|cl|IM
      AGp998L206082|AI888746 wn22h10.x1 NCI_CGAP_Gas4
      Homo sapiens cDNA clone IMAGE:2446243 3' similar
      to contains element MSR1 repetitive element ;,
      mRNA sequence.

<400> 176
tttttttttt tttttttttt tttttttttt tttttttttt tttttttttt tgggtttttt 60
ttttttttaa aaaagggctt aaatttaaaa ttctcaaaaa aaacaaaaaa aaaaaaaccc 120
cttgggtcca agccaaaacc ccgggaaaac ccccctaaaa cagggcgggg ggccctacaa 180
aaaaaacaaa aaagggccaa gggaaattta aaacatttaa atccttaaag gtccaccagg 240
aaaaaggggg cccccccccc gggaaatcat tttaaaaaaa aacaccgggc att 293

<210> 177
<211> 404
<212> DNA
<213> unknown

<220>
<223> >sr|IMAGp998D085368.2|cl|IMAGp998D085368|gb|BX1070
      55|oid|234548491 BX107055 Barstead colon HPLRB7
      Homo sapiens cDNA clone IMAGp998D085368 ;
      IMAGE:2173015, mRNA sequence.

<221> misc_feature
<222> 9
<223> n=a, t, c, or g

<400> 177
ctagtaatna ctagtaatat tactagtgaa ttcactagta atgccagagc caacctggac 60
ttcctgtcat tttcacaatc ttggggctga tgaagaaggg ggtgggggga gatctacagg 120

agctcagcat ggcatctgtg atattcctgc ccaacataca tgacagaatc caatcatgga 180
aaagcaccaa aaaacccaag ttgaaggaca agtctaccaa gtagctggcc aggtaatctt 240
cacaaaggtc cgtgtcatga aagacaaagc agcagaaact ttcaaattag aggggacagc 300
taaatgcaac acatggtctt ggattgtttt gttataaagg acattatcat gaagtctgaa 360
taaagtctgt agatgagatg agagaattcc ctgtttttag gaaa 404

<210> 178
<211> 270
<212> DNA
<213> unknown

<220>
<223> >sr|IMAGp998D20611.1|cl|IMAGp998D20611|gb|BX115533
      |oid|234565441 BX115533
      Soares_multiple_sclerosis_2NbHMSP Homo sapiens
      cDNA clone IMAGp998D20611 ; IMAGE:276835, mRNA
      sequence

<221> misc_feature
<222> 267, 270
<223> n=a, t, c, or g

<400> 178
atattaacat aattatgttg gagagaagaa aaacccacac ttagaagtca ggtttctttt 60
aaaactcctt gttacaaata gaatcaatga ttatatctca gacataataa tgggtcaaac 120
tcacaaaatc agtgctacaa aatacagtat ttttccatca cttttagttg taatgtacac 180
tatgtacaga aagataatac tacctatttt aaactaaata tagatgaaca aacagcttgt 240
taaaaaccag aatcagggct tataaanagn 270

<210> 179
<211> 669
<212> DNA
<213> unknown

<220>
<223> >sr|IMAGp998B14742.3|cl|IMAGp998B14742|gb|BX100503
      |oid|234535382 BX100503 Soares_fetal_heart_NbHH19W
      Homo sapiens cDNA clone IMAGp998B14742 ;
      IMAGE:327085, mRNA sequence

<221> misc_feature
<222> 558
<223> n=a, t, c, or g

<400> 179
tagtgttttc ttaaaataag actgatttaa agcttatcta gcagatagtt ttaaacacag 60
attaaagaaa actagattgg gtacaaatgt ttggcttccc aagtgagagg ccacatgtac 120
atcaacagta ctttccaact taacactacg acccccattt tgatggtgac agaattggaa 180
acgggggaca gtgatgagag catcaccctc ctgtcactta cagatggtcc cttgggactg 240
gatttttcct gggagaccat tgaccccaag aaccctgaaa acatgatatg cggtagcaga 300
atggattaga ttgctgttag ctttctggta ggtgagggga cctgaaaaga acagggccac 360
cagcatttca agggaacagc cattccaatt ttcagaggtt gattctcttg gaattatttg 420
acctgaggcc ataggaaagc aaagggttaa aaaagtcacg tttgacatgc cagcctgggg 480
agctggacat tggatacccg tgtgagcctc gtggaacacc ataatcaaat gtgaaaatta 540
gagttgatta attaaagnaa aagaatttaa gtctttattt ctaaactgat gcatgaggac 600
ccccagtgag caatattccc ccaaggcaaa tcaaccaaaa gcaaaactgt tttgaaaaaa 660
aaaaaaaaa 669

<210> 180

<211> 711
<212> DNA
<213> unknown

<220>
<223> >sr|IMAGp998B07407.1|cl|IMAGp998B07407|gb|BX111875
      |oid|234558127 BX111875 Soares fetal liver spleen
      1NFLS Homo sapiens cDNA clone IMAGp998B07407 ;
      IMAGE:208806, mRNA sequence

<221> misc_feature
<222> 676
<223> n=a, t, c, or g

<400> 180
ctcattcttg gcaggatggc ttctcatcgt ctgctcctcc tctgccttgc tggactggta 60
tttgtgtctg aggctggccc tacgggcacc ggtgaatcca agtgtcctct gatggtcaaa 120
gttctagatg ctgtccgagg cagtcctgcc atcaatgtgg ccgtgcatgt gttcagaaag 180
gctgctgatg acacctggga gccatttgcc tctgggaaaa ccagtgagtc tggagagctg 240
catgggctca caactgagga ggaatttgta gaagggatat acaaagtgga aatagacacc 300
aaatcttact ggaaggcact tggcatctcc ccattccatg agcatgcaga ggtggtattc 360
acagccaacg actccggccc ccgccgctac accattgccg ccctgctgag cccctactcc 420
tattccacca cggctgtcgt caccaatccc aaggaatgag ggacttctcc tccagtggaa 480
ctgaaggacg agggatggga tttcatgtaa ccaagagtat tccattttta ctaaagcagt 540
gttttcacct catatgctat gttagaagtc caggcagaga caataaaaca ttcctgtgaa 600
aggcactttt cattccactt taacttgatt ttttaaattc ccttattgtc ccttccaaaa 660
aaaagagaat caaaanttta caaagaatca aaggaattct agaaagtatc t          711

<210> 181
<211> 474
<212> DNA
<213> unknown

<220>
<223> >sr|IMAGp998C16522.3|cl|IMAGp998C16522|gb|BX097461
      |oid|234529295 BX097461 Soares fetal liver spleen
      1NFLS Homo sapiens cDNA clone IMAGp998C16522 ;
      IMAGE:242631, mRNA sequence

<400> 181
aggacagaga cgctggctag gccgccctcc ccactgttac caacatgaag ctgctcgcag 60
caactgtgct actcctcacc atctgcagcc ttgaaggagc tttggttcgg agacaggcaa 120
aggagccatg tgtggagagc ctggtttctc agtacttcca gaccgtgact gactatggca 180
aggacctgat ggagaaggtc aagagcccag agcttcaggc cgaggccaag tcttactttg 240
aaaagtcaaa ggagcagctg acacccctga tcaagaaggc tggaacggaa ctggttaact 300
tcttgagcta tttcgtggaa cttggaacac agcctgccac ccagtgaagt gtccagacca 360
ttgtcttcca accccagctg gcctctagaa cacccactgg ccagtcctag agctcctgtc 420
cctacccact ctttgctaca ataaatgctg aatgaatcca aaaaaaaaaa aaaa       474

<210> 182
<211> 462
<212> DNA
<213> unknown

<220>
<223> >gi|4739393|gb|AI655414|sr|IMAGp998O055540.1|cl|IM
      AGp998O055540|AI655414 ts98e03.x1 NCI_CGAP_GC6
      Homo sapiens cDNA clone IMAGE:2239324 3' similar
      to gb:S70057 GASTRIN/CHOLECYSTOKININ TYPE B

RECEPTOR (HUMAN);, mRNA sequence.

<400> 182
ttcaggagga agccaaacaa tttttatttc ttgaacagtt tgggtaagga aggagagggc 60
agggccacgg tccttaactg gaaagggatg aagaaccttt tcagtgcaga acagcctgtt 120
ggtcagaggt atgagattag gcctgacggt attttcagtg ctgattggga agggtcactg 180
tgaggtcaaa ctaggagcat cccagagtca gtcctgtgcc aggctccata gtctctaaaa 240
caatcagtgc cattaatcta tgtgtgtgag aggcaggtca gcccttgtca gagttcccac 300
tgtgtagaaa tagttcactg ctagggacgc tgtgtctctc caaggtcagt gtcatgtttc 360
tatgggccag gctcagtccc atatcagagg catgcctcct ttcccatgta ctgctccatt 420
cttattcctc ttgtgtcagg taagctacct tttcctgtgc gt 462

<210> 183
<211> 466
<212> DNA
<213> unknown

<220>
<223> >sr|IMAGp998A244944.2|cl|IMAGp998A244944|gb|BX0909
      80|oid|234516327 BX090980 NCI_CGAP_GC6 Homo
      sapiens cDNA clone IMAGp998A244944 ;
      IMAGE:2010143, mRNA sequence

<400> 183
atggcttaca tattcaaaag gttggatttg ggaagcaatg ctaagcagtg gaatggacat 60
cgacatagag agatcagctc cacacttata ctctgccact caacttcccc atgtgacttg 120
aggatcactc taactccaaa acatagcaag ctcgcggaac atcagggttc atgcaaagta 180
ttccaaggag ccccttgaag caacagaatg gattgctctt ctatggtgga atggcaccct 240
ggatgattaa aaccgtagca gcaaacaaaa cctccatcaa gtaagaattc agagtgtgag 300
atatcacgca cagccacgcg tggatcttta tatacgtgtc aatgtgtttg attgtatttt 360
tgctttcaaa gtatgtattg agcatttctt ctaggtcctc agtaacatct ttttttaaaa 420
aaaataaatg cttagggaat tgttttatat taaaaaaaaa aaaaaa 466

<210> 184
<211> 16966
<212> DNA
<213> unknown

<220>
<223> >Homosapiens
       chromosome:NCBI34:4:74736257:74753222:1

<400> 184
agcttttctc ttctgtcaac cccacacgcc tttggcacaa tgaagtgggt aacctttatt 60
tcccttcttt ttctctttag ctcggcttat tccaggggtg tgtttcgtcg agatgcacgt 120
aagaaatcca tttttctatt gttcaacttt tattctattt tcccagtaaa ataaagtttt 180
agtaaactct gcatctttaa agaattattt tggcatttat ttctaaaatg gcatagtatt 240
ttgtatttgt gaagtcttac aaggttatct tattaataaa attcaaacat cctaggtaaa 300
aaaaaaaaaa ggtcagaatt gtttagtgac tgtaattttc ttttgcgcac taaggaaagt 360
gcaaagtaac ttagagtgac tgaaacttca cagaataggg ttgaagattg aattcataac 420
tatcccaaag acctatccat tgcactatgc tttatttaaa aaccacaaaa cctgtgctgt 480
tgatctcata aatagaactt gtatttatat ttattttcat tttagtctgt cttcttggtt 540
gctgttgata gacactaaaa gagtattaga tattatctaa gtttgaatat aaggctataa 600
atatttaata atttttaaaa tagtattctt ggtaattgaa ttattcttct gtttaaaggc 660
agaagaaata attgaacatc atcctgagtt tttctgtagg aatcagagcc caatattttg 720
aaacaaatgc ataatctaag tcaaatggaa agaaatataa aaagtaacat tattacttct 780
tgttttcttc agtatttaac aatccttttt tttcttccct tgcccagaca agagtgaggt 840
tgctcatcgg tttaaagatt tgggagaaga aaatttcaaa gccttgtaag ttaaaatatt 900
gatgaatcaa atttaatgtt tctaatagtg ttgtttatta ttctaaagtg cttatatttc 960

cttgtcatca gggttcagat tctaaaacag tgctgcctcg tagagttttc tgcgttgagg 1020
aagatattct gtatctgggc tatccaataa ggtagtcact ggtcacatgg ctattgagta 1080
cttcaaatat gacaagtgca actgagaaac aaaaacttaa attgtattta attgtagtta 1140
atttgaatgt atatagtcac atgtggctaa tggctactgt attggacagt acagctctgg 1200
aacttgcttg gtggaaagga ctttaatata ggtttccttt ggtggcttac ccactaaatc 1260
ttctttacat agcaagcatt cctgtgctta gttgggaata tttaattttt tttttttttt 1320
aagacagggt ctcgctctgt cgcccaggct ggagtgcagt ggcgcaatct cggctcactg 1380
caaactccgc ctcccgggtt cacgccattc tcctgcctca gcctcccgag tagctgggac 1440
tacaggcgcc cgccatcacg cccggctaat cttttgtatt tttagtagag atggggtttc 1500
accgtgtgcc aggatggtct caatctcctg acatcgtgat ctgcccacct cggcctccca 1560
aagtgctggg attacaggag tgagccaccg cgcccggcct atttaaatgt tttttaatct 1620
agtaaaaaat gagaaaattg tttttttaaa agtctaccta atcctacagg ctaattaaag 1680
acgtgtgtgg ggatcaggtg cggtggttca cacctgtaat cccagcactt tggaaggctg 1740
atgcaggagg attgcttgag cccaggagtt caagaccagc ctgggcaagt ctctttaaaa 1800
aaaacaaaac aaacaaacaa aaaaattagg catggtggca catgcctgta gtcctagcta 1860
cttaggaggc tgacgtagga ggatcgtttg gacctgagag gtcaaggcta cagtgagcca 1920
tgattgtgcc actgcactcc agcctgggtg acagagtgag actctgtctc aaaaaagaaa 1980
aaggaaatct gtggggtttg ttttagtttt aagtaattct aaggacttta aaaatgccta 2040
gtcttgacaa ttagatctat ttggcataca atttgcttgc ttaatctatg tgtgtgcata 2100
gatctactga cacacgcata catataaaca ttagggaact accattctct ttgcgtagga 2160
agccacatat gcctatctag gcctcagatc atacctgata tgaataggct ttctggataa 2220
tggtgaagaa gatgtataaa agatagaacc tatacccata catgatttgt tctctagcgt 2280
agcaacctgt tacatattaa agttttatta tactacattt ttctacatcc tttgtttcag 2340
ggtgttgatt gcctttgctc agtatcttca gcagtgtcca tttgaagatc atgtaaaatt 2400
agtgaatgaa gtaactgaat ttgcaaaaac atgtgttgct gatgagtcag ctgaaaattg 2460
tgacaaatca cttgtaagta cattctaatt gtggagattc tttcttctgt ttgaagtaat 2520
cccaagcatt tcaaaggaat tttttttaag ttttctcaat tattattaag tgtcctgatt 2580
tgtaagaaac actaaaaagt tgctcataga ctgataagcc attgtttctt ttgtgataga 2640
gatgctttag ctatgtccac agttttaaaa tcatttcttt attgagacca aacacaacag 2700
tcatggtgta tttaaatggc aatttgtcat ttataaacac ctctttttaa aatttgaggt 2760
ttggtttctt tttgtagagg ctaataggga tatgatagca tgtatttatt tatttattta 2820
tcttatttta ttatagtaag aacccttaac atgagatcta ccctgttata tttttaagtg 2880
tacaatccat tattgttaac tacgggtaca ctgttgtata gcttactcat cttgctgtat 2940
taaaactttg tgcccattga ttagtaaccc ctcgtttcgt cctcccccag ccactggcaa 3000
ccagcattat actctttgat tctatgagtt tgactacttt agctacctta tataagtggt 3060
attatgtact gtttatcttt ttatgactga cttatttccc ttagcatagt gcattcaaag 3120
tccaaccatg ttgttgccta ttgcagaatt tccttctttt caaggctgaa taatattcca 3180
gtgcatgtgt gtaccacatt ttctttatcc attaatttgt tgattgatag acatttaggt 3240
tggtttctaa catcttgact atcatgaata gtgttgcaat gaacacagga gagctactat 3300
ctcttagaga tgatatcatg gtttttatca tcagaaaaca cccactgatt tctatgctaa 3360
ttttgttacc tgggtggaat aatagtacag ctatatattc ctcattttag atatctttgt 3420
atttctacat acaataaaaa agcagagtac ttagtcatgt tgaagaactt taaactttta 3480
gtatttccag atcaatcttc aaaacaagga caggtttatc tttctctcac cactcaatct 3540
atatatacct cttgtgggca aggccagttt ttatcactgg agcctttccc ctttttatta 3600
tgtacctctc cctcacagca gagtcaggac tttaacttta cacaatacta tggctctaca 3660
tatgaaatct taaaaataca taaaaattaa taaattctgt ctagagtagt atattttccc 3720
tggggttaca gttactttca taataaaaat tagagataag gaaaggactc atttattgga 3780
aagtgatttt aggtaacatt tctggaagaa aaatgtctat atcttaatag tcacttaata 3840
tatgatggat tgtgttactc ctcagttttc aatggcatat actaaaacat ggccctctaa 3900
aaagggggca aatgaaatga gaaactctct gaatgttttt ctcccctagg tgaattcacc 3960
tgctgcttag aagcttattt tctcttgatt tctgttataa tgattgctct taccctttag 4020
ttttaagttt caaaatagga gtcatataac tttccttaaa gctattgact gtctttttgt 4080
cctgttttat tcaccatgag ttatagtgtg acagttaatt cttatgaaaa ttatatagag 4140
atggttaaat catcagaaac tgtaaacctc gattgggagg ggaagcggat ttttaaatga 4200
tttcctgacc aagcttaacc agtatattaa atcctttgta ctgttctttg gctataaaga 4260
aaaaaggtac tgtccagcaa ctgaaacctg ctttcttcca tttagcatac cctttttgga 4320
gacaaattat gcacagttgc aactcttcgt gaaacctatg gtgaaatggc tgactgctgt 4380
gcaaaacaag aacctgagag aaatgaatgc ttcttgcaac acaaagatga caacccaaac 4440
ctcccccgat tggtgagacc agaggttgat gtgatgtgca ctgcttttca tgacaatgaa 4500

gagacatttt tgaaaaagta agtaatcaga tgtttatagt tcaaaattaa aaagcatgga 4560
gtaactccat aggccaacac tctataaaaa ttaccataac aaaaatattt tcaacattaa 4620
gacttggaag ttttgttatg atgatttttt aaagaagtag tatttgatac cacaaaattc 4680
tacacagcaa aaaatatgat caaagatatt ttgaagttta ttgaaacagg atacaatctt 4740
tctgaaaaat ttaagataga caaattattt aatgtattac gaagatatgt atatatggtt 4800
gttataattg atttcgtttt agtcagcaac attatattgc caaaatttaa ccatttatgc 4860
acacacacac acacacacac acacttaacc cttttttcca catacttaaa gaatgacaga 4920
gacaagacca tcatgtgcaa attgagctta attggttaat tagatatctt tggaatttgg 4980
aggttctggg gagaatgtcg attacaatta tttctgtaat attgtctgct atagaaaagt 5040
gactgttttt ctttttcaaa atttagatac ttatatgaaa ttgccagaag acatccttac 5100
ttttatgccc cggaactcct tttctttgct aaaaggtata aagctgcttt tacagaatgt 5160
tgccaagctg ctgataaagc tgcctgcctg ttgccaaagg tattatgcaa aagaatagaa 5220
aaaaagagtt cattatccaa cctgattttg tccattttgt ggctagattt agggaacctg 5280
agtgtctgat acaaactttc cgacatggtc aaaaaagcct tccttttatc tgtcttgaaa 5340
atctttcatc tttgaaggcc tacactctcg tttcttcttt taagatttgc caatgatgat 5400
ctgtcagagg taatcactgt gcatgtgttt aaagatttca ccacttttta tggtggtgat 5460
cactatagtg aaatactgaa acttgtttgt caaattgcac agcaaggggc cacagttctt 5520
gtttatcttt tcatgataat ttttagtagg gagggaattc aaagtagaga attttactgc 5580
atctagatgc ctgagttcat gcattcattc cataaatata tattatggaa tgctttattt 5640
tcttttctga ggagtttact gatgttggtg gaggagagac tgaaatgaat tatacacaaa 5700
atttaaaaat tagcaaaatt gcagcccctg ggatattagc gtactctttc tctgactttt 5760
ctcccacttt taaggctctt tttcctggca atgtttccag ttggtttcta actacatagg 5820
gaattccgct gtgaccagaa tgatcgaatg atctttcctt ttcttagaga gcaaaatcat 5880
tattcgctaa agggagtact tgggaattta ggcataaatt atgccttcaa aatttaattt 5940
ggcacagtct catctgagct tatggagggg tgtttcatgt agaatttttc ttctaatttt 6000
catcaaatta ttcctttttg tagctcgatg aacttcggga tgaagggaag gcttcgtctg 6060
ccaaacagag actcaagtgt gccagtctcc aaaaatttgg agaaagagct ttcaaagcat 6120
ggtaaatact tttaaacata gttggcatct ttataacgat gtaaatgata atgcttcagt 6180
gacaaattgt acatttttat gtattttgca aagtgctgtc aaatacattt ctttggttgt 6240
ctaacaggta gaactctaat agaggtaaaa atcagaatat caatgacaat ttgacattat 6300
ttttaatctt ttcttttcta aatagttgaa taatttagag gacgctgtcc tttttgtcct 6360
aaaaaaaggg acagatattt aagttctatt tatttataaa atcttggact cttattctaa 6420
tggttcatta tttttataga gctgtaggca tggttcttta tttaattttt taaagttatt 6480
tttaattttt gtggatacag agtaggtata catatttacg gggtatatga gatattttga 6540
tataagtata caacatatat aatcccttta tttaatttta tcttcccccc aatgatctaa 6600
aactatttgc ttgtcctttt atgtcttata gttaaattca gtcaccaact aagttgaagt 6660
tacttcttat ttttgcatag ctccagctct gatcttcatc tcatgttttt gcctgagcct 6720
ctgttttcat attacttagt tggttctggg agcatacttt aatagccgag tcaagaaaaa 6780
tactagctgc cccgtcaccc acactcctca cctgctagtc aacagcaaat caacacaaca 6840
ggaaataaaa tgaaaataat agacattatg catgctctct agaaactgtc aattgaactg 6900
tatttgctca tcattcctac catctacacc accaaaatca accaaattta tgaaaaaaaa 6960
cagccccaac ataaaattat acacagataa acaggctatg attggttttg ggaaagaagt 7020
cacctttacc tgatttaggc aactgtgaaa tgactagaga atgaagaaaa ttagacgttt 7080
acatcttgtc atagagtttg aagatagtgc tggatctttc tttttataag taagatcaat 7140
aaaaactccc tcattctgta gaagttatga tttcttttct aagagacctt tagaagtcag 7200
aaaaaatgtg tttcaattga gaaaaaagat aactggagtt tgtgtagtac ttcccagatt 7260
ataaaatgct tttgtatgta ttatctaatt taatcctcaa aacttcttca atttagcatg 7320
ttgtcatgac actgcagagg ctgaagctca gagaggctga gccctctgct aacaagtcct 7380
actgctaaca agtgataaag ccagagctgg aagtcacatc tggactccaa acctgatgct 7440
tctcagcctg ttgccccttt tagagttcct ttttaatttc tgcttttatg acttgctaga 7500
tttctaccta ccacacacac tcttaaatgg ataattctgc cctaaggata agtgattacc 7560
atttggttca gaactagaac taatgaattt taaaaattat ttctgtatgt ccattttgaa 7620
ttttcttatg agaaatagta tttgcctagt gttttcatat aaaatatcgc atgataatac 7680
cattttgatt ggcgattttc tttttagggc agtagctcgc ctgagccaga gatttcccaa 7740
agctgagttt gcagaagttt ccaagttagt gacagatctt accaaagtcc acacggaatg 7800
ctgccatgga gatctgcttg aatgtgctga tgacagggta aagagtcgtc gatatgcttt 7860
ttggtagctt gcatgctcaa gttggtagaa tggatgcgtt tggtatcatt ggtgatagct 7920
gacagtgggt tgagattgtc ttctgtgctt tcgtctgtcc tatcttcaat ctttccctgc 7980
ctatggtggt ggtacctttc tgtttttaac ctggctataa attaccagat aaacccattc 8040

actgatttgt aactcctttc agtcatgctc taactgtaaa tgaaggctta aactgaagta 8100
gaacagttac aaggttttac ttggcagaac atcttgcaag gtagatgtct aagaagattt 8160
tttttctttt ttttaagaca gagtttcgct cttgtttccc aggctggggt gcaatggtgt 8220
gatcttggct cagcgcaacc tctgcctcct gggttcaagt gattctcatg cctcagcctc 8280
ccaagtagct gggattacag gcatgcgcca ccacacctgg ctaattttgt atttttagta 8340
gaggcggggt ttcaccatat tgtccagact ggtctcgaac tcctgacctc aggtgatcca 8400
cccgccttgg cctcccaaag tgctgggatt acaggcatga gccaccttgc ccagcctaag 8460
aagatttttt gagggaggta ggtggacttg gagaaggtca ctacttgaag agatttttgg 8520
aaatgatgta tttttcttct ctatattcct tcccttaatt aactctgttt gttagatgtg 8580
caaatatttg gaatgatatc tcttttctca aaacttataa tattttcttt ctccctttct 8640
tcaagattaa acttatgggc aaatactaga atcctaatct ctcatggcac tttctggaaa 8700
atttaaggcg gttattttat atatgtaagc agggcctatg actatgatct tgactcattt 8760
ttcaaaaatc ttctatattt tatttagtta tttggtttca aaaggcctgc acttaatttt 8820
gggggattat ttggaaaaac agcattgagt tttaatgaaa aaaacttaaa tgccctaaca 8880
gtagaaacat aaaattaata aataactgag ctgagcacct gctactgatt agtctatttt 8940
aattaagtgg gaatgttttt gtagtcctat ctacatctcc aggtttagga gcaaacagag 9000
tatgttcata gaaggaatat gtgtatggtc ttagaataca atgaatatgt tctgccaact 9060
taataaaggt ctgaggagaa agtgtagcaa tgtcaattcg tgttgaacaa tttccaccaa 9120
cttacttata ggcggacctt gccaagtata tctgtgaaaa tcaagattcg atctccagta 9180
aactgaagga atgctgtgaa aaacctctgt tggaaaaatc ccactgcatt gccgaagtgg 9240
aaaatgatga gatgcctgct gacttgcctt cattagctgc tgattttgtt gaaagtaagg 9300
atgtttgcaa aaactatgct gaggcaaagg atgtcttcct gggcatgtaa gtagataaga 9360
aattattctt ttatagcttt ggcatgacct cacaacttag gaggatagcc taggcttttc 9420
tgtggagttg ctacaatttc cctgctgccc agaatgtttc ttcatccttc cctttcccag 9480
gctttaacaa tttttgaaat agttaattag ttgaatacat tgtcataaaa taatacatgt 9540
tcatggcaaa gctcaacatt ccttactcct taggggtatt tctgaaaata cgtctagaaa 9600
cattttgtgt atatataaat tatgtatact tcagtcattc attccaagtg tatttcttga 9660
acatctataa tatatgtgtg tgactatgta ttgcctgtct atctaactaa tctaatctaa 9720
tctagtctat ctatctaatc tatgcaatga tagcaaagaa gtataaaaag aaatatagag 9780
tctgacacca ggtgctttat atttggtgaa aagaccagaa gttcagtata atggcaatat 9840
ggtaggcaac tcaattacaa aataaatgtt tacatattgt cagaagttgt ggtgataaac 9900
tgcatttttg ttgttggatt atgataatgc actaaataat atttcctaaa attatgtacc 9960
ctacaagatt tcactcatac agagaagaaa gagaatattt taagaacata tctctgccca 10020
tctatttatc agaatccttt tgagatgtag tttaaatcaa acaaaatgtt aataaaaata 10080
acaagtatca ttcatcaaag acttcatatg tgccaagcag tgtgtgcttt gtgtagatta 10140
tgtcatatag ttctcataat ccaccttccg agacagatac tatttatttt ttgagacaga 10200
gttttactct tgttgcccag gctggagtgc aatggtgcca tctcggctca ccacaacctc 10260
cgcctcccag gttcaagcga ttctcctgcc tcagcctcct gggattacag gcatgcacca 10320
ccatgcctgg ctaattttgt atttttagta gagatggggt ttcaccatgt tggtcagact 10380
ggtctcaaac tcctgacctc tggtgatatg cctgcctcag cctcctaaag tgctgggatt 10440
acaggcatga gccactgtgc ccagccgaca gatactatta ttatttccat tctaccgaga 10500
aggagactaa ggctctgatc atttaaataa gttgcctaag gtgatgcagt gatataagta 10560
gcagagctag gaattgagcc ttggtaactt taactctgga ccccaagtcc ttagctacta 10620
agctttactg catggggttt agtcaaatta agacttttgg aatatgagtt acttttgaga 10680
ttagctttgt gatatttttt gtgctcattt gtccaacaaa gtctatttta ttttcatctt 10740
aattaggttt ttgtatgaat atgcaagaag gcatcctgat tactctgtcg tgctgctgct 10800
gagacttgcc aagacatatg aaaccactct agagaagtgc tgtgccgctg cagatcctca 10860
tgaatgctat gccaaagtgg taggtttatt gttggaaaaa aatgtagttc tttgactgat 10920
gattccaata atgagaaaga aaaataatgc aagaatgtaa aatgatatac agtgcaattt 10980
agatcttttc ttgagatggt ttcaattctg gaatcttaaa catgaaagaa aaagtagcct 11040
tagaatgatt aacaaaattt agactagtta gaatagaaag atctgaatag agcaatctct 11100
aaaaaatttt gatctttttt tctctttttc acaatcctga gaacaaaaaa aaattaaatt 11160
taaatgttaa ttagaagata tttaacttag atgtaaagtg agttaacctg attccaggat 11220
taatcaagta ctagaattag tatcttatgg caaattatag aacctatccc tttagaatat 11280
tttcaaatct ttttgaggat gtttaggaat agttttacaa gaaattaagt taggagagga 11340
aatctgttct ggaggatttt tagggttccc actagcatat gtaatggttt ctgaactatt 11400
cagaatcaga gaaaactcat ttttcctgct ttcaagaagc tactgtatgc caggcaccat 11460
gcacaaacaa tgaccaacgt aaaatctctc attttggaga gcctggaatc taactggaaa 11520
ggtgaactaa taataataat atgtacaatc atagccatca tttattaaac ttttattata 11580

tgcaaggcac tgtttaattt cattagctta cctggtttac agagcagctc tatgagatga 11640
gtgccatctt tgcccctatt ttagggataa ggattctgaa atgtggagat ggtaagtaaa 11700
attgcacaac tgaagaatga gttacatgac ttggctcaaa tactggtcat tgaactccag 11760
agcctgaata ttcttaacca cttacatgat gcaagctcac caaataaata gttcgaatgt 11820
attgtgacag agcggcattg atattcatct attcatgtgg ctttgagtag gaagaagaaa 11880
ggatatcatt ctgaccagag gggtgaaaaa caacctgcat ctgatcctga ggcataatac 11940
tattaacaca attcttttat gtttcagttc gatgaattta aacctcttgt ggaagagcct 12000
cagaatttaa tcaaacaaaa ttgtgagctt tttgagcagc ttggagagta caaattccag 12060
aatgcgtaag taatttttat tgactgattt tttttatcaa tttgtaatta tttaagactt 12120
aatatatgag ccacctagca tagaactttt aagaatgaaa atacattgca tatttctaat 12180
cactctttgt caagaaagat aggagaggag agataaaata gttgatgggg tggagaggtc 12240
tatatttgaa tgtagtctaa aaattgttct cttaagattg gaagtatgta ggctgggagg 12300
gtaaatacca aatcttggta tatcagaact gagcatgtcc cttgaaggtt aagaaatagt 12360
taatgggcaa atagagcatg gcaatatttt gtagagcagc aagtagtagg ccttgaatag 12420
atgtcgctca aaaagtaata tgtaagctga acacaaaaat gtaacaaatg aatttagata 12480
catatttgaa tattaaattc aggttgtttg ggagatgcac ctagtctttg atggttaaac 12540
ctttccctcc atagaagaga cagagacaga atggcttgct ggactaatgt cccaattcaa 12600
tagagtctta tctatgaagg ttaaaaacaa gaagagacat attatacagt agatatttat 12660
tgtgtggctc atacacatgg tgctcttctg attatggatt ttagagataa taacagtgaa 12720
caagacatag tttctttcct cgagtagatt aaagtcatac attgactttt aatggtgact 12780
ggcattctta atacatgatt attatatatt aggtaccatg tcagattaat tataatactt 12840
tactactttt aatttaaccc ttgaactatc cctattgagt cagatatatt tccttccatt 12900
ttctacttgt atctttcaag tttagcatat gctgatacat atgaagctct ctccaggttt 12960
tattgaaaga agaaattaat aaatttatta atgtcactga attaggcaac tcactttccc 13020
aagattatgc aagtggtaca ggtggaactc aaagccaagt ttaactagtt gttcaggaga 13080
atgttttcta ccctccacta acccactact ctgcagatgg agataatatg atgaatggaa 13140
catagcaaca tcttagttga ttccggccaa gtgttctctg ttttatctac tatgttagac 13200
agtttcttgc cttgctgaaa acacatgact tctttttttc aggctattag ttcgttacac 13260
caagaaagta ccccaagtgt caactccaac tcttgtagag gtctcaagaa acctaggaaa 13320
agtgggcagc aaatgttgta aacatcctga agcaaaaaga atgccctgtg cagaagacta 13380
tgtgagtctt taaaaaaata taataaatta ataatgaaaa aattttacct ttagatattg 13440
ataatgctag ctttcataag cagaaggaag taatgtgtgt gtgtgcatgt ttgtgtgcat 13500
gtgtgtgtgc atgcacgtgt gtgtatgtgt gatattggca gtcaaggccc cgaggatgat 13560
aatttttttt tttttttgga gacggagtct cgctttgttg tccaggctgg agtgcagtgg 13620
tgccatctcg gctcactgca acctccgcct cccaggttca agccattctc ctgcctcagc 13680
ctcccaagta gctgggacta caggtgcatg ccaccatgcc tggctaattt tttgtatttt 13740
tagtagaaaa ttttcagctt cacctctttt gaatttctgc tctcctgcct gttctttagc 13800
tatccgtggt cctgaaccag ttatgtgtgt tgcatgagaa aacgccagta agtgacagag 13860
tcaccaaatg ctgcacagaa tccttggtga acaggcgacc atgcttttca gctctggaag 13920
tcgatgaaac atacgttccc aaagagttta atgctgaaac attcaccttc catgcagata 13980
tatgcacact ttctgagaag gagagacaaa tcaagaaaca aacgtgagga gtatttcatt 14040
actgcatgtg tttgtagtct tgatagcaag aactgtcaat tcaagctagc aactttttcc 14100
tgaagtagtg attatatttc ttagaggaaa gtattggagt gttgccctta ttatgctgat 14160
aagagtaccc agaataaaat gaataacttt ttaaagacaa aatcctctgt tataatattg 14220
ctaaaattat tcagagtaat attgtggatt aaagccacaa tagaataaca tgttaggcca 14280
tattcagtag aaaaagatga acaattaact gataaatttg tgcacatggc aaattagtta 14340
atgggaacca taggagaatt tatttctaga tgtaaataat tattttaagt ttgccctatg 14400
gtggccccac acatgagaca aacccccaag atgtgacttt tgagaatgag acttggataa 14460
aaaacatgta gaaatgcaag ccctgaagct caactcccta ttgctatcac aggggttata 14520
attgcataaa atttagctat agaaagttgc tgtcatctct tgtgggctgt aatcatcgtc 14580
taggcttaag agtaatattg caaaacctgt catgcccaca caaatctctc cctggcattg 14640
ttgtctttgc agatgtcagt gaaagagaac cagcagctcc catgagtttg gatagcctta 14700
ttttctatag cctccccact attagctttg aagggagcaa agtttaagaa ccaaatataa 14760
agtttctcat ctttatagat gagaaaaatt ttaaataaag tccaagataa ttaaattttt 14820
aaggatcatt tttagctctt taatagcaat aaaactcaat atgacataat atggcacttc 14880
caaaatctga ataatatata attgcaatga catacttctt ttcagagatt tactgaaaag 14940
aaatttgttg acactacata acgtgatgag tggtttatac tgattgtttc agttggtctt 15000
cccaccaact ccatgaaagt ggattttatt atcctcatca tgcagatgag aatattgaga 15060
cttatagcgg tatgcctgag ccccaaagta ctcagagttg cctggctcca agatttataa 15120

tcttaaatga tgggactacc atccttactc tctccatttt tctatacgtg agtaatgttt 15180
tttctgtttt ttttttttct ttttccattc aaactcagtg cacttgttga gctcgtgaaa 15240
cacaagccca aggcaacaaa agagcaactg aaagctgtta tggatgattt cgcagctttt 15300
gtagagaagt gctgcaaggc tgacgataag gagacctgct ttgccgagga ggtactacag 15360
ttctcttcat tttaatatgt ccagtattca tttttgcatg tttggttagg ctagggctta 15420
gggatttata tatcaaagga ggctttgtac atgtgggaca gggatcttat tttacaaaca 15480
attgtcttac aaaatgaata aaacagcact ttgtttttat ctcctgctct attgtgccat 15540
actgttaaat gtttataatg cctgttctgt ttccaaattt gtgatgctta tgaatattaa 15600
taggaatatt tgtaaggcct gaaatatttt gatcatgaaa tcaaaacatt aatttattta 15660
aacatttact tgaaatgtgg tggtttgtga tttagttgat tttataggct agtgggagaa 15720
tttacattca aatgtctaaa tcacttaaaa ttgcccttta tggcctgaca gtaacttttt 15780
tttattcatt tggggacaac tatgtccgtg agcttccgtc cagagattat agtagtaaat 15840
tgtaattaaa ggatatgatg cacgtgaaat cactttgcaa tcatcaatag cttcataaat 15900
gttaattttg tatcctaata gtaatgctaa tattttccta acatctgtca tgtctttgtg 15960
ttcagggtaa aaaacttgtt gctgcaagtc aagctgcctt aggcttataa catcacattt 16020
aaaagcatct caggtaacta tattttgaat tttttaaaaa agtaactata atagttatta 16080
ttaaaatagc aaagattgac catttccaag agccatatag accagcaccg accactattc 16140
taaactattt atgtatgtaa atattagctt ttaaaattct caaaatagtt gctgagttgg 16200
gaaccactat tatttctatt ttgtagatga gaaaatgaag ataaacatca aagcatagat 16260
taagtaattt tccaaagggt caaaattcaa aattgaaacc aaagtttcag tgttgcccat 16320
tgtcctgttc tgacttatat gatgcggtac acagagccat ccaagtaagt gatggctcag 16380
cagtggaata ctctgggaat taggctgaac cacatgaaag agtgctttat agggcaaaaa 16440
cagttgaata tcagtgattt cacatggttc aacctaatag ttcaactcat cctttccatt 16500
ggagaatatg atggatctac cttctgtgaa ctttatagtg aagaatctgc tattacattt 16560
ccaatttgtc aacatgctga gctttaatag gacttatctt cttatgacaa catttattgg 16620
tgtgtcccct tgcctagccc aacagaagaa ttcagcagcc gtaagtctag gacaggctta 16680
aattgttttc actggtgtaa attgcagaaa gatgatctaa gtaatttggc atttatttta 16740
ataggtttga aaaacacatg ccatttacaa aataagactt atatttgtcc ttttgttttt 16800
cagcctacca tgagaataag agaaagaaaa tgaagatcaa aagcttattc atctgttttt 16860
ctttttcgtt ggtgtaaagc caacaccctg tctaaaaaac ataaatttct ttaatcattt 16920
tgcctctttt ctctgtgctt caattaataa aaaatggaaa gaatct 16966

<210> 185
<211> 364
<212> DNA
<213> unknown

<220>
<223> >gi|4292568|gb|AI433922|sr|IMAGp998K105256.1|cl|IM
      AGp998K105256|AI433922 ti11f05.x1 NCI_CGAP_Pan1
      Homo sapiens cDNA clone IMAGE:2130177 3' similar
      to TR:Q08805 Q08805 SALIVARY PROLINE-RICH PROTEIN
      L ;, mRNA sequence

<400> 185
tttttttttt tttttttttt tttttttttt tttttttttt tttttttttt tttttttttt 60
tttttttttt tttttttttt tttttttttt ttttggggcg gtcccaaaac caaccctttt 120
aattggaaaa aaaggggaaa cccccccccc ccgggggggg gggggggaaa aaaaaaaaaa 180
accccctttt caaaaaaagg cgggggggga accaattccc cccccgggga aacccccccc 240
ccccaaaacc ctggaagttc cctgggaaaa aaaaaaaaaa aagggggggg gggaaaaaag 300
gggggacccc cccaaacccc tttgcccccc cccccaaggg gggggaaaaa ccccccggtc 360
cccc 364

<210> 186
<211> 474
<212> DNA
<213> unknown

<220>
<223> >ImageID=1752104 IMAGP971B2475_S1_M13-RP CC
      FromEXP of IMAGp971B2475_s1_M13-RP.exp April 26,
      2002 12:41

<400> 186
catcatcatc cccactgtgg acaggtcgca gtaagaacag gagagtcaca tcattctaat 60
aatggtctca gagatacatc acaatgactc ccctgggcag aaagaaggga taagagtcac 120
atcacctgtg ggctaggccc agagatgtca ctcttacttc tgtgggcatg tctcaggctg 180
gagaggagaa tcacattacc taaacactgg accaagaaat acgtcacagt ctttctcatg 240
ggcaaagtcc aggacctcgg caaaacctga gtcctgtcct ctcgctttcc tccccgtgca 300
cagcgagctt caccacttgc tccgcacctt ctccatcaac tactacctgt ccctgtgatc 360
tgtccagtcg cccaactaaa gtgctcagca ggtcagcagc gcggcgagct tctatgcagg 420
cgtggggtct ggggctcctg gatctctgtg tcccatttca cagagattgc cacc       474

<210> 187
<211> 667
<212> DNA
<213> unknown

<220>
<223> >sr|IMAGp998G193982.2|cl|IMAGp998G193982|gb|BX0901
      35|oid|234514632 BX090135 NCI_CGAP_GC4 Homo
      sapiens cDNA clone IMAGp998G193982 ;
      IMAGE:1571370, mRNA sequence

<400> 187
ctcagcccac ctgcacccag gtgaaataaa cagccatgta gctcacacaa cgcctgtttg 60
gtggtctctt cacacggacg cacatgaaat ttggtgccct gactcggatc gggggacctc 120
ccttgggaga tcaatcccct gtcctcctgt tctttgctcc ctgagaaaga tccacctatg 180
acttcaggtc ctcagactga ccagcccaag gaacatctca ccaattttaa atcagcggcc 240
aggcgttcct ccagaacctc ctcccacagg aacttgctac acgtgctgga aatctggcaa 300
ctggaccaag gaatgcccgc agcccgggat tcctaagccg catcccatct gtgtgggacc 360
ccacggaaaa tcggactgtt caactcacct ggcagccact cccagagccc ctggaactct 420
ggcccaaggc tctctgactc cttcccagat cttctcggcc tagcggttga agactgacac 480
tgcccgatca cctcggaagc cccctagacc atcacggacg ccgagcttca gatacgcgca 540
tgaaaggaag attaagaaaa acaaaaaaat gtaaatttac tggacactga atatatacta 600
agtactgctt aagtgattta catgtcaaga ttaaaatatt ttgctttact ttaaaaaaaa 660
aaaaaaa                                                           667

<210> 188
<211> 444
<212> DNA
<213> unknown

<220>
<223> >gi|5340943|gb|AI793227|sr|IMAGp998P194992.3|cl|IM
      AGp998P194992|AI793227 qz35g10.x5 NCI_CGAP_Kid11
      Homo sapiens cDNA clone IMAGE:2028930 3', mRNA
      sequence

<400> 188
tgtgcttcag tgatttattg agctttaact tcgggccagg gcagcagtag gcgccgctga 60
gaaaagatga atgaacaagt ccccaaacca aaaataagca gcccactcct ccaaaataaa 120
cacccactcc cagcattaag tttaaattac aatcactcag ggctcaggac tttcttgttg 180
gcagcagctg ttggcttcat agctttgacc attctgccac ctctctggga tcccacagcc 240
catctttgag tgtctgtttt cagttcaaga cagctagctc atgccagcac tggcagaggt 300
gagtccccaa ctgcacacag gcacctactg gtataggtct ggcagatttg agttcggata 360

gagttgccat cacttcctcg ctgtctggag ctgcacgcgt taatatgcca ttagagcccc 420
agtttcttca tatgccagta gcac 444

<210> 189
<211> 476
<212> DNA
<213> unknown

<220>
<223> >gi|5110011|gb|AI741723|sr|IMAGp998F015873.1|cl|IM
      AGp998F015873|AI741723 wg22c01.x1
      Soares_NSF_F8_9W_OT_PA_P_S1 Homo sapiens cDNA
      clone IMAGE:2365824 3', mRNA sequence

<400> 189
tttttttttt tttttttttg catttaaata tgggctcaag tagtaggact tggctgagtt 60
tattactctc aagagtcaaa cagaaagaga aggagtgagg aaaccaagaa acagtggggg 120
tcaggctggg agtagaggaa gtactagaac ttgggttggc agagttcatc tttgtagttc 180
aagaagtctg aagttttaca gttatttgca tcacctggat agaagacttt cttggcttgc 240
aaagctttct gccacatcca taaagatctt gtttttattt acttgctggt tttctttatt 300
tctttatttc atgaggctga atagaaatga aggaaacttg gttggtgatt ctagaagctg 360
ctctctggga tgatctcctt gttaatgatc ccctatggat tcatctttga tgggcaataa 420
agaattgctt tcttgaggga cgcacagctt cctcagacct cttcatgcta ctgcag 476

<210> 190
<211> 417
<212> DNA
<213> unknown

<220>
<223> >gi|3930893|gb|AI288116|sr|IMAGp998A054888.1|cl|IM
      AGp998A054888|AI288116 qv88a03.x1 NCI_CGAP_Ut2
      Homo sapiens cDNA clone IMAGE:1988620 3' similar
      to gb:J03075 PROTEIN KINASE C SUBSTRATE, 80 KD
      PROTEIN, HEAVY CHAIN (HUMAN);, mRNA sequence

<400> 190
tttttttttt tttttttttt tttttttttt tttttttttt tttttttttt tttttttttt 60
tttttttttt tttttttttt tggggggggg gaaaaaaata aatggatggg gggccaaggg 120
aaaaacatcc acaaaccccc caaaaaaggg ggggcagaaa cagggctggg gggggggggg 180
caaaacacca aggggggggg cccaaggggg gaaacccccc taacccattt ttaaaggggg 240
ggggggcccg gaaaagggaa ccaccccccc cggggcccca aaaaagggga aaaaacccca 300
aaaagggcct gccccccaaa aagagggcaa aaaaaccaca ggcagcccca aagggggggg 360
gggacacgac tgaaggggcg ggtttaaacc ctttttaagg ggttttgcgc ccaacca 417

<210> 191
<211> 488
<212> DNA
<213> unknown

<220>
<223> >sr|IMAGp998A04528.1|cl|IMAGp998A04528|gb|BX101034
      |oid|234536444 BX101034 Soares fetal liver spleen
      1NFLS Homo sapiens cDNA clone IMAGp998A04528 ;
      IMAGE:244875, mRNA sequence.

<400> 191
aaatatgtga ttttctctaa tgtaacttcc tcttaggatg tttcaccaag gaaatattga 60
gagagaagtc ggccaggtag gatgaacaca ggcaatgact gcgcagagtg gattaaaggc 120

aaaagagaga agagtccagg aaggggcggg gagaagcctg ggtggctcag catcctccac 180
gggctgcgcc gtctgctcgg ggctgagctg gcgggagcag tttgcgtgtt tgggtttttt 240
aattgagatg aaattcaaat aacctaaaaa tcaatcactt gaaagtgaac aatcagcggc 300
atttagtaca tccagaaagt tgtgtaggca ccacctctgt cacgttctgg aacattctgt 360
catcaccccg tgaagcaatc atttcccctc ccgtcttcct cctcccctgg caactgctga 420
tcgactttgt gtctctgttg tctaaaatag gttttccctg ttctggacat ttcatataaa 480
tggaatca 488

<210> 192
<211> 526
<212> DNA
<213> unknown

<220>
<223> >sr|IMAGp998H22207.1|cl|IMAGp998H22207|gb|BX096159
      |oid|234526691 BX096159 Soares placenta Nb2HP Homo
      sapiens cDNA clone IMAGp998H22207 ; IMAGE:140997,
      mRNA sequence

<400> 192
ctggaagacc tgggctgggc cgattgggtg ctgtcgccac gggaggtgca agtgaccatg 60
tgcatcggcg cgtgcccgag ccagttccgg gcggcaaaca tgcacgcgca gatcaagacg 120
agcctgcacc gcctgaagcc cgacacggtg ccagcgccct gctgcgtgcc cgccagctac 180
aatcccatgg tgctcattca aaagaccgac accggggtgt cgctccagac ctatgatgac 240
ttgttagcca aagactgcca ctgcatatga gcagtcctgg tccttccact gtgcacctgc 300
gcggaggacg cgacctcagt tgtcctgccc tgtggaatgg gctcaaggtt cctgagacac 360
ccgattcctg cccaaacagc tgtatttata taagtctgtt atttattatt aatttattgg 420
ggtgaccttc ttggggactc gggggctggt ctgatggaac tgtgtattta tttaaaactc 480
tggtgataaa aataaagctg tctgaactgt taaaaaaaaa aaaaaa 526

<210> 193
<211> 755
<212> DNA
<213> unknown

<220>
<223> >ImageID=451838 IMAGP971M0340_S1 CC   FromEXP of
      IMAGp971M0340_s1.exp April 26, 2002 13:37

<221> misc_feature
<222> 580, 694, 704
<223> n=a, t, c, or g

<400> 193
agaaacatgt atttcagcaa acccatccag tgtaccacgt aaaacctggt gggccagtaa 60
atctcctgac aataaacctg tttggtatgg tcttgatatg aacagagggt ctcagttcgc 120
ttatggagac caccaatcac ctaatacagc cattactcag atgacttttt tgcgcctttt 180
atcaaaagaa gcctcccaga acatcactta catctgtaaa aacagtgtag gatacatgga 240
cgatcaagct aagaacctca aaaaagctgt ggttctcaaa ggggcaaatg acttagatat 300
caaagcagag ggaaatatta gattccggta tatcgttctt caagacactt gctctaagcg 360
gaatggaaat gtgggcaaga ctgtctttga atatagaaca cagaatgtgg cacgcttgcc 420
catcatagat cttgctcctg tggatgttgg cggcacagac caggaattcg gcgttgaaat 480
tgggccagtt tgttttgtgt aaagtaagcc aagacacatc gacaatgagc accaccatca 540
atgaccaccg ccattcacaa gaactttgac tgtttgaagn tgatcctgag actcttgaag 600
taatggctga tcctgcatca gcattgtata tatggtctta agtgcctggc ctccttatcc 660
ttcagaatat ttattttact tacaatcctc aagntttaat tgantttaaa tatttttcaa 720
tacaacaggt taggttttaa gatgaccaat gacaa 755

<210> 194

<211> 467
<212> DNA
<213> unknown

<220>
<223> >gi|4740154|gb|AI656175|sr|IMAGp998K055550.1|cl|IM
      AGp998K055550|AI656175 tt38e03.x1 NCI_CGAP_GC6
      Homo sapiens cDNA clone IMAGE:2243068 3', mRNA
      sequence

<400> 194
actttgtgtc attcagttta gagcaattta atcttttttt ctccaaatcc atttttgaag 60
ctgagtttaa cttttgcaac ccatggcaaa tcttaaatgc cctcatttac caatctttac 120
caaactccta tttaagcctc taaaagtcaa tactggccat cagacccaaa tttcagaaga 180
caatagtgaa aaattactta cgtttaatct ccagtcgtgt cccttggccg aaggtgatcc 240
acagtgttaa cttaattact ttcccctaaa caaaaatctc ttttcgctgt taatatcact 300
aacctgaccg atgcagagaa aatctacaat tgagatgcct cacttaactg gctagcgctt 360
ggctgttcct taagatgaac tacttttcta tcccttactc atctgacttt ttgaaagaat 420
ctggtactct tttgaattga cctgagctca tatctcaaac acaaaaa 467

<210> 195
<211> 474
<212> DNA
<213> unknown

<220>
<223> >sr|IMAGp998A065260.2|cl|IMAGp998A065260|gb|BX1195
      12|oid|234573224 BX119512 NCI_CGAP_Kid11 Homo
      sapiens cDNA clone IMAGp998A065260 ;
      IMAGE:2131469, mRNA sequence

<400> 195
tcttaatttt ttttttttaa ttggaaaact cgatctctag gaggaaagaa aaaaaaggcc 60
tgggtcccag aaaactggtt gtagccctag cttcttaatt tattggccat gtgactatag 120
actatttgct tacttgctct ggtcctcagt tttctggtct gcagaatggg tatcttgatg 180
tgtgttgttc ttctcctcca cgatatgaaa ccaaaggcct ttatagcttg ttttctgagg 240
ctccttccag gtgaaaatcc tataactccc aataaaacag ctggagaggc tgtctctact 300
gccttttctg tctacccagg agtttccttg gttgatggga agctgtttaa gaactgaaat 360
gagagagaag aggcagacag acccaaccgc tacagctcct atcagaaagg gcagcgcccg 420
tggaattgta ggtagcctgt tactggctca gagaacaagc aggcctactt atgt 474

<210> 196
<211> 498
<212> DNA
<213> unknown

<220>
<223> >sr|IMAGp998J076121.2|cl|IMAGp998J076121|gb|BX0968
      30|oid|234528032 BX096830 NCI_CGAP_Pr22 Homo
      sapiens cDNA clone IMAGp998J076121 ;
      IMAGE:2461158, mRNA sequence

<400> 196
   gcccccacct tcccatccag acagccgagc tcgctccgga acttgtcatc tccaacgaca 60
   aaaggagctt ttgccactga ctcggccgtg tcctgacacc tccagaacgc aggtgctggc 120
   gcccgttctg cctgggaccc cgggaacctc tcctgccgga agccggacgg cagggatggg 180
   ccccaacttc gccctgccca cttgacttca ccaaatccct tcctggagac tgaacctggt 240
   gctcaggagc gaaggactgt gaacttgtgg cctgaagagc cagagctagc tctggccacc 300
   agctggcgca cgtcaccctg ctcccacccc acccccaagt tctaaggtct tttcagagcg 360
   tggaggtgtg gaaggagtgg ctgctctcca aactatgcca aggcggcggc agagctggtc 420
   ttctggtctc cttggagaaa ggttctgttg ccctgattta tgaactctat aatagagtat 480
   ataggttttg tacctttt 498

<210> 197
<211> 584
<212> DNA
<213> unknown

<220>
<223> >sr|IMAGp998L10662.1|cl|IMAGp998L10662|gb|BX102297
      |oid|234538974 BX102297 Soares fetal liver spleen
      1NFLS Homo sapiens cDNA clone IMAGp998L10662 ;
      IMAGE:296601, mRNA sequence

<221> misc_feature
<222> 512, 538, 552, 570, 579, 584
<223> n=a, t, c, or g

<400> 197
tccgatatgg cctcaatgaa gtgaagtgca aagggaatga gtcttccttg tgggattgtc 60
ctgccagacg ctggggccat agtgagtgtg ggcacaagga agacgctgca gtgaattgca 120
cagatatttc agtgcagaaa accccacaaa aagccacaac aggtcgctca tcccgtcagt 180
catcctttat tgcagtcggg atccttgggg ttgttctgtt ggccattttc gtcgcattat 240
tcttcttgac taaaaagcga agacagagac agcggcttgc agtttcctca agaggagaga 300
acttagtcca ccaaattcaa taccgggaga tgaattcttg cctgaatgca gatgatctgg 360
acctaatgaa ttcctcagga ggccattctg agccacactg aaaaggaaaa tgggaattta 420
taacccagtg agttcagcct ttaagatacc ttgatgaaga cctggactat tgaatggagc 480
agaaattcac ctctctcact gactattaca gntgcatttt tatggagttc ttcttctnct 540
atgattccta anactgctgc tgaatttatn aaaattaanc ttgn 584

<210> 198
<211> 732
<212> DNA
<213> unknown

<220>
<223> >ImageID=114388 IMAGP971C043_S1 CC   FromEXP of
      IMAGp971C043_s1.exp April 26, 2002 12:42

<400> 198
aaattccctc tggggatcta tgagctaatg agtttcttca tgcttatccc atgtgatgtt 60
gggaatccag actacagggc aaagtagata ctttaactcc attttacaga taagagtgag 120
gttcagagag atgattgagt tagtgaggac tgagcctggc ctcaaactcg agttgactac 180
aagcacctct tctctgcaca gtgttactca tgaaagagac ttccctgcaa gacctgggcc 240
ttttacttca aagggcaaag agacttattt ttcctttcat gcctttccat ggcccactct 300
ttcattctgg tttcaggttg ccagtctggc tgcagtcctc tgtcattggt cggtaacacc 360
ctgggtcata ttccacactt aaagatggag gtaattgctt caagatgtgg ccctttaagg 420
agagaatagg ttgaaaggca aattcttttt ttcagcttta gtcagagcct gagttttgga 480
aaaaggaaat gatgagttgc tccgagatat aaactttctt tcatctctta gaagaagtat 540
ttgtccttct gaaaaggtta gtggcagttt tgtccattga ttttttttct aaacctttat 600
tacccagttt catggtccat cacagtaagt aaaacaaacc accctccccc agaccacctc 660
cctccttatt tgcacaggga accttcaaac aattgggagc agaagcagaa tgatgcctgt 720
gaagcctttg cc 732

<210> 199
<211> 528
<212> DNA
<213> unknown

<220>
<223> >sr|IMAGp998G06783.2|cl|IMAGp998G06783|gb|BX118770
      |oid|234571916 BX118770 Soares_fetal_heart_NbHH19W
      Homo sapiens cDNA clone IMAGp998G06783 ;
      IMAGE:342941, mRNA sequence

<221> misc_feature
<222> 438, 463, 498, 517
<223> n=a, t, c, or g

<400> 199
tcttatacta atttatacaa agatattaag gccctgttca ttaagaaatt gttcccttcc 60
cctgtgttca atgtttgtaa agattgttct gtgtaaatat gtctttataa taaacagtta 120
aaagctgaca gttcgccctt actcttggag gtcatgttca ggaggggcat tcctttcccc 180
tgggggtcat gggtgtcccc atgcccacat attgcacgtg cagggaggta agtgcctgca 240
tcccaaatcg gttctaggtc aactggcctc aaactgattt gccatgagct cacaaaatga 300
atccctatgc ttaatgacca ggtcacataa aatccagccc acttacaggt tttctggcat 360
ctgtttgggt gtcctaattt ttttggcagt gtcatttgaa gaattttttt aaagcagttt 420
atttaagaac atactganta aatgcaggat cgctactaaa aantgctttg tatccttggt 480
gggtgtcttc tgctattnta tctacttttg aacactntca ggactttt          528

<210> 200
<211> 489
<212> DNA
<213> unknown

<220>
<223> >sr|IMAGp998K08276.1|cl|IMAGp998K08276|gb|BX090262
      |oid|234514887 BX090262 Soares infant brain 1NIB
      Homo sapiens cDNA clone IMAGp998K08276 ;
      IMAGE:47615, mRNA sequence

<400> 200
tttttttttt ttttctggaa ggttctcagg tctttatttg ctctctcaaa ttccaggaat 60
tgacttattt aattaatcca tcaacctctc atagcaaata tttgagaaaa caaatttata 120
ttcagattct tattttcagt agggaagtaa gaagttgcag ctcagtgcac gtaaagttga 180
gacagagatg gagacatcca gccccacctc tctggaacaa gaaagatgac tggggaggaa 240
acacaggtca gcatgggaac aggggtcaca gtggacacaa gggtgggctg tctctccacc 300
tcctcacatt atgctaacag ggacgcagac acattcaggt gcctttgcag aaagagatgc 360
cagaggctct tgaagtcaca aaggggaggc gtgaagaaat cctgcatctc agtccctcac 420
aagacagctg tctcaggctt ttcaagctgt gagagacaca tcagagccct gggcactgtc 480
gctggacgc                                                         489

<210> 201
<211> 364
<212> DNA
<213> unknown

<220>
<223> >gi|2224139|gb|AA494352|sr|IMAGp998F152230.1|cl|IM
      AGp998F152230|AA494352 ne27c08.s1 NCI_CGAP_Co3
      Homo sapiens cDNA clone IMAGE:898574 3' similar to
      contains element LTR5 LTR5 repetitive element ;,
      mRNA sequence.

<400> 201
ctttcactca ttggtttatt cccgcactcc ttccctcact cattcattca ttgactcttg 60
caggtagtgc acaccaaggg tgtgaagggg tgaggtgtct ggttctctgc tcagacctct 120

gcaagtgccc gcagaggtgt tctctgctca gacctctgca agtgcccgca gaggtgactg 180
ggcggcctcc acttctggcc tggccttgca cccctgtgct cttgggggcc atggctgggc 240
agggcaggtg tccccctctg cacaggccca gtgtgtctca gcctcagagc tggcagggca 300
gggcaggcat cccctctgca ctggcccagt gtctctcagc ctcagaacca gatgtgagaa 360
gacg 364

<210> 202
<211> 3159
<212> DNA
<213> unknown

<220>
<223> >ENST00000322244 Coding sequence

<400> 202
atggaaggat ccgagcctgt ggccgcccat cagggggaag aggcgtcctg ttcttcctgg 60
gggactggca gcacaaataa aaatttgccc attatgtcaa cagcatctgt ggaaatcgat 120
gatgcattgt atagtcgaca gaggtacgtt cttggagaca cagcaatgca gaagatggcc 180
aagtcccatg ttttcttaag tgggatgggt ggtcttggtt tggaaattgc aaagaatctt 240
gttcttgcag ggattaaggc agttacaatt catgatacag aaaaatgcca agcatgggat 300
ctaggaacca acttctttct cagtgaagat gatgttgtta ataagagaaa cagggctgaa 360
gctgtactta aacatattgc agaactaaat ccatacgttc atgtcacatc atcttctgtt 420
cctttcaatg agaccacaga tctctccttt ttagataaat accagtgtgt agtattgact 480
gagatgaaac ttccattgca gaagaagatc aatgactttt gccgttctca gtgccctcca 540
attaagttta tcagtgcaga tgtacatgga atttggtcaa ggttattttg tgatttcggt 600
gatgaatttg aagttttaga tacaacagga gaagaaccaa aagaaatttt catttcaaac 660
ataacgcaag caaatcctgg cattgttact tgccttgaaa atcatcctca caaactggag 720
acaggacaat tcctaacatt tcgagaaatt aatggaatga caggtttaaa tggatctata 780
caacaaataa cggtgatatc gccatttctt tttagtattg gtgacaccac agaactggaa 840
ccatatttac atggaggcat agctgtccaa gttaagactc ctaaaacagt tttttttgaa 900
tcactggaga ggcagttaaa acatccaaag tgccttattg tggattttag caaccctgag 960
gcacctttag agattcacac agctatgctt gccttggacc agtttcagga gaaatacagt 1020
cgcaagccaa atgttggatg ccaacaagat tcagaagaac tgttgaaact agcaacatct 1080
ataagtgaaa ccttggaaga gaagcctgat gtaaatgctg acattgtgca ttggctctct 1140
tggactgccc aaggcttttt atctccactt gctgcagcag taggaggtgt tgccagccaa 1200
gaagtattga aagctgtaac aggaaaattt tctcctttgt gccagtggtt atatcttgaa 1260
gcagcagata ttgttgaatc actaggcaaa cctgaatgtg aagaatttct cccacgagga 1320
gatagatatg atgccttaag agcttgcatt ggagacactt tgtgtcagaa actgcaaaat 1380
ttaaacatct tcttagtagg gtgtggagcc ataggctgtg aaatgttgaa aaattttgct 1440
ttacttggtg ttggcacaag caaagagaaa ggaatgatta cagttacaga tcctgacttg 1500
atagagaaat ccaacttaaa tagacagttc ctatttcgtc ctcatcacat acagaaacct 1560
aaaagctaca ctgctgctga tgctactctg aaaataaatt ctcaaataaa gatagatgca 1620
cacctgaaca aagtatgtcc aaccactgag accatttaca atgatgagtt ctatactaaa 1680
caagatgtaa ttattacagc attagataat gtggaagcca ggagatacgt agacagtcgt 1740
tgcttagcaa atctaaggcc tcttttagat tctggaacaa tgggcactaa gggacacact 1800
gaagttattg taccgcattt gactgagtct tacaatagtc atcgggatcc cccagaagag 1860
gaaataccat tttgtactct aaaatccttt ccagctgcta ttgaacatac catacagtgg 1920
gcaagagata agtttgaaag ttccttttcc cacaaacctt cattgtttaa caaattttgg 1980
caaacctatt catctgcaga agaagtctta cagaagatac agagtggaca cagtttagaa 2040
ggctgttttc aagttataaa gttacttagc agaagaccta gaaattggtc ccagtgtgta 2100
gaattagcaa gattaaagtt tgaaaaatat tttaaccata aggctcttca gcttcttcac 2160
tgtttccctc tggacatacg attaaaagat ggcagtttat tttggcagtc accaaagagg 2220
ccaccctctc caataaaatt tgatttaaat gagcctttgc acctcagttt ccttcagaat 2280
gctgcaaaac tatatgctac agtatattgt attccatttg cagaagagga cttatcagca 2340
gatgccctct tgaatattct ttcagaagta aagattcagg aattcaagcc ttccaataag 2400
gttgttcaaa cagatgaaac tgcaaggaaa ccagaccatg ttcctattag cagtgaagat 2460
gagaggaatg caattttcca actagaaaag gctattttat ctaatgaagc caccaaaagt 2520
gaccttcaga tggcagtgct ttcatttgaa aaagatgatg atcataatgg acacatagat 2580
ttcatcacag ctgcatcaaa tcttcgtgcc aaaatgtaca gcattgaacc agctgaccgt 2640

ttcaaaacaa agcgcatagc tggtaaaatt atacctgcta tagcaacaac cactgctaca 2700
gtttctggct tggttgcctt ggagatgatc aaagtaactg gtggctatcc atttgaagct 2760
tacaaaaatt gttttcttaa cttagccatt ccaattgtag tatttacaga gacaactgaa 2820
gtaaggaaaa ctaaaatcag aaatggaata tcatttacaa tttgggatcg atggaccgta 2880
catggaaaag aagatttcac cctcttggat ttcataaatg cagtcaaaga gaagtatgga 2940
attgagccaa caatggtggt acagggagtc aaaatgcttt atgttcctgt aatgcctggt 3000
catgcaaaaa gattgaagtt aacaatgcat aaacttgtaa aacctactac tgaaaagaaa 3060
tatgtggatc ttactgtgtc atttgctcca gacattgatg gagatgaaga tttgccggga 3120
cctccagtaa gatactactt cagtcatgac actgattaa 3159

<210> 203
<211> 166
<212> DNA
<213> unknown

<220>
<223> >gi|2524282|gb|AA620343|sr|IMAGp998A022575.1|cl|IM
      AGp998A022575|AA620343 af07b01.s1
      Soares_testis_NHT Homo sapiens cDNA clone
      IMAGE:1030921 3', mRNA sequence.

<400> 203
ttttggtagc tattgaatca gggccacaca tttaattgat attatgatca agatgttcaa 60
ggcaaaaaat actattactt atttaatgtg gaacaagtct agtctttctc ttgagctccc 120
acctgctggt taggaggcaa caatgttatt tggatcctgt ttagag 166

<210> 204
<211> 408
<212> DNA
<213> unknown

<220>
<223> >sr|IMAGp998E122300.2|cl|IMAGp998E122300|gb|BX0954
      46|oid|234525261 BX095446 NCI_CGAP_Co3 Homo
      sapiens cDNA clone IMAGp998E122300 ; IMAGE:925427,
      mRNA sequence

<400> 204
aaatatgtat aaatgtgggc accgtcattt gtctagaaaa atctatagta aattgttttg 60
ttttcctttc attgcaaagt ctcatacttg ttcttccaaa atcagttcta tgagttgctt 120
taaaattagc ctgggacgtc caagaagtgt taagacatct gcagtaactg gggcagccag 180
tccctggagg ccaactcagg gaacaacaaa ctccttcgcc aagccgtcca cacatgccag 240
ctgatggtct aggacccaag tatgtgccaa gagtgaggtg tcttacttct aaggagaggc 300
aagcagtttg ctatgcaagc gggtcgctgg ccctggacct aaagatcttc tctctaactc 360
catattcttt aaataaactt gtcttttatg aggaaaaaaa aaaaaaaa 408

<210> 205
<211> 398
<212> DNA
<213> unknown

<220>
<223> >gi|879189|sr|IMAGp998C12278|cl|IMAGp998C12278|gb|
      H14369|H14369 ym18f06.s1 Homo sapiens cDNA clone
      48417 3'.

<221> misc_feature
<222> 105, 342, 363
<223> n=a, t, c, or g

<400> 205
tttttttttt ttgccttttc atttgtcttt ttattttaca tttaaaaagt ggttattgct 60
ctaagtggat cagaggtaag gacctctccc ctagggagcc ttggncttgc agccccattc 120
agcagggatg gaagtcacaa gacaatgagt ggagcctcat gccctcccat gaggaagccc 180
ttagtattgc tgacatctgc cctttatcct gtctctcctc cccagtgctg tcacacttgg 240
gcaaagcaga gtggtggcag acccagcctt gagagctctt gtaggaccgg aaggaagggg 300
cggtcattag gtgatgggct tctgggctct ctgggcttgg gngaccaatg ctcttattca 360
ttncattttc aacaaatatt taactggagg taccgggc 398

<210> 206
<211> 654
<212> DNA
<213> unknown

<220>
<223> >sr|IMAGp998E191782.3|cl|IMAGp998E191782|gb|BX1105
      07|oid|234555393 BX110507 Soares_testis_NHT Homo
      sapiens cDNA clone IMAGp998E191782 ; IMAGE:726522,
      mRNA sequence

<400> 206
caaattcaac ccaccacata ggccctcccg aggccacctc tgcgtcatct ttgtctacag 60
attctgtgtc cgtcttcctc tcctcattga ctctgcatga aggggatttc tggtggagta 120
agggtagcag ctcttgccgc acgtgcagag agagaggaac ttccagtgcc cgctatggag 180
ccacagccca cagcatgggg gaagtcccca tccagaggca gtgctgcagc tagaggtagc 240
tccagagtcc tccgggccct gcaccgatac agccaaagac cagcaaagcg acaagctgcc 300
agacctcatg ccacctgccg tagccactgg gctcagccct ggagctgaga gcatcgctgg 360
agatagacgt ggcagagaag aggttgcgag catggcccca gccagcagct cccacgctgc 420
ccctagtcct gggcatggag cgagccttgg tgtcagagac cagggtgtgc agtctgagct 480
cctctacctt actaaagaga ggcctctttt atttaccaga gccacagccc tgctgcctca 540
ggaccttttc attctgccgg tgctggggct gtctatctgc aagctggagg tgttgagagc 600
aggaaaaggg aggctgtgag gaaggggttc gggcagctcc tcctgctctc agag 654

<210> 207
<211> 417
<212> DNA
<213> unknown

<220>
<223> >sr|IMAGp998O124335.2|cl|IMAGp998O124335|gb|BX0992
      72|oid|234532920 BX099272
      Soares_fetal_heart_NbHH19W Homo sapiens cDNA clone
      IMAGp998O124335 ; IMAGE:1707107, mRNA sequence

<221> misc_feature
<222> 20, 26
<223> n=a, t, c, or g

<400> 207
agcatggccc cagccctggn acaagnacac gttctgccag ggcaaagctg atgggctcta 60
tcccaatcct cgggaacggt ccagcttcta cagctgtgca gcggggcggc tgttccagca 120
aagctgcccg acaggcctgg tgttcagcaa ctcctgcaaa tgctgcacct ggaattgagt 180
cgctaaagcc cctccagtcc cagctttgag gctgggccca ggatcactct acagcctgcc 240
tcctgggttt tccctggggg ccgcaatctg gctcctgcag gcctttctgt ggtcttcctt 300
tatccaggct ttctgctctc agccttgcct tccttttttc tgggtctcct gggctgcccc 360
tttcacttgc aaaataaatc tttggtttgt gcccctcttc ccaaaaaaaa aaaaaaa 417

<210> 208

<211> 767
<212> DNA
<213> unknown

<220>
<223> >sr|IMAGp998K102640.2|cl|IMAGp998K102640|gb|BX0989
      17|oid|234532208 BX098917 Soares_testis_NHT Homo
      sapiens cDNA clone IMAGp998K102640 ;
      IMAGE:1056129, mRNA sequence

<221> misc_feature
<222> 7, 759
<223> n=a, t, c, or g

<400> 208
cgcaagnata atggattgtc aaaaaggagg caatatggca aacggaagtt gtttggacct 60
gaagtcagaa gaatcaagtt cagctcactt tgattctgat gggatctacc aaagcatgtt 120
tgttgatatg atcaatccaa aagagaaggt gagactgatg aacaggagga ctggagtctt 180
gtagtaggag gagtaaatgg gattcatatc tcacatcagc accttattta tttgacactg 240
ctgatactgt taatttaact actaaagtct ggatgtcctg gcatggcaga caaaagtttc 300
attcattatc cagtgaaaca ctgaaatgaa attaggatga gcaagtcact gagttctttg 360
gaacgggaat cctcacggtt ctcctacagt cctaagaatg cttacaaaac aacgggctta 420
tatcaatgcc agatctggaa gccatgggct aacttgacaa gcccactgga tttgacttta 480
gcttcttgct ctagccagag gttaccaaca cctaaacaca aaatagacta tctgctcctg 540
actgctgcag aactaaagag ctgactttat tcttgagttc tttattcaga gtcccagaaa 600
attatttcta aaatgcatgc tgggataact ttcaaaatca ggatttggtt tactttgcaa 660
cagatattac cacacaaaaa atattaaatt ttagaaaaca agacaaatca tctttaactt 720
aatgccctag tcttccttaa caaaagcaaa gacacaaant taagaag 767

<210> 209
<211> 468
<212> DNA
<213> unknown

<220>
<223> >gi|4735332|gb|AI651353|sr|IMAGp998F035711.1|cl|IM
      AGp998F035711|AI651353 wb05c02.x1 NCI_CGAP_GC6
      Homo sapiens cDNA clone IMAGE:2304770 3' similar
      to contains Alu repetitive element;contains
      element TAR1 repetitive element ;, mRNA sequence

<400> 209
cttctttttg cttctctttt ctttctttct ttctttcttt ctttctttct ttctttcttt 60
ttctttttct ctcttcccct ctttctttcc tgccttcctg cctttcttct tttcttcttt 120
cctcccttcc tcccttcctt ctttcctccc gcctcagcct cccaaagtgc tgggatgact 180
ggcgggaggc accatgcctg cttggcccaa agagaccctc ttggaaagtg agacgcagag 240
agcgccttcc agtgatctca ttgactgatt tagagacggc atctcgctcc gtcaccccgg 300
cagtggtgcc gtcgtaactc actccttgca gcgtggacgc tcctggactc gagcgatcct 360
tccacctcag cctccagagt acagagcctg ggaccgcggg cacgcgccac tgtgcccaca 420
ccgtttttaa ttgttttttt ttcccccgag acagagtttc actctcgt 468

<210> 210
<211> 726
<212> DNA
<213> unknown

<220>
<223> >sr|IMAGp998O184348.2|cl|IMAGp998O184348|gb|BX1054
      24|oid|234545229 BX105424

Soares_pregnant_uterus_NbHPU Homo sapiens cDNA
      clone IMAGp998O184348 ; IMAGE:1712105, mRNA
      sequence.

<221> misc_feature
<222> 637, 721
<223> n=a, t, c, or g

<400> 210
acggatttta ttaaaaccta tgttattttt tgttgctaat catttgacac ttaatcgaaa 60
cagaataagt tactgtggaa aaaaggatga tccaactcct gaggattgcc gcctgtgtgt 120
ctttaattga ttatctttga tagttagagt tatttttcag atttttcccc ctcatactgg 180
attgtgatgc tctgatatga gcatgcaatg actgatacag aaataggctg gagcttcggt 240
gtgtgctttt gctatacagc ttacagttct aatgcctgat gtagagatga tgaatttctc 300
ctttttaaac ctatcttctg tttactcctt ttgcattatg aaggccctct tcaatgatgt 360
atgtttctga tggaatcaga aaatttcaca ctagaaaagt gtacctaact tgattcagtg 420
tgagctcttg ggacagggag gggagccagg ggtagagctt aaagaagctg gctctgtaag 480
agttgtatat caaagtacct tttgctacaa ggtgcatata gaagtctaag ataagaaatg 540
taccctctat actttatttt tcatgcccct atcatgtttg gtgtgcctta aatcttacct 600
ttaaatgaag actctatgga ttgtttacaa atgaagnttt acactaggac cagatcgtcc 660
atacatgatt ggtcttctac aataataatg ttcaattctt tcattttaaa tctttcttta 720
nggtca                                                            726

<210> 211
<211> 391
<212> DNA
<213> unknown

<220>
<223> >gi|1512724|sr|IMAGp998B201153|cl|IMAGp998B201153|
      gb|AA037588|AA037588 zk36d10.r1 Soares pregnant
      uterus NbHPU Homo sapiens cDNA clone 484915 5'

<221> misc_feature
<222> 85, 96, 167, 174, 218, 242, 297, 312, 332, 366
<223> n=a, t, c, or g

<400> 211
ccaaattgct caacagtatg tatgtttaga ggggttagac tccttttgaa aatctggata 60
tctaaccacc tacttaaatc tgttngatag tgtcanacca cccccaccct tgatcctccc 120
acccccaaga aaaagaaaag atgctcctgt taaatgaagg ctctttnggg gtangtgcta 180
ggtatactct tggctagatg gattcagctc agaatttntg actttttaaa gctaagtcct 240
tngtagtcac atggttgact tgaaaggatg aggtggtgtg tgcatttatg acgcttntcc 300
tacccccctg anagaaactt cattggccgc cntggttact caccttgaag agagtaccat 360
ccattntacg tgaaaaacac atctgtcacc c                                391

<210> 212
<211> 658
<212> DNA
<213> unknown

<220>
<223> >sr|IMAGp998P08567.1|cl|IMAGp998P08567|gb|BX099307
      |oid|234532990 BX099307
      Soares_placenta_8to9weeks_2NbHP8to9W Homo sapiens
      cDNA clone IMAGp998P08567 ; IMAGE:260215, mRNA
      sequence

<221> misc_feature

<222> 564, 584, 601, 612, 616, 619, 627, 630, 644, 656
<223> n=a, t, c, or g

<400> 212
tctcgtctca catttgcact ctctccttcc cacattctgt gctcagctca ctcagtggtc 60
agcgtttcct gcacacttta cctctcatgt gcgtttcccg gcctgatgtt gtggtggtgt 120
gcggcgtgct cactctctcc ctcatgaaca cccacccacc tcgtttccgc agcccctgcg 180
tgctgctcca gaggtgggtg ggaggtgagc tgggggctcc ttgggccctc atcggtcatg 240
gtctcgtccc attccacacc atttgtttct ctgtctcccc atcctactcc aaggatgccg 300
gcatcaccct gagggctccc ccttgggaat ggggtagtga ggccccagac ttcaccccca 360
gcccactgct aaaatctgtt ttctgacaga tgggttttgg ggagtcgcct gctgcactac 420
atgagaaagg gactcccatt tgcccttccc tttctcctac agtccctttt gtcttgtctg 480
tcctggctgt ctgtgtgtgt gccattctct ggacttcaga gccccctgag ccagtcctcc 540
cttcccagcc tccctttggg cctncctaac tccacctagg ctgncaggga ccggagtcag 600
ntggttcaag gncatnggna gctctgnctn caagtctacc cttnccttcc ggactnct 658

<210> 213
<211> 548
<212> DNA
<213> unknown

<220>
<223> >sr|IMAGp998A191006.1|cl|IMAGp998A191006|gb|BX1180
      17|oid|234570410 BX118017
      Soares_fetal_liver_spleen_1NFLS_S1 Homo sapiens
      cDNA clone IMAGp998A191006 ; IMAGE:428442, mRNA
      sequence

<400> 213
cagatctcta ccagctgctc cagagactct tcaaaagcca ctcatctctg gagggattgc 60
tcaaagccct gagccaggct agcacagatc ctaaggaatc aacatctccc gagaaacgtg 120
acatgcatga cttctttgtg ggacttatgg gcaagaggag cgtccagcca gactctccta 180
cggatgtgaa tcaagagaac gtccccagct ttggcatcct caagtatccc ccgagagcag 240
aataggtact ccacttccgg actcctggac tgcattagga agacctcttt ccctgtccca 300
atccccaggt gcgcacgctc ctgttaccct ttctcttccc tgttcttgta acattcttgt 360
gctttgactc cttctccatc ttttctacct gaccctggtg tggaaactgc atagtgaata 420
tccccaaccc caatgggcat tgactgtaga ataccctaga gttcctgtag tgtcctacat 480
taaaaatata atgtctctct ctattcctca acaataaagg atttttgcat atgaaaaaaa 540
aaaaaaaa 548

<210> 214
<211> 528
<212> DNA
<213> unknown

<220>
<223> >sr|IMAGp998B02152.1|cl|IMAGp998B02152|gb|BX110160
      |oid|234554699 BX110160 Soares infant brain 1NIB
      Homo sapiens cDNA clone IMAGp998B02152 ;
      IMAGE:31971, mRNA sequence

<400> 214
tttttttttt ttttgaaggg acaccgattt attgagtagc agagctgagg gaaggcgtag 60
gatggctcca gcttccggtc agtggctaca tggtcagttc catgatggcg ttgacgatgt 120
cactgtggtt gtctctcaga gcccgcacgg ccttggccct ggacacattg gcctgcgcca 180
tcaccagctc aatgtcacgc agttccagcc ccgcctcgtc cacctcttcc tcctcctcct 240
cttcctcttc cttgcactcc agcctcaccc ggggcctggg tgctgactca gggaccaagg 300
ctgagggctc tgagggcacc ttaaacttct cagctgcggc tttgtgcact tgctgggaca 360
ggtcctcaat cttggcctcg ccaaagacca cataagtgtc tgaggctggg ctcttgaaga 420

catcaggctt ggcgatgaca aagaggatgt tcttggactt ctggatggtg atcctggtga 480
ctccctgaat ctgccgcaag cccagctttg acattgcctt tcgggcct 528

<210> 215
<211> 2341
<212> DNA
<213> unknown

<220>
<223> >Homo
      sapiens|chromosome:NCBI34:7:64716658:64718998:1

<400> 215
   gcagattctc caggcccagc atctgcctca ccgtggcccc ccacaagcca agcgcctgcc 60
   tttcagcagc ctctacacac ccagctcctg ccacccaatg gctctttagg ccaagctcat 120
   acctcacgat gatttttcca ggcccaactt ttgtctcatg gcaaccttcc ctggccaagt 180
   ttccacctat ttcctggcag cctggacagg cccaggtcct gccacacact ggcctctcta 240
   cgcccagctc atgcctcaca gtggcctctc caggcccagc tcctgtcccg ggacatcatc 300
   tccaggccca aaacttcctc aagtcggcct ctccaggccc agttgctgcc tcccggcatt 360
   ctctccaggc ctagctcttc ctcctggctg tatctacaag accaactcct gcctcacaac 420
   aaccttttat ggctcagctc ctgcccaact actgccggcc tttgtaggcc caaaacttcc 480
   tcaagtcaag ctctttaggc ccaccttctg ccttgcagtg gcctgtacag acccagctct 540
   ggcttgagaa cagcctctgc aggccctgct cttgcctctt agctccctct ccaggcccat 600
   ctcttgcctc acagtggctt ccgtgggcca agttcccgcc tgcctcccag cagcctcaac 660
   aggcctagct cctccctcac aatggcttgt ttaggtccag ttgatgcctc tggcaacctg 720
   tccaggccca gctcctgcct cacactggcc tctctaggcc gaggtccttt ctcatactgg 780
   cctgtttagg cccagctcat tcctcttgtc atctctccag gcccagcttt tgcctgttgt 840
   tggcctctac ctcacagtgc accttccagt cccacctctt gcctcaccat ggcctcctct 900
   gaccaggttc ctgcctttcg gcagcctcta caggcctagc tgctgcctcc caatggcctt 960
   tgtaggccac gctcatgcct cactgtggcc tttccaggcc tagctttcgc tttttggcca 1020
   ctccaggccc agaacttccc ccagtcagcc tctccaggcc cagctcttcc tcccagcaac 1080
   ctctgcaggc ccaaatcatc ctcaaattgg cctcttcttt cccagctcct gcctcctggt 1140
   ggcctctgaa gacccaaatc gtcctccagt tggtttttcc aggcccagct cctgcctttt 1200
   ggtggcctct ccaggtgcaa aacttcctcc catcagcctg tccaggccca gctcatgcct 1260
   cttggtggcc ttctcaggcc ctgcttttga cttggtggcc tcttcaggcc cagaacttga 1320
   actcaagtca gcctctccag gcccagctcc tgccttctta aggtctgtac aggcccagcc 1380
   tctacctcac agcggactct ccacacccag ctcttgcctc actgtagcct ccccagtcca 1440
   aaactcctgc cttttggcag cttcgacaag cccagctcct gcctttcaat gacctcttta 1500
   ggccccgctc attccttaca acggcctttc caggcccagt tttccctttt tggcggcctc 1560
   tccaggccca gaacttcctc aagtcggcct ctttaggccc agttgctgcc tcctggcatc 1620
   ctctgcaggc cgagctcttc ctccctgctg tgtctacagg cccaactcct gcctcacaac 1680
   aacctccttg gactcagctt ctgcccagct cctggtggcc tttgtaggct caaaattttc 1740
   tcaaatcaag ctctccaggc ctactgtcag cctcgtggca gcctaaacag gcccagctcc 1800
   tgcctgacaa tggcctctcc aggcttttct cctgcctcgc agcaggcttt ccaggcccag 1860
   ctcttgcctc atggtggcct tccccggcca tgttcctatc tgacttctgg cagcctcaac 1920
   cggcccagct tctgcctcac actggcctct ctaggcccag ctcctttttc acagtggcct 1980
   cactaggccc atctcctacc tcagatctgc ctcccaagac ccagctcctg tctcatggtg 2040
   gtctctctta caccagctcc tgcctcacaa tggcctcgtc tggcccatct tctgcctcac 2100
   agtggccact caaggcccat cttttgcctc atggtagcct cttctggttt tgctcttgcc 2160
   tcacagttgc ctcttccaga tccagcttta agcctttgat ggtcaacagc atcaaggagc 2220
   ctaaagcttc cctggactct catttgttca ctttacagca gagtgcctta gcaaaaactg 2280
   tctcttaacc ttgagagtgg atttctgaca aatcgatagt aaattctgcc tgtgtggttt 2340
   c 2341

<210> 216
<211> 407
<212> DNA
<213> unknown

<220>
<223> >gi|944178|gb|R85772|sr|IMAGp998I12605.2|cl|IMAGp9
      98I12605|R85772 yq23e06.s1 Soares retina N2b4HR
      Homo sapiens cDNA clone IMAGE:274643 3', mRNA
      sequence.

<400> 216
gacacaattc caatgtgtga acatttcagt tgtcatgttt cattcaacaa caccagtctc 60
ccaacaaact ggctcaaatt tcagttacct tggtattcag atgagtaact gcatctcagt 120
ttcttcagtc cacatattac aatgtgaata acagatgtac attgtgatca gtgaccggtc 180
acgtcacttc cttcaaagtc tgtcaatgat tggtcattgc ccttctgtta tttggctcac 240
acacagacag caaaacatgt gttgccttcc tgtctctcag cagtagacag atactataga 300
ccaaataatt taaccattaa agcactacaa ctattgggat aatgctcttg ggtcacccta 360
attctcttgg gggattgaaa ttaaaatgtg aaaatttgct ttatacc             407

<210> 217
<211> 585
<212> DNA
<213> unknown

<220>
<223> >gi|4373025|gb|AI479857|sr|IMAGp998F015343.1|cl|IM
      AGp998F015343|AI479857 tm70c01.x1 NCI_CGAP_Brn25
      Homo sapiens cDNA clone IMAGE:2163456 3' similar
      to TR:Q13231 Q13231 CHITOTRIOSIDASE PRECURSOR. ;,
      mRNA sequence.

<221> misc_feature
<222> 319
<223> n=a, t, c, or g

<400> 217
cattcagtaa ctatttattg gatgtctaat acatgcttag tatggtctct ggtcatgcaa 60
cgaatgttaa ttcccctgtc tctttgaagg aaggactaat ggctggacac taatattaaa 120
agagtcagtg gtattcaata aaagggcttg ggagagtagt gctcttgtca tgaaatagca 180
cttcagtctg gaaatcctct aagtgatttc tgcactcagt tcctgccgta gcgtctggat 240
gagggggtat cggccctggt tgcaggagaa gccggcaaag tcatttaagt ccagtgccca 300
gaccatggcc ccgcccagnc ccttctgctt cagatagctg accttggttt tgaagctctc 360
cacatcatca aagcccaccc actggttgtc ccggaagatg tagggcacct tctgatcctg 420
gattctctgt ttggtggccc ccttccaaga gcagactata taataggcca acatcccttc 480
tttcttggtg aaggggcctg gagggccaca ccctgtgtgt ggggccccct actcttggtt 540
cttatgaggc ggcagggaga aggagccccc cgaggtggcc tcccc               585

<210> 218
<211> 534
<212> DNA
<213> unknown

<220>
<223> >sr|IMAGp998B171824.3|cl|IMAGp998B171824|gb|BX1158
      55|oid|234566085 BX115855 Soares_testis_NHT Homo
      sapiens cDNA clone IMAGp998B171824 ; IMAGE:742576,
      mRNA sequence.

<400> 218
ttggcagaca tgaggcaacg attgctcccg tccgtcacca gccttctcct tgtggccctg 60
ctgtttccag gatcgtctca agccagacat gtgaaccact cagccactga ggctctcgga 120
gaactcaggg aaagagcccc tgggcaaggc acaaacgggt ttcagctgct acgccacgca 180
gtgaaacggg acctcttacc accgcgcacc ccaccttacc aagggacagg ccagcagcac 240

agacaacgct gtggataagg agagtagaga cttcctcttc ctgcctcctg tcttctggag 300
ttgtgaccgc agggtggccc aggtggatcg gcttcagagg ccaggaggca gctctctgca 360
gccgaagagc aggagcctca cccagggtct gtggctctga ctaagcctgg actgcctctc 420
ggctgtgctc cgtggactgg ctcctcaggg atccatgtga gagaccggag tatgatcctc 480
agtgcgagga caaataaaag tagtgattat gtccacccca aaaaaaaaaa aaaa 534

<210> 219
<211> 741
<212> DNA
<213> unknown

<220>
<223> >sr|IMAGp998L24923.2|cl|IMAGp998L24923|gb|BX096399
      |oid|234527172 BX096399 Soares pineal gland 3NbHPG
      Homo sapiens cDNA clone IMAGp998L24923 ;
      IMAGE:396839, mRNA sequence.

<221> misc_feature
<222> 687, 713
<223> n=a, t, c, or g

<400> 219
ttagtcccta attctagacc ttacccattt tacacacact attagctaaa tcgtgttcct 60
gaggcatggc tctgattact ttactctggc tttagccttg gcctcatccg ttaaattgaa 120
actgttcacc agacctgtat gcattaaaaa tttttttatt agaaaaatca gtgtcttgct 180
agccatgtat gatagctatc aaattgatca ttctgtaaat ggtagcaata agtgtatcaa 240
cactttgacc tcctctgtgt ctagtaacat ttgcatatag tacaactgag cacatagcac 300
cagaatattg gaacagatgc ttctccaaag ccaagggttg ccttacattt agagtgggaa 360
aagaggaaaa tgagttagca gaggagcagc ggggaggggg gtaaattaca ggacataatt 420
tgcttctaaa aatttgtgtt tcgtgttgta gttatataat taacttttct attgtactga 480
ttgacagatt tggcacagac atcctctcat tgtgaatata tatgaatata tcagagtttt 540
ggactctggg gtatatttac ataaacacaa gtattctgaa agcattgata accacactgg 600
tgtctcttaa ttttgtctat aagatgacct tataaaatcc ttcaagaatt atttaaatca 660
agtgtacgaa gctctgcagt tttttgntat gtctgaaata ttcattaaaa agnttattaa 720
aacctgaaaa aaaaaaaaaa a 741

<210> 220
<211> 393
<212> DNA
<213> unknown

<220>
<223> >sr|IMAGp998E135802.2|cl|IMAGp998E135802|gb|BX0968
      12|oid|234527996 BX096812 NCI_CGAP_Lu24 Homo
      sapiens cDNA clone IMAGp998E135802 ;
      IMAGE:2338548, mRNA sequence.

<400> 220
cccctcttcc caaagatgtg gtgacttaag aggctctcta agcacactgt tgactccaaa 60
acatccgcag gtcagagcca ggtgggaagg tggtccgtgc aggatgtgcc aggccctgtg 120
gcaggtcttg ccccatgagt ccatatgcca gtgggcagtt tccagtacac aggtgatggc 180
agccagagcg cggccccaag tgcaacacgt tctttggtta ttggctcaga agcctggaaa 240
caggagcttc tgcggacctt cacgcacgtg ttattcccaa aatgccacta gatggcagca 300
gagaagaact tttccctgat ttaagcgctc tgcgagcagc cactcagcat tacactaaga 360
attcagaccc ctggtactca ggggtcttcg aga 393

<210> 221
<211> 620
<212> DNA

<213> unknown

<220>
<223> >sr|IMAGp998D171153.1|cl|IMAGp998D171153|gb|BX0985
      06|oid|234531385 BX098506
      Soares_pregnant_uterus_NbHPU Homo sapiens cDNA
      clone IMAGp998D171153 ; IMAGE:484960, mRNA
      sequence.

<400> 221
cgaagggacc ctgggcgtct gaggccctga tccgggcggg ccttcaggat gttagcagtc 60
cctgtccggt tgaaggtagg aagccgaaaa cccgagtggg ggacgaacag acttacctcc 120
tgtcccgcaa aagatccact agatcgtcgt ctccaaaacc tgagagaccg cgaacgggtt 180
ccagagccgc agcgttctct cagacctggc gttcaggaag attctagaga gcacgggcag 240
gtcccggagg tctcagaccc gcaggcctgc ggggcggggg cggggtggcg cccaggacga 300
ctccccgggc tcagcttggc tgcctgcctc cttctcaaac aatgagtacc tactgtgtgt 360
agaagcagca ggacagcctg tcatcactgg gaaagaggcc agaagagccg tttggccagg 420
gtctccaatt taggctttca acattatctc taaagaaggt tatacattat gtcggctcca 480
ctggctgatg ggatcaaaga ccctccttgt atatccttaa acctgtactc tattaccagg 540
ccactgtgcc tgtggagcac tgtaagttcc taccttttaa taaattttat aattatcttg 600
tgagcaaaaa aaaaaaaaaa                                             620

<210> 222
<211> 696
<212> DNA
<213> unknown

<220>
<223> >sr|IMAGp998F17593.1|cl|IMAGp998F17593|gb|BX117945
      |oid|234570266 BX117945 Soares melanocyte 2NbHM
      Homo sapiens cDNA clone IMAGp998F17593 ;
      IMAGE:269968, mRNA sequence.

<400> 222
ctactgtacc ctggcctggg aacacattgc cctccccaca caaaactgga gggcttggct 60
tctgcgtgtg agaaatcaac atttttaaag cacttgcctt ctaccaaccc cagcttgcaa 120
tcactgggcc ttcccctcct atccaagggg ttggaggggc cccttggctc tccttttggc 180
aggaggagcc tgcttcatta caccaatgac tctgccatcc ccctccctgg ccctagaccc 240
caaacacatc tccctctacc caatttactc ttctcgcccc acctagggac agattccccc 300
tgctcttttt gtcctagaaa ccccgctagt ttgggatggt agcgtctggg gtggggaggg 360
cttccccttc cccactcgag ggtgcgggtg gggaaggggg ggtggggtgg agacagccct 420
ggggcaggga ggatggtctc tccactgtag aaagtagagt aggattgtgg tcagacttaa 480
tttgaggcat ctagtgaaga cacgtacaaa tccaccaagg aaaaagattt caaaagcaaa 540
ataaaagcgg gaaataaaac agacccaaga ataatcaagt caaagtgatg ttgcacaaaa 600
tgcagagaaa ccaagaaggg ggagggttaa tgtattaaat gtgctattaa gaacttaatt 660
ttattaaaag tactattact taaaaaaaaa aaaaaa                           696

<210> 223
<211> 215
<212> DNA
<213> unknown

<220>
<223> >gi|917103|sr|IMAGp998N11360|cl|IMAGp998N11360|gb|
      H41051|H41051 yp52c06.s1 Homo sapiens cDNA clone
      191050 3'.

<221> misc_feature
<222> 16, 36, 38, 44, 54, 123, 170, 211

<223> n=a, t, c, or g

<400> 223
tccaacccgc ggggancagg tgtccccgga ctgggntnct gttnggaaga tggncgaccc 60
cgggcatgag ctggcagcag cactattacg gcggctcgcg gccaaattcg cgccctcgcc 120
ggnccaccgc acagctggct gggcacagca ttgactacag ccaggagatn cacctgaaaa 180
taagcaagaa aatcgcccag ctcaaccaag nttaa 215

<210> 224
<211> 473
<212> DNA
<213> unknown

<220>
<223> >sr|IMAGp998I152328.2|cl|IMAGp998I152328|gb|BX0952
      10|oid|234524789 BX095210 NCI_CGAP_Co3 Homo
      sapiens cDNA clone IMAGp998I152328 ; IMAGE:936278,
      mRNA sequence.

<400> 224
atgacctctt tagactcagc tcattttcac tgctacatct tcaagccatt ctcctgcctc 60
ttggcaacct ctagtggccc agcttctgcc tcacagcagc ctctccatgc atgcctagct 120
cctgcctctt tagggaactt acaggcctaa aactttctta atttgggctt ctcaggccca 180
gctcctgcct tctgttggcc tctacaggcc tggcatcatc ctttcaacag cctctttagg 240
cccggcctct ccaggaccaa aacatcctta agtcaacctc accaggcccg gctcctgtct 300
ccttgcggcc tccagaggcc gagcttttgc ctgccaatgg cctctctagc cccagctttt 360
gcctgccaat ggcctctcta gccccagctc ctgcctctca gcaccctctt taggcccagc 420
tcctgcctta ataaatttga ataaattatt gttatgtgaa aaaaaaaaaa aaa 473

<210> 225
<211> 279
<212> DNA
<213> unknown

<220>
<223> >sr|IMAGp998I112449.2|cl|IMAGp998I112449|gb|BX1058
      75|oid|234546131 BX105875 NCI_CGAP_Pr12 Homo
      sapiens cDNA clone IMAGp998I112449 ; IMAGE:982738,
      mRNA sequence

<400> 225
aattcgcggc cgcgtcgact tttttttttt ttatcgtttt gttttaaata taaagagcta 60
ttttagctct cttgaatcta aaaaagcaac ttaagtttgt cggttactgc tattgggatt 120
accttatgaa atcataacat aatgttgttt ttaaatatga aatcattttc ctacgaattt 180
cccaatttgt gtttcataaa cattccagta agagaaaatg attgattaca aagcagaata 240
tcaaaggttt ttaataaatt gtcgacgcgg ccgcgaatt 279

<210> 226
<211> 547
<212> DNA
<213> unknown

<220>
<223> >sr|IMAGp998A17282.1|cl|IMAGp998A17282|gb|BX091082
      |oid|234516531 BX091082 Soares infant brain 1NIB
      Homo sapiens cDNA clone IMAGp998A17282 ;
      IMAGE:50008, mRNA sequence.

<400> 226

tttttttttt ttttaacact tcttaaatat aaagttttgc aacaaacctt tgatttatat 60
caaatataat taaaaattaa caaatgaata cattgtatca aaaagtacga agatatacat 120
tatcatgcac atacttgtat atatgtatat atgtatatat gttacatgca cacataactt 180
gttcaaacta cagccactgc ttgactggtt ccagatgcag acaactgggt gatgcacttc 240
aaatttggca gctcactatg agattcaggg gacatatatg atagccggag aagacacagt 300
gagaaacaaa acccaaagtc aggaaaacaa gacataatgt ctcctttccc cgctccctgt 360
agttttcatc aattccaatg cctagaagag tctagacaag ccagctaggc tgtgtgggtt 420
tagaaaagtg gattctcgac ccaacactgc ctggggaaat ggctgtggtt ctgcatggaa 480
tggacacatt cgacacccaa gataacatgg gctgggggct ggggaacaag agacaaaacg 540
tcagtgt 547

<210> 227
<211> 328
<212> DNA
<213> unknown

<220>
<223> >gi|3246373|gb|AI026885|sr|IMAGp998J014173.1|cl|IM
      AGp998J014173|AI026885 ov92c01.x1
      Soares_testis_NHT Homo sapiens cDNA clone
      IMAGE:1644768 3', mRNA sequence.

<400> 227
ttttgtattc actagacttt tatttcgttc cagggcttat gacagagccc aagagagtca 60
gagaagcttt ctacaaattc agctgtcgca ttcattttct tacacactag agttgccact 120
taaaaatact aaaataaaat aaaaaattaa tgttgaccat ctaacaggaa tacttgactc 180
agctactaag ttacaaattt gcttcatttt tggaagacat ctagcatctg agaattggac 240
ttttcccaga agttcacttg gtaacctcag gtaagtcctg gttcatcctg gagaggatcc 300
gaggggaaag cctgatggga cccacggc 328

<210> 228
<211> 262
<212> DNA
<213> unknown

<220>
<223> >gi|4892849|gb|AI682667|sr|IMAGp998O235762.1|cl|IM
      AGp998O235762|AI682667 wc64e12.x1 NCI_CGAP_Pan1
      Homo sapiens cDNA clone IMAGE:2323438 3', mRNA
      sequence.

<400> 228
   tttttttttt tttttttttt tttttttttt tttttttttt ttttttttta aacaattaaa 60
   aaatttatgg gcttccgggg gccccccatc agcgggcaaa aaggcccctt taagcaaaag 120
   gttttcagtt ttcccgggcc ccatttggtg aacaggaacc catccctggg gaaaattctt 180
   tttaaaaaaa ctgggcaaaa accccttggg ccgggaaggg ggattttttt ttttactttt 240
   ccttcccccc attttttcct tt 262

<210> 229
<211> 477
<212> DNA
<213> unknown

<220>
<223> >sr|IMAGp998C01743.1|cl|IMAGp998C01743|gb|BX099107
      |oid|234532590 BX099107 Soares_fetal_heart_NbHH19W
      Homo sapiens cDNA clone IMAGp998C01743 ;
      IMAGE:327480, mRNA sequence.

<221> misc_feature
<222> 470
<223> n=a, t, c, or g

<400> 229
gcaaggctag aaagatgcta cgttgggcct tcagtcacct gatcagcaga gagactctca 60
gctgtggtac tgccctgtaa gaacctgccc ccgcaaaact ctggagtccc tgggacacac 120
cctatccaag aagacccagg ggtggaacag cggctgctgt tgctcctggc ctcatcagcc 180
tccaaactca accacaacca gctgcctctg cagttggaca agacttggcc cccggacaag 240
actcgcccag cacttgcggc tgggcccggg gagcagtgag tggaaatccc ccacgagggt 300
ctagctctac cacattcagg aggcctcagg aggccagcct gccatgagag cacatgtcct 360
ctggccagga gtagtggctg agctctgtga tcgctgtgat gtggacccag cctccaggga 420
gcagagtgtc ggggatggag gggcccagcc tggactgact gctacttccn gtctctg 477

<210> 230
<211> 468
<212> DNA
<213> unknown

<220>
<223> >gi|4081545|gb|AI344339|sr|IMAGp998D175081.1|cl|IM
      AGp998D175081|AI344339 tc03g09.x1 NCI_CGAP_Co16
      Homo sapiens cDNA clone IMAGE:2062816 3' similar
      to contains TAR1.t3 TAR1 repetitive element ;,
      mRNA sequence.

<400> 230
gcggccgcca aactccgtaa ggatggcgtc ggccgcccgg cctgcccctc cctgggcccc 60
gctgccctgc acctccgcct tcagccatag gcgctggagg ttgtgctgac gctcgaaggt 120
ccagaccagg gcctcctcgc gactgcgggc aaaggtgcac cggtttcggt ggcgccggca 180
gccgagccca gggctgtagt agtggcagac ttggtagcac aggggccttg ggaaggtggg 240
ccggcagccc accacgcgcc agaccttgct cttggtggcc tgcttaaagc gggccagcag 300
gatctcgcgg gagcagtcat gctccaccct acggaggacg taggtgcttt cattgagccg 360
ctgggtgcaa cgggaggcag cccagaccca ggtccaccag ggcacacagc cgggccaggg 420
acgggcctct ggtggccgcg gggctgttgg gcagcagggt ggaccctg 468

<210> 231
<211> 367
<212> DNA
<213> unknown

<220>
<223> >sr|IMAGp998B2474.1|cl|IMAGp998B2474|gb|BX089396|o
      id|234513140 BX089396 Soares infant brain 1NIB
      Homo sapiens cDNA clone IMAGp998B2474 ;
      IMAGE:23877, mRNA sequence.

<221> misc_feature
<222> 55
<223> n=a, t, c, or g

<400> 231
tttttttttt ttttaagtaa tggtaaagtt tatttcattt ttaataacaa ttagnaggac 60
aaaatgttta aaatttgcag tttaaaacat gcacattcac aaaaggccaa tgacacataa 120
cactgcatag aaataatatt actcaatttt aataactata aaacacagtg catcaaacat 180
caagataaac aaacctgaga aaactactat aaccacagat tcaatactct ccactcatgc 240
agcttcacaa tttctacagc agtttcagca ggaatggttt ttcagggagc tgaaaatact 300
actttatctt taacgcaaaa ctgcagtttt ctgtagtagc tgccttccaa ggctgccctg 360
tcttttc 367

<210> 232
<211> 498
<212> DNA
<213> unknown

<220>
<223> >sr|IMAGp998I094494.2|cl|IMAGp998I094494|gb|BX0959
      09|oid|234526189 BX095909 Soares_testis_NHT Homo
      sapiens cDNA clone IMAGp998I094494 ;
      IMAGE:1837520, mRNA sequence

<400> 232
gcaacctcta gtggcccagc ttctgcctca cagcagcctc tccatgcatg cctagctcct 60
gcctctttag ggaacttaca ggcctaaaac tttcttaatt tgggcttctc aggcccagct 120
cctgccttct gttggcctct acaggcctgg catcatcctt tcaacagcct ctttaggccc 180
ggcctctcca ggaccaaaac atccttaagt caacctcacc aggcccggct cctgtctcct 240
tgcggcctcc agaggccgag cttttgcctg ccaatggcct ctctagcccc agcttttgcc 300
tgccaatggc ctctctagcc ccagcttctg cctttcatcg gtctctccag gcttagctcc 360
tttctcttca cggcctctgc aggcctaaaa cttcctcaat ttggcatctc caggcccagc 420
tcctgcctcc aggccgcctc tgcaggccta tctcaagcct tacaacagcc tctttacccc 480
cagctccttt ctccgact                                                498

<210> 233
<211> 591
<212> DNA
<213> unknown

<220>
<223> >sr|IMAGp998B08733.1|cl|IMAGp998B08733|gb|BX102114
      |oid|234538608 BX102114
      Soares_senescent_fibroblasts_NbHSF Homo sapiens
      cDNA clone IMAGp998B08733 ; IMAGE:323623, mRNA
      sequence.

<221> misc_feature
<222> 462, 482, 513, 558, 564
<223> n=a, t, c, or g

<400> 233
ataactgata cttagctaac aattgtccat aacttgttta agtgtaagaa ataccatttc 60
aggaatgaaa aagaaggaat actactttct aagaaagaag atcttataat agacatattt 120
agtaggttaa actactcctt tgaaagaata agttttggtt caaagtcaat aatgaagacg 180
agatttgttt ttctcttctc tggctgattt ttaaggatat tatgtagttc atttagttaa 240
cagaattgaa atgtttgata tggcaagata ggtatggtaa tttcaaagtg aattgggaat 300
tcctctggct catagaaccc tttttttttt cctttaagta ttcttgagat acaaaaaaaa 360
aaagtaaata aaatttcaaa aaaaaagttc cggatctgtt tttaagctcc atctggtcct 420
cataacctgc aagatttttc ttaaaacctt tcagctgaaa gngggggtaa aggtggagta 480
anctgtggat tcgcttctgt tgtcttttaa aangtcaaat atataacatg taattttttt 540
aaaaaccacc agatacanaa atgngcttta acatcagcct gaaacctaaa t          591

<210> 234
<211> 8483
<212> DNA
<213> unknown

<220>
<223> >Homo
      sapiens|chromosome:NCBI34:4:155279369:155287851:1

<400> 234
ataggtaaaa caggatgtaa agtttatata caagaatata atgtttatct gaaatattta 60
cagtgttggt taaagcaata tttttacaac ttttaaaggt aaactactat gtatattaca 120
ggtaagctac aatgggttta atttgcaaaa gttaagtaag aaatgtttta aacaaggctt 180
aaagtactca agtcaattat aaaatttata tcttttgcct tttacttgaa gaaatcatgc 240
tatagaaatg gttaatgtgc ttctaataaa tggaagtatt gtagctggaa tgtgatacat 300
gtaacagttt aagttcccat tgaaggtata aaatgatgaa ttgttgtaag acttagacac 360
tgagtctcag tctggagctg atgaagatgt tgagataaca gccagcttta tctcaacagg 420
gtttgtgacc cacaagtttg ggccacagag aaaattgaag caatttgcat gttatgacaa 480
cctcagtggg aagtgaaaat cagctgactc aaaacaacaa acaacaacca accagaccca 540
agtcacagtt gcacctattc aaaactagct ttaaagtgag ctatttttaa acttcataaa 600
aatattcatg attttattag tttgaatatt tctacaagat tcgggtgggc ttttccttta 660
ggtgaaaaca gctatccact cctgtggcct tataactcag gaaatgctgg ggatgcaaac 720
gtgcaaaagg cagggggaag ctgcccaggc tgagactgga gcagctagga gtgtgcttgg 780
ggaacgggag ctgagatccc ggagcagaaa tggtcagccg tgctctggag caggcctgtc 840
ggagccatca ggatgccggg actagcactg cagcttgcgg atgctgcggg agatgcgctt 900
gaactctctc tgccccttct gccaccgctt caccgaggtg atcaccagct ccccaccctg 960
tttctgtccc atgaccagat agggcgcgtt gatgtcgttc atctcctcac aggtgcactg 1020
caagctgtct ttgagccaca gcaccgattt cttcaggtcc ctttcggaca caccgttcag 1080
cttgtaaatg gtcttgctct tggtctccag gatgattttg gtatctcggt tgatgtaggt 1140
tatctccttc acttttattt tcagtgctat gagggagggc agaaagagtc atgccagtta 1200
taatgaggga atacagtata cctcccccca ttctgccaac tcgtgtgact tggcctggat 1260
gaggaaagag aggcttccta gagctaatgg gaccctcaga ggctgcagat cgggggttgg 1320
gagctagggg cagcaggtgg ctccactgct aaagcctcaa agcatttgcc aggcacttgg 1380
tttccaatca gccatttttt caaaactctc tctttgcggg actcaattaa cttttcttct 1440
cctttcctca ctcacccttg tgtttgtaag aaagttgcat gtggtgtgtg ctctctccaa 1500
caaatgactt cctctgttgg aaacttaagc atgaaaagaa atgcctgact tttaggtgaa 1560
atatgatccc tctgtggccg taaagtttaa actcaattta ttatgcaaga ggtttttgga 1620
caaacctgta aggaaactgt gtcacgcaat cattgttttc taaagatcag attacaggga 1680
aggagaattg atcttacagc tgcatttgat ttggtaaagc aggagaaaca atatgcaagt 1740
ctctttttaa cagcacaacg tggttagtca taaaagtgtt tttctgaaga cctcatcctt 1800
atatgctctc atttgccagt ccaaggccaa ggcgtttacg gatcatatat gtactgtttc 1860
caaaatgtat atgtagactc gttagaatgc gaagaccaaa agtaagtggt ttactataaa 1920
ggacacgttt tatagtcatg aacagtatgc tctgggaaag aaagccctac aaacgtgtat 1980
gctggaatta aatttcaaga ctccatgaaa gcaggaaaag aatagaattt taaggttaga 2040
agggatcatc ccattaaaat tgcttgttaa tacacaaaga atcttaaatc cagagaagtt 2100
tagtgacttg catgaggtca cccagtttag gttgtttgct agaactccag ttgccaaacc 2160
tcctcctgta ataccactgt ggtgtcttga ggcaatgtcc atgtttttgt agtggttgca 2220
gcataaactt gcccagctgc aatgtgtcct aagaccctgt gtgcccctga cagctattct 2280
gtatagatga tgtgtaagta tgacttcctg actttgatgg agtttaatct atgtttaaat 2340
tagtaataga ggaagatttt tctgtgttgc gtacaactgt ttttgagatt ttcttttttt 2400
tttttttaaa tatcagaatg ggaatttgac tgagtatggc tttctagtca accaggaaat 2460
tatagaatct ctagcaggta aatcaaaaat gctatgactt ggccaaccat ggttttgtct 2520
ggtatgatat gcatctgaac tgtggtactg aattctcagt ttttgagact caagtcgtgg 2580
aataagccat acttgtggaa atgtttcatg cttttgtgtc ctctgcattg ggagtagggt 2640
gttggataga cctcaccatg gtgggagaaa tggactgatt acactttacc tgggagcctt 2700
gtctgtagta caggtagggc atatataaga tttcacctgg ccaaatgaaa cattctcaag 2760
ctccaaacat cagaaaaaac catcactttt aaccaggagg aagatttttt agtcatcttt 2820
gtctagctct aaaataacta attttgcaaa tgtttatcat gagaaaacat tagggatttt 2880
ctccctggat tttcagctag gcccaaacag agagaattga atcccccaca taacctttcc 2940
tgtcctggag gatgtgactg aggatgcagg atgtgatcgc aggcagaggt gatgatactt 3000
tctggtccta caataggttg tatatatagg aaggtagttc tgaaccattt tttattcacc 3060
agtacttgca ccactgaatt tcccttgtac ttcaggagca cagtagggaa gaggtgatgc 3120
cttcccagag tagtcaagtc aaataaatca gatttctatg cttgttcctc ctacttttcc 3180
attattcaca aagaacagag gcaagatgga atagtgctta taaaccaata atcttatgat 3240
gcagttctta aaattctgct tcattatccc tctatgggtc aaaaagagtg aagaaataga 3300
aaacaaagct tgtgaggtat ctatgcatgt gataccttaa gtgtgataac ttctgtgttg 3360
ttttgaaagg ctttctctct gtaaggaggg agcacactaa ggcacacagg catccatgtt 3420

ttggctcatg tcttgatagg gcatccttag aaagggggtc ttctgtaagg tcagccacaa 3480
agcaagcaag taataggtgt gtgtgcatgt ttcccaaagc caattgttat ggagctccac 3540
agtgtaacaa tcaacttttc atggaagtca ggaaggatct gttttatgtg cagtgcaaag 3600
gatataaact gccagaagaa catgatattt tataggcttt ttaatagaaa ttttataaaa 3660
tgatattttt ctatcttgcc cttaatgaag tgatgtagca tgggggctga gaactgtgtt 3720
ttggtaaggc tctggtgatt tggaattatc tgctgctaac atgcctggct cttagccagt 3780
ttgagggttg tatacatttg aaaacaggca tatgaaaccc cgtcagcccc atttgtggct 3840
cctcctgagg ggaaaagttg ctaaggactt tgagctacct cccttctttc tctttcctca 3900
gcagtgtgaa gcctttccca acaccgggac tatggtagtt ctgggaccag tcagagttaa 3960
acccaggaca ctttctggaa gagacctgga gttgtcaaag agcagggatt ggatggttcc 4020
agagttttag ggacctgacc aggcctgaga tttctgggat ggcaaggtaa ggagagtgag 4080
agctggagtg atttccctca gtctcgagga ctatgttgaa tatgtagaag tgttaatatg 4140
ggtctattag actccattat tgattgacag agcagggaac atatctgctt atgagtaagt 4200
tcagccttgt gcctgaataa atactttttt aaaaggtacc aataattcca acacaccaaa 4260
aggtacctgc ccttggaaaa ggataaaagc aagaatgcct ctgggtgaag ttcctggtca 4320
ttcccaactc tgaatttagc attgatcctg tgtctagctg ggctcctctt ctgacctgag 4380
agccctgaag tcagcagatg aggtgctggt agagttccat gtagacaaag ccccagctct 4440
ctttcccctt ataatcctgt cttcagggga gtgacttgca acttatggaa catatcacat 4500
gtgtttttta ttgtccccaa acaccatgtg atctatcatg tatcttgcta aagtaaattt 4560
ctaagctttt tttttccttt ttaaaactat tttcgaaact gtggcaaatg tgtctagact 4620
tagtcataca aaatttacaa aaacctttta aaagggattt taagatggtg ttttaaggtt 4680
gttaggtgtt ccatgttagg tgtcttctca gtaagccact cttctttttc aacttaagga 4740
acccctcctt gacaaattat taatcagtgc cttggatata ttcactaatg gtagaacaca 4800
gaggtttaaa atcattatgg gttataaaga aagtaaaaat atttttattt gtggccaatt 4860
taaagccttg aatctgtttg gctggggcta atatttacac ttccatccct ggtgcacatg 4920
ttaagtaatc aaaagaaaat agaactcggg attatgcctg tcggcaaaaa aaaaaaaaaa 4980
aaaaaaggga tttaaaatgt gccactggaa attgataatc tgaataattt tcattatttg 5040
attattggct taacagaaca atgagtctca cctaacaaga tacaactcaa tgctagacag 5100
ttacataagg aggaaactag gatagatcaa gttagatgat tttgggatac atgagctata 5160
atggtgtttt acccaacctt tatctagcaa catcataatg atttgcacct tgaagtttta 5220
ctggtaaata aaacttctga atgtgaaatc tgttgttgtt gttgtattac ttaccaaaat 5280
catttttaca aagcgtttcc attatgtcgt tgtcatcatc atttttattt ttgcaggctt 5340
cacatacctt tggagctaga aatgtgaaaa acagtcttta gtaagtttgc ttaaaagaga 5400
acagtaggtg gcgtttgtaa acaactcaaa gggacttatc cagacaaaag tagcaattgt 5460
cctatttaac ctcagcttct gcctcctcta taaaagcagg aacactcaat cctaagaaag 5520
ttttcccatg cttcccaatt tttttggggg ggaggggtgg tgggagcaaa atgagaatgc 5580
ctatatgttg ttgtgtttaa taaattatga ctctcaaaag ggagttgtgg gcagagtata 5640
caagtgtatg tagttatagt taactgagac agggtgcagc ttttcagctc atcctcccaa 5700
cctctgggaa attcagtctg aaaaagttat tcccaaggcc ttttgaagga gaaagcaccc 5760
aaagaaccaa agatacttga ggaatttggg aaaactggta actagaaaat atagacaaaa 5820
gattgtcttt ctcagccaca ggacaccagt gtagttggct ctactcggca gagctctgta 5880
tctcaggggg gccaagtctg gactgggagg ggtgactggc tagcgttcca catgtgggct 5940
gtttccacct tgaaaacaca tctcactaat tacaacagcc tgctctaagt ggttctgtgt 6000
caccttgtgc ctctgccaag attgtgcttg agttaggcag cattcatttc attgttatct 6060
gcaagctccc tggtacaggt tcttcttgcc cttctgtgcc tgtttcaaac tctcaaaaca 6120
aacaaataaa taaacctgtt tctgaagcta atcaaagacg aagcaaaaat ggtttagtgg 6180
tctgatcaaa cttaggattt gtatactagc acaattatca gaagacctgc taaaagctct 6240
tctaagagtt tgatttcttg ggagaagtaa aaaaaaaaaa aaaagaacaa ttatattgcc 6300
cttctctcat ttataaatgt gttaattatg tctttaaaat ttaggtgaaa tgagattttc 6360
actaatgtga aatttaatgt tcatagaaaa ctatgtgact taatagaagc gttatttatc 6420
aaattagaaa aatcacaaag ctggaatttt gaagaacata aacttttatt ttgaaggaga 6480
aaagtggctt tttggagaaa tgtaagatgc gtaatgttgc tgctgatagt gggaagaaat 6540
aaaagcaaca tacataaaac tgcccttaag tcccctcacc caaacaaaat caaacatttc 6600
caattaggag ctctcaggat gtatataaaa tattactaac ctgtattggt tggtctatgg 6660
tacatgttgg acaaatgttt gaaaaatgcc aaacatttgc aataaattag aaatctaacc 6720
aataagatga ttgcaaactc atggtccctg ccaggaaaga ttgctaaaga actttgcatt 6780
tgctttaact aatgatgtct gtaggaaaac tactagagat tgaaagggag aggctgctga 6840
agccctgaaa gggattagga agctcccttt tccaacccag gggacccaaa tcagctcaga 6900
ggcattgtat atgttttcct ttcagaaaaa atattgagag cccatgaggt tttcattgct 6960

ttgatggttt ttgcacagga actctccctc cgttttctaa aaacttggga atctcttttg 7020
catgattttc tccagctaat caaaaccact cctaagttaa aaactgatag caatagaaaa 7080
gtgatttcca gagattccct ttatgccctc ttcctcaagt atctgagcag ttacaaccaa 7140
tgttcaattt tccacacact ccgtgccagt cccggtttta tttccaaaat ctaaattgac 7200
aaaggacaaa aagaccctta aaaggttatc tacttgtgca tcctggtttt cctttctgta 7260
caggaaatgt tcctgtgact agaatcctgc ccagtggtag gaaggtgcag aatgaggctg 7320
agcgaggggc ctctggccag aatgctcacc acaaactcct ccaccaaaca ccacttccaa 7380
cgctctgcgc agctccagtg ctagcatctt taacaataat cgtgtatcta ttttttttta 7440
aaccgccgtt cagttcttcg tttttttagt tttgttgttt ttgtttgttt gttttatttt 7500
taaactgccc ttaagtatag ctggtacaac tcgggaggag aatgcgcgag acttgggcgg 7560
cgtgggaggg gtgcctcaaa tttgatacag gcttgttgtg atgacagacc aggtcaggca 7620
gaacttctgc ccttcccgct actggcaccc caagcaggga tgcactggga tgcgtggcag 7680
gggcgggatc tcctgggagc gtctcagccc agcagggagt ggggaagcaa gagggaaggc 7740
ttaccttcct cggtggctgg caggaggtgg tcgctgctag cgagggggat gcaaaggtcg 7800
ttgtcctggg ggaaacggtc gcactcaagc atgtcgggcc aggggaagcc gaaggcggac 7860
atgaccgggg cgcagcggtc cttcacctgc acgcagagcg agtggcatgg ctggatggtc 7920
tcgtctaggt catcgaggca gacgggggcg aagagcgagc acaggaactt cttggtgtcc 7980
gggtggcact gcttcatgac cagcgggatc caagcgccgg cctgctccag cacctccttc 8040
atggtctcgt ggcccagcag gttgggcagc cgcatgttct ggtattcgat gccgtggcac 8100
agctgcaggt tggcagggat gggcttgcaa ttgctgcgct tgtaggagaa gtcgggctgg 8160
ccaaagagga agagcccgcg cgccgagccc aggcagcagt gcgaggcgag gaagagcagc 8220
agcagcgagc cagggccctg cagcatcgtg ggcgcgcgac cccgaggggg cagagggagc 8280
ggagccgggg aagggcgagg cggccggagt tcgagcttgt cccgggcccg ctctcttcgc 8340
tgggtgcgac tcggggcccc gaaaagctgg cagccggcgg ctggggcgcg gagaagcggg 8400
acaccgggag gacagcgcgg gcgaggcgct gcaagcccgc gcgcagctcc ggggggctcc 8460
gacccggggg agcagaatga gcc 8483

<210> 235
<211> 153
<212> DNA
<213> unknown

<220>
<223> >gi|2714120|gb|AA704202|sr|IMAGp998K211090.1|cl|IM
      AGp998K211090|AA704202 zj77e11.s1
      Soares_fetal_liver_spleen_1NFLS_S1 Homo sapiens
      cDNA clone IMAGE:460940 3', mRNA sequence.

<400> 235
aaatactttt actgggatac tcagtggtag aaaatggtac ttggacagtt gaactgattt 60
ttagcagtaa ccaagatctg tcagctctga gcttggggaa catcattctg tttggtaccc 120
aaagcaacaa gtgactattg agcaggggtt acc 153

<210> 236
<211> 382
<212> DNA
<213> unknown

<220>
<223> >gi|2186431|gb|AA461311|sr|IMAGp998A181964.1|cl|IM
      AGp998A181964|AA461311 zx65b09.s1
      Soares_total_fetus_Nb2HF8_9w Homo sapiens cDNA
      clone IMAGE:796313 3', mRNA sequence.

<400> 236
ttacaatttt actctttctt agcaccaact ccatcatgca agactgacag ggaagaatca 60
caacatgtaa aagctaaaag aaacccctta tacagtccag tgcttacaac ctttaaaaca 120

cattaaaagc ttgaggaatt tgttaacatg caaattccca ggcctaacca cagagacact 180
gatttagatg atatggggta gatcccagta aagcagccca ggtgtttttg aaatagatgg 240
atcatgaacc aaactttagg aaataacaat atactgcctc acttttagat taagaaattt 300
aaactcagag agcttagtga ttttcctgag gccatgtaat tagctagggg caaagaagac 360
attagactag tagcctccta tc 382

<210> 237
<211> 672
<212> DNA
<213> unknown

<220>
<223> >sr|IMAGp998H04617.1|cl|IMAGp998H04617|gb|BX100150
      |oid|234534676 BX100150
      Soares_multiple_sclerosis_2NbHMSP Homo sapiens
      cDNA clone IMAGp998H04617 ; IMAGE:279219, mRNA
      sequence.

<221> misc_feature
<222> 588, 592, 604, 618, 621, 623, 630, 644, 645, 661, 663, 664,
       669, 672
<223> n=a, t, c, or g

<400> 237
atacctgata cgcgcttctg ggctttgtct gtggctgcca ggtggtcagc gtgtttacgg 60
aggaagagga cagctttgat ttcattggtg gatttgatcc atttcctctc taccatgtca 120
atgaaaagcc atccagtctc ttgtccaagc aggtgtactt gcctgcgtaa gtgaggaaac 180
agctgatcct gctcctgtgg cctccagcct cagcgaccga ccagtgacaa tgacaggagc 240
tcccaggcct tgggacgcgc ccccacccag caccccccag gcggccggca gcacctgccc 300
tgggttctaa gtactggaca ccagccaggg cggcagggca gtgccacggc tggctgcagc 360
gtcaagagag tttgtaattt cctttctctt aaaaaaaaaa aagaaaagaa aacatacaaa 420
agaaaaggca aaaccccaca tgcccacctc ctctggcaac atgggggtca cagctctgcc 480
cccaggctgt cgtctcgtcg aggagcccct ccctcaggtg cccacctggg gctgctggac 540
cctcgggctg caagcactgc tgctgggatg cagccctccc caggaagnca angcgaggcc 600
cganacccct caagcggnga ngncccctgn tgaacatgga gggnntccta acccccaaac 660
ncnngccana an 672

<210> 238
<211> 551
<212> DNA
<213> unknown

<220>
<223> >sr|IMAGp998I12670.3|cl|IMAGp998I12670|gb|BX089595
      |oid|234513538 BX089595 Soares_fetal_lung_NbHL19W
      Homo sapiens cDNA clone IMAGp998I12670 ;
      IMAGE:299603, mRNA sequence

<400> 238
gaggagaaga aatagaagaa agttctgcaa caaccatgtc tgagaacttc cagtattaat 60
gtcagacacc aaaccaaaga tccaggaagc tccgagaaca ccaggcagaa taaatgccaa 120
caacctacac ttggacatat aattttcaaa ctatatgaaa taaaagataa aggaaaactc 180
tgaaagaaac cagaggtggg gcagaaaaca ccttacctac agagacacaa agataagaac 240
tgcattcaac attgcagaaa ctgtgaaagc aagaagacag tgaaatgaaa aattcaaaat 300
gttgacagaa aaaaacccac caacctaagt ttctgtaccc actgaaaaca cccttcaaaa 360
gtgaaggaga attaaggcct tcctcagaaa aataaaaatt caagaaactt gttgccagga 420
gacctgtctt gcaagaaatt ttaaatgaaa ttctttaggg aaacaaaaga tatgtaactg 480
aaacctggat caacattttt ttaaaaagag cattaaagaa agaattgtgg tacaataaaa 540
aaaaaaaaaa a 551

<210> 239
<211> 239
<212> DNA
<213> unknown

<220>
<223> >gi|5445722|gb|AI825133|sr|IMAGp998D145711.1|cl|IM
      AGp998D145711|AI825133 wb04h07.x1 NCI_CGAP_GC6
      Homo sapiens cDNA clone IMAGE:2304733 3', mRNA
      sequence.

<400> 239
tttttttttt tttttttttt tttttttttt ttcaaaaatg gaaaaatttt atgtttttta 60
tataaccatt ccctagggtt aacagatttt atttttatga tacatatctt taagtggggg 120
cccactgggg acatagcaaa tttgcaataa atccttatta agctgaaggg aactaacaaa 180
aaccattaat tacccctttt ttaacaccca aaccagtttt ggtttttaag ggtttgggg  239

<210> 240
<211> 525
<212> DNA
<213> unknown

<220>
<223> >sr|IMAGp998L06178.1|cl|IMAGp998L06178|gb|BX113044
      |oid|234560465 BX113044 Soares infant brain 1NIB
      Homo sapiens cDNA clone IMAGp998L06178 ;
      IMAGE:38015, mRNA sequence.

<221> misc_feature
<222> 1, 13, 35
<223> n=a, t, c, or g

<400> 240
nacaacactg agnactcacg ataaaacaga cggcncatgt ctacaactat atccccccac 60
ctgtgggtca gatgtgccaa aaccccatct acatgcagaa ggaaggagac ccagtagcct 120
attaccgaaa cctgcaagag ttcagctata gcaacctgga ggagaaaaaa gaagagccag 180
ccacacctgc ttacacaata agtgccactg agctgctaga aaagcaggcc acaccaagag 240
agcctgagct gctgtattaa aatattgctg agcgagtcaa ggaacttccc agcgcaggcc 300
tagtccacta taacttttgt accttaccta aaaggcagtt tgccccttcc tatgaatctc 360
gacgccaaaa ccaagacaga atcaataaaa ccgttttata tggaactccc aggaaatgct 420
ttgtggggca gtcaaaaccc aaccaccctt tactgcaagc taagccgcaa tcagaaccgg 480
actacctcga agttctggaa aaacaaactg caatcagtca gctgt                525

<210> 241
<211> 364
<212> DNA
<213> unknown

<220>
<223> >sr|IMAGp998I135799.2|cl|IMAGp998I135799|gb|BX1007
      17|oid|234535810 BX100717 NCI_CGAP_Lu24 Homo
      sapiens cDNA clone IMAGp998I135799 ;
      IMAGE:2337492, mRNA sequence.

<400> 241
ttttttttta catcgacagt tgtattagag aaaagcttaa agacaaggaa aaattaagga 60
aatgaggcac aaaacgtaga gtagatacaa ccaacaaaac caaattggat tctttgagaa 120
gattagtaca cttgagaaag ccctggtaca ctagtcagag aaaagagagc agggacaaat 180

tacaatgtca gaatctacga acccttcaga cataaaaact tgaggctgtc tcaaaagtca 240
gtgggaaacg ttaaatgcaa ttgacaaatt actaggaaaa aactgctatc agaagaaata 300
gaacatgtta ctagctcctg tctttcagaa atcttaccta tatcttgtca aaaaaaaaaa 360
aaaa 364

<210> 242
<211> 493
<212> DNA
<213> unknown

<220>
<223> >sr|IMAGp998D154762.2|cl|IMAGp998D154762|gb|BX1032
      41|oid|234540862 BX103241
      Soares_total_fetus_Nb2HF8_9w Homo sapiens cDNA
      clone IMAGp998D154762 ; IMAGE:1940318, mRNA
      sequence.

<400> 242
aggtttttcc agaattagtc tggatcagtg cctaacaaag ctgttattaa ttctccacac 60
atgagtctgg ctaactgggc cagctctcat gttttactcc tgtctggcta caaatagcac 120
tgtgcatagt agccctgttt ccccatggtg ttggactgtg gtaaagtatg ttgtgacata 180
ctggaattct aataatcctg ttgtaagtat gtacagaatc tatgtaactt attgttgaca 240
tacagaggtt tgggaagtag tctaatggac attccttaca atagaatggc tgggaaggga 300
aagaaaaaaa aaaaaaaact acctaactcc aatcttaatg ttgtagtttt atagcaaaca 360
aaaattgtca gctttttgta ttgaaaggtc agtggtggta agacaaggtg tcttgtaaat 420
taagatttta agaagtgcat taaaatgttt tagaattact ctgggaattc tgtatatcac 480
actaaaattt tta 493

<210> 243
<211> 751
<212> DNA
<213> unknown

<220>
<223> >sr|IMAGp998P20366.1|cl|IMAGp998P20366|gb|BX097677
      |oid|234529727 BX097677 Soares fetal liver spleen
      1NFLS Homo sapiens cDNA clone IMAGp998P20366 ;
      IMAGE:193411, mRNA sequence.

<400> 243
caccctgcag ccccagcact gcctggctcc acgtgcctcc tggtctcagt atggcgctgt 60
cctgggttct tacagtcctg agcctcctac ctctgctgga agcccagatc ccattgtgtg 120
ccaacctagt accggtgccc atcaccaacg ccaccctgga ccggatcact ggcaagtggt 180
tttatatcgc atcggccttt cgaaacgagg agtacaataa gtcggttcag gagatccaag 240
caaccttctt ttactttacc cccaacaaga cagaggacac gatctttctc agagagtacc 300
agacccgcca gaaccagtgc ttctataact ccagttacct gaatgtccag cgggagaatg 360
ggaccgtctc cagatacgag ggaggccgag aacatgttgc tcacctgctg ttccttaggg 420
acaccaagac cttgatgttt ggttcctacc tggacgatga gaagaactgg gggctgtctt 480
tctatgctga caagccagag acgaccaagg agcaactggg agagttctac gaagctctcg 540
actgcttgtg cattcccagg tcagatgtca tgtacaccga ctggaaaaag gataagtgtg 600
agccactgga gaagcagcac gagaaggaga ggaaacagga ggagggggaa tcctagcagg 660
acacagcctt ggatcaggac agagacttgg gggccatcct gcccctccaa cccgacatgt 720
gtacctcagc tttttccctc acttgcatca a 751

<210> 244
<211> 276
<212> DNA
<213> unknown

<220>
<223> >gi|3785069|gb|AI216028|sr|IMAGp998C034512.1|cl|IM
      AGp998C034512|AI216028 qh10e02.x1
      Soares_NFL_T_GBC_S1 Homo sapiens cDNA clone
      IMAGE:1844282 3', mRNA sequence.

<400> 244
tttgtgtgat cgcacttatt taacatttat tttgagttag ggtgtgagtg ctggggcagg 60
tgactaacca tcccctgccc tctgtggtgg cctgagagag aagcaggaat gccccgtgca 120
ggttggggat cagcaaacag tctagaccag cttctctcct gtatcccgtt tggagagtcc 180
ctgggtcagg ggccagtcca tcctgcagca ttgaagggcg tgaggaatga gcagggctgt 240
ggggctcaca gagcaggcag ctctggggag aagcgg                           276

<210> 245
<211> 468
<212> DNA
<213> unknown

<220>
<223> >sr|IMAGp998G01115.1|cl|IMAGp998G01115|gb|BX111632
      |oid|234557641 BX111632 Soares fetal liver spleen
      1NFLS Homo sapiens cDNA clone IMAGp998G01115 ;
      IMAGE:122136, mRNA sequence.

<400> 245
acagttctcc ctgcccctag tatgcagcag catttgtttc cccttggtaa tacacccgag 60
tcagtaaacc ccttatttgc ttgttggttc agtgcccaga agggcccaaa acagcaggtg 120
agcctcaatg tacaattcag tgagctgttg tcttgtgttc cccggaagca ttcctcctgt 180
aaaaaccaag ccctccaaac cagtagaact ccagttgagg ggacaggtga gaggaaccac 240
tcctacttct atcccttgat tcctggaccc gtgaatcctg gttataggag agacaacaca 300
tgcaaagcag aaccttgggg gcagcaccca aaagttgtct cccaagtggt gctgtaacgg 360
agtcttcacg aggccgtttc actctaatgt ttgtccagct gcttcctcct gctgagatac 420
gtggtatgac tgtgattacc tggtcacaag cccattgcct cactttct             468

<210> 246
<211> 752
<212> DNA
<213> unknown

<220>
<223> >sr|IMAGp998B22787.1|cl|IMAGp998B22787|gb|BX109149
      |oid|234552677 BX109149 Soares_fetal_heart_NbHH19W
      Homo sapiens cDNA clone IMAGp998B22787 ;
      IMAGE:344373, mRNA sequence.

<400> 246
aggctagtgg ctggacctgc actcagatgg caattcaggt ccagttcatg agatcttgga 60
ctgttttgac aaatgatttg gggaaaggga tagggagaga gttgtcagta ccgatgctga 120
gactgtgaga gccatttctg agtggaggtg aggaaaaacg gagcgatgac cgtgagggtc 180
tttgcgtggc gcctcgagac cacacctggg aacggtgact ttggaactgg ctgacgtgtt 240
tatttggtgt gaggtcaact acagctctga gggcaataac atgatcctca taaaacagac 300
cccccaggct gggcctcagc ccgcttgctg ccaagcaggg gtggtggcct aataagtgag 360
acattgctat caactctctg taaatggtgg gttcattcac tctgtggatg acatagcctg 420
ttctttgccc ttctgtgtca gccccattga ttgcttttta accaccttct tgctttgtgc 480
tgcttctttc agatgaaaag gaagggaaaa gagacttcat ttccataccc actaaccatg 540
ccaacagaag gctttgtgaa gaaggaagta aaagctatcg gtaggagtta gaaagaaaca 600
tttctagtta caagaacagc agaaaaagac ctgaacttca gagtggacca caaatgaata 660
tgaattaata acacagatct cttttcaaaa gccaacacaa aatgaggatg tgttttttgc 720
aaaagcagtt gttcttgcca ctgggcttat gc                               752

<210> 247
<211> 505
<212> DNA
<213> unknown

<220>
<223> >sr|IMAGp998J134174.2|cl|IMAGp998J134174|gb|BX1068
      51|oid|234548083 BX106851 Soares_testis_NHT Homo
      sapiens cDNA clone IMAGp998J134174 ;
      IMAGE:1645164, mRNA sequence.

<221> misc_feature
<222> 9
<223> n=a, t, c, or g

<400> 247
accaccagna atgtgccttt tccttatcaa aaagaattgc tgccccttac aggacaggag 60
gactccaatt gtgaccaagt tggagcagcc ttgagccaca actcagactt attttcattc 120
tctgctcatt ggccattagg aacccgatcc ttatgacctg cagtgacctg ctgctaccag 180
aactgactcg cttcaaagtg acggatgaaa cccatatgga gatattcctg gggtgggtag 240
gccagttgga agacagagtg tttacacctg agagaggact tgagaatttt actcagtcac 300
gttgctcact gctgcgcact cctgcagacg catcccagtg gactctgcgc tgcatcccca 360
gagtacagcg agtttcccgc ctcccgttag agatccaaca catatttttt ttgaaagaag 420
aaactgatgg aagacaggaa gcaaaaagga gaggaaggaa agggatagga aggaggggaa 480
ggaaagggaa aggaaggaaa agaaa                                       505

<210> 248
<211> 327
<212> DNA
<213> unknown

<220>
<223> >gi|711665|sr|IMAGp998D04101|cl|IMAGp998D04101|gb|
      T83377|T83377 ye03h02.s1 Homo sapiens cDNA clone
      116691 3'.

<221> misc_feature
<222> 72, 238, 278, 294, 320
<223> n=a, t, c, or g

<400> 248
ttttaacata aaacagggct atttattttt attattaaac atgagtactt ctgaaatatg 60
acatttcatg gnacataaaa tgatgtgtat gacaaaatct gttgctaact ccaaatatta 120
acttaatata gcttagaaaa atgacataat atactctgca tttttctgac cattctattt 180
ttacaagtac tccagttata tttagcattc aatttaaaag acaagtgcac tgaaggantt 240
tgaggttata atataattaa cccaaatatt atataacnat acacacttaa atgnctggtt 300
gtggtttatt ggatttggcn gggtatc                                     327

<210> 249
<211> 327
<212> DNA
<213> unknown

<220>
<223> >gi|711665|sr|IMAGp998D04101|cl|IMAGp998D04101|gb|
      T83377|T83377 ye03h02.s1 Homo sapiens cDNA clone
      116691 3'.

<221> misc_feature
<222> 72, 238, 278, 294, 320
<223> n=a, t, c, or g

<400> 249
ttttaacata aaacagggct atttattttt attattaaac atgagtactt ctgaaatatg 60
acatttcatg gnacataaaa tgatgtgtat gacaaaatct gttgctaact ccaaatatta 120
acttaatata gcttagaaaa atgacataat atactctgca tttttctgac cattctattt 180
ttacaagtac tccagttata tttagcattc aatttaaaag acaagtgcac tgaaggantt 240
tgaggttata atataattaa cccaaatatt atataacnat acacacttaa atgnctggtt 300
gtggtttatt ggatttggcn gggtatc 327

<210> 250
<211> 334
<212> DNA
<213> unknown

<220>
<223> >sr|IMAGp998O0470.2|cl|IMAGp998O0470|gb|BX101214|o
      id|234536804 BX101214 Soares infant brain 1NIB
      Homo sapiens cDNA clone IMAGp998O0470 ;
      IMAGE:22040, mRNA sequence.

<221> misc_feature
<222> 168
<223> n=a, t, c, or g

<400> 250
tttttttttt ttttaaaggt tagagaatcc aagtttatta gaaacactcc aacaaaaaac 60
aaaggtgaga agagagggcc cagcccacct ccactcctcc ctttcctcca gaacagaata 120
ccagtttgta tccggcaaac tggctccttc cccagggttg gtcccagngg ggatttacat 180
ggcactgcca aagcaggacg ggagccctag tcctcagggc actgcaggat gtcataggtc 240
acgtagccca cttggtccac ctggttcaac tcactccggg aactcacgcg ccagtagaag 300
cggtcctggc agaaataggc tttctctcgg tact 334

<210> 251
<211> 474
<212> DNA
<213> unknown

<220>
<223> >sr|IMAGp998A125385.2|cl|IMAGp998A125385|gb|BX1135
      74|oid|234561525 BX113574 NCI_CGAP_Ut2 Homo
      sapiens cDNA clone IMAGp998A125385 ;
      IMAGE:2179475, mRNA sequence.

<400> 251
tttttttttt ttttcttgtg caaatgcttt actgagggag tactttctgg agaaacctgt 60
gagggaacca ggaaaactgg ataggccaag gggtcagacc aagcagagag gtggttccag 120
gagaggcctg gcctcagccc gatcccagga acaatttgag agtgtcattc aacccagaca 180
atcctgcaca gacacggggg agctggcaat tgtaccctcc atggtgtcac tctcctggcc 240
atgagtgaca ggtggcctct tggtgggaga ggtggggcag ggggaaggca gctcccacta 300
gaccagggca gtatttaggg gaaggaggca gattggagca gttggctgcc aacacctggc 360
ctcactcagg gagaggggcc ttgggcctgg atgatatctg ggtgaggtac ccacaccatc 420
tgctgcaacc tctgtgtatc cattatgggc tcacgccatt cacaggcatg aatt 474

<210> 252
<211> 406
<212> DNA

<213> unknown

<220>
<223> >sr|IMAGp998D194160.2|cl|IMAGp998D194160|gb|BX0988
      70|oid|234532114 BX098870 Soares_testis_NHT Homo
      sapiens cDNA clone IMAGp998D194160 ;
      IMAGE:1639650, mRNA sequence.

<221> misc_feature
<222> (0)...(0)
<223> n=a, t, c, or g

<400> 252
caggttgtag tgacctagcc tgcaaaaatg agctccagna cacccctgag caaggttaaa 60
agaaaaaaaa caaattcctt tactgtctct cctgtactga actattagtt atgactatgt 120
ttgccaatgc ttatatttag taaaatttat aaattcctgt ttttctttca acgtagctgg 180
aaggtcacta gctatacaag gccacaagtt atgccaagtc aacagttatg ccataaatta 240
catgacttgt aatttatgta attaccgcct ttgttttgct cttgtatact agcctatata 300
agctaaactc tgtctttgtt tggggctcag gtttttaaat gtgaatccac tgagccagtg 360
cgtaccttaa aataaatatc ctcctatatt caaaaaaaaa aaaaaa 406

<210> 253
<211> 486
<212> DNA
<213> unknown

<220>
<223> >gi|2191401|gb|AA465234|sr|IMAGp998L132010.1|cl|IM
      AGp998L132010|AA465234 aa24g07.s1 NCI_CGAP_GCB1
      Homo sapiens cDNA clone IMAGE:814236 3', mRNA
      sequence.

<400> 253
acattttcaa tcattttaat gaaaagggcg acacagccat ggtgtgttca gatactcagc 60
atgacaccgc ctgcaagctc tattgtcttt cgctgattaa actgggctgg ttgagaatta 120
catttctgca cccatggttc cgctgtggtg aacaccggga gtaagcaagc tcttattcag 180
gtcccaagca agccacagcc agcatgtcag aaagcaagat tcaagtcagg gtccccttgg 240
cttcgactgg ctctactgga tcaaacagct ccagcgggga cagaggcagc aggaaagagc 300
ttcctcaata aggaaattga agagcaggag gaaggaaaaa ataacctgaa actcagatgg 360
gcaagattag gggtggggag tgagagccag agatcatgtg tcagcaccca gagcggagga 420
gaaagaattc tgattccagt ccacaccacc ctagactgca gcattgagca ccccagggct 480
ggcgtt 486

<210> 254
<211> 591
<212> DNA
<213> unknown

<220>
<223> >gi|2186490|gb|AA461370|sr|IMAGp998G081965.1|cl|IM
      AGp998G081965|AA461370 zx70f04.r1
      Soares_total_fetus_Nb2HF8_9w Homo sapiens cDNA
      clone IMAGE:796831 5' similar to SW:ECE1_BOVIN
      P42891 ENDOTHELIN-CONVERTING ENZYME 1 ;, mRNA
      sequence.

<400> 254
aaatattgct gataatggag gcctgcggga agcttttagg gcttacagga aatggataaa 60
tgacagaagg cagggacttg aggagcctct tctaccaggc atcacattca ccaacaacca 120

gctcttcttc ctgagttatg ctcatgtgag gtgcaattcc tacagaccag aagctgcccg 180
agaacaagtc caaattggtg ctcacagtcc ccctcagttt agggtcaatg gtgcaattag 240
taactttgaa gaattccaga aagcttttaa ctgtccaccc aattccacga tgaacagagg 300
catggactcc tgccgactct ggtagctggg acgctggttt atggcatcct gagacagttg 360
cacagtgcca gcggaggctt gcactgagcc ttcatcgccc attgctttag gcctggagac 420
tttcattttt agtgcatttt cattatttgg gtaggtgacc tgcttggatc tagacagcat 480
ctgttcaaag ttgtagggct tataaagtgg aatataagaa tgaactaagt atgtttcttt 540
agaaaatcaa accaacaaaa ataaatccct aggctacttt tgttaaaatg c 591

<210> 255
<211> 693
<212> DNA
<213> unknown

<220>
<223> >sr|IMAGp998B072455.2|cl|IMAGp998B072455|gb|BX1183
      94|oid|234571164 BX118394 NCI_CGAP_Pr21 Homo
      sapiens cDNA clone IMAGp998B072455 ; IMAGE:984870,
      mRNA sequence.

<400> 255
tgaaattccc ggcgctcacg cactactggc ccctgatccg gttcttggtg cccctgggca 60
tcaccaacat agccctcgac ttcggggagc agccatctcc tttttatatt tacaagaggt 120
cacaagagga cagttaaaac tgatgacgcc taaggacaga tcacacgtaa tagaagagct 180
gccgcagccg gttcccatta gaagggcctc caagaacaac acacacagga actcgcctgc 240
tcttctcctg atggtccaca gtcacccctg ccctttgagc aggaggagcg tgacctcctg 300
ccacagctac agccacaatt attgaaattg tggtcaacat tccatttaaa gcattaaagt 360
ctgttgtttt agtcctgact aagcagtagg cttctatgga ttgggtattt tttttttttt 420
acccacccaa atgtgtttta tgttgagatg tttcgagttt ttttgggggg ttgagggtgg 480
tgcaggtcgt gaggggcaag agctgctgca taggagataa ttctctttaa ctgattaatg 540
tttttgcttt attcaacagc tattgaatta ggcagtctgt acctttagtg ttttccagaa 600
caaatgtgtt gaagtggcag aagggtggaa gaattgaaaa ttattcttgc acagttgcac 660
ctgagttgta ttattgctcc atgtctcctt atg 693

## Description

### Field of the Invention

The present invention provides biomarkers that characterize testicular carcinoma in situ (CIS), which is the precursor for classical testicular seminoma and non-seminomas. The same biomarkers may also be used to characterize gonadoblastoma, a CIS-like pre-cancerous lesion found in dysgenetic gonads.

### Background of the invention

Testicular carcinoma in situ (CIS) is the common precursor of nearly all testicular germ cell tumors (TGCTs) that occur in young adults. The incidence of TGCTs has increased markedly over the past several decades, and these tumors are now the most common type of cancer in young men.

CIS cells may transform into either a seminoma or a non-seminoma. The seminoma retains a germ-cell-like phenotype, whereas the tumour known as non-seminoma or teratoma retains embryonic stem cell features; pluripotency and the ability to differentiate into virtually all somatic tissues. Non-seminomas comprise embryonal carcinoma (EC), various mixtures of differentiated teratomatous tissue components and extraembryonic tissues, such as yolk sac tumour and choriocarcinoma. In addition to CIS, gonadoblastoma, a CIS-like lesion occurs in dysgenetic testes and intersex gonads, which frequently may contain some ovarian structures.

The only difference between CIS and gonadoblastoma concerns the overall architecture of the lesion in the surrounding gonad (CIS is found usually in single rows along the basement membrane and there is a single layer of Sertoli cells between CIS cells and the lumen of seminiferous tubules, while gonadoblastoma contains nests (clumps) of CIS-like cells surrounded by small somatic granulosa-like cells and sometimes spermatogonia-like cells). The morphology and gene expression patterns of CIS cells and gonadoblastoma cells are indistinguishable (Jørgensen et al. Histopathology 1997), therefore, further in this application, the present inventors use the term CIS for both precursor lesions.

The most commonly used marker for CIS in clinical practice is placental-like alkaline phosphatase (ALPP or PLAP) (Manivel, JC, et al. Am J Surg Pathol 1987, 11:21-9), which is also a known marker of primordial germ cells. Epidemiological evidence and evidence based on immunohistochemical comparative studies of proteins expressed in CIS and fetal germ cells indicate that CIS is an inborn lesion, probably arising from gonocytes in early fetal life and progress to an overt TGCT after puberty. The precise nature of the molecular events underlying the initiation of malignant transformation from the gonocyte to the CIS cell has not yet been elucidated, and the following progression into overt tumors remains largely unknown.

In order to identify new markers for early detection of CIS, the present inventors analyzed gene expression in CIS cells and tumors in a systematic manner, by employing both a differential display (DD) methodology and by using a cDNA microarray covering the entire human transcriptome.

### Summary of the invention

In the microarray studies the present inventors have circumvented detecting changes of gene expression not related to CIS (as CIS cells constitute only a small percentage of cells in a typical tissue sample) by using testis tissues containing different amounts of tubules with CIS and searched for genes regulated in a manner proportional to the content of CIS in the sample.

Selected candidates for CIS and tumor markers from both the DD and the microarray study have been verified by semi-quantitative reverse transcriptase PCR (RT-PCR) and the expressing cell type determined by in situ hybridization. Furthermore, the present inventors use antibodies against the some promising candidates and used immunohistochemistry to identify the expressing cell types in tissues with CIS/gonadoblastoma and in tumors derived from CIS.

The present inventors also investigated the gene expression profile (using the same large cDNA microarray) of testicular seminoma and embryonic carcinoma (EC, an undifferentiated component of non-seminomas) and compared that to the expression profile of CIS.

This facilitated identification of genes that mark the progression of CIS into either the seminoma or the non-seminoma. Results were confirmed by RT-PCR and markers were localized to tumor cells by in situ hybridization as well as for selected markers at the protein level by immunohistochemistry.

Carcinoma in situ (CIS) is the common precursor of histologically heterogeneous testicular germ cell tumors, which have markedly increased in frequency in the recent decades and now are the most common malignancy of young adult men. Using genome-wide gene expression profiling, the present inventors identified more than 200 genes highly expressed in testicular CIS, including many never reported in testicular neoplasms. Expression was further verified by semi-quantitative RT-PCR and in situ hybridization, and by immunohistochemistry for a few protein products, for which antibodies were available.

### Description of the invention

The biomarkers disclosed herein can be used for the detection of CIS and gonadoblastoma but also biomarkers for the detection of the seminomas and non-seminomas derived from CIS and thus for the progression of CIS is included in the present invention.

The biomarkers were identified using differential display and genome-wide cDNA microarray analysis and subsequent verified by other methods, including in situ hybridization, which showed that the CIS markers were expressed in CIS cells.

For some biomarkers localization to CIS cells and some CIS-derived overt tumours was also done at the protein level by immunohistochemistry.

The present invention thus relates to the use of the identified biomarkers to identify CIS and the status of CIS derived cancers in human samples such as semen samples, blood, testicular biopsies, and biopsies from any other human tissue.

In one aspect, the present invention relates to a method for determining the presence of precursors of germ cell tumours in an individual. Such a diagnostic method would comprise determining the expression profile of a group of markers in a sample, and concluding from said expression profile whether or not the sample contains precursors of germ cell tumours, wherein the group of markers consists of markers selected independently from the markers listed in table 1, whereby the number of markers in the group is between one and the total number of markers listed in table 1.

Specifically, the method comprises the following steps:
a) determining the expression profile of a group of markers in said sample, wherein the group of markers consists of at least one marker selected independently from the markers listed in table 1;
b) comparing said expression profile with a reference expression profile;
c) identifying whether the expression profile is different from said reference expression profile and
evaluating whether the sample contains precursors of germ cell tumours if the expression profile is different from the reference expression profile.

In the present context the term "precursors of germ cell tumours" relates to the commonly accepted fact that CIS is a common precursor for all germ cell tumours of the adolescents and young adults. CIS is also known as intratubular germ cell neoplasia of the unclassified type, testicular intraepithelial neoplasia and gonocytoma in situ, with the last term probably the most accurate from the biological point of view.

In the present context the term "expression profile" relates to assaying the level of transcripts, such as mRNA, either relative to a reference value, relatively to another transcript or sample and/or absolutely values. In example cDNA micro arrays measure the abundance of a transcript in two different samples relative to each other, whereas e.g. real-time PCR measures the absolute copy number of transcripts in the sample. Both relative and absolute measurements enables a person skilled in the art to make a diagnostic decision based on the data presented.

As known to the skilled addressee, an expression profile of a transcript could also be obtained from e.g. amplified RNA, aRNA, and/or complementary DNA, cDNA, by various techniques. Thus, the present invention also relates to any methods using such manipulations. The genes listed in table 1 are in no particular order, and thus both the listed and the complementary strand is an object of the present inventions.

The sequences listed in table 1, may represent only a part of the sequence of the gene listed, e.g. an EST sequence, fragment of sequence or similar. However, the full genomic sequence, the coding sequence, untranslated regions, and promoter regions is an object of the present invention.

In general, assaying the level of transcripts, and thus determining the expression profile of a group of markers can be made by several technical approaches, such as, but not limited to, micro arrays, filter arrays, DNA chips, gene chips, differential display, SAGE, northern blot, RT-PCR, quantitative PCR, PCR, real-time PCR or any other known technique known to the skilled addressee

When assaying levels of expression of the sequences presented in table 1, the levels refers to the measurable expression level of a given nucleic acid. The level of expression of a nucleic acid is determined by methods well known in the art. "Differentially expressed" or "changes in the level of expression" may refer to an increase or decrease in the measurable expression level of a given nucleic acid and "Differentially expressed" when referring to microarray analysis means the ratio of the expression level of a given nucleic acid in one sample and the expression level of the given nucleic acid in another sample is not equal to 1.0. "Differentially expressed" when referring to microarray analysis according to the invention also means the ratio of the expression level of a given nucleic acid in one sample and the expression level of the given nucleic acid in another sample is greater than or less than 1.0 and includes greater than 1.2 and less than 0.7, as well as greater than 1.5 and less than 0.5. A nucleic acid also is said to be differentially expressed in two samples if one of the two samples contains no detectable expression of the nucleic acid.

Absolute quantification of the level of expression of a nucleic acid can be accomplished by including known concentration(s) of one or more control nucleic acid species, generating a standard curve based on the amount of the control nucleic acid and extrapolating the expression level of the "unknown" nucleic acid species from the hybridization intensities of the unknown with respect to the standard curve. The level of expression is measured by hybridization analysis using labeled target nucleic acids according to methods well known in the art. The label on the target nucleic acid can be a luminescent label, an enzymatic label, a radioactive label, a chemical label or a physical label. Preferably, target nucleic acids are labeled with a fluorescent molecule. Preferred fluorescent labels include, but are not limited to, fluorescein, amino coumarin acetic acid, tetramethylrhodamine isothiocyanate (TRITC), Texas Red, Cy3 and Cy5.

Concluding from said expression profile whether or not the sample contains precursors of germ cell tumours can be obtained by assaying the expression profile and determine if the markers are either present and/or up-regulated and/or down-regulated. Depending on the experimental set-up the marker genes may appear up-regulated but could also appear down-regulated, if e.g. the ratio were switched. The settings in the present application focus on the up-regulated markers.

Depending on the assay method and set-up the measurement of the marker genes may be done in reference to either another transcript, a sample or measured absolutely.

The group of markers consists of markers selected independently from the markers listed in table 1, the number of markers in the group is between one and the total number of markers listed in table 1, such as 1 marker, 2 markers, 3 markers, such as 4 markers, such as 5 markers, e.g. 6 markers, 7 markers, 8 markers, such as 9 markers, e.g. 10 markers, such as 11 markers, e.g. 12 markers, such ac 13 markers, 14 markers, e.g. 15 markers, such as 16 markers, such as 17 markers, e.g. 18 markers, 19 markers, e.g. 20 markers, such as 21 markers, 22 markers, 23 markers, e.g. 24 markers, such as 25 markers, such as 30 markers, such as 40 markers, e.g. 50 markers, such as 60 markers, such as 70 markers, e.g. 80 markers, e.g. 90 markers, such as 100 markers, such as 120 markers, e.g. 140 markers, such as 150 markers, e.g. 170 markers, e.g. 200 markers, e.g. 220 markers, e.g. 240 markers, such as 255 markers.

The term "sample" could be a semen sample, a blood sample, a serum sample, a urine sample, a testicular biopsy sample, or a biopsy from any other human tissue. The marker genes described here may be used to identify CIS or seminoma or non-seminoma cells in other clinical samples than those related to the testis and semen samples, i.e. in extra-gonadal brain tumors or blood samples from patients with disseminated seminoma.

In a presently preferred embodiment the sample is a semen sample.

The general scope of the present invention relates to detection of precursors of germ cell tumours in various samples as described above. It is obvious to the skilled addressee that the methods described herein enables a person skilled in the art to detect the presence of and/or the development of even very small amounts of precursors of germ cell tumours in the selected samples. In a presently preferred embodiment the methods described herein enables detection of at least one cell which is a precursors of germ cell tumours.

The expression profiles and as described below the intensity values are indicative of precursors of germ cell tumours since these data are found significantly more often in patients with a precursors of germ cell tumours than in patients without the precursors of germ cell tumours.

As used herein, "expression pattern" or "expression profile" comprises the pattern (i.e., qualitatively and/or quantitatively) of expression of one or more expressed nucleic acid sequences where one or more members of the set are differentially expressed.

As the skilled addressee would recognise the present invention enables a person skilled in the art in both diagnosing and/or monitoring precursors of germ cell tumours in a patient by detecting the expression level(s) of one or more genes described in table 1.

The present invention also relates to use of any of the sequences/genes/mRNAs listed in the present application for diagnostics of precursors of germ cell tumours located in the testis or in all other locations, using any method that can detect the products on the mRNA level (PCR, hybridisation, micro-array, etc.) or on the protein level (immunoassays, Western blot, protein-microarrays etc.) in any human samples including semen samples, blood, tissue samples, urine, etc. in patients or healthy individuals, the sequences selected from the group consisting of SEQ ID NO 1-255 are of particularly value.

In other words, the present invention relates to a method for diagnosing precursors of germ cell tumours, the method comprising the steps of assaying an expression level for each of a predetermined number of markers of precursors of germ cell tumours in a sample; and, identifying said precursors of germ cell tumours if at least one of said expression levels is greater than a reference expression level.

Alternatively, the method for determining the presence of precursors of germ cell tumours in a sample may be based on measurements of the polypetides expressed by the nucleotide sequences comprising the markers selected from the group of SEQ ID NO: 1 to SEQ ID NO:255.

Thus, another aspect of the present invention relates to a method for determining the presence of precursors of germ cell tumours in an individual comprising determining the intensity signal(s) of a group of markers in a patient sample, and concluding from said intensity signal(s) whether the patient sample contains of precursors of germ cell tumours, wherein the group of markers consists of markers selected independently from the markers listed in table 1, whereby the number of markers in the group is between one and the total number of markers listed in table 1.

Such a method comprises the following steps:
a) determining the intensity signal of the proteins encoded by each of the members of the group of markers in said sample, wherein the group of markers consists of at least one marker selected independently from the markers listed in table 1
b) comparing each individual intensity signal with a corresponding reference intensity signal;
c) identifying whether each individual intensity signal is different from said corresponding reference intensity signal and
evaluating whether the sample contains precursors of germ cell tumours if the intensity signal is different from the corresponding reference intensity signal.

The term "intensity signal" relates to assaying the protein level of the proteins encoded by the transcripts from table 1. Any immuno-assay is able to perform such measurement e.g. ELISA, RIA, Western blots, or dot blots. Also other methods capable of measuring the amount of a protein quantitatively or relatively. Such techniques include, but are not limited to; mass spectrometry base measurements, protein microarrays, ProteinChip® (Ciphergen) and derived methods.

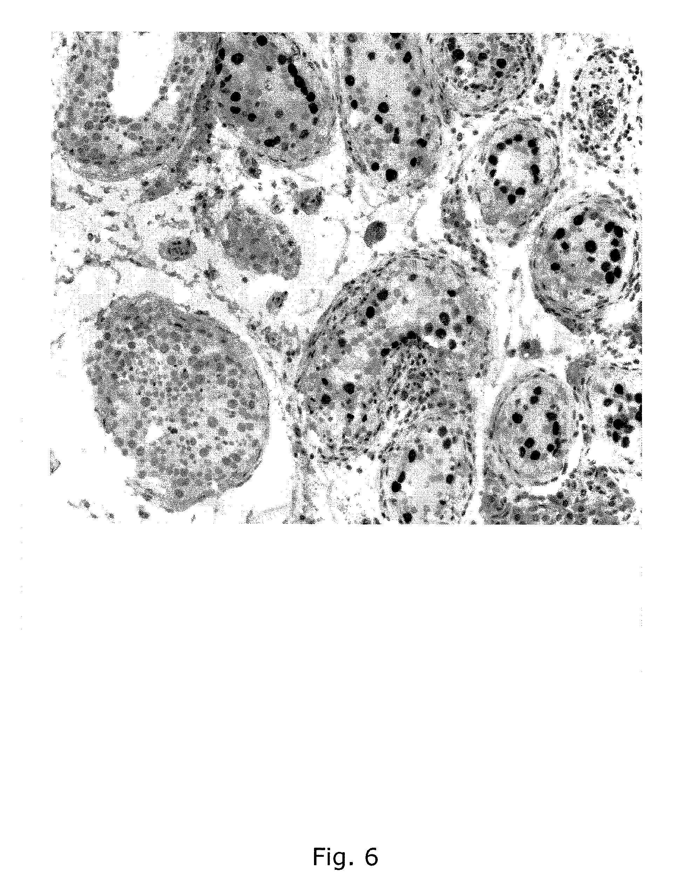

As exemplified below, the present inventors are generating antibodies against the gene products of the present inventions. Figure 6 displays an example of one such antibody used in an immuno-staining of an tissue section comprising CIS cells.

Antibodies generated against the gene products of the present invention may enable monitoring of metastasis and invasion of germ cell tumours.

As shown in table 1, several markers are up-regulated in highly abundant CIS cells, thus in one embodiment, the present invention can be performed on such up-regulated markers. Such specific markers are the markers selected from the group consisting of SEQ ID NO 1-157.

A presently preferred embodiment of the present invention further relates to a method according to the invention, wherein the group of markers consists of at least one marker which in a sample with CIS in 100% of the seminiferous tubules is up-regulated more than 7 fold when compared to a reference sample, such as 7.1 fold, e.g. 7.2 fold, such as 7.5 fold, e.g. 8 fold, e.g. 9 fold, such as 10 fold, e.g. 11 fold, e.g. 12 fold, such as 13 fold, 14 fold, 15 fold, e.g. 16 fold, such as 17 fold, 18 fold, 19 fold, e.g. 20 fold, such as 25 fold, e.g. 30 fold, 35 fold, 40 fold, e.g. 45 fold, such as 50 fold, e.g. 55 fold, e.g. 60 fold, such as 65 fold, e.g. 70 fold, such as 75 fold, e.g. 80 fold, such as 85 fold, e.g. 90 fold, e.g. 95 fold, such as 100 fold.

In another embodiment of the present invention some markers are, in an sample with CIS in 100% of the seminiferous tubules, up-regulated more than 4.3 fold but less than 7 fold. Such markers are presently considered as medium-potential markers, thus the applicability of these markers are considered to be less useful than the markers with higher abundance in CIS cells.

In yet another embodiment of the present invention some markers are, in a sample with CIS in 100% of the seminiferous tubules, up-regulated more than 3 fold but less than 4.3 fold. Such markers are presently considered as markers of lesser potential, thus the applicability of these markers for diagnosis is considered low when compared to the markers with higher abundance in CIS cells, however still capable of adding valuable information for the purposes of the present invention.

Another presently preferred embodiment of the present invention relates to a method according to the invention, wherein the expression profile distinguishes between classical testicular seminoma and testicular non-seminoma in gonads or in extra-gonadal localizations.

When the present inventors compared tissue samples comprising non-seminoma (EC-cells) versus CIS cells and seminoma cells, the markers defined in SEQ ID NO 158 to SEQ ID NO 202 showed an up-regulation of more than 5 fold, whereas the CIS cells and seminoma cells showed an up-regulation of less than 2 fold.

Thus, the markers defined in SEQ ID NO 158 to SEQ ID NO 202 enable a person skilled in the art to distinguish non-seminoma from CIS and seminoma, since these markers are all upregulated in non-seminoma as described in table 1 and the examples below, when compared to samples containing CIS and/or seminoma.

When the present inventors compared tissue comprising seminoma cells versus CIS cells and non-seminoma cells, the markers defined in SEQ ID NO 203 to SEQ ID NO 255 showed an up-regulation of more than 3 fold, whereas the CIS cells and non-seminoma cells showed an up-regulation of less than 2 fold.

Thus, the markers defined in SEQ ID NO 203 to SEQ ID NO 255 enable a person skilled in the art to distinguish classical seminoma from CIS and/or non-seminoma, since these markers are all upregulated in seminoma as described in table 1 and the examples below, when compared to samples containing CIS and/or non-seminoma.

One presently preferred embodiment relates to a method which can distinguish seminoma from non-seminoma.

Another presently preferred embodiment relates to a method which can distinguish seminoma from CIS.

Yet another presently preferred embodiment relates to a method which can distinguish non-seminoma from CIS.

Therefore, one aspect of the present invention relates to a method for monitoring progression from precursors of germ cell tumours to overt cancer in an individual comprising determining the expression profile and/or the intensity signal(s) of a group of markers in a sample and concluding from expression profile and/or the intensity signal(s), whether the sample contains precursors of germ cell tumours, wherein the group of markers consists of markers selected independently from the markers listed in table 1, whereby the number of markers in the group is between one and the total number of markers listed in table 1.

In one detailed embodiment the method comprises the following steps:
a) determining the expression profile of a group of markers in said sample, wherein the group of markers consists of at least one marker selected independently from the markers listed in table 1;
b) comparing said expression profile with a reference expression profile;
c) identifying whether the expression profile is different from said reference expression profile and
evaluating the development stage of precursors of germ cell tumours to overt cancer cells if the expression profile is different from the reference expression profile.

Alternatively, the method comprises the following steps:
a) determining the intensity signal of the proteins encoded by each of the members of the group of markers in said sample, wherein the group of markers consists of at least one marker selected independently from the markers listed in table 1
b) comparing each individual intensity signal with a corresponding reference intensity signal;
c) identifying whether each individual intensity signal is different from said corresponding reference intensity signal and
evaluating the development stage of precursors of germ cell tumours to overt cancer cells if the intensity signal is different from the corresponding reference intensity signal.

In yet another aspect, the present invention relates to a method for identifying precursors of germ cell tumours in a sample from a mammal, the method comprising
assaying said sample by a quantitative detection assay and determining the expression profile and/or the intensity signal(s) of at least one marker selected from the group consisting of
   SEQ ID NO: 1 to SEQ ID NO: 255
comparing said expression profile and/or the intensity signal(s) with reference value(s)
identifying whether the expression profile and/or the intensity signal(s) of at least one marker from the sample is significantly different from the reference value, and
evaluating whether the sample contains precursors of germ cell tumours if the expression profile and/or the intensity signal(s) is different from the reference value.

As the skilled addressee would recognise the present invention is useful as a method for determining the presence of precursors of germ cell tumours in an individual comprising detecting the presence or absence of at least one expression product, wherein said at least one expression product comprise a nucleotide sequence selected from the group consisting of SEQ ID 1-255 in a sample isolated from the individual.

In another aspect, the present invention also relates to a method of assessing the efficacy of a therapy for inhibiting precursors of germ cell tumours in a subject, the method comprising
comparing the expression profile of a group of markers in a first sample obtained from the subject prior to providing at least a portion of the therapy to the subject and the expression profile of a group of markers in a second sample obtained from the subject following provision of the portion of the therapy, wherein a significantly altered expression profile of the marker(s) in the second sample, relative to the first sample, is an indication that the therapy is efficacious for inhibiting precursors of germ cell tumours in the subject
wherein the group of markers consists of markers selected independently from the markers listed in table 1 and whereby the number of markers in the group is between one and the total number of markers listed in table 1.

Alternatively, the above method aspect can also be assessed by comparing the intensity signals of the proteins encoded by each of the member of the group of markers instead of comparing the expression profiles.

The method of assessing the efficacy of a therapy of the present invention also relates to methods for identifying, evaluating, and monitoring drug candidates for the treatment of precursors of germ cell tumours and of course any of the later stages. According to the invention, a candidate drug or therapy is assayed for its ability to decrease the expression of one or more markers of precursors of germ cell tumours. In one embodiment, a specific drug or therapy may reduce the expression of markers for a specific type or subclass of precursors of germ cell tumours described herein. Alternatively, a preferred drug may have a general effect on precursors of germ cell tumours and decrease the expression of different markers characteristic of different types or classes of precursors of germ cell tumours. In one embodiment, a preferred drug decreases the expression of a precursors of germ cell tumours marker by killing precursors of germ cell tumour cells or by interfering with their replication.

Current trends in stem cell research enable a person skilled in the art of various treatments comprising transplanting or regeneration of tissue by the use of e.g. embryonal stem cells.

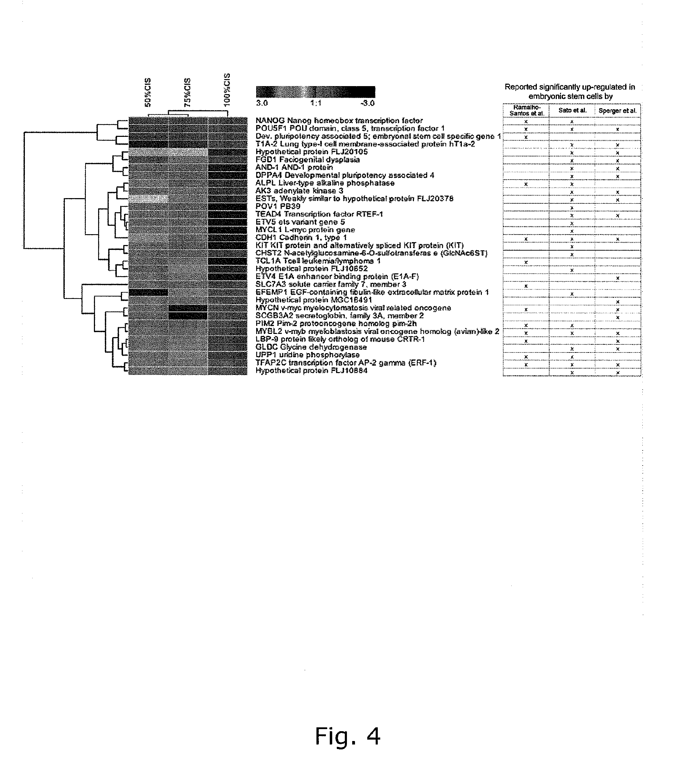

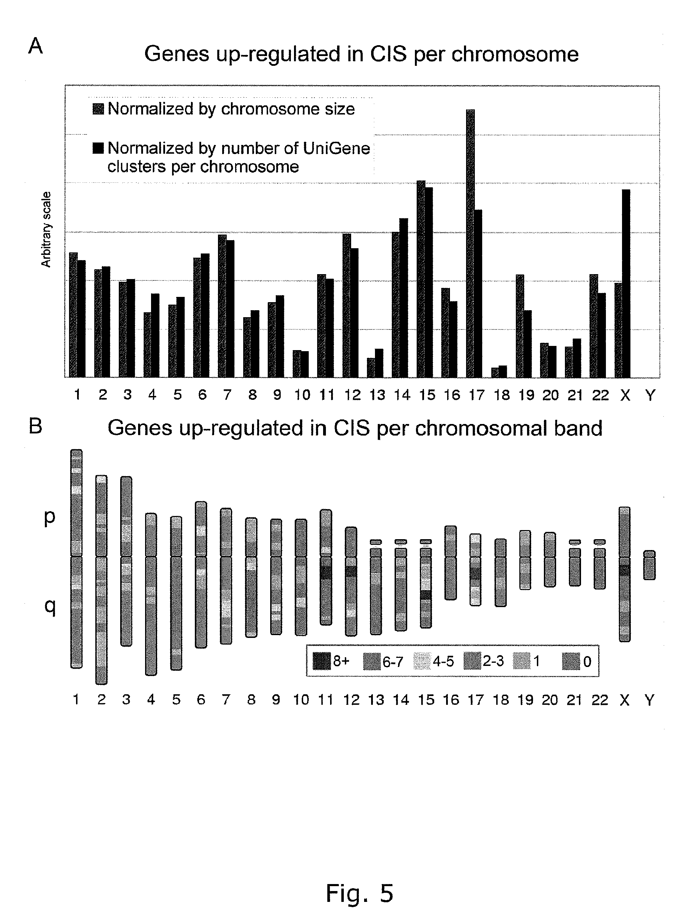

In the present application the present inventors have identified that CIS cells have many features, which are embryonal stem cell like, see figure 4 and figure 5 and the examples below.

CIS cells thus both are embryonal stem cell like and cancer precursor cells. Thus, by use of the present markers a person skilled in the art would be able to judge, if the e.g. tissue derived from embryonal stem cells contains cells, which are likely to become cancer precursor cells, and thus represent valuable information for consideration before transplanting e.g. an embryonal stem cells.

In another aspect, the present invention also relates to a methods for analysing the presence of undifferentiated stem cells highly likely to progress to precursors of germ cell tumours and/or cancer in an individual.

Such methods comprise the following steps in one embodiment:
a) determining the expression profile of a group of markers in said sample, wherein the group of markers consists of at least one marker selected independently from the markers listed in table 1;
b) comparing said expression profile with a reference expression profile;
c) identifying whether the expression profile is different from said reference expression profile and
evaluating whether the sample contains undifferentiated stem cells highly likely to progress to precursors of germ cell tumours and/or cancer in the individual if the expression profile is different from the reference expression profile.

Alternatively, such methods comprise the following steps in a second embodiment:
a) determining the intensity signal of the proteins encoded by each of the members of the group of markers in said sample, wherein the group of markers consists of at least one marker selected independently from the markers listed in table 1
b) comparing each individual intensity signal with a corresponding reference intensity signal;
c) identifying whether each individual intensity signal is different from said corresponding reference intensity signal and
evaluating whether the sample contains undifferentiated stem cells highly likely to progress to precursors of germ cell tumours and/or cancer in the individual if the intensity signal is different from the reference expression profile.

Testicular carcinoma in situ (CIS) is the common precursor of nearly all testicular germ cell tumors (TGCTs) that occur in young adults. The incidence of TGCTs has increased markedly over the past several decades, and these tumors are now the most common type of cancer in young men, thus representing an increasing health problem. There is a current need in the art of potential screening methods to identify the patients in an early non-invasive stage for the purpose of e.g. treatment strategy.

Thus, in another aspect, the present invention also relates to a method for screening for the presence of precursors of germ cell tumours in an individual of a population, said method comprising determining the expression profile and/or the intensity signal(s)of a group of markers in a sample obtained from said individual, and concluding from the expression profile and/or the intensity signal(s) whether the sample contains at least one precursor cell of germ cell tumours, wherein the group of markers consists of markers selected independently from the markers listed in table 1, whereby the number of markers in the group is between one and the total number of markers listed in table 1.

### Tested Individual Markers

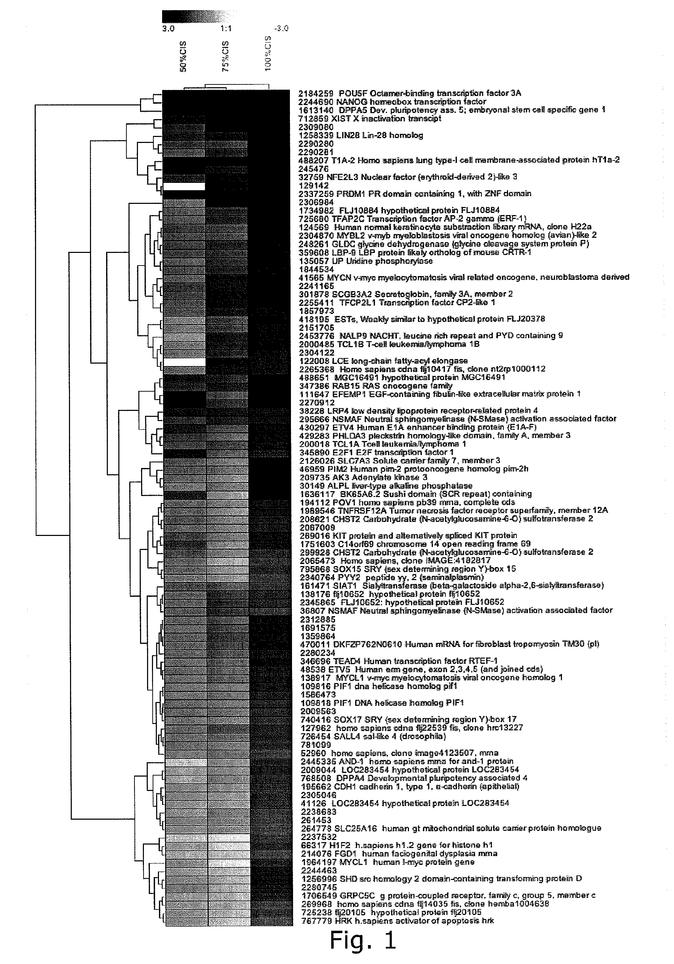

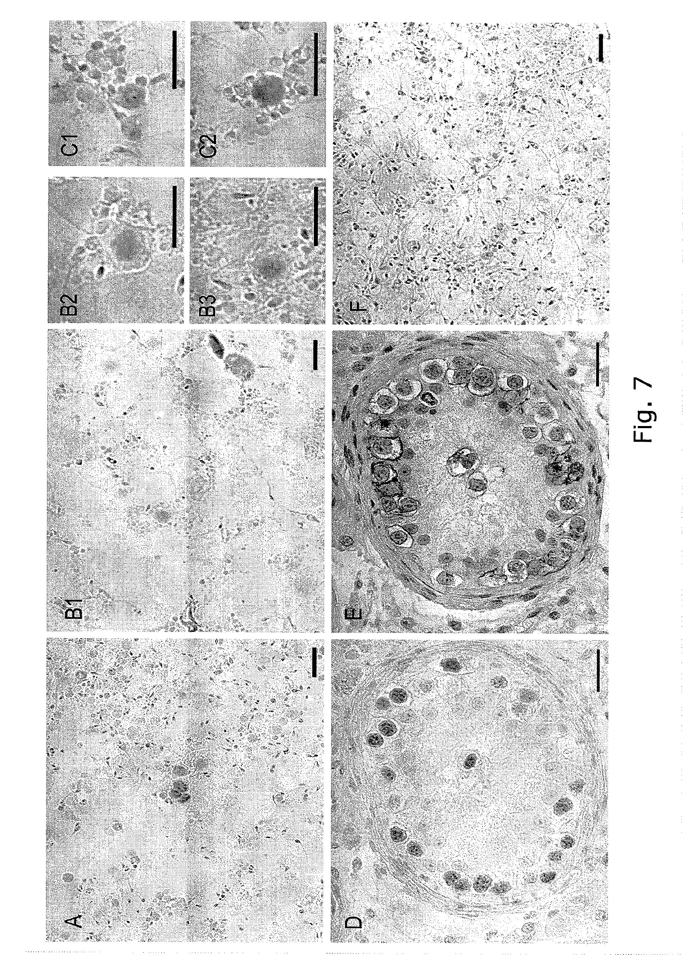

A range of the genes listed in tabel 1 has been further investigated at the protein level. Initially the genes from figure 1 which are highly expressed at the RNA level were studied at the protein level on tissue sections containing CIS using immunohistochemical detection. The antibodies which stained only CIS or tumour cells in testicular tissue sections were further used to stain semen samples spreads (cytospins) on a microscope slide.

One embodiment of the present invention relates to a method according to the present invention, wherein the marker is selected from the group consisting of AP-2γ, NANOG, TCL1A, PIM-2, and E-cadherin .

### AP-2γ

AP-2γ is encoded by the *TFAP2C* gene mapped on chromosome 20q13.2 and is a member of a family of DNA-binding transcription factors, which includes AP-2α, AP-2β, AP-2γ, TFAP-28 and TFAP-2ε. During vertebrate embryogenesis, these proteins play important roles in the development and differentiation of the neural tube, neural crest derivatives, skin, heart and urogenital tissues and show overlapping, but distinct, patterns of expression within these tissues.

AP-2γ is required within the extra-embryonic lineages for early post-implantation development, but apparently does not play an autonomous role within the embryo proper. Mice lacking AP-2γ die at day 7.5-8.5 post coitum due to malformation of extra-embryonic tissues.

In the above genome-wide gene expression profiling study of CIS cells, we have identified a number of genes expressed also in embryonic stem cells and foetal gonocytes but not in adult germ cells, thus providing many possible new markers for CIS. One of such genes was TFAP2C (mapped to chromosome 20q13.2), which encodes the transcription factor activator protein-2 (AP-2γ).

By immunhistochemical detection of the AP-2γ protein the present inventors investigated the protein expression in a large range ot tissues including fetal and malignant tissues. The present inventors found that AP-2γ protein expression was very resticted and only found in early gonocytes and other not fully differentiated cells. Also it was found to be very solid nuclear marker of testicular CIS cells and derived neoplastic cells (figure 6).

The present inventors disclose AP-2γ as a novel marker for foetal gonocytes and neoplastic germ cells, including testicular CIS, with a role in pathways regulating cell differentiation and a possible involvement in oncogenesis.

The fact that AP-2γ protein was not expressed in normal adult reproductive tract but was abundant in nuclei of CIS and tumour cells, which are better protected than the cytoplasm from degradation in semen due to a structural strength, prompted us to analyse the value of AP-2γ for detection of CIS and/or tumour cells in ejaculates in a series of patients and controls.

In an investigation of 104 andrological patients, presented in Example 10, cells positive for AP-2γ were found only in semen samples from patients diagnosed a priori with testicular neoplasms and, surprisingly, in a 23-year-old control subject with oligozoospermia and no symptoms of a germ cell tumour (figure 7).

Testicular biopsies performed during the follow-up of the 23-year-old control subject revealed widespread CIS in one testis, thus proving a diagnostic value of the new assay. Optimisation of the method is in progress, but a preliminary assessment suggests that the method may have a place in screening of germ cell neoplasia in at-risk patients. The local ethics committee approved the studies of AP-2γ expression and participants gave written informed consent.

The number of patients that we have screened for the presence of immunoreactive AP-2γ is continuously increasing as we are testing patients as they appear in our clinic. So far we have tested 347 andrological patients (including the 104 mentioned above) and found CIS-like cells positive for AP-2γ in semen samples of 3 patients, including the first case described above (a 23-year-old subfertile control subject with widespread CIS confirmed by testicular biopsies). Two other patients are currently under clinical follow-up and being considered for testicular biopses.

Thus, in a particular preferred embodiment, the present invention relates to a method according to the present invention, wherein the marker is AP-2γ.

### TCL-1A

TCL1A is a powerful oncogene that, when overexpressed in both B and T cells, predominantly yields mature B-cell lymphomas. TCL1A both in vitro and in vivo, increase AKT kinase activity and as a consequence enhances substrate phosphorylation. In vivo, TCL1A stabilizes the mitochondrial transmembrane potential and enhances cell proliferation and survival. TCL1 forms trimers, which associate with AKT in vivo and TCL1A is thus an AKT kinase coactivator that promotes AKT-induced cell survival and proliferation.

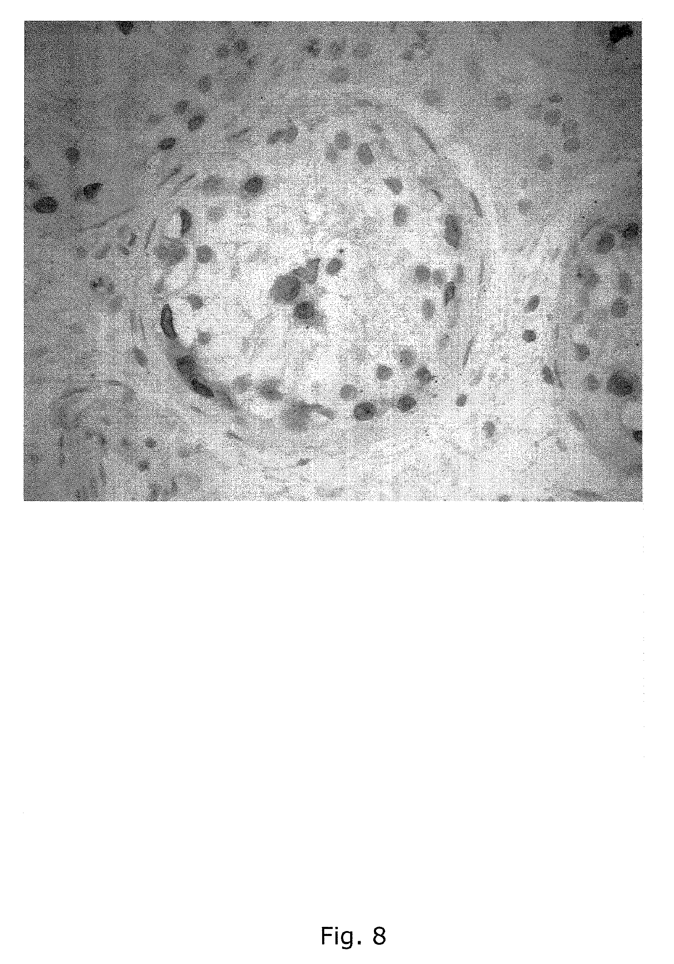

Expression of the TCL1A protein in testicular CIS as detected by immunohistochemical staining is shown in figure 8.

In another particular preferred embodiment, the present invention relates to a method according to the present invention, wherein the marker is TCL-1A.

### NANOG

Nanog-deficient Mouse inner cell mass (ICM) fail to generate epiblast and only produce parietal endoderm-like cells. Mouse Nanog-deficient embryonic stem (ES) cells loose pluripotency and differentiate into extraembryonic endoderm lineage. Nanog is thus a critical factor in maintaining pluripotency in both ICM and ES cells.
Elevated Nanog expression from transgene constructs is sufficient for clonal expansion of ES cells, bypassing Stat3 and maintaining POU5F1 (Oct4) levels.

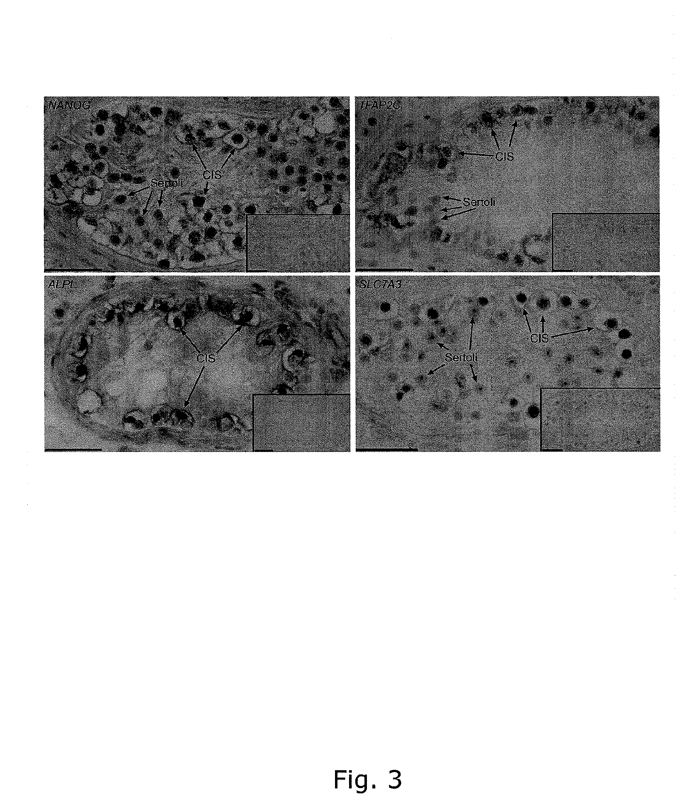

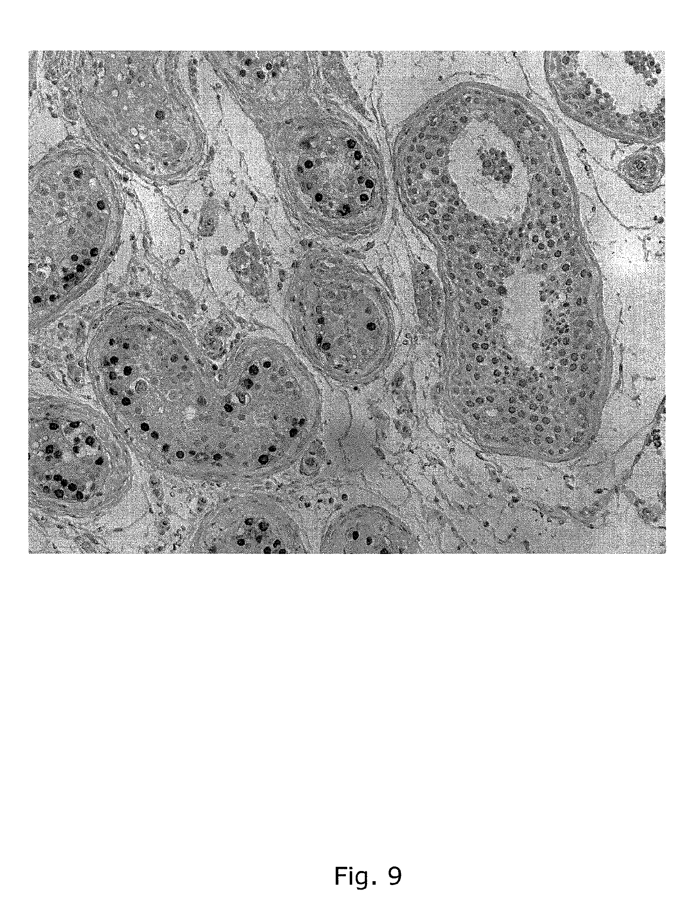

NANOG was found to be highly expressed in CIS both at the RNA and protein level. Immunohistochemical detection of NANOG is shown in figure 9 and at the RNA level in figure 1, 2, and 3.

In another particular preferred embodiment, the present invention relates to a method according to the present invention, wherein the marker is NANOG.

### PIM-2 and E-cadherin

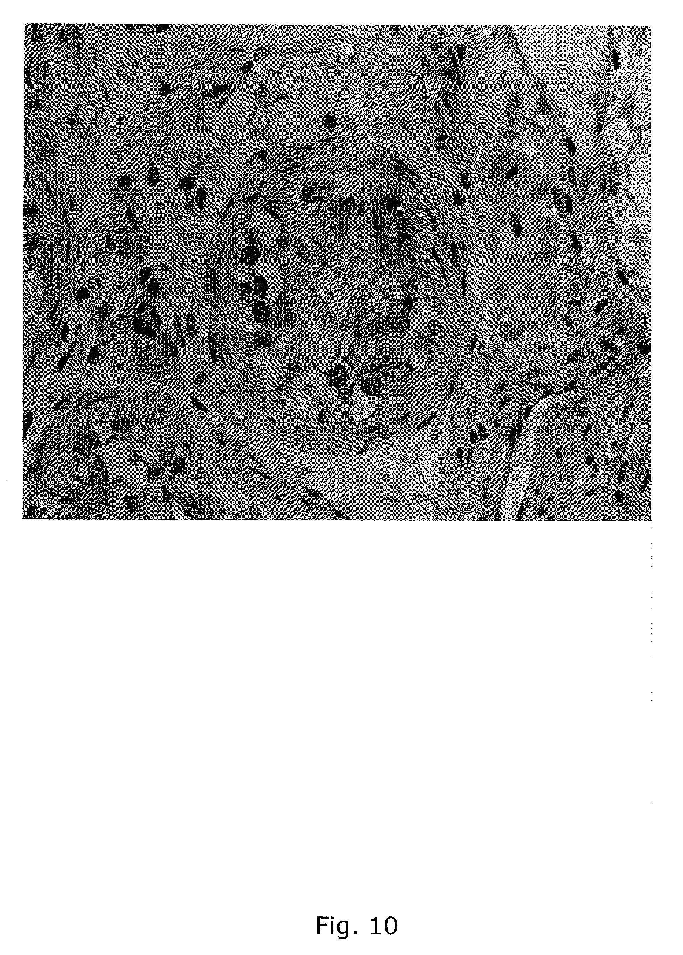

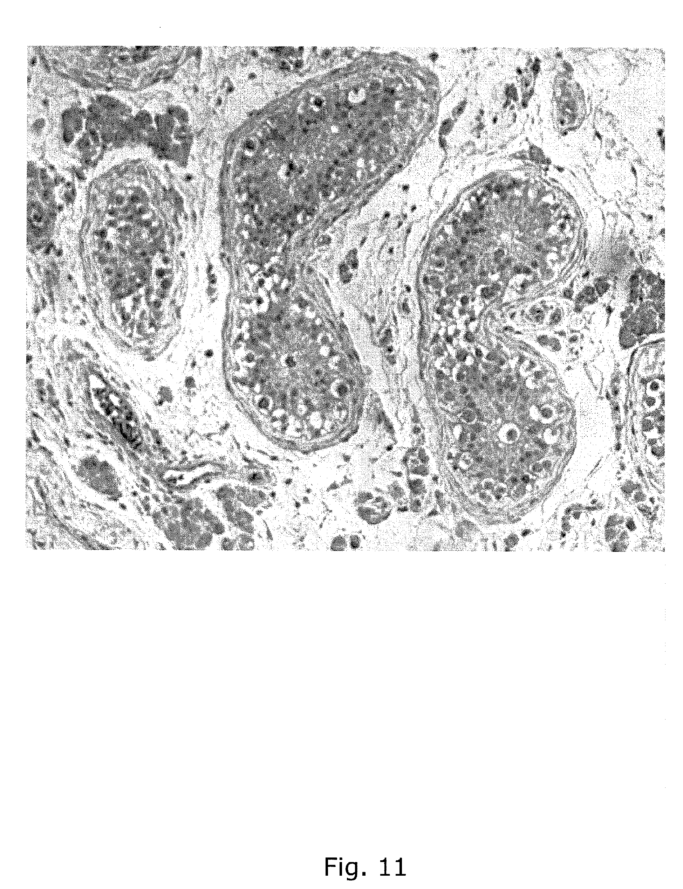

Similar to AP-2γ, TCL1A, and NANOG, the proteins encoded by the E-cadherin gene and the PIM-2 oncogene were also found highly expressed in CIS cells. Immunohistochemical detection of E-cadherin is shown in figure 10 and PIM-2 in figure 11.

In another particular preferred embodiment, the present invention relates to a method according to the present invention, wherein the marker is PIM-2 and/or E-cadherin.

Antibodies directed against the proteins encoded by the genes in table 1 were used for immunohistochemical investigations on sections from different tissues including testicular CIS.

The present application furthermore particularly relates to a method according to the present invention, wherein the intensity signal is determined by immunocytological detection (immuno staining).

As the skilled person will know, any of the methods of the present invention may be used for screening for the presence of precursors of germ cell tumours in individual of a population, either alone or in any combination.

In another aspect, the present invention also relates to a kit for use in an assay or method as defined in the present application.

In one embodiment, such kit may be a diagnostic array comprising: a solid support; and a plurality of diagnostic agents coupled to said solid support, wherein each of said agents is used to assay the expression level of at least one of the markers shown in table 1, wherein each of said diagnostic agents is selected from the group consisting of PNA, DNA, and RNA molecules that specifically hybridize to a transcript from a marker of precursor of germ cell tumours or wherein each of said diagnostic agents is an antibody that specifically binds to a protein expression product of a marker of precursor of germ cell tumours.

In another embodiment, the present invention further provides databases of marker genes and information about the marker genes, including the expression levels that are characteristic of precursor of germ cell tumours. According to the invention, marker gene information is preferably stored in a memory in a computer system. Alternatively, the information is stored in a removable data medium such as a magnetic disk, a CDROM, a tape, or an optical disk. In a further embodiment, the input/output of the computer system can be attached to a network and the information about the marker genes can be transmitted across the network.

Preferred information includes the identity of a predetermined number of marker genes the expression of which correlates with a particular type of precursor of germ cell tumours. In addition, threshold expression levels of one or more marker genes may be stored in a memory or on a removable data medium. According to the invention, a threshold expression level is a level of expression of the marker gene that is indicative of the presence of a particular type or class precursor of germ cell tumours.

In a preferred embodiment, a computer system or removable data medium includes the identity and expression information about a plurality of marker genes for several types or classes of precursor of germ cell tumours disclosed herein. In addition, information about marker genes for normal testis tissue may be included. Information stored on a computer system or data medium as described above is useful as a reference for comparison with expression data generated in an assay of testis tissue of unknown disease status

It should be understood that any feature and/or aspect discussed above in connection with the methods according to the invention apply by analogy to the uses according to the invention.

All patent and non-patent references cited in the present application, are hereby incorporated by reference in their entirety.

As will be apparent, preferred features and characteristics of one aspect of the invention may be applicable to other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention will hereinafter be described by way of the following non-limiting Figures and Examples.

### Figure legends

### Figure 1

Hierarchical clustering of genes found by filtering data so only genes 4 fold or more up-regulated in the sample with CIS in 100% of the tubules and 2 fold in the samples with 50 and 75% tubules with CIS. Hierarchical clustering was done using Euclidean distances and average linkage. Some redundancy between entries is seen and implies consistency in the results. The image clone number (Unique ID on the array) is reported in front of the gene names and can be used to search the German Resource Center for Genome Research for further sequence information, http://www.rzpd.de.

### Figure 2

Further analysis of selected genes that in microarray study were found up-regulated in CIS. A) Examples of the semi-quantitative RT-PCR on a spectrum of testicular tissue samples. The gene name is on the right side of the panes. In the gels of NANOG, and POU5F1 the beta-actin-marker band of 800 bp is visible. Representative DNA fragments from all RT-PCR amplifications were excised, cloned, and sequence verified. 129142 is an Image clone number. B) Corresponding quantifications of NANOG and POU5F1 and the ratio between the two. Quantification was done by digitalizing the image of the agarose gel. M=100 base pair marker, SEM=seminoma, TER=teratoma, EC=embryonal carcinoma, CIS=testicular tissue showing 100% tubules with carcinoma in situ, NOR=normal testicular tissue, CIS A= CIS amplified for microarray analysis, H2O=control. The CIS sample (to the right) is the same sample used in the microarray analysis.

### Figure 3

Analysis of the cellular expression in tubules with CIS by in situ hybridization of NANOG, SLC7A3, TFAP2C and ALPL showing that the expression was confined to CIS cells (arrows). CIS cells are seen as a stained ring with a stained nucleus in the center, while the rest appears empty, due to large amounts of glycogen washed out during the tissue processing. Staining was in addition seen in Sertoli cells nuclei, probably due to over-staining necessary to visualize low abundance transcripts (arrows), since this was also seen with the sense probe. The sense control is from neighbouring sections of the same tubule as the anti-sense and inserted in the lower right corner. The scale bar corresponds to 50 µm.

### Figure 4

Genes from figure 1 reported to be up regulated in ESC. Data on mouse ESC are from Ramalho-Santos et al. (Ramalho-Santos, M, et al. Science 2002, 298:597-600) whereas data on human ESC are from Sato et al. (Sato, N, et al. Dev Biol 2003, 260:404-13) and Sperger et al. (Sperger, JM, et al. Proc Natl Acad Sci U S A 2003, 100:13350-5). The overlap between genes reported in these studies and genes identified in CIS (fig. 1) amount to roughly 35% but may be even greater due to differences in gene annotation and RNA references used.

### Figure 5

Chromosomal distribution of genes more than 2 fold up-regulated in the sample with 100 % tubules having CIS. A) Distribution per chromosome and normalized by the size of the chromosomes (blue bars) or by the number of UniGene clusters on each chromosome (red bars). Chromosomal size was acquired from Ensembl, http://www.ensembl.org/, and number of UniGene clusters (Build 34 version 3) from NCBI, http://www.ncbi.nlm.nih.gov/. B) Detailed distribution of the number of up-regulated genes in each chromosomal band. The color-coding is outline in the inserted box.

### Figure 6.

Immuno-staining of transcription factor AP-2γ (TFAP2C) on a cross-section of testicular tissue containing both normal seminiferous tubules without CIS and tubules with CIS lesion. The CIS cells stain darkly and the signal is located to the nucleus of the CIS cells.

### Figure 7

A. Semen sample from a control patient spiked with CIS cells that are strongly stained with AP-2γ.

B1. AP-2γ-positive CIS cells in the semen sample of the 23-years-old subfertile control subject diagnosed with CIS by this assay.

B2, B3. Examples of AP-2γ stained cells in the 23-years-old subfertile control subjectdiagnosed with CIS by this assay, higher magnification.

C1, C2. Examples of AP-2γ stained cells in a patient known a priori to harbour testicular cancer.

D. Histological features of the biopsy of the left testis of the 23-years-old subfertile control subject with AP-2γ staining.

E. placental alkaline phosphatase (PLAP) staining showing CIS cells both adjacent to the tubular membrane and in the lumen. F. Semen sample from a control patient, with no AP-2γ-positive cells. Scale bar =25 µm.

### Figure 8

Immuno-staining of TCL1A on a cross-section of testicular tissue containing tubules with CIS lesion. The CIS cells stain highly red and the signal is located to the nucleus of the CIS cells.

### Figure 9

Immuno-staining of homeobox protein NANOG on a cross-section of testicular tissue containing both normal seminiferous tubules without CIS and tubules with CIS lesion. The CIS cells stain darkly and the signal is located to the nucleus of the CIS cells.

### Figure 10

Immuno-staining of E-cadherin on a cross-section of testicular tissue containing tubules with CIS lesion. The CIS cells stain dark red and the signal is located to the cytoplasm or cellular membrane of the CIS cells.

### Figure 11

Immuno-staining of PIM-2 on a cross-section of testicular tissue containing tubules with CIS lesion. The CIS cells stain dark red and the signal is located to the nucleus of the CIS cells.

### Tables

**Table I**

| **ID** | **Database Entry [RZPD]** | **IMAGE clone** | **Feature description** | **Genename** | **Chromosome** | **50% CIS** | **75% CIS** | **100% CIS** | **Seminoma** | **EC** | **Seminom/EC** |
|---|---|---|---|---|---|---|---|---|---|---|---|
| SEQ ID No: | IMAGp998N195554;IMAGp971M1 | 224469 | Nanog homeobox | NANOG | 12p13.2 | 10,3 | 15,4 | 33,2 | | | |
| 1 | 993 | 0 | | | | | | | | | |
| SEQ ID No: | IMAGp998GO8223;IMAGp971H04 | 147103 | hXBP-1 transcription factor | hXBP1 | | 2,0 | 30,0 | 25,5 | | | |
| 2 | 10 | | | | | | | | | | |
| SEQ ID No: | IMAGp998F08222;IMAGp971F04 | 146695 | | | | 23,8 | 14,7 | 24,6 | | | |
| 3 | 10 | | | | | | | | | | |
| SEQ ID No: | IMAGp998A06220;IMAGp971B06 | 145805 | TMF1 TATA element modulatory factor 1 | TMF1 | | 24,8 | 27,6 | 16,6 | | | |
| 4 | 10 | | | | | | | | | | |
| SEQ ID No: | IMAGp998D203167;IMAGp971C | 125833 | LIN28 lin-28homolog(C.elegans) | LIN28 | 1p35.3 | 3,6 | 4,7 | 15,0 | | | |
| 5 | 959 | 9 | | | | | | | | | |
| SEQ ID No: | IMAGp998D054091;IMAGp971C0 | 161314 | developmental pluripotency associated 5; | embryonal | 11p15.4 | 10,1 | 14,0 | 15,0 | | | |
| 6 | 466 | 0 | stem cell specific gene 1 | | | | | | | | |
| SEQ ID No: | IMAGp998K241161;IMAGp971D | 488207 | Homo sapiens lung type-I cell membrane- | T1A-2 | 1p36 | 6,6 | 7,3 | 14,8 | | | |
| 7 | 341 | | associated protein hT1a-2 (hT1a-2) mRNA | | | | | | | | |
| SEQ ID No: | IMAGp998J095673;IMAGp971I09 | 229028 8 | | | | 2,9 | 3,2 | 14,2 | | | |
| 8 | 130 | 0 | | | | | | | | | |
| SEQ ID No: | IMAGp998117S722;IMAGp97111 | 230908 | | | | 3,3 | 8,7 | 14,2 | | | |
| 9 | 96 | 0 | | | | | | | | | |
| SEQ ID No: | IMAGp998J10S673;1MAGp971E1 | 229028 | | | | 2,5 | 3,8 | 12,9 | | | |
| 10 | 7130 | 1 | | | | | | | | | |
| SEQ ID No: | IMAGp998N115245;IMAGp971I0 | 212602 | SLC7A3 solute carrier family 7 (cationic | SLC7A3 | Xq12 | 2,6 | 2,1 | 12,3 | | | |
| 11 | 390 | 6 | amino acid transporter, y+ system), | | | | | | | | |
| | | | member 3 | | | | | | | | |
| SEQ ID No: | IMAGp998J05529;IMAGp971I052 | 245476 | | 14 | 6,7 | 4,5 | 11,4 | | | | |
| 12 | 2 | | | | | | | | | | |
| SEQ ID No: | IMAGp998A03577;IMAGp971A03 | 263690 | | 6 | NA | 2,7 | 10,0 | | | | |
| 13 | 24 | | | | | | | | | | |
| SEQ ID No: | IMAGp998H234408;IMAGp971G2 | 173498 | FLJ10884 hypothetical protein FLJ10884 | 1p32.1 | 2,3 | 3,7 | 10,0 | | | | |
| 14 | 474 | 2 | | | | | | | | | |
| SEQ ID No: | IMAGp998G13226;IMAGp971F06 | 148260 | | | 19,4 | 16,4 | 9,9 | | | | |
| 15 | 10 | | | | | | | | | | |
| SEQ ID No: | IMAGp998J23134;IMAGp971H23 | 129142 | | 7 | NA | 5,8 | 9,1 | | | | |
| 16 | 6 | | | | | | | | | | |
| SEQ ID No: | IMAGp998K225545;IMAGp971L2 | 224116 | | | 2,9 | 4,8 | 9,0 | | | | |
| 17 | 192 | 5 | | | | | | | | | |
| SEQ ID No: | IMAGp998N121162;IMAGp97101 | 488651 | MGC16491 hypothetical protein MGC16491 | 1p35.3 | 3,8 | 3,8 | 8,6 | | | | |
| 18 | 341 | | | | | | | | | | |
| SEQ ID No: | IMAGp998H07676;IMAGp971G10 | 301878 | SCGB3A2 secretoglobin, family 3A, | SCGB3A2 5q32 | 2,7 | 5,2 | 8,3 | | | | |
| 19 | 31 | | member 2 | | | | | | | | |
| SEQ ID No: | IMAGp998B171780;IMAGp971G1 | 725680 | TFAP2C transcription factor AP-2 gamma | TFAP2C 20q13.2 | 2,4 | 3,8 | 8,2 | | | | |
| 20 | 744 | | (activating enhancer binding protein 2 | | | | | | | | |
| | | | gamma) | | | | | | | | |
| SEQ ID No: | IMAGp998A2488;IMAGp971B232 | 111647 | EFEMP1 EGF-containing fibulin-like | EFEMP1 2p16 | 5,6 | 3,1 | 8,2 | | | | |
| 21 | | | extracellular matrix protein 1 | | | | | | | | |
| SEQ ID No: | IMAGp998H01154;IMAGp971C01 | 32759 | NFE2L3 nuclear factor (erythroid-derived | NFE2L3 7p15-p14 | 3,5 | 6,1 | 8,1 | | | | |
| 22 | 113 | | 2)-like 3 | | | | | | | | |
| SEQ ID No: | IMAGp998J02274;IMAGp971K01 | 46959 | Human pim-2 protooncogene homolog | PIM2 Xp11.23 | 2,5 | 2,5 | 8,0 | | | | |
| 23 | 117 | | pim-2h mRNA, complete cds | | | | | | | | |
| SEQ ID No: | IMAGp998L10121;IMAGp971M18 | 124569 | Human normal keratinocyte substraction | library 2 | 2,9 | 2,9 | 8,0 | | | | |
| 24 | 5 | | mRNA, clone H22a, complete sequence | | | | | | | | |
| SEQ ID No: | IMAGp998J075711;IMAGp971J12 | 230487 | MYBL2 v-myb myeloblastosis viral | MYBL2 | 2,7 | 2,8 | 7,7 | | | | |
| 25 | 94 | 0 | oncogene homolog (avian)-like 2 | | | | | | | | |
| SEQ ID No: | IMAGp998N06536;IMAGp971P15 | 248261 | glycine dehydrogenase (glycine cleavage | GLDC 9p22 | 2,8 | 3,7 | 7,5 | | | | |
| 26 | 22 | | system protein P) | | | | | | | | |
| SEQ ID No: | IMAGp998M17826;IMAGp971N10 | 359608 | LBP protein likely ortholog of mouse CRTR- | LBP-9 | 2q14 | 2,8 | 3,2 | 7,4 | | | |
| 27 | 34 | | 1 | | | | | | | | |
| SEQ ID No: | IMAGp998P11794;IMAGp971O09 | 347386 | RAB15 RAB15, member RAS onocogene | RAB15 | 14 | 3,4 | 4,3 | 7,4 | | | |
| 28 | 35 | | family | | | | | | | | |
| SEQ ID No: | IMAGp998O08148;IMAGp971M0 | 30149 | Human mRNA for liver-type alkaline | ALPL | 1p36.1- | 1,9 | 2,4 | 7,3 | | | |
| 29 | 5112 | | phosphatase | | p34 | | | | | | |
| SEQ ID No: | IMAGp998C095623;IMAGp971I0 | 227091 | | | | 5,1 | 3,7 | 7,3 | | | |
| 30 | 993 | 2 | | | | | | | | | |
| SEQ ID No: | IMAGp998O205798;IMAGp971P1 | 233725 | PRDM1 PR domain containing 1, with ZNF | PRDM1 | 6 | 6,0 | 6,4 | 7,1 | | | |
| 31 | 197 | 9 | domain | | | | | | | | |
| SEQ ID No: | IMAGp998A10192;IMAGp971B10 | 135057 | UP uridine phosphorylase | UP | | 2,3 | 3,3 | 7,1 | | | |
| 32 | 8 | | | | | | | | | | |
| SEQ ID No: | IMAGp998F20979;IMAGp971E20 | 418195 | ESTs, Weakly similar to hypothetical protein | FLJ20378 | [Homo | 1,5 | 3,0 | 6,9 | | | |
| 33 | 38 | | sapiens] | | | | | | | | |
| SEQ ID No: | IMAGp998B095717;IMAGp971C1 | 230698 | | | | 2,0 | 6,9 | 6,7 | | | |
| 34 | 895 | 4 | | | | | | | | | |
| SEQ ID No: | IMAGp998M125582;IMAGp971H0 | 225541 | transcription factor CP2-like 1 | TFCP2L1 | | 2,5 | 4,9 | 6,7 | | | |
| 35 | 7127 | 1 | | | | | | | | | |
| SEQ ID No: | IMAGp998M154512;IMAGp971N1 | 184453 | | | | 2,5 | 3,5 | 6,6 | | | |
| 36 | 778 | 4 | | | | | | | | | |
| SEQ ID No: | IMAGp998M144547;IMAGp971G | 185797 | | | | 2,4 | 4,3 | 6,4 | | | |
| 37 | 0981 | 3 | | | | | | | | | |
| SEQ ID No: | IMAGp998L105312;IMAGp971P0 | 215170 | HLXB9 homeo box HB9 | HLXB9 | | 1,7 | 3,1 | 6,3 | | | |
| 38 | 590 | 5 | | | | | | | | | |
| SEQ ID No: | IMAGp998D201008;IMAGp971D2 | 429283 | pleckstrin homology-like domain, family A, | PHLDA3 | 1q31 | 3,1 | 2,7 | 6,2 | | | |
| 39 | 339 | | member 3 | | | | | | | | |
| SEQ ID No: | IMAGp998H065732;IMAGp971C0 | 231288 | | | | 2,2 | 3,1 | 6,1 | | | |
| 40 | 596 | 5 | | | | | | | | | |
| SEQ ID No: | IMAGp998H084295;IMAGp971G0 | 169157 | COL22A1 collagen, type XXII, alpha 1 | COL22A1 | | 2,0 | 3,3 | 6,1 | | | |
| 41 | 772 | 5 | | | | | | | | | |
| SEQ ID No: | IMAGp998F176102;IMAGp971E1 | 245377 | NACHT, leucine rich repeat and PYD | NALP9 | | 1,3 | 4,3 | 6,1 | | | |
| 42 | 5132 | 6 | containing 9 | | | | | | | | |
| SEQ ID No: | IMAGp998K013431;IMAGp971J0 | 135986 | | | | 2,4 | 3,5 | 6,0 | | | |
| 43 | 1106 | 4 | | | | | | | | | |
| SEQ ID No: | IMAGp998M23174;IMAGp971P24 | 41558 | | | 1 | 6,0 | 5,9 | 6,0 | | | |
| 44 | 114 | | | | | | | | | | |
| SEQ ID No: | IMAGp998C17793;IMAGp971B17 | 346696 | Human transcription factor RTEF-1 (RTEF1) | TEAD4 | 12p13.2- | 2,0 | 2,8 | 5,9 | | | |
| 45 | 34 | | mRNA, complete cds | | p13.3 | | | | | | |
| SEQ ID No: | IMAGp998O144918;IMAGp971P1 | 200048 | T-cell leukemia/lymphoma 1B or TCL6 T- | TCL1B, | 14q32.1 | 1,7 | 4,0 | 5,9 | | | |
| 46 | 3122 | 5 | cell leukemia/lymphoma 6 | TCL6 | | | | | | | |
| SEQ ID No: | IMAGp998O021010;IMAGp971O0 | 430297 | Human E1A enhancer binding protein | ETV4 | 17q21 | 3,8 | 3,1 | 5,8 | | | |
| 47 | 139 | | (E1A-F) mRNA, partial cds | | | | | | | | |
| SEQ ID No: | IMAGp998E201114;IMAGp971J1 | 470011 | Human mRNA for fibroblast tropomyosin | DKFZP76 | 1p36.13 | 2,2 | 3,3 | 5,7 | | | |
| 48 | 941 | | TM30 (pl) | 2N0610 | | | | | | | |
| SEQ ID No: | IMAGp998H224299;IMAGp971D2 | 169312 | | | | 2,1 | 2,8 | 5,6 | | | |
| 49 | 472 | 5 | | | | | | | | | |
| SEQ ID No: | IMAGp998G195647;IMAGp971J1 | 228023 | | | | 2,3 | 3,0 | 5,5 | | | |
| 50 | 8129 | 4 | | | | | | | | | |
| SEQ ID No: | IMAGp998K06166;IMAGp971K05 | 38228 | low density lipoprotein receptor-related | LRP4 | 11p11.2- | 4,2 | 4,5 | 5,5 | | | |
| 51 | 114 | | protein 4 | | p12 | | | | | | |
| SEQ ID No: | IMAGp998D03384;IMAGp971E04 | 200018 | H.sapiens mRNA for Tcell | TCL1A | 14q32.1 | 2,7 | 3,1 | 5,5 | | | |
| 52 | 15 | | leukemia/lymphoma 1 | | | | | | | | |
| SEQ ID No: | IMAGp998E01223;IMAGp971D06 | 147048 | Homo sapiens endothelial cell protein | PROCR | 20 | 14,1 | 7,6 | 5,4 | | | |
| 53 | 10 | | C/APC receptor (EPCR) mRNA, complete | | | | | | | | |
| | | | cds | | | | | | | | |
| SEQ ID No: | IMAGp998D054941;IMAGp971D0 | 200904 | | | | 1,3 | 2,3 | 5,3 | | | |
| 54 | 184 | 4 | | | | | | | | | |
| SEQ ID No: | IMAGp998A17115;IMAGp971B10 | 122008 | long-chain fatty-acyl elongase | LCE | 4q25 | NA | 5,2 | 5,3 | | | |
| 55 | 4 | | | | | | | | | | |
| SEQ ID No: | IMAGp998K035709;IMAGp971JO | 230412 | | | | 1,8 | 3,7 | 5,3 | | | |
| 56 | 394 | 2 | | | | | | | | | |
| SEQ ID No: | IMAGp998F13278;IMAGp971K05 | 48538 | ETV5 ets variant gene 5 (ets-related | ETV5 | 3q28 | 2,1 | 2,8 | 5,3 | | | |
| 57 | 119 | | molecule) | | | | | | | | |
| SEQ ID No: | IMAGp998B06202;IMAGp971A06 | 138917 | Human L-myc protein gene, complete cds | | 1 | 1,9 | 2,8 | 5,2 | | | |
| 58 | 9 | | | | | | | | | | |
| SEQ ID No: | IMAGp998E1783;IMAGp971E141 | 109816 | DNA helicase homolog PIF1 | PIF1 | 15q22.1 | 1,9 | 2,5 | 5,1 | | | |
| 59 | | | | | | | | | | | |
| SEQ ID No: | IMAGp998L095608;IMAGp971N0 | 226536 | Homo sapiens cDNA FLJ10417 fis, clone | | 7 | 2,0 | 4,7 | 5,1 | | | |
| 60 | 9128 | 8 | NT2RP1000112 | | | | | | | | |
| SEQ ID No: | IMAGp998E11660;IMAGp971E03 | 295666 | | | | 3,2 | 4,0 | 5,1 | | | |
| 61 | 30 | | | | | | | | | | |
| SEQ ID No: | IMAGp998K051891;IMAGp971K0 | 768508 | developmental pluripotency associated 4 | DPPA4 | | 1,2 | 2,4 | 4,9 | | | |
| 62 | 650 | | | | | | | | | | |
| SEQ ID No: | IMAGp998J14406;IMAGp971J111 | 208621 | | E2IG2 | | 2,5 | 2,1 | 4,8 | | | |
| 63 | 7 | | | | | | | | | | |
| SEQ ID No: | IMAGp998B231782;IMAGp971B2 | 726454 | sal-like 4 (Drosophila) | SALL4 | 20q13.13 | 2,1 | 2,8 | 4,8 | | | |
| 64 | 344 | | | | -q13.2 | | | | | | |
| SEQ ID No: | IMAGp998M024021;IMAGp971P0 | 158647 | | | | 1,9 | 2,6 | 4,8 | | | |
| 65 | 165 | 3 | | | | | | | | | |
| SEQ ID No: | IMAGp998C105092;IMAGp971B0 | 206700 | | | | 2,2 | 2,2 | 4,7 | | | |
| 66 | 286 | 9 | | | | | | | | | |
| SEQ ID No: | IMAGp998I19131;IMAGp971E20 | 127962 | Homo sapiens cDNA FLJ22539 fis, clone HRC13227 | | 4 | 2,2 | 3,2 | 4,7 | | | |
| 67 | 6 | | | | | | | | | | |
| SEQ ID No: | IMAGp998F246080;IMAGp971E2 | 244533 | Homo sapiens mRNA for AND-1 protein | AND-1 | 14q22.1 | 0,9 | 2,1 | 4,7 | | | |
| 68 | 2131 | 5 | | | | | | | | | |
| SEQ ID No: | IMAGp998B03791;IMAGp971F11 | 345890 | E2F transcription factor 1 | E2F1 | | 4,6 | 2,8 | 4,7 | | | |
| 69 | 34 | | | | | | | | | | |
| SEQ ID No: | IMAGp998N15372;IMAGp971M16 | 195662 | cadherin 1, type 1, E-cadherin (epithelial) | CDH1 | 16q22.1 | 1,7 | 2,1 | 4,7 | | | |
| 70 | 15 | | | | | | | | | | |
| SEQ ID No: | IMAGp998G201924;IMAGp971G2 | 781099 | | | | 2,2 | 2,6 | 4,6 | | | |
| 71 | 151 | | | | | | | | | | |
| SEQ ID No: | IMAGp998E1983;IMAGp971E212 | 109818 | | | | 2,1 | 2,3 | 4,5 | | | |
| 72 | | | | | | | | | | | |
| SEQ ID No: | IMAGp998C09200;IMAGp971D09 | 138176 | hypothetical protein FLJ10652 | FLJ10652 | 12p12.1 | 2,3 | 3,5 | 4,5 | | | |
| 73 | 9 | | | | | | | | | | |
| SEQ ID No: | IMAGp998I204942;IMAGp971J20 | 200956 | | | | 2,1 | 2,5 | 4,4 | | | |
| 74 | 84 | 3 | | | | | | | | | |
| SEQ ID No: | IMAGp998H171818;IMAGp971I1 | 740416 | SRY (sex determining region Y)-box 17 | SOX17 | 8q11.22 | 2,0 | 2,2 | 4,4 | | | |
| 75 | 747 | | | | | | | | | | |
| SEQ ID No: | IMAGp998P175394;IMAGp971N1 | 218329 | Homo sapiens cDNA FLJ12742 fis, clone | NT2RP2000644 | | NA | 10,5 | 4,3 | | | |
| 76 | 8124 | 6 | | | | | | | | | |
| SEQ ID No: | IMAGp998F105821;IMAGp971B1 | 234586 | | | | 2,3 | 3,7 | 4,4 | | | |
| 77 | 898 | 5 | | | | | | | | | |
| SEQ ID No: | IMAGp998G01671;IMAGp971H03 | 299928 | Homo sapiens GN6ST mRNA for N- | CHST2 | 3q24 | 2,8 | 2,1 | 4,2 | | | |
| 78 | 30 | | acetylglucosamine-6-O-sulfotransferas e | | | | | | | | |
| | | | (GlcNAc6ST) | | | | | | | | |
| SEQ ID No: | IMAGp998D125539;IMAGp971F1 | 223868 | | | | 1,4 | 2,5 | 4,2 | | | |
| 79 | 691 | 3 | | | | | | | | | |
| SEQ ID No: | IMAGp998C22571;IMAGp971D21 | 261453 | | | | 1,8 | 2,4 | 4,1 | | | |
| 80 | 23 | | | | | | | | | | |
| SEQ ID No: | IMAGp998M24260;IMAGp971I23 | 161471 | Sialyltransferase (CMP-N- | SIAT1 | 3q27-q28 | 2,7 | 3,0 | 4,1 | | | |
| 81 | 12 | | acetylneuraminate-beta-galactoside alpha- | | | | | | | | |
| | | | 2,6-sialyltransferase) | | | | | | | | |
| SEQ ID No: | IMAGp998N11579;IMAGp971K18 | 264778 | Solute carrier family 25 (mitochondrial | SLC25A1 | 10q21.3- | 1,6 | 2,3 | 4,1 | | | |
| 82 | 23 | | carrier; Graves disease autoantigen), | 6 | q22.1 | | | | | | |
| | | | member 16 | | | | | | | | |
| SEQ ID No: | IMAGp998E15164;IMAGp971H10 | 36807 | Homo sapiens neutral sphingomyelinase | NSMAF | | 2,4 | 3,7 | 4,0 | | | |
| 83 | 113 | | (N-SMase) activation associated factor | | | | | | | | |
| SEQ ID No: | IMAGp998N06288;IMAGp971L03 | 52960 | Homo sapiens, clone IMAGE4123507, mRNA | | | 2,0 | 2,8 | 4,0 | | | |
| 84 | 118 | | | | | | | | | | |
| SEQ ID No: | IMAGp998M18194;IMAGp971N06 | 136121 | Homo sapiens cDNA FLJ10247 fis, clone | 14 | 4,8 | 2,4 | 4,0 | | | | |
| 85 | 8 | | HEMBB1000705 | | | | | | | | |
| SEQ ID No: | IMAGp998J155722;IMAGp971K1 | 230910 | | | 2,6 | 2,4 | 4,0 | | | | |
| 86 | 295 | 2 | | | | | | | | | |
| SEQ ID No: | IMAGp998B21790;IMAGp971E13 | 345524 | integral membrane protein 2C | ITM2C 2q37 | 2,8 | 2,3 | 4,0 | | | | |
| 87 | 34 | | | | | | | | | | |
| SEQ ID No: | IMAGp998J09792;IMAGp971C04 | 346472 | Homo sapiens, clone IMAGE4123507, mRNA | | 2,2 | 2,9 | 3,9 | | | | |
| 88 | 134 | | | | | | | | | | |
| SEQ ID No: | IMAGp998H175550;IMAGp971H2 | 224300 | | 3 | 2,1 | 2,2 | 3,9 | | | | |
| 89 | 092 | 8 | | | | | | | | | |
| SEQ ID No: | IMAGp998E24312;IMAGp971F24 | 172415 | Solute carrier family 35 (UDP-galactose | SLC35A2 Xp11.23- | 2,1 | 2,6 | 3,9 | | | | |
| 90 | 12 | | transporter), member A2 | p11.22 | | | | | | | |
| SEQ ID No: | IMAGp998J081153;IMAGp971J10 | 485095 | LOC388610 (LOC388610), mRNA | | 2,2 | 2,2 | 3,8 | | | | |
| 91 | 40 | | | | | | | | | | |
| SEQ ID No: | IMAGp998G18196;IMAGp971H14 | 136745 | hypothetical protein FLJ20171 | FLJ20171 8q21.3 | 3,2 | 2,4 | 3,8 | | | | |
| 92 | 8 | | | | | | | | | | |
| SEQ ID No: | IMAGp998F241862;IMAGp971F2 | 757271 | | | 2,5 | 2,8 | 3,8 | | | | |
| 93 | 248 | | | | | | | | | | |
| SEQ ID No: | IMAGp998B09229;IMAGp971B04 | 149288 | Nedd4 family interacting protein 2 | NDFIP2 13q22.2 | 5,7 | 4,9 | 3,7 | | | | |
| 94 | 10 | | | | | | | | | | |
| SEQ ID No: | IMAGp998E11127;IMAGp971H14 | 126322 | Caspase 4, apoptosis-related cysteine | CASP4 | 5,1 | 2,6 | 3,7 | | | | |
| 95 | 5 | | protease | | | | | | | | |
| SEQ ID No: | IMAGp998N02421;IMAGp971N05 | 214465 | Oxysterol binding protein-like 3 | OSBPL3 7p15 | 4,2 | 4,0 | 3,6 | | | | |
| 96 | 18 | | | | | | | | | | |
| SEQ ID No: | IMAGp998B145536;IMAGp971B1 | 223748 | | | 1,1 | 2,2 | 3,6 | | | | |
| 97 | 592 | 5 | | | | | | | | | |
| SEQ ID No: | IMAGp998C144120;IMAGp971D1 | 162426 | | | 1,5 | 2,7 | 3,6 | | | | |
| 98 | 066 | 1 | | | | | | | | | |
| SEQ ID No: | IMAGp998M232973;IMAGp971E1 | 118406 | | | 2,5 | 3,2 | 3,6 | | | | |
| 99 | 958 | 2 | | | | | | | | | |
| SEQ ID No: | IMAGp998D051943;IMAGp971D0 | 788308 | Cell cycle progression 8 protein | CPR8 15q21.1 | 2,0 | 2,2 | 3,6 | | | | |
| 100 | 552 | | | | | | | | | | |
| SEQ ID No: | IMAGp998M031831;IMAGp971M | 745514 | | | 4,1 | 3,3 | 3,5 | | | | |
| 101 | 0448 | | | | | | | | | | |
| SEQ ID No: | IMAGp998K243513;IMAGp971L2 | 139137 | | | 1,3 | 3,3 | 3,5 | | | | |
| 102 | 358 | 5 | | | | | | | | | |
| SEQ ID No: | IMAGp998B165468;IMAGp971J0 | 221137 | Annexin A3 | ANXA3 4q13-q22 | 2,6 | 2,7 | 3,5 | | | | |
| 103 | 4132 | 5 | | | | | | | | | |
| SEQ ID No: | IMAGp998G024171;IMAGp971L0 | 164392 | | | 1,4 | 4,0 | 3,5 | | | | |
| 104 | 469 | 9 | | | | | | | | | |
| SEQ ID No: | IMAGp998K15174;IMAGp971L16 | 41539 | Homo sapiens mRNA for KIAA0644 protein, | KIAA064 7p21.1 | 1,8 | 3,6 | 3,4 | | | | |
| 105 | 114 | | complete cds | 4 | | | | | | | |
| SEQ ID No: | IMAGp998I04624;IMAGp971H09 | 281931 | cytoplasmic polyadenylation element | CPEB1 15q24.2 | 1,4 | 2,6 | 3,4 | | | | |
| 106 | 26 | | binding protein | | | | | | | | |
| SEQ ID No: | IMAGp998M24235;IMAGp971N24 | 151871 | hypothetical protein FLJ22662 | FLJ22662 12p13.31 | 2,6 | 2,2 | 3,4 | | | | |
| 107 | 11 | | | | | | | | | | |
| SEQ ID No: | IMAGp998F16280;IMAGp971H22 | 49499 | Dedicator of cytokinesis 11 | DOCK11 | 2,3 | 2,8 | 3,4 | | | | |
| 108 | 117 | | | | | | | | | | |
| SEQ ID No: | IMAGp998B23677;IMAGp971A24 | 302134 | H.sapiens mRNA for SOX-4 protein | SOX4 6p23 | 2,0 | 2,4 | 3,4 | | | | |
| 109 | 31 | | | | | | | | | | |
| SEQ ID No: | IMAGp998N204778;IMAGp971F1 | 194670 | ESTs, Weakly similar to C Chain C, Human | Activated Protein C | 2,5 | 3,4 | 3,4 | | | | |
| 110 | 184 | 7 | [H.sapiens] | | | | | | | | |
| SEQ ID No: | IMAGp998E163300;IMAGp971F1 | 130943 | | | 2,4 | 2,9 | 3,3 | | | | |
| 111 | 2104 | 1 | | | | | | | | | |
| SEQ ID No: | IMAGp998P192044;IMAGp971O2 | 827394 | ash2 (absent, small, or homeotic)-like | ASH2L | 2,3 | 2,6 | 3,3 | | | | |
| 112 | 055 | | (Drosophila) | | | | | | | | |
| SEQ ID No: | IMAGp998I144113;IMAGp971I13 | 162171 | hypothetical protein LOC130576 | | 0,6 | 3,5 | 3,3 | | | | |
| 113 | 66 | 7 | | | | | | | | | |
| SEQ ID No: | IMAGp998N045486;IMAGp971L0 | 221856 | | | 1,4 | 2,2 | 3,3 | | | | |
| 114 | 2126 | 3 | | | | | | | | | |
| SEQ ID No: | IMAGp998I16328;IMAGp971J141 | 178647 | Myosin VA (heavy polypeptide 12, myoxin) | MYO5A 15 | 2,1 | 2,0 | 3,3 | | | | |
| 115 | 3 | | | | | | | | | | |
| SEQ ID No: | IMAGp998C14658;IMAGp971A13 | 294853 | UDP-glucose ceramide glucosyltransferase | UGCG 9 | 1,8 | 2,5 | 3,2 | | | | |
| 116 | 30 | | | | | | | | | | |
| SEQ ID No: | IMAGp998M174328;IMAGp971I1 | 170437 | | | 2,2 | 2,5 | 3,2 | | | | |
| 117 | 573 | 6 | | | | | | | | | |
| SEQ ID No: | IMAGp998D065777;IMAGp971B0 | 232891 | Vacuolar protein sorting 13A (yeast | VPS13A | 2,0 | 2,7 | 3,2 | | | | |
| 118 | 496 | 7 | | | | | | | | | |
| SEQ ID No: | IMAGp998D01196;IMAGp971D19 | 136656 | Sorting nexin 10 | SNX10 7p21.1 | 1,6 | 4,9 | 3,2 | | | | |
| 119 | 9 | | | | | | | | | | |
| SEQ ID No: | IMAGp998B2070;IMAGp971A131 | 22170 | Homo sapiens family with sequence | FAM16AX Xp22.32 | 2,0 | 2,1 | 3,2 | | | | |
| 120 | 10 | | similarity 16, member A, X-linked | | | | | | | | |
| SEQ ID No: | IMAGp998I17258;IMAGp971G18 | 160600 | Human mRNA for complement control | IF 4q25 | 6,5 | 2,3 | 3,2 | | | | |
| 121 | 12 | | protein factor I | | | | | | | | |
| SEQ ID No: | IMAGp998M04179;IMAGp971M1 | 130347 | Dysferlin, limb girdle muscular dystrophy | DYSF 2p13.3- | 2,9 | 2,3 | 3,2 | | | | |
| 122 | 07 | | 2B (autosomal recessive) | p13.1 | | | | | | | |
| SEQ ID No: | IMAGp998K175764;IMAGp971N2 | 232410 | | | 1,8 | 2,8 | 3,2 | | | | |
| 123 | 2120 | 4 | | | | | | | | | |
| SEQ ID No: | IMAGp998J11128;IMAGp971H04 | 126826 | | | 0,8 | 2,7 | 3,2 | | | | |
| 124 | 5 | | | | | | | | | | |
| SEQ ID No: | IMAGp998H195650;IMAGp971K1 | 228141 | | | 1,3 | 2,3 | 3,1 | | | | |
| 125 | 8129 | 0 | | | | | | | | | |
| SEQ ID No: | IMAGp998P025858;IMAGp971N0 | 236030 | | | 1,5 | 2,6 | 3,1 | | | | |
| 126 | 499 | 5 | | | | | | | | | |
| SEQ ID No: | IMAGp998M03208;IMAGp971N10 | 141482 | Solute carrier family 38, member 1 | SLC38A1 | 1,9 | 2,5 | 3,1 | | | | |
| 127 | 9 | | | | | | | | | | |
| SEQ ID No: | IMAGp998H07795;IMAGp971C05 | 347574 | Full length insert cDNA clone ZD86G04 | | 2,1 | 2,6 | 3,1 | | | | |
| 128 | 35 | | | | | | | | | | |
| SEQ ID No: | IMAGp998A112011;IMAGp971D1 | 814354 | | | 1,3 | 2,9 | 3,1 | | | | |
| 129 | 5103 | | | | | | | | | | |
| SEQ ID No: | IMAGp998A205772;IMAGp971F2 | 232693 | Timeless homolog (Drosophila) | TIMELESS | 1,5 | 2,1 | 3,1 | | | | |
| 130 | 296 | 9 | | | | | | | | | |
| SEQ ID No: | IMAGp998I09280;IMAGp971J021 | 49203 | DNA-damage-inducible transcript 4-like | DDIT4L 4q23 | 2,9 | 5,5 | 3,1 | | | | |
| 131 | 17 | | | | | | | | | | |
| SEQ ID No: | IMAGp998J24653;IMAGp971I182 | 293111 | Human cDNA for uracil-DNA glycosylase | UNG 12 | 1,8 | 2,7 | 3,0 | | | | |
| 132 | 9 | | | | | | | | | | |
| SEQ ID No: | IMAGp998N161887;IMAGp971I2 | 767055 | Sema domain, immunoglobulin domain | SEMA3A | 1,9 | 2,3 | 3,0 | | | | |
| 133 | 350 | | (Ig), short basic domain, secreted, | | | | | | | | |
| | | | (semaphorin) 3A | | | | | | | | |
| DD genes | | | | | | | | | | | |
| SEQ ID No: | 134 | | THIOREDOXIN INTERACTING PROTEIN; | TXNIP | | | | | | | |
| | | | UPREGULATED BY 1,25- | | | | | | | | |
| | | | DIHYDROXYVITAMIN D-3 | | | | | | | | |
| SEQ ID No: | 135 | | CALDESMON (CDM). | CALD1 | | | | | | | |
| SEQ ID No: | 136 | | HLA CLASS II HISTOCOMPATIBILITY | CD74 | | | | | | | |
| | | | ANTIGEN, GAMMA CHAIN (HLA-DR | | | | | | | | |
| | | | ANTIGENS ASSOCIATED INVARIANT | | | | | | | | |
| | | | CHAIN) (IA ANTIGEN-ASSOCIATED | | | | | | | | |
| | | | INVARIANT CHAIN) (II) (P33) (CD74 | | | | | | | | |
| | | | ANTIGEN). | | | | | | | | |
| SEQ ID No: | 137 | | COMPLEMENT COMPONENT C7 | C7 | | | | | | | |
| | | | PRECURSOR | | | | | | | | |
| SEQ ID No: | 138 | | SMALL INDUCIBLE CYTOKINE B16 | CXCL16 | | | | | | | |
| | | | (TRANSMEMBRANE CHEMOKINE CXCL16) | | | | | | | | |
| | | | (SR- PSOX) (SCAVENGER RECEPTOR FOR | | | | | | | | |
| | | | PHOSPHATIDYLSERINE AND OXIDIZED | | | | | | | | |
| | | | LOW DENSITY LIPOPROTEIN) | | | | | | | | |
| SEQ ID No: | 139 | | CYTOCHROME B REDUCTASE 1; | CYBRD1 | | | | | | | |
| | DUODENAL CYTOCHROME B | | | | | | | | | | |
| SEQ ID No: 140 | INTERFERON-INDUCED TRANSMEMBRANE FITM1 | | | | | | | | | | |
| | PROTEIN 1 (INTERFERON-INDUCED | | | | | | | | | | |
| | PROTEIN 17) (INTERFERON-INDUCIBLE | | | | | | | | | | |
| | PROTEIN 9-27) (LEU-13 ANTIGEN) (CD225 | | | | | | | | | | |
| | ANTIGEN). | | | | | | | | | | |
| SEQ ID No: 141 | SAL-LIKE PROTEIN 4 (ZINC FINGER SALL4 | | | | | | | | | | |
| | PROTEIN SALL4). | | | | | | | | | | |
| SEQ ID No: 142 | ENDOPLASMIN PRECURSOR (94 KDA TRA1 | | | | | | | | | | |
| | GLUCOSE-REGULATED PROTEIN) (GRP94) | | | | | | | | | | |
| | (GP96 HOMOLOG) (TUMOR REJECTION ANTIGEN 1). | | | | | | | | | | |
| SEQ ID No: 143 | ALPHA 2 TYPE I COLLAGEN; COLLAGEN I, COL1A2 | | | | | | | | | | |
| | ALPHA-2 POLYPEPTIDE; COLLAGEN OF | | | | | | | | | | |
| | SKIN, TENDON AND BONE, ALPHA-2 CHAIN | | | | | | | | | | |
| SEQ ID No: 144 | HISTONE DEACETYLASE 3 (HD3) (RPD3- HDAC3 2). | | | | | | | | | | |
| SEQ ID No: 145 | HLA CLASS II HISTOCOMPATIBILITY HLA-DRA | | | | | | | | | | |
| | ANTIGEN, DR ALPHA CHAIN PRECURSOR | | | | | | | | | | |
| SEQ ID No: 146 | DYNAMIN-LIKE 120 KDA PROTEIN, OPA1 | | | | | | | | | | |
| | MITOCHONDRIAL PRECURSOR (OPTIC | | | | | | | | | | |
| | ATROPHY 1 GENE PROTEIN). | | | | | | | | | | |
| SEQ ID No: 147 | BULLOUS PEMPHIGOID ANTIGEN 1 BPAG1 | | | | | | | | | | |
| | ISOFORMS 1/2/3/4/5/8 (230 KDA | | | | | | | | | | |
| | BULLOUS PEMPHIGOID ANTIGEN) (BPA) | | | | | | | | | | |
| | (HEMIDESMOSOMAL PLAQUE PROTEIN) | | | | | | | | | | |
| | (DYSTONIA MUSCULORUM PROTEIN) | | | | | | | | | | |
| SEQ ID No: 148 | DECORIN PRECURSOR (BONE DCN | | | | | | | | | | |
| | PROTEOGLYCAN II) (PG-S2) (PG40) | | | | | | | | | | |
| SEQ ID No: | 149 | | LEUKOCYTE SURFACE ANTIGEN CD47 | CD47 | | | | | | | |
| | | | PRECURSOR (ANTIGENIC SURFACE | | | | | | | | |
| | | | DETERMINANT PROTEIN OA3) (INTEGRIN | | | | | | | | |
| | | | ASSOCIATED PROTEIN) (IAP) (MER6). | | | | | | | | |
| SEQ ID No: | 150 | | b2-microglobulin | | | | | | | | |
| SEQ ID No: | 151 | | 60S RIBOSOMAL PROTEIN L27 | RPL27 | | | | | | | |
| SEQ ID No: | 152 | | 40S RIBOSOMAL PROTEIN S16 | RPS16 | | | | | | | |
| SEQ ID No: | 153 | | UNCHARACTERIZED HYPOTHALAMUS PROTEIN HCDASE NM_018479 | | | | | | | | |
| SEQ ID No: | 154 | | Overexpressed in CIS | OIC1 | | | | | | | |
| SEQ ID No: | 155 | | Ensambl EST ENSESTT00000023592 | | | | | | | | |
| SEQ ID No: | 156 | | Chromosome 16 clone | | | | | | | | |
| SEQ ID No: | 157 | | Chromosome-1 clone hypothetical protein XP_211586 | | | | | | | | |
| EC genes | | | | | | | | | | | |
| SEQ ID No: | IMAGp998F065401;IMAGp971I0 | 218573 | S100A14 S100 calcium binding protein A14 S100A14 | 1,157 | 1,335 | 1,685 | 0,6 24,36 | 0,105 | | | |
| 158 | 6125 | 3 | | 5 | | | | | | | |
| SEQ ID No: | IMAGp998J065499;IMAGp971I22 | 222346 | transmembrane 4 superfamily member 3 TM4SF3 | 3,165 | 0,88 | 0,9625 | 0,6 17,62 | 0,05 | | | |
| 159 | 120 | 1 | | | | | | | | | |
| SEQ ID No: | IMAGp998A05535;IMAGp971B06 | 247564 | Homo sapiens transcribed sequence with moderate 10 | 1,102 | 1,15 | 1,4575 | 1,29 15,53 | NA | | | |
| 160 | 21 | | similarity to protein ref:NP_060190.1 (H.sapiens) | 5 | | | | | | | |
| | | | hypothetical protein FLJ20234 [Homo | | | | | | | | |
| SEQ ID No: | IMAGp998B19790;IMAGp971B09 | 345522 | SEC13-like 1 (S. cerevisiae) | 2,332 | 0,845 | 0,5625 | 0,61 15,03 | 0,065 | | | |
| 161 | 34 | | | | 5 | | 5 | | | | |
| SEQ ID No: | IMAGp998M105514;IMAGp971K1 | 222929 | | 2,367 | 0,87 | 0,5475 | 0,715 14,86 | 0,085 | | | |
| 162 | 0120 | 7 | | | 5 | | 5 | | | | |
| SEQ ID No: | IMAGp9980095541;IMAGp971P0 | 223971 | Homo sapiens transcribed sequence with weak similarity to | 1,21 | 1,23 | 0,95 | 0,93 13,86 | 0,06 | | | |
| 163 | 592 | 2 | protein ref:NP_038602.1 (M.musculus) L1 repeat, Tf subfamily, | | | | | | | | |
| | | | member 18 [Mus musculus] | | | | | | | | |
| SEQ ID No: | IMAGp998D121896;IMAGp971D1 | 770267 | | 0,79 | 0,705 | 0,5075 | 0,695 13,47 | 0,04 | | | |
| 164 | 150 | | | | | | 5 | | | | |
| SEQ ID No: | IMAGp998H235725;IMAGp971H1 | 231021 | | | | 0,852 | 0,61 | 0,835 | 1,05 | 13,38 | 0,06 |
| 165 | 195 | 4 | | | | 5 | | | | | |
| SEQ ID No: | IMAGp998P03372;IMAGp971O04 | 195698 | Human fibrinogen beta chain gene, | FGB | 4q28 | 1,205 | 1,36 | 1,105 | 0,8 11,69 | | 0,08 |
| 166 | 15 | | complete mRNA | | | | | | | 5 | |
| SEQ ID No: | IMAGp998B071870;IMAGp971C0 | 760230 | Homo sapiens secreted cement gland | AGR2 | 7p21.3 | 0,78 | 0,725 | 0,71 | 1,2 | 11,05 | 0,06 |
| 167 | 749 | | protein XAG-2 homolog (hAG-2/R) mRNA, | | | | | | | | |
| | | | complete cds | | | | | | | | |
| SEQ ID No: | IMAGp998D08822;IMAGp971C07 | 357847 | coiled-coil domain containing 2 | CCDC2 | | 2,14 | 0,85 | 0,56 | 0,67 | 9,57 | 0,105 |
| 168 | 35 | | | | | | | | | | |
| SEQ ID No: | IMAGp998I08822;IMAGp971C06 | 357967 | KIAA1712 protein | KIAA1712 | | 2,145 | 0,865 | 0,58 | 0,89 | 9,5 | 0,065 |
| 169 | 35 | | | | | | | | | | |
| SEQ ID No: | IMAGp998D09822;IMAGp971C09 | 357848 | Homo sapiens similar to hypothetical protein | FLJ20958 | | 1,955 | 0,875 | 0,605 | 0,655 9,415 | | 0,095 |
| 170 | 35 | | (LOC285177), mRNA | | | | | | | | |
| SEQ ID No: | IMAGp998K045001;IMAGp971H0 | 203225 | chromosome 4 open reading frame 7 | C4orf7 | | 0,795 | 1,015 | 1,055 | 1,405 | 9,35 | 0,095 |
| 171 | 285 | 1 | | | | | | | | | |
| SEQ ID No: | IMAGp998K125400;IMAGp971C1 | 218547 | | | | 0,665 | 0,82 | 1,165 | 1,02 | 9,3 | 0,08 |
| 172 | 9125 | 5 | | | | | | | | | |
| SEQ ID No: | IMAGp998K151776;IMAGp971P2 | 724358 | keratin 19 | KRT19 | 15 | 0,88 | 0,91 | 0,77 | 0,56 | 8,88 | 0,065 |
| 173 | 043 | | | | | | | | | | |
| SEQ ID No: | IMAGp998I164581;IMAGp971L1 | 187093 | fibronectin 1 | FN1 | | 1,742 | 0,8 | 0,685 | 0,755 | 8,875 | 0,09 |
| 174 | 480 | 5 | | | | 5 | | | | | |
| SEQ ID No: | IMAGp998F044825;IMAGp971I0 | 196454 | | | | 0,827 | 1,325 | 0,955 | 0,915 | 8,615 | 0,105 |
| 175 | 3121 | 7 | | | | 5 | | | | | |
| SEQ ID No: | IMAGp998L206082;IMAGp971L1 | 244624 | | | | 0,872 | 0,79 | 0,9425 | 1,185 | 8,475 | 0,15 |
| 176 | 9131 | 3 | | | | 5 | | | | | |
| SEQ ID No: | IMAGp998D085368;IMAGp971A0 | 217301 | Homo sapiens caudal-type homeobox gene | CDX2 | 13q12.3 | 1,185 | 0,97 | 0,985 | 1,34 | 8,415 | 0,165 |
| 177 | 792 | 5 | 2 (CDX2) sequence | | | | | | | | |
| SEQ ID No: | IMAGp998D20611;IMAGp971E20 | 276835 | TPA regulated locus | TPARL | 4q12 | 1,06 | 1,46 | 0,8625 | 1,125 8,395 | | 0,1 |
| 178 | 25 | | | | | | | | | | |
| SEQ ID No: | IMAGp998B14742;IMAGp971C24 | 327085 | discs, large (Drosophila) homolog- | DLGAP1 | 8 | 2,612 | 0,98 | 0,755 | 0,695 | 7,83 | 0,095 |
| 179 | 33 | | associated protein 1 | | | 5 | | | | | |
| SEQ ID No: | IMAGp998B07407;IMAGp971E04 | 208806 | Human serum prealbumin gene, complete | TTR | 18q12.1 | 0,832 | 0,965 | 0,95 | 0,925 | 7,675 | 0,175 |
| 180 | 17 | | cds | | | 5 | | | | | |
| SEQ ID No: | IMAGp998C16522;IMAGp971C15 | 242631 | Human mRNA for apolipoprotein AII | APOA2 | 1q21-q23 | 0,615 | 0,85 | 1,065 | 1,12 | 7,51 | 0,28 |
| 181 | 21 | | precursor | | | | | | | | |
| SEQ ID No: | IMAGp998O055540;IMAGp971O0 | 223932 | cholecystokinin B receptor | CCKBR | | 1,115 | 0,855 | 1,45 | 0,995 | 7,25 | 0,11 |
| 182 | 692 | 4 | | | | | | | | | |
| SEQ ID No: | IMAGp998A244944;IMAGp971B2 | 201014 | | | | 0,85 | 1,15 | 1,785 | 1,21 | 7,175 | 0,12 |
| 183 | 484 | 3 | | | | | | | | | |
| SEQ ID No: | IMAGp998I15843;IMAGp971H14 | 366038 | SERUM ALBUMIN PRECURSOR | Q8IUK7 | | 0,64 | 1,28 | 1,01 | 0,86 | 7,01 | 0,24 |
| 184 | 36 | | | | | | | | | | |
| SEQ ID No: | IMAGp998K105256;IMAGp971F0 | 213017 | | | | 0,56 | 0,88 | 0,8925 | 0,79 | 6,835 | 0,31 |
| 185 | 2120 | 7 | | | | | | | | | |
| SEQ ID No: | IMAGp998B094453;IMAGp971B2 | 175210 | | | | 1,227 | 0,855 | 0,77 | 0,63 | 6,765 | 0,26 |
| 186 | 475 | 4 | | | | 5 | | | | | |
| SEQ ID No: | IMAGp998G193982;IMAGp971E1 | 157137 | Homo sapiens transcribed sequence with moderate similarity to | | | 1,232 | 1,335 | 1,49 | 1,82 | 6,69 | 0,11 |
| 187 | 864 | 0 | protein pir:S30392 (H.sapiens) S30392 phospholipase A2 | | | 5 | | | | | |
| | | | homolog (clone AF-5) - human retrotransposon | | | | | | | | |
| SEQ ID No: | IMAGp998P194992;IMAGp971N1 | 202893 | ESTs, Moderately similar to JE0065 retroviral proteinase-like | | | 1,482 | 1,23 | 1,7875 | 1,33 | 6,685 | 0,15 |
| 188 | 785 | 0 | protein [H.sapiens] | | | 5 | | | | | |
| SEQ ID No: | IMAGp998F015873;IMAGp971H0 | 236582 | | | | 3,56 | 1,845 | 0,87 | 1,83 | 6,505 | 0,305 |
| 189 | 199 | 4 | | | | | | | | | |
| SEQ ID No: | IMAGp998A054888;IMAGp971B0 | 198862 | | | | 1,562 | 0,88 | 1,02 | 0,925 | 6,45 | 0,125 |
| 190 | 4121 | 0 | | | | 5 | | | | | |
| SEQ ID No: | IMAGp998A04528;IMAGp971A02 | 244875 | Human cystathionine-beta-synthase (CBS) | CBS | 21q22.3 | 1,327 | 0,925 | 0,795 | 1,735 | 6,33 | 0,085 |
| 191 | 22 | | mRNA | | | 5 | | | | | |
| SEQ ID No: | IMAGp998H22207;IMAGp971I22 | 140997 | Growth differentiation factor 15 | GDF15 | 19p13.1- | 1,095 | 1,18 | 1,0975 | 1,515 | 6,23 | 0,14 |
| 192 | 10 | | | | 13.2 | | | | | | |
| SEQ ID No: | IMAGp998P151066;IMAGp971M0 | 451838 | | | | 2,61 | 1,385 | 0,7125 | 0,915 | 5,995 | 0,115 |
| 193 | 340 | | | | | | | | | | |
| SEQ ID No: | IMAGp998K055550;IMAGp971D0 | 224306 | | | 2 | 1,825 | 2,145 | 1,45 | 1,49 | 5,965 | 0,27 |
| 194 | 892 | 8 | | | | | | | | | |
| SEQ ID No: | IMAGp998A065260;IMAGp971A0 | 213146 | ETS2 v-ets erythroblastosis virus E26 | ETS2 | | 3,292 | 1,38 | 1,58 | 0,695 | 5,965 | 0,22 |
| 195 | 890 | 9 | oncogene homolog 2 (avian) | | | 5 | | | | | |
| SEQ ID No: | IMAGp998J076121;IMAGp971M0 | 246115 | Inhibitor of DNA binding 3, dominant | ID3 | | 1,747 | 0,93 | 1,0025 | 0,85 | 5,85 | 0,13 |
| 196 | 4102 | 8 | negative helix-loop-helix protein | | | 5 | | | | | |
| SEQ ID No: | IMAGp998L10662;IMAGp971G05 | 296601 | H.sapiens mRNA for M130 antigen | CD163 | 12p13.3 | 4,722 | 1,315 | 0,565 | 1,79 | 5,77 | 0,115 |
| 197 | 30 | | cytoplasmic variant 2 | | | 5 | | | | | |
| SEQ ID No: | IMAGp998D0595;IMAGp971C043 | 114388 | | | | 3,07 | 2,04 | 1,7 | 0,775 | 5,765 | 0,165 |
| 198 | | | | | | | | | | | |
| SEQ ID No: | IMAGp998G06783;IMAGp971L03 | 342941 | APLN apelin | APLN | Xq25- | 1,497 | 3,02 | 1,8725 | 0,905 | 5,75 | 0,13 |
| 199 | 33 | | | | 26.3 | 5 | | | | | |
| SEQ ID No: | IMAGp998K08276;IMAGp971K12 | 47615 | Human MHC HLA-B7 class I cell surface | HLA-B | 6p21.3 | NA | 1,735 | 0,74 | 1,925 | 5,71 | 0,27 |
| 200 | 119 | | glycoprotein heavy chain mRNA, complete | | | | | | | | |
| | | | cds | | | | | | | | |
| SEQ ID No: | IMAGp998F152230;IMAGp971F1 | 898574 | | | | 1,545 | 0,95 | 1,065 | 1,68 | 5,7 | 0,15 |
| 201 | 655 | | | | | | | | | | |
| SEQ ID No: | IMAGp998O075514;IMAGp971L0 | 222934 | FLJ10808 hypothetical protein FLJ10808 | FLJ10808 | | 3,182 | 2,86 | 1,83 | 0,515 | 5,69 | 0,155 |
| 202 | 3120 | 2 | | | | 5 | | | | | |
| Seminoma | genes | | | | | | | | | | |
| SEQ ID No: | IMAGp998A022575;IMAGp971C0 | 103092 | | | | 0,755 | 1,165 | 0,81 | 5,92 | 0,59 | 19,32 |
| 203 | 257 | 1 | | | | | | | | | |
| SEQ ID No: | IMAGp998E122300;IMAGp971F1 | 925427 | | | | 0,765 | 1,1 | 0,8375 | 7,37 | 0,47 | 11,74 |
| 204 | 455 | | | | | | | | | | |
| SEQ ID No: | IMAGp998C12278;IMAGp971H11 | 48417 | FRCP2 likely ortholog of mouse fibronectin | FRCP2 | | 1,432 | 1,48 | 3,045 | 3,135 | 0,135 | 10,50 |
| 205 | 117 | | type III repeat containing protein 2 | | | 5 | | | | | 5 |
| SEQ ID No: | IMAGp998E191782;IMAGp971A1 | 726522 | Homo sapiens similar to Envoplakin (210 kDa paraneoplastic | | | 0,975 | 1,125 | 0,8925 | 5,3 | 0,895 | 7,96 |
| 206 | 744 | | pemphigus antigen) (p210) (210 kDa cornified envelope | | | | | | | | |
| | | | precursor) (LOC339237), mRNA | | | | | | | | |
| SEQ ID No: | IMAGp9980124335;IMAGp971Pl | 170710 | Human chitotriosidase precursor mRNA, | CHIT1 | 1q31-q32 | 1,427 | 1,065 | 0,82 | 13,945 | 0,92 | 7,67 |
| 207 | 173 | 7 | complete cds | | | 5 | | | | | |
| SEQ ID No: | IMAGp998K102640;IMAGp971L1 | 105612 | | | | 1,05 | 0,68 | 1,265 | 5,195 | 0,17 | 7,165 |
| 208 | 957 | 9 | | | | | | | | | |
| SEQ ID No: | IMAGp998F035711;IMAGp971E1 | 230477 | | | | 1,51 | 1,23 | 1,85 | 8,875 | 0,705 | 5,34 |
| 209 | 095 | 0 | | | | | | | | | |
| SEQ ID No: | IMAGp998O184348;IMAGp971P2 | 171210 | ALL1 fused gene from 5q31 | AF5Q31 | 5q31 | 0,947 | 0,795 | 0,97 | 6,575 | 0,585 | 4,925 |
| 210 | 373 | 5 | | | | 5 | | | | | |
| SEQ ID No: | IMAGp998B201153;IMAGp971E1 | 484915 | RNA binding motif protein 5 | RBM5 | 3p21.3 | 1,427 | 1,15 | 1,485 | 4,95 | 0,92 | 4,665 |
| 211 | 741 | | | | | 5 | | | | | |
| SEQ ID No: | IMAGp998P08567;IMAGp971P05 | 260215 | Homo sapiens cDNA FLJ31733 fis, clone | | 12 | 0,942 | 0,955 | 1,2775 | 3,575 | 0,68 | 4,6 |
| 212 | 23 | | NT2RI2006943 | | | 5 | | | | | |
| SEQ ID No: | IMAGp998A191006;IMAGp971B1 | 428442 | tachykinin 3 (neuromedin K, neurokinin | TAC3 | 12q13- | 0,637 | 1,045 | 0,8875 | 7,06 | 0,45 | 4,41 |
| 213 | 539 | | beta) | | q21 | 5 | | | | | |
| SEQ ID No: | IMAGp998B02152;IMAGp971I04 | 31971 | Human mRNA for KIAA0363 gene, partial | KIAA036 | 7p15.1 | 1,512 | 1,515 | 1,78 | 3,735 | 0,32 | 4,3 |
| 214 | 112 | | cds | 3 | | 5 | | | | | |
| SEQ ID No: | IMAGp998M23288;IMAGp971N21 | 52650 | Human erythroblastosis virus oncogene homolog 2 (ets-2) | | | 0,365 | 0,635 | 0,3925 | 3,1 | 0,335 | 4,14 |
| 215 | 118 | | | | | | | | | | |
| SEQ ID No: | IMAGp998I12605;IMAGp971E15 | 274643 | | | | NA | NA | NA | 4,11 | 0,41 | 4,115 |
| 216 | 25 | | | | | | | | | | |
| SEQ ID No: | IMAGp998F015343;IMAGp971E0 | 216345 | Chitinase 1 (chitotriosidase) | CHIT1 | | 0,85 | 1,205 | 0,9575 | 4,56 | 0,675 | 3,98 |
| 217 | 391 | 6 | | | | | | | | | |
| SEQ ID No: | IMAGp998B171824;IMAGp971E1 | 742576 | H.sapiens HE2 mRNA | SPAG11 | 8 | 1,005 | 1,11 | 0,855 | 3,635 | 1,78 | 3,9 |
| 218 | 447 | | | | | | | | | | |
| SEQ ID No: | IMAGp998L24923;IMAGp971O24 | 396839 | | | 13 | 1,1 | 0,845 | 0,7575 | 3,235 | 0,5 | 3,89 |
| 219 | 38 | | | | | | | | | | |
| SEQ ID No: | IMAGp998E135802;IMAGp971F1 | 233854 | chitinase 1 (chitotriosidase) | CHIT1 | | 1,165 | 1,05 | 1,015 | 3,89 | 0,935 | 3,65 |
| 220 | 597 | 8 | | | | | | | | | |
| SEQ ID No: | IMAGp998D171153;IMAGp971F2 | 484960 | hypothetical protein BC007899 | LOC1488 | 1p36.11 | 0,577 | 0,765 | 0,55 | 4,18 | 1,28 | 3,595 |
| 221 | 341 | | | 98 | | 5 | | | | | |
| SEQ ID No: | IMAGp998F17593;IMAGp971B15 | 269968 | Transcribed sequence with strong similarity to protein 1 | | | 1,495 | 1,645 | 4,11 | 5,04 | 0,51 | 3,49 |
| 222 | 24 | | pdb:14GS (H.sapiens) B Chain B, Glutathione S- | | | | | | | | |
| | | | Transferase P1-1 Apo Form 1 | | | | | | | | |
| SEQ ID No: | IMAGp998N11360;IMAGp971O14 | 191050 | | | | 0,89 | 0,95 | 0,9675 | 6,375 | 0,77 | 3,47 |
| 223 | 14 | | | | | | | | | | |
| SEQ ID No: | IMAGp998I152328;IMAGp971I21 | 936278 | Transcribed sequence with moderate similarity to protein | | | 0,525 | 0,76 | 0,7275 | 3,06 | 0,37 | 3,425 |
| 224 | 56 | | pir:S52491 (H.sapiens) S52491 polyadenylate binding protein II | | | | | | | | |
| | | | - human | | | | | | | | |
| SEQ ID No: | IMAGp998I112449;IMAGp971G0 | 982738 | | | | 1,74 | 1,31 | 0,855 | 5,835 | 0,97 | 3,3 |
| 225 | 5108 | | | | | | | | | | |
| SEQ ID No: | IMAGp998A17282;IMAGp971B18 | 50008 | Calcium channel, voltage-dependent, L | CACNA1B 9 | | 1,167 | 1,35 | 1,155 | 3,475 | 0,6 | 3,285 |
| 226 | 117 | | type, alpha 1B subunit | | | 5 | | | | | |
| SEQ ID No: | IMAGp998J014173;IMAGp971J04 | 164476 | Golgi autoantigen, golgin subfamily a, | GOLGA6 | | 3,282 | 1,34 | 0,91 | 12,645 | 1,23 | 3,26 |
| 227 | 69 | 8 | member 6 | | | 5 | | | | | |
| SEQ ID No: | IMAGp998O235762;IMAGp971O2 | 232343 | | | | 0,875 | 1,355 | 1,1025 | 3,445 | 0,465 | 3,24 |
| 228 | 2130 | 8 | | | | | | | | | |
| SEQ ID No: | IMAGp998C01743;IMAGp971D01 | 327480 | Collagen, type XXIII, alpha 1 | COL23A1 5 | | 1,195 | 1,345 | 1,61 | 3,25 | 0,495 | 3,05 |
| 229 | 32 | | | | | | | | | | |
| SEQ ID No: | IMAGp998D175081;IMAGp971F1 | 206281 | hypothetical protein LOC200213 | LOC200213 | | 1,412 | 1,425 | 1,7625 | 3,165 | 0,48 | 3,025 |
| 230 | 286 | 6 | | | | 5 | | | | | |
| SEQ ID No: | IMAGp998B2474;IMAGp971D231 | 23877 | ATPase, aminophospholipid transporter | ATP8A1 4 | | 2,292 | 1,815 | 1,565 | 3,275 | 0,58 | 3,015 |
| 231 | 10 | | (APLT), Class I, type 8A, member 1 | | | 5 | | | | | |
| SEQ ID No: | IMAGp998I094494;IMAGp971I09 | 183752 | Homo sapiens transcribed sequence with moderate similarity to | | | 0,6 | 0,89 | 0,7725 | 3,37 | 0,28 | 3,005 |
| 232 | 78 | 0 | protein pir:S52491 (H.sapiens) S52491 polyadenylate binding | | | | | | | | |
| | | | protein II - human | | | | | | | | |
| SEQ ID No: | IMAGp998B08733;IMAGp971A03 | 323623 | DKFZP564O1863 protein | DKFZP56 | 12p12.3 | 1,155 | 1,335 | 0,95 | 5,315 | 1,005 | 2,985 |
| 233 | 33 | | | 401863 | | | | | | | |
| SEQ ID No: | IMAGp998I11564;IMAGp971K13 | 258898 | Homo sapiens secreted frizzled-related | SFRP2 | | 1,917 | 1,6 | 1,4625 | 5,425 0,595 | | 2,86 |
| 234 | 23 | | protein 2 (SFRP2), mRNA | | | 5 | | | | | |
| SEQ ID No: | IMAGp998K211090;IMAGp971I2 | 460940 | | | | 0,865 | 1,26 | 1,445 | 5,265 | 0,545 | 2,86 |
| 235 | 040 | | | | | | | | | | |
| SEQ ID No: | IMAGp998A181964;IMAGp971E0 | 796313 | | | 1,41 | 1,25 | 3,07 | 4,805 | 1,27 | 2,85 | |
| 236 | 253 | | | | | | | | | | |
| SEQ ID No: | IMAGp998H04617;IMAGp971G03 | 279219 | chromosome 20 open reading frame 58 | C20orf58 20 | 1,202 | 1,075 | 1,0825 | 3,935 | 0,495 | 2,845 | |
| 237 | 26 | | | | 5 | | | | | | |
| SEQ ID No: | IMAGp998I12670;IMAGp971L04 | 299603 | | | 1,35 | 2,66 | 1,9625 | 4,195 | 1,03 | 2,81 | |
| 238 | 30 | | | | | | | | | | |
| SEQ ID No: | IMAGp998D145711;IMAGp971D2 | 230473 | | | 1,045 | 0,615 | 1,2575 | 4,38 | 1,46 | 2,78 | |
| 239 | 194 | 3 | | | | | | | | | |
| SEQ ID No: | IMAGp998L06178;IMAGp971H02 | 38015 | Slit and trk like gene 2 | SLITRK2 | 1,055 | 1,42 | 1,2175 | 4,23 | 0,615 | 2,78 | |
| 240 | 115 | | | | | | | | | | |
| SEQ ID No: | IMAGp998I135799;IMAGp971C2 | 233749 | Hypothetical gene supported by AK126852 (LOC400516), mRNA | | 1,905 | 0,87 | 0,76 | 3,9 | 0,995 | 2,645 | |
| 241 | 497 | 2 | | | | | | | | | |
| SEQ ID No: | IMAGp998D154762;IMAGp971D1 | 194031 | disco-interacting protein 2 (Drosophila) | DIP2 21q22.3 | 1,092 | 1,245 | 1,325 | 3,34 | 0,65 | 2,52 | |
| 242 | 183 | 8 | homolog | | 5 | | | | | | |
| SEQ ID No: | IMAGp998P20366;IMAGp971O19 | 193411 | Human mRNA for alphal-acid glycoprotein | ORM1,OR 9q31-q32 | 0,865 | 0,81 | 1 | 4,54 | 0,675 | 2,435 | |
| 243 | 15 | | (orosomucoid) | M2 | | | | | | | |
| SEQ ID No: | IMAGp998C034512;IMAGp971C0 | 184428 | transglutaminase 3 (E polypeptide, | TGM3 | 0,69 | 1,465 | 0,54 | 3,72 | 0,705 | 2,415 | |
| 244 | 279 | 2 | protein-glutamine-gamma- | | | | | | | | |
| | | | glutamyltransferase) | | | | | | | | |
| SEQ ID No: | IMAGp998G01115;IMAGp971E01 | 122136 | | 2 | 0,762 | 1,05 | 1,045 | 4,26 | 0,51 | 2,315 | |
| 245 | 103 | | | | 5 | | | | | | |
| SEQ ID No: | IMAGp998B22787;IMAGp971A22 | 344373 | | 9 | 0,527 | 0,99 | 2,345 | 5,42 | 0,505 | 2,305 | |
| 246 | 34 | | | | 5 | | | | | | |
| SEQ ID No: | IMAGp998J134174;IMAGp971J16 | 164516 | hypothetical protein LOC340094 | | 1,265 | 0,94 | 0,7825 | 4,99 | 0,93 | 2,26 | |
| 247 | 69 | 4 | | | | | | | | | |
| SEQ ID No: | IMAGp998D04101;IMAGp971A03 | 116691 | | | 1,05 | 1,035 | 1,165 | 6,91 | 0,79 | 2,23 | |
| 248 | 4 | | | | | | | | | | |
| SEQ ID No: | IMAGp998D04101;IMAGp971A03 | 116691 | | | 1,05 | 1,035 | 1,165 | 6,91 | 0,79 | 2,23 | |
| 249 | 4 | | | | | | | | | | |
| SEQ ID No: | IMAGp998O0470;IMAGp971O031 | 22040 | Human type IV collagenase mRNA, | MMP9 | 20q11.2- | 1,082 | 1,05 | 0,9175 | 6,705 | 2,12 | 2,095 |
| 250 | 10 | | complete cds | | q13.1 | 5 | | | | | |
| SEQ ID No: | IMAGp998A125385;IMAGp971D1 | 217947 | phospholipase A2, group IID | PLA2G2D | 1p36.12 | 1,355 | 1,02 | 0,8925 | 4,54 | 1,095 | 2,05 |
| 251 | 4124 | 5 | | | | | | | | | |
| SEQ ID No: | IMAGp998D194160;IMAGp971F1 | 163965 | | | | 1,485 | 1,225 | 1,395 | 3,85 | 0,62 | 2,03 |
| 252 | 767 | 0 | | | | | | | | | |
| SEQ ID No: | IMAGp998L132010;IMAGp971K1 | 814236 | | | | 0,635 | 1,06 | 0,685 | 4,8 | 0,735 | 2,025 |
| 253 | 355 | | | | | | | | | | |
| SEQ ID No: | IMAGp998G081965;IMAGp971G1 | 796831 | Human metalloendopeptidase homolog | PHEX | Xp22.2- | 1,417 | 1,31 | 1,135 | 3,235 | 0,74 | 2 |
| 254 | 553 | | (PEX) mRNA, complete sequence | | p22.1 | 5 | | | | | |
| SEQ ID No: | IMAGp998B072455;IMAGp971B0 | 984870 | Similar to ankylosis, progressive homolog | | | 1,347 | 1,025 | 0,99 | 85,23 | 1,39 | 1,12 |
| 255 | 956 | | | | | 5 | | | | | |

### Table I

This lists features descriptions of all the sequences. In most instances the RZPD (German Resource Center for Genome Research) and IMAGE clone numbers are reported. In addition to that, also the best applicable feature description and gene name as well as chromosomal localization is reported. In the last 6 columns results from the microarray study are reported. 50%, 75%, and 100% CIS reflects analysis on samples with CIS in 50%, 75%, and 100% of the tubules in a given sample. Seminoma reflects a sample with classical seminoma and EC from a sample with embryonal carcinoma. Seminoma/EC reflects an individual experiment with seminoma vs. EC.

### Table II.

Summary and evaluation of the results of the AP-2γ staining of semen samples from the various patient categories. The staining was classified using an arbitrary score 0-5, after a systematical and independent evaluation by two investigators (CHH and ERM), who had no prior knowledge of the diagnosis. The group with testicular neoplasms included patients with the following tumours: 5 seminomas, 4 non-seminomas, 1 mixed GCT and 2 unilateral CIS, which had not proceeded to an invasive tumour, and the subfertile patient diagnosed with CIS in this study. The group of patients with other diseases comprised: 1 benign testis tumour (Leydig adenoma), 1 malignant lymphoma, 2 Hodgkin's disease, 1 glioblastoma, 1 Wegeners granulomatosis, 1 Crohn's disease, 1 bone tumour, 1 torsio testis, 1 pituitary tumour, 1 Klinefelter, 1 XYY male. The healthy controls were recruited among apparently healthy young men attending our department for other projects and 59 subfertile men, of whom 16 had normospermia and 43 had oligospermia. Statistical analysis of data was performed using the Confidence Interval Analysis programme, version 2.0, developed by D.G. Altman, D. Machin, T.N. Bryant and M.J. Gardner.

**Table III**

| **Studied groups** | **N** | **Mean age** | **AP-2**γ**-positive** |
|---|---|---|---|
| | | (years) | N (%, 95% CI) |
| Testicular tumour or CIS before operation / irradiation | 13^{#} | 33 | 6^{#} (46.2%, Cl 23-71%) |
| Testicular cancer after operation, no contralateral CIS | 7 | 32 | 0 (0%, CI 0-35%) |
| Other diseases | 12 | 29 | 0 (0%, CI 0-24%) |
| Healthy controls | 14 | 32 | 0 (0%, CI 0-22%) |
| Subfertile patients with normospermia | 16 | 32 | 0 (0%, CI 0-19%) |
| Subfertile patients with oligospermia | 43^{#} | 34 | 1^{#} (2.3%, CI 0.4-12%) |
| ^{#} Including the patient diagnosed with CIS in the course of the study. | | | |

Summary of the immunohistochemical staining for AP-2γ. Abbreviations: N=number; wg=weeks of gestation; AIS=androgen insensitivity syndrome; AMH=anti-Müllerian hormone; TDS=testicular dysgenesis syndrome; TGCT=germ cell tumor; CIS=carcinoma *in situ.* Staining intensity: +++: strong staining, ++: moderate staining, +: weak staining, +/-: very weak staining, -: no positive cells detected.

| **Age** | ***N*** | **Histology / Diagnosis** | **AP-2**γ (staining intensity and approximate % of positive cells in a section) | **Remarks** |
|---|---|---|---|---|
| **Normal testes:** | | | | |
| 10-22 wg | 11 | Normal fetal testes | - to ++ (75-100%) | 3 samples negative |
| 23-26 wg | 4 | Normal fetal testes | +/- to + (5-50%) | |
| 27-42 wg | 2 | Normal fetal testes | +/- (<10%) | |
| 0-2½ months | 2 | Normal infantile testes | - to + (25%) | |
| 3-4 months | 5 | Normal infantile testes | +/- (single cells or <10%) | |
| ½-2½ years | 3 | Normal infantile testes | - | |
| 3-11 years | 7 | Normal pre-pubertal testes | - | |
| 13-16 years | 1 | Normal testes with complete spermatogenesis | - | |
| **Normal ovaries:** | | | | |
| 9 wg | 1 | Normal fetal ovary | + (single cells) | Oogonia+ |
| 12-16 wg | 5 | Normal fetal ovaries | +/-to+ + | Oogonia+, oocytes- |
| 17-26 wg | 7 | Normal fetal ovaries | +/- to ++ | Oogonia+, oocytes- |
| 27-41 wg | 9 | Normal fetal ovaries | + to ++ | Oogonia+, oocytes- |
| Adult | 1 | Normal adult ovary | - | |
| **Dysgenetic and intersex gonads:** | | | | |
| 15 wg | 1 | Fetal testis (mosaic isochromosomeY) | - | AMH+, Oct4- |
| 20 wg | 2 | Fetal testes (AIS, 46,XY females) | + (10%) | |
| 4 months | 1 | Infantile testis (AIS, 46,XY females) | + (5%) | |
| 14 months | 1 | Ovotestis (46 XX hermaphrodite) | +/- | Gonocytes positive, oocytes negative |
| 9 months - 6 | 3 | Pre-pubertal testes (AIS, 46 XY females) | + to +++ (2-10%) | Gonocytes with CIS characteristics, |
| years | | | | PLAP+ or -. |
| 2-17 | 6 | Pre-pubertal testes (AIS, 46 XY females) | - | |
| 2½ years | 1 | Infantile testis with decreased number of germ | - | |
| | | cell (idiopathic genital ambiguity) | | |
| 9½ years | 1 | Pre-pubertal testis (Prader-Willy's syndrome) | - | |
| 15 years | 1 | Leydig-cell hyperplasia (adrenogenital | - | |
| | | syndrome) | | |
| 15 years | 1 | 46,XY female, 17βHD mutation | - | |
| 18 years | 1 | Dysgenetic testis with CIS and gonadoblastoma | ++ (90-100%) | |
| | | (45,X/46XY male) | | |
| 25 years | 1 | Klinefelter, bilateral testicular cryptorchidism | - | |
| 20-39 years | 9 | Adult testes with TDS (undifferentiated tubules, | - | |
| | | microliths) but without CIS or TGCT | | |
| 30-48 years | 3 | Adult testes with TDS (undifferentiated tubules, | - | |
| | | microliths) and contralateral TGCT | | |
| **Testicular carcinoma *in situ* and tumour samples:** | | | | |
| Adult | 5 | CIS adjacent to seminomas | + to +++ (90-100%) | |
| Adult | 5 | CIS adjacent to non-seminomas | + to +++ (90-100%) | |
| Adult | 2 | CIS adjacent to mixed TGCT | ++ (90-100%) | |
| Adult | 2 | CIS without overt TGCT | +++ (90-100%) | |
| Adult | 12 | Classical, homogenous seminomas | + to +++ (90-100%) | |
| Adult | 12 | Non-seminomas | +/- to ++ | Various epithelial and stromal |
| | | | | elements positive |
| Adult | 2 | Spermatocytic seminomas | - | |
| Adult / 6 years | 3 | Leydig cell tumors | - to + | Cytoplasmic, background? |
| 14 years | 1 | Gonadoblastoma | +++ | |
| Adult | 1 | Testicular B-cell lymphoma | - | |
| **Other tissue samples:** | | | | |
| Fetal, | 1 of | Liver, heart, thymus, thyroid gland, kidney and | - | |
| 9-20 wg | each | adrenal gland | | |
| Adult | 11 | Normal breast tissues | + to ++ (90-100%) | Subpopulation of basally situated duct |
| | | | | cells |
| Adult | 47 | Infiltrating ductal breast adenocarcinomas | 7 samples +/- (<10%) | |
| | | | 7 samples + (50%) | |
| | | | 33 samples - | |
| Adult | 1 | Breast mucinous carcinoma | - | |
| Adult | 4 | Breast medullary carcinomas | 2 samples +/- (90-100%) | |
| Adult | 3 | Ductal breast carcinoma in situ | +/- | Single basal cells |
| Adult | 2 | Tubular breast cancers | - | |
| Adult | 1 | Infiltrating nodular breast adenocarcinoma | - | |
| Adult | 4 | Lung | + | Epithelial pneumocytes positive |
| Adult | 7 | Skin | +/- (25%) | Basal cells |
| Adult | 1 | Kidney | +/- (50%) | Single cells in canaliculi, glomeruli- |
| Adult | 1-3 | Skeletal muscle, heart muscle, stomach, | - | |
| | of | esophagus, small intestine, colon, liver, spleen, | | |
| | each | pancreas, salivary gland, pituitary gland, adrenal | | |
| | | gland, thyroid gland, parathyroid gland, thymus | | |
| | | gland, tonsil, epididymis, uterus, cervix, ovarian | | |
| | | serous adenocarcinoma, prostate gland, | | |
| | | prostatic cancer, omentum, peripheral nerve, | | |
| | | cerebral cortex, cerebellum | | |
| **Cell lines** | | | | |
| NT2 cells | Day 0,3,15 of retinoic acid treatment | | - to + | In all samples a subpopulation of cells |
| | | | | were stained. |
| 2102Ep | Day 0,1,3,7,10 of retinoic acid treatment | | ++ | In all samples most cells were stained. |
| MCF7 | Exposed to ICI-182,780, ethanol or 1nM | | - to + | In all samples a subpopulation of cells |
| | estradiol for 24 or 48 h. | | | were stained. |

### Examples

### Materials and methods

### Tissue samples

The testicular tissue samples were obtained directly after orchidectomy and macroscopic pathological evaluation. The use of the orchidectomy samples was approved by the Regional Committee for Medical Research Ethics in Denmark. Samples of homogeneous overt tumors (seminoma or EC) and of testicular parenchyma preserved in the vicinity of a tumor were excised. Each testicular sample was divided into several tissue fragments: 2 - 3 fragments were snap-frozen at -80° C for nucleic acid extraction, several adjacent fragments were fixed overnight at 4° C in Stieve's fluid or paraformaldehyde (PFA), and embedded in paraffin. Fixed sections were subsequently stained with haematoxylin and eosin (HE) or with an antibody against PLAP for histological evaluation. Most samples of testicular parenchyma contained variable numbers of tubules with CIS cells. Samples where no tubules with CIS were found and complete spermatogenesis was present, were used as a normal reference. In an attempt to limit the uncertainty about cellular differences between fragments from the same patient, the present inventors also made a direct print of the frozen fragment that specifically was used for RNA isolation. After staining with a PLAP antibody, the present inventors could roughly confirm the approximate proportion of tubules with CIS as established by histology in adjacent tissue fragments. Samples of tissue containing approximately 50%, 75%, and 100% tubules with CIS cells were chosen for microarray analysis along with the above-mentioned samples of overt tumours and the normal testicular tissue (serving as the common reference). Additional samples of teratomas and embryonal carcinomas were also used in the differential display screening and in the RT-PCR.

### Labeling of testicular RNA and hybridization to microarrays

Total RNA was purified using the Macherey & Nagel kit, DNase digested, and the RNA quality evaluated using the Agilent Bioanalyzer 2100 (Agilent Technologies Inc., CA, USA), 5 µg of total RNA was linearly amplified using the RiboAmp RNA amplification kit (Arcturus GmbH, Germany) yielding a 400-700 fold amplification of the mRNA. The amplified mRNA was reevaluated on the Bioanalyzer 2100 and 5 µg was then subjected to in-direct labeling with Cy3 and Cy5 (Amersham Biosciences) using the Atlas Glass Fluorescent labeling kit (BD Biosciences Clonetech, CA, USA).

The labeled RNA probes were combined and hybridized at 42° C over night to a 52.000 element cDNA microarray representing the complete Unigene database. The microarray consists of two slides, which are hybridized on top of each other with the labeled probe in-between. Production and handling of the microarray is described elsewhere (http://embl-h3r.embl.de/). Hybridized slides were washed at 42° C for 5 min in 2XSSC, 0.1 % SDS and 10 min in 0.2XSSC, 0.1 % SDS, rinsed, dipped in isopropanol, and dried.

Slides were scanned on a GenePix 4000B scanner (Axon Instruments Inc., Union City, CA, USA) using settings where overall intensity of each channel was equal. The generated images were then analyzed in ChipSkipper (http://chipskipper.embl.de/) and quantified using a histogram segmentation. Flagged spots and controls were taken out and quantified spots were normalized using a framed median ratio centering (frame = 200 genes). The average of dye-swaps was used and spots that did not dye-swap or showed a big standard deviation were sorted out. Data was then imported into software Genesis where hierarchical clustering was done using average linkage and Euclidian distances.

### Differential display competitive PCR screening

Total RNA was prepared using the RNeasy kit from Qiagen as described by the manufacturer (Qiagen, Hilden, Germany). RNA samples were double DNAse digested, first "on-column" and subsequently after the RNA had been purified. cDNA was prepared using one-base-anchored AAGCT11V (V= A, C or G) downstream primers. DDRT-PCR (Liang and Pardee, 1992) was subsequently performed using either one- or two-base-anchored (AAGCT11VN; N = A, C, G or T) primers in combination with different of upstream primers. Bands of interest were cut from the DDRT-PCR gels and reamplified using the up- and downstream primers that displayed the bands, but the downstream primer was extended at the 5'-end with a T7-promotor sequence (taatacgactcactatagggAAGCT11V; T7 promotor sequence in small letters), allowing direct sequencing on ALFexpress sequencers (Amersham-Pharmacia Biotech, Uppsala, Sweden), using a Cy5-labelled T7-promotor complementary primer. The DDRT-PCR method has been described in detail previously (Jorgensen, M, et al. Electrophoresis 1999, 20:230-40).

### Verification by RT-PCR

The expression of a selected set of the regulated genes identified in the microarray analysis was verified with RT-PCR as described previously. In brief, cDNA was synthesized using a dT24 primer. Specific primers targeting each mRNA were designed spanning intron-exon areas to avoid amplification of genomic DNA. PCR was performed in 30 µl of (final concentrations): 12 mM Tris-HCl, pH 8.3; 50 mM KCl; 1.9 mM MgCl2; 0.1 % Triton X-100; 0.005 % Gelatine; 250 µM dNTP; and 30 pmol of each primer. H2O was used as a negative control and β-actin was used as an internal control in all PCR reactions, resulting in an approximately 800 base pair actin fragment. Cycle conditions: 1 cycle of 2 min. at 95° C; 30 - 40 cycles (depending on the intensity of bands) of: 30 sec. at 95° C, 1 min. at 62° C, 1 min. at 72° C and finally 1 cycle of 5 min. at 72° C. PCR products were resolved on 1.5 % agarose gels and visualized by ethidium bromide staining. Digital images of the agarose gels were quantified by the STORM phosphor imager software (ImageQuant, Amersham Biosciences). In a few of the RT-PCR analyses of less abundant transcripts, no bands were detectable after PCR and nested primers were designed. One µl from the first PCR reaction was transferred to a new reaction containing the nested primers and analyzed as above.

### Preparation of biotin-labeled probes and in situ hybridization

In situ hybridization was performed to confirm that the identified transcripts were indeed expressed in CIS cells. Probes for in situ hybridization were prepared by re-amplification of the PCR products using nested primers specific to the individual products and with an added T7- or T3-promotor sequence. PCR conditions were: 5 min. 95° C; 5 cycles of: 30 sec. 95° C, 1 min. 45° C, 1 min. 72° C; 20 cycles of 30 sec. 95° C, 1 min. 65° C, 1 min. 72° C and finally 5 min. 72° C. The resulting PCR product was purified on a 2 % low melting point agarose gel and sequenced from both ends, using Cy5-labelled primers complementary to the added T3 and T7 tags. Aliquots of about 200 ng were used for in vitro transcription labeling, using the MEGAscript-T3 (sense) or MEGAscript-T7 (anti-sense) kits, as described by the manufacturer (Ambion, Houston, TX, USA). The composition of the 10X-nucleotide mix was: 7.5 mM ATP, GTP and CTP, 3.75 mM UTP, 1.5 mM biotin-labeled UTP. To estimate quantity and labeling efficiencies, aliquots of the labeled RNA product were analyzed by agarose gel electrophoresis, and dotted onto nitrocellulose filters and developed as described below.
In situ hybridization was performed essentially as described previously (Nielsen, JE, et al. Eur J Histochem 2003, 47:215-22), except for one additional step needed to remove mercury from sections fixed in Stieve fixative. After deparaffination, mercury was removed by 15 min. treatment with potassium iodide (KI/I ratio 2/1), followed by three washes in DEPC water. The iodine was subsequently removed by incubation in sodium thiosulfate pentahydrate (5 % w/v, 10 min.) followed by washing (4X DEPC water). Subsequently, the procedure was, in brief, as follows: sections were re-fixed in 4 % PFA, treated with proteinase K (Sigma, St. Louis MO USA) (1.0 - 5.0 µg/ml), post-fixed in PFA, pre-hybridized 1 h at 50° C, and hybridized over night at 50° C with biotinylated antisense and - sense control probes. Excess probe was removed with 0,1X SSC (60° C) 3X33 min.

Visualization was performed with streptavidin conjugated with alkaline phosphatase (1:1000) (Roche Diagnostics GmbH, Mannheim, Germany) followed by a development with BCIP / NBT (Nielsen, JE, et al. Eur J Histochem 2003, 47:215-22).

### Example 1

Genes identified by microarray

By analyzing testicular samples containing increasing amounts of tubules with CIS the present inventors identified differentially expressed genes as compared to the normal reference with complete spermatogenesis and no CIS cells present.

From normalized scatter plots of gene expression in CIS-containing-tissue versus normal testis tissue, the present inventors observed that approximately 200-300 genes were significantly up-regulated in CIS-containing tissues. Data was filtered so that only genes that were up-regulated in the CIS samples and had low standard deviation were considered for further analysis. The up-regulated genes were a heterogeneous group including transcription factors, such as TFAP2C (ERF-1), LBP-9, TEAD4 (RTEF-1), NFE2L3 (NRF3) and POU5F1, solute carriers e.g. SLC25A16, SLC7A3, oncogenes e.g. RAB-15, TCL1B (TML1), MYBL2 (B-MYB), PIM2 and many more genes, which are listed in Table X. Some of these genes were previously described as CIS markers, e.g. KIT (c-KIT), POV1 (PB39), MYCN (N-MYC) and POU5F1 (OCT-3/4), but the majority were novel, not previously characterized genes.

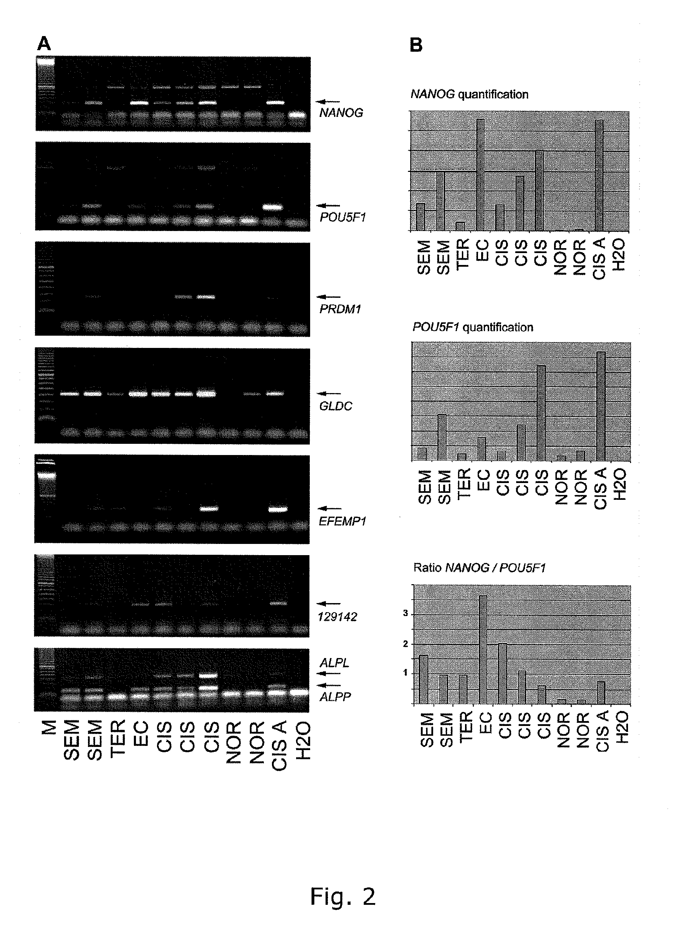

A hierarchical cluster analysis with average linkage of the top most up-regulated genes (minimum 4 fold increase in the sample with CIS in 100 % of the tubules and more than 2 fold in the other samples) is shown in figure 1. For most of the genes, a gradual increase in expression was observed with increase in the percent of tubules with CIS. This strongly indicates that expression of these genes is linked to the CIS phenotype and indeed to the actual amount of tubules with CIS in the testis. To verify that, the present inventors re-analyzed the expression of selected genes by RT-PCR, in a panel of samples covering a broad spectrum of different testicular neoplasms and normal testicular tissue (fig. 2A). We also investigated the expression of the non-tissue specific alkaline phosphatase (ALPL) simultaneously with the previously known CIS marker PLAP and found that they were expressed in a similar manner (fig. 2A).

Both the microarray and the RT-PCR analysis indicated that the observed up-regulation of genes in the samples with CIS were linked to the CIS phenotype and thus to CIS cells. However, it cannot be excluded that the high expression of some genes (e.g. SIAT1, TCL1A, TCL1B), may be due to the presence of infiltrating lymphocytes, which are commonly seen in patients with CIS, but not present in the normal testis (Jahnukainen, K, et al. Int J Androl 1995, 18:313-20). In addition, some transcripts up-regulated in the tissues with CIS may originate from immature Sertoli cells, which are sometimes present in testes with CIS, but such dysgenetic changes are relatively rare, so this possibility is less likely.

To confirm the cellular localization, the present inventors performed in situ hybridization for some of the identified genes on tissue sections with CIS. All transcripts tested (NANOG, ALPL, TFAP2C, and SLC7A3) were localized to CIS cells. The present inventors also observed in some cases variable staining of Sertoli cell nuclei, which was most probably unspecific, since similar, but lighter staining was observed for the control sense probe. However, in all cases, the strongest signal was from CIS cells.

### Example 2

Genes identified by Differential Display
To obtain knowledge on gene expression changes between the different stages in the development from non-malignant testicular tissue through CIS to the malignant stage of a germ cell cancer, the present inventors performed a systematic comparison by the DDRT-PCR method (Jorgensen, M, et al. Electrophoresis 1999, 20:230-40). For the initial screening the present inventors analysed six testicular biopsy samples: two with homogenous classical seminoma, two containing almost exclusively CIS tubules, of which one was from an intraabdominal CIS and two with normal spermatogenesis. The undescended testis with CIS was of our interest, because in the hypothesis of Testicular Dysgenesis Syndrome, both testicular cancer and undescended testes are symptoms of maldeveloped genitalia.

We performed 402 primer combinations, and assuming that each competitive PCR reaction gives rise to approximately 100 bands on a DDRT gel and that each band corresponds to one mRNA, this investigation allowed comparison of about 40.000 mRNAs. We excised and sequenced 56 bands, which showed overexpression (ranging from 1-10 fold) in at least one of the CIS samples. Several mRNAs were detected with more than one primer combination; for instance, overexpression of CCND2 was detected with three different primer combinations. The RT-PCR analysis verified overexpression of 24 known genes, 4 transcripts corresponding to ESTs and 1 sequence not annotated as a gene.

### Example 3

Antibodies against the genes
Several vendors of commercial antibodies were searched for antibodies to the proteins encoded by the identified genes highly expressed in testicular CIS. In addition, new antibodies against protein product of the newly identified CIS markers genes are going to be generated.

### Example 4

Identification of markers for CIS progression into seminoma or non-seminoma

Besides identification of highly specific CIS markers the present inventors also identified markers for the progression of CIS either into seminoma or the non-seminoma. Currently it is not known why some CIS cells give rise to a seminoma and others non-seminomas. It is however speculated that CIS may be predisposed to the one or the other. By identification of the markers for CIS progression together with the CIS markers, the present inventors will be able to: 1) identify CIS cells in a subject 2) predict whether the subject is predisposed to either a seminoma or a non-seminoma, or may develop a combined tumour 3) evaluate to what degree the CIS in the subject has evolved into a seminoma or a non-seminoma 4) monitor during a possible subsequent treatment whether or not all CIS cells or derived tumours have been successfully eradicated.

### Example 5

Kit development
A diagnostic kit for screening for a panel of the marker genes can be provided. Such a kit may be in different forms, depending whether the gene expression in a sample will be screened at the RNA or at the protein level. The most obvious form would be a kit based on reverse transcriptase polymerase chain reaction (RT-PCR). By picking out a battery of the most promising CIS marker genes, RNA purified from a human sample can be tested for the presence of CIS marker genes at the transcript level. The present inventors can also take advantage of the high throughput microarray technique for assaying the expression of hundreds of genes simultaneously. This could be in the format of a microarray focused on genes identified here as CIS markers. Additional microarrays e.g. containing markers for the progression into seminoma or nonseminoma can also be developed.

At the protein level, the present inventors can also develop two approaches: either select a limited number of best markers and make an ELISA-based assay using robust and well validated specific antibodies. The present inventors can also develop a protein-microarray using a large number of antibodies against a broader panel of markers, including those previously known, spotted on a glass slide.

### Example 6

New nuclear markers
One very important finding from the study of CIS markers at the protein level was to find proteins localized to the nucleus of the CIS cells. This is important because CIS cells shed to seminal fluid are exposed to different pH and high concentrations of proteases that may damage the cell membrane (and the proteins located within, e.g. receptors) before the ejaculation takes place. A nuclear marker will be better retained than a cell surface marker and thus will increase the possibility for detection in semen samples.

### Example 7

Human samples for detection of CIS
The most convenient and least invasive biological sample to detect CIS cells will be semen (ejaculate). In our earlier studies the present inventors established that some CIS cells are present in semen samples in patients with CIS or overt germ cell tumours in the testes. However, in some cases of early invasion/metastasis, it may also be possible to detect CIS/early tumour cells in a blood sample. Testicular tissue in the form of i.e. biopsies, is nowadays used to detect CIS, and the novel markers can be used to confirm the results of e.g. a CIS array screen, before the final treatment (e.g. orchidectomy) takes place. Yet, other human samples e.g. urine may perhaps be used in the future, e.g. in very young boys, but as only single cells are expected to be detected in urine, careful testing and validation has to be performed.

### Example 8

CIS cells are embryonic stem cell-like
Among the genes highly expressed in CIS, the present inventors noticed a range of genes known to be expressed primarily in embryonic stem cells (ESC) and/or primordial germ cells (PGC), such as POU5F1 DPPA4, DPPA5, and NANOG. The present inventors therefore performed a systematic comparison of genes highly expressed in CIS with genes recently reported in the literature to be highly expressed in both human and mouse ESC (Ramalho-Santos, M, et al. Science 2002, 298:597-600;Sato, N, et al. Dev Biol 2003, 260:404-13;Sperger, JM, et al. Proc Natl Acad Sci U S A 2003, 100:13350-5). As shown in figure 4, many of the genes found in ESC were also highly expressed in our CIS samples. Among the 100 highest expressed CIS marker genes (listed in figure 1), 34 genes were reported in ESC. The overlap might even be greater, as many of the genes included on the array are yet uncharacterized and thus may be differently annotated on the microarrays used to expression profile ESC. Excluding genes with no functional description (gene name) leads to an approximately 47% overlap. Furthermore, studies where a relative level of expression in ESC cell compared to a reference was reported (Ramalho-Santos, M, et al. Science 2002, 298:597-600;Sperger, JM, et al. Proc Natl Acad Sci U S A 2003, 100:13350-5), the ranking of the expression level seemed to correlate between CIS and ESC, though with some exceptions. The studies the present inventors compared with, in addition all have used different reference samples to explore ESC specific genes and therefore not always agree on the expression in ESC (fig. 4).

Furthermore, the chromosomal distribution of the over-expressed genes in CIS may be associated with a non-random genomic amplification in certain "hot-spot" areas. The present inventors therefore created a map of the chromosomal distribution of the genes highly expressed in CIS. Searching for the chromosomal localization of these genes and normalizing with the length of each chromosome revealed that there was an over-representation of up-regulated genes on chromosomes 7, 12, 14, 15, and especially chromosome 17 (fig. 5) Plotting the density of up-regulated genes in each chromosomal band allowed us to narrow down hot-spots to smaller regions, with 17q as the most pronounced region. Recurrent genomic gain of chromosomes 17q and 12 was recently discovered in cultured human ESCs (Draper, JS, et al. Nat Biotechnol 2004, 22:53-4;Pera, MF Nat Biotechnol 2004, 22:42-3). It was postulated that cultured ESC might gain extra chromosome material in these areas after freezing and thawing, which could inhibit apoptosis and increase their self-renewal in culture etc. (Draper, JS, et al. Nat Biotechnol 2004, 22:53-4). This again indicates that CIS cells may have strong similarities to embryonic stem cells and the identified CIS markers may thus be used to identify transplanted embryonic stem cells how have evolved into CIS cells leading to uncontrolled growth of the embryonic stem cells.

### Example 9

Transcription factor AP-2γ is a developmentally regulated marker of testicular carcinoma *in situ* and germ cell tumors

### Purpose

Transcription Factor Activator Protein-2γ (*TFAP2C,* AP-2γ) was previously reported in extraembryonic ectoderm and breast carcinomas, but not in the testis. the above gene expression study we detected AP-2γ in carcinoma *in situ* testis (CIS, or intratubular germ cell neoplasia, IGCN), precursor of testicular germ cell tumors (TGCT). In this example we aimed to investigate the expression pattern of AP-2γ and to shed light on this factor in germ cell differentiation and the pathogenesis of germ cell neoplasia.

### Experimental Design

We analyzed expression pattern of AP-2γ at the RNA and protein level in normal human tissues and a panel of tumors and tumor-derived cell lines. In the gonads, we established the ontogeny of expression of AP-2γ in normal and dysgenetic samples. We also investigated the regulation of AP-2γ by steroids and retinoic acid.

### Results

We detected abundant AP-2γ in testicular CIS and in TGCTs of young adults, and confirmed differential expression of AP-2γ in somatic tumors. We found that AP-2γ expression was regulated by retinoic acid in an embryonal carcinoma cell-line (NT2). The investigation of ontogeny of AP-2γ protein expression in fetal gonads revealed that it was confined to oogonia/gonocytes and was down-regulated with germ cell differentiation. In some pre-pubertal intersex cases, AP-2γ was detected outside of the normal window of expression, probably marking neoplastic transformation of germ cells.

### Conclusions

AP-2γ is developmentally regulated and associated with the undifferentiated phenotype in germ cells. This transcription factor may be involved in self-renewal and survival of immature germ cells and tissue-specific stem cells. AP-2γ is a novel marker of testicular CIS and CIS-derived tumors.

The aim of this study was to explore the possible role of AP-2γ in the pathogenesis of reproductive tumors, especially in the testis, and to further investigate the timing of neoplastic transformation of germ cells. To that end, we determined a comprehensive expression profile of AP-2γ in a large panel of normal tissues, tumors and tumor-derived cell lines, established the ontogeny of AP-2γ in normal and dysgenetic human gonads and studied regulation of the AP-2γ expression in selected cell lines.

### MATERIALS AND METHODS

Tissue samples

The Regional Committee for Medical Research Ethics in Denmark approved the use of human tissue samples for the studies of novel genes expressed in germ cell cancer.

The tissue samples from adults with testicular neoplasms were obtained directly after orchidectomy and macroscopic pathological evaluation. Each testicular sample was divided into several tissue fragments, which were either snap-frozen at -80°C for nucleic acid extraction, or fixed overnight at 4°C in Stieve's fluid, buffered formalin or paraformaldehyde (PFA), and subsequently embedded in paraffin.

Tissue sections were stained with haematoxylin and eosin (HE) or with antibodies against placental alkaline phosphatase (PLAP or ALPP) for histological evaluation (Giwercman, A, et al. APMIS 1991, 99:586-94).

A series of 31 of overt testicular tumors were analyzed by immunohistochemistry, including classical seminomas, various non-seminomatous tumor components, spermatocytic seminomas, Leydig cell tumors and a testicular B-cell lymphoma (listed in Table III). Moreover 14 samples of testicular CIS were analyzed: twelve, which were from tissue adjacent to a TGCT, and two, which had not progressed to an overt tumor, but only had signs of micro-invasion. Furthermore, we included 8 samples containing normal testicular tissue from either patients with prostate cancer or from tissue surrounding a GCT. A representative panel of tissues adjacent to the microscopically examined samples (seminomas, nonseminomatous tumors and specimens with CIS) was analyzed by RT-PCR and ISH. RNAs for RT-PCR were isolated from homogeneous tumors and from specimens containing the largest possible number of tubules with CIS, as judged by microscopical examination of adjacent tissue fragments.

The series for immunohistochemical analysis of the ontogeny of expression of AP-2γ in normal and dysgenetic gonads included paraffin embedded specimens from tissue archives of the Rigshospitalet, Copenhagen University Hospital. The 40 normal fetal tissue samples (19 testicular and 21 ovarian specimens) were obtained after induced or spontaneous abortions and stillbirths, mainly due to placental or maternal problems. The developmental age was calculated from the date of the last menstrual bleeding, supported by the foot size of the fetus. Normal postnatal testicular samples (N=16) were obtained either from autopsies of infants who died suddenly of causes unrelated to the reproductive system or as testicular biopsies performed in boys with acute leukemia for monitoring the spread of disease. Pathology specimens comprised a series of 20 overtly dysgenetic gonads from individuals with intersex disorders or other components of the testicular dysgenesis syndrome (TDS)(Skakkebaek, NE, et al. Hum Reprod 2001, 16:972-8), and 12 testicular biopsies with mild dysgenetic features (e.g. undifferentiated tubules and/or microliths) with or without CIS, performed for diagnostic reasons from young adult men with sub-fertility or with contralateral germ cell tumors.

In addition 130 samples from extragonadal tissues were examined. Panels of normal tissues and mammary gland tissues with or without neoplastic lesions were investigated using commercial tissue arrays (MaxArray Human Breast Carcinoma and Human Normal Tissue Microarray slides, Zymed, S. San Francisco, CA, USA). All specimens are listed in Table III.

### Studies in cell lines

The following established cancer cell lines were grown in standard conditions (5% CO₂, 37°C): embryonal carcinoma NT2 and 2102EP lines, and a mammary tumor-derived MCF7 line. Total RNA was isolated for RT-PCR as described below, while for the immunohistochemical staining of the protein, the cells were spun in a cyto-centrifuge or directly grown on microscopic slides.

For analysis of the effect of retinoic acid (RA) on the AP-2γ expression (Andrews, PW Dev Biol 1984, 103:285-93), NT2 cells were grown in DMEM medium (10% FBS, 2 mM L-glutamine, 25 IU/ml penicillin, 25 µg/ml streptomycin) and stimulated with 10 µM RA (Sigma-Aldrich, St. Louis, MO) for 0-15 days to induce differentiation; 2102Ep cells were grown in DMEM medium with added 100 µM β-mercaptoethanol, and stimulated with 10 µM RA for 0-10 days. A possible estrogen regulation of AP-2γ was analyzed in MCF7 cells, which were grown in steroid-depleted DMEM medium (5% dextran-charcoal stripped FBS, 1 x non-essential amino acids, 1 nM insulin, 2 mM L-glutamine, 25 IU/ml penicillin, 25 µg/ml streptomycin) for 6 days and then exposed to 1 nM estradiol, ICI-182,780 (anti-estrogen), or ethanol (vehicle) for 24 or 48 hours.

### RT-PCR

Total RNA was purified using the NucleoSpin RNAII kit as described by the manufacturer (Macherey-Nagel, Düren, Germany). RNA samples were DNAse digested, and cDNA was synthesized using a dT₂₀ primer. Specific primers were designed for *TFAP2C* (TFAP2C-ex6: ATCTTGGAGGACGAAATGAGAT, TFAP2C-ex7: CAGATGGCCTGGCTGCCAA), spanning intron-exon boundaries. RT-PCR was performed in duplex in 30 µl of (final concentrations): 12 mM Tris-HCl, pH 8.3; 50 mM KCl; 1.9 mM MgCl2; 0.1 % Triton X-100; 0.005 % gelatine; 250 µM dNTP; 30 pmol of each primer. For control of PCR load and cDNA synthesis the expression of the marker *gene ACTB* was analyzed with the following primers (ACTB-ex4: ACCCACACTGTGCCCATCTA, ACTB-ex6: ATCAAAGTCCTCGGCCACATT). Cycle conditions for all PCR reactions: 1 cycle of 2 min. at 95°C; 40 cycles of: 30 sec. at 95°C, 1 min. at 62°C, 1 min. at 72°C and finally 1 cycle of 5 min. at 72°C. PCR products were run on 1.5% agarose gels and visualized by ethidium bromide staining. Fragment size was 205 base pairs for *TFAP2C* and 815 for *ACTB.* Representative bands were excised, cloned (with TOPO®Cloning Invitrogen, Carlsbad, CA, USA) and sequenced for verification.

### In situ hybridization (ISH)

Probes for ISH were prepared by use of specific primers (1^{st} primer combination: AAGAGTTTGTTACCTACCTTACT, CATCAATTTGACATTTCAATGGC, 2^{nd} primer combination AATTAACCCTCACTAAAGGGTTAAAGAGCCTTCACT, TAATACGACTCACTATAGGGCTAAGTGTGTGG) containing an added T3- or T7-promotor sequence, respectively (promotor sequences underlined). PCR conditions were: 5 min. 95° C; 5 cycles of: 30 sec. 95°C, 1 min. 45°C, 1 min. 72°C; 20 cycles of 30 sec. 95°C, 1 min. 65°C, 1 min. 72°C and finally 5 min. 72°C. The resulting PCR product was purified on a 2 % low melting point agarose gel and sequenced from both ends, using Cy5-labelled primers complementary to the added T3 and T7 tags. Aliquots of about 200 ng were used for *in vitro* transcription labeling, using the MEGAscript-T3 (sense) or MEGAscript-T7 (anti-sense) kits, as described by the manufacturer (Ambion, Houston, TX, USA). The composition of the 10x-nucleotide mix was: 7.5 mM ATP, GTP and CTP, 3.75 mM UTP, 1.5 mM biotin-labeled UTP. To estimate quantity and labeling efficiencies, aliquots of the labeled RNA product were analyzed by agarose gel electrophoresis, dotted onto nitrocellulose filters and developed. ISH was performed on 3-6 different samples as described previously (Hoei-Hansen, CE, et al. Mol Hum Reprod 2004, 10:423-31).

Immunohistochemistry
A commercially available monoclonal anti-AP-2γ antibody (6E4/4:sc-12762, kindly provided for testing by Santa Cruz Biotechnology Inc., Santa Cruz, CA, USA) was used. The antibody was validated by western blots by the manufacturer. In addition, several other antibodies were used as controls, in particular to detect CIS cells (anti-PLAP, a monoclonal antibody from BioGenex, San Ramon, CA, USA; anti-OCT3/4, C-20, sc 8629, Santa Cruz Biotechnology), and to control for integrity of fetal and infantile testis samples obtained at autopsies (anti-AMH, a monoclonal antibody kindly provided by R. Cate, Biogen). The immunohistochemical staining was performed using a standard indirect peroxidase method, as previously described for other antibodies (Giwercman, A, et al. APMIS 1991, 99:586-94;Rajpert-De Meyts, E, et al. Hum Reprod 2004). Briefly, the dewaxed and rehydrated sections were heated in a microwave oven to unmask the antigen. Sections fixed in formalin or PFA were heated in a 5% urea, pH 8.5, whereas sections fixed in Stieve's fluid were heated in TEG buffer, pH 9.0 (TRIS 6.06g/5L, EGTA 0.95g/5L). Subsequently, the sections were incubated with 0.5% H₂O₂ to inhibit the endogenous peroxidase, followed by diluted non-immune goat serum (Zymed, S. San Francisco, CA, USA) to block unspecific binding sites. The incubation with the primary anti-AP-2γ antibody diluted 1:25 - 1:100 was carried out overnight at 4°C. For tissues fixed in Stieve's fluid the primary antibody was diluted in Background Reducing antibody diluent (DakoCytomation, Glostrup, Denmark). For negative control, a serial section from each block was incubated with a dilution buffer. Subsequently, a secondary biotinylated goat-anti-mouse link antibody was applied (Zymed, S. San Francisco, CA, USA), followed by the horseradish peroxidase-streptavidin complex (Zymed, S. San Francisco, CA, USA). Between all steps the sections were thoroughly washed. The bound antibody was visualized using aminoethyl carbazole substrate (Zymed, S. San Francisco, CA, USA). Most sections were lightly counterstained with Mayer's haematoxylin to mark unstained nuclei.

The sections were examined under a light microscope (Zeiss) and scored systematically by two investigators (CHH and ERM). The staining was assessed using an arbitrary semi-quantitative score of the percentage of cells stained in a section and their staining intensity: +++: strong staining, ++: moderate staining, +: weak staining, +/-: very weak staining, neg: no positive cells detected.

### RESULTS

AP-2γ is a new marker for testicular carcinoma *in situ* and derived germ cell tumors

After the discovery of the high expression of AP-2γ in testicular tissues harboring CIS cells (5), we extensively mapped the expression at the mRNA level by reverse transcriptase PCR (RT-PCR) in a panel of testicular neoplasms and tumor-derived cell lines. Subsequently, we determined cellular localization of AP-2γ transcripts by *in situ* hybridization (ISH), and at the protein level by immunohistochemistry in a comprehensive panel of normal and neoplastic tissues.

RT-PCR showed a high expression of AP-2γ in samples of seminoma, teratoma, embryonal carcinoma and CIS as compared to normal testicular tissue, which showed only a trace of reaction in one biopsy from a patient with an adjacent TGCT, and could encompass a few CIS or micro-invasive tumor cells (Fig. 8). Expression in CIS cells was approximately equivalent in tissue containing CIS adjacent to overt tumors and in CIS at the micro-invasive stage. For a few CIS and tumor samples the expression was confirmed in the database of our earlier study by differential display PCR (DDRT-PCR) (Hoei-Hansen, CE, et al. Mol Hum Reprod 2004, 10:423-31)(results not shown). ISH revealed cytoplasmic staining in CIS cells, seminoma, embryonal carcinoma and to lesser extent in some components of teratomas (Fig. 9). ISH in normal testicular tissue was not completely conclusive; in contrast to IHC, which did not show any detectable protein in normal germ cells, a reaction was observed in some spermatocytes and spermatids (results not shown). The presence of *TFAP2C* transcripts in the normal germ cells with a simultaneous lack of the protein product cannot, therefore, be excluded. This could be due to translational regulation of AP-2γ in the testis. A similar discrepancy between expression at the mRNA and the protein level was previously described for AP-2α in colon cancer (Ropponen, KM, et al. J Clin Pathol 2001, 54:533-8). Immunohistochemistry demonstrated a high abundance of AP-2γ protein in CIS. CIS cells adjacent to TGCTs were uniformly strongly positive for AP-2γ regardless of the tumor type, and equally strong in CIS present in the testis and as an isolated pre-malignant lesion. Gonadoblastoma, a CIS-like pre-malignant lesion of severely dysgenetic gonads was also strongly positive (Fig. 10). Differential expression was however observed in the overt TGCTs: seminomas were strongly positive, whereas a heterogeneous pattern was detected in the non-seminomas (Fig. 9). Embryonal carcinoma demonstrated strongest staining, teratomas displayed staining in some epithelial and stromal elements but many highly differentiated components were negative. In all TGCT specimens staining was present exclusively in the nucleus. No expression was detected in spermatocytic seminoma. In non-germ-cell-derived testicular tumors no expression was detected in a B-cell lymphoma and two Leydig cell tumors (one testicular and one adrenal). One Leydig-Sertoli cell tumor was unexpectedly positive, however, as the staining was confined to the cytoplasm, this may be an unspecific reaction, which is quite frequently observed for various antibodies in the protein and glycoprotein-filled Leydig cells. A similar but much weaker unspecific staining in Leydig cell cytoplasm was also observed in a few specimens of testicular parenchyma. In general, the intensity of staining varied somewhat depending on the fixative used, with stronger staining in tissues fixed with PFA and formalin compared to those fixed in Stieve's and Bouin. In addition, in samples fixed in Stieve's fluid or in high-percentage formalin, there was a tendency to an unspecific staining of erythrocytes. The complete results of the immunohistochemical staining are listed in Table III and representative examples are shown in Figures 9, 10 and 11.

### The expression of AP-2γ is developmentally regulated in human gonads

To investigate whether the high expression of AP-2γ in CIS cells is linked to their gonocyte-like phenotype, as is the case for a number of other markers for CIS cells (Rajpert-De Meyts, E, et al. APMIS 2003, 111:267-78; discussion 278-9), we established the ontogeny of expression of AP-2γ by immunohistochemistry in a panel of specimens of normal human fetal testis and ovaries during development.

The AP-2γ protein was detected in almost all gonocytes in testes from 10 weeks of gestation (earlier samples were not available) until approximately 22 weeks of gestation (Fig. 10), although a few of our analyzed tissue samples failed to show expression. Expression was gradually reduced after week 22, but persisted in a few cells until approximately 4 months of postnatal age. Afterwards, AP-2γ protein was not detected in spermatogonia in normal pre-pubertal, post-pubertal or adult testes.

In fetal ovaries AP-2γ expression was seen in oogonia in high amounts in early gestational ages and to a lesser extent in third trimester. The number of positive oogonia was decreasing with the increasing number of oocytes that entered the meiotic prophase, which were AP-2γ negative. No expression was seen in adult ovary, but only few samples were analyzed.

### Changes of the expression pattern of AP-2γ in intersex and dysgenetic gonads

Subsequently, we asked the question whether or not the normal developmental pattern of the expression of AP-2γ is changed in testicular dysgenesis and disorders of sex differentiation, conditions with a markedly increased risk of germ cell neoplasia. Expression of AP-2γ in dysgenetic gonads was analyzed only by immunohistochemistry, because the specimens were available only as paraffin-embedded blocks from tissue archives. The pattern of expression in these specimens was in general similar to that in the normal testes, but in some samples expression deviated from the observed pattern. Expression of AP-2γ was not observed in a sample from a male fetus gestational age 15 weeks with mosaic isochromosome Y, similar to previously observed lack of OCT3/4 (Rajpert-De Meyts, E, et al. Hum Reprod 2004). Staining for AMH was positive, suggesting that the integrity of proteins should be preserved in the sample. Prolonged expression of AP-2γ was seen in cells resembling gonocytes in testicular cords in an ovotestis from a 14-month old XX hermaphrodite, but not in the oocytes present in the ovarian part. In a few of the dysgenetic samples neoplastic, pre-invasive CIS or gonadoblastoma was present and these showed a high expression of AP-2γ. In three cases staining was observed in foci of germ cells that morphologically were classified as somewhere in between gonocytes and CIS (see Fig. 10). In two of the cases (girls with AIS of age 9 months and 6 years, respectively) PLAP was also positive. The third was from a testis removed from a 2-year old girl with AIS, where a very small focus of AP-2γ positive cells was detected. We were not able to locate this focus morphologically in adjacent sections cut before and after, and stainings with PLAP and OCT3/4 were accordingly negative.

Expression of AP-2γ is associated with cell differentiation or proliferation and is cell type dependent

In order to define the extent of expression and a possible link to hormonal stimulation, expression of AP-2γ was analyzed in some tissues that are endocrinologically activated and in a panel of normal somatic tissues. In normal female mammary tissue expression was seen in a subpopulation of basally situated epithelial duct cells, and expression was also detected in some, but not all, premalignant or manifest malignant changes in the female breast (Fig. 11, Table III). In samples from skin, the basal layer of keratinocytes was positive, and weaker staining could be seen in the adjacent layers of epidermis (Fig. 11), which is in concert with the previously reported pattern. In normal adult kidney AP-2γ expression was seen in a subset of cells in proximal tubules. Finally, AP-2γ expression was present in lung tissue, in pneumocytes lining alveoles. All other analyzed tissue samples were negative (Table III). We cannot exclude that the AP-2γ expression may be developmentally regulated in some tissues, in analogy to the testis. We could not gain access to a full spectrum of human fetal tissues at different developmental stages, but we have examined and found no AP-2γ expression in the following fetal tissues from the first or second trimester of gestation: epididymis, liver, heart, thymus, thyroid gland, kidney and adrenal gland.

Finally, regulation of AP-2γ expression was studied *in vitro* in three cell lines. We showed by RT-PCR that AP-2γ was RA-regulated in NT2 cells in a biphasic manner. Strong expression was observed in cells without RA-induced differentiation, after 3 days of RA treatment (which at that point induced cell proliferation) the expression was somewhat stronger and then declined after 15 days when the cells have differentiated (Fig. 8). A heterogeneous staining for AP-2γ protein was seen in NT2 cells by immunocytochemistry in all analyzed samples, regardless of the length of RA treatment. It was not possible to establish the pattern quantitatively, because of differences in intensity from cell to cell. In contrast, in the 2102Ep cells, which do not differentiate with RA treatment, expression was unaffected by RA treatment at the RNA level, and immunocyto-chemical staining was uniformly strong in all analyzed cells. In MCF7 cells, expression was similar in cells exposed to estradiol, the anti-estrogen ICI, or ethanol, both at the RNA level, and at the protein level, where expression was seen in a subset of cells in all analyzed cells, regardless of treatment (Fig. 11). For examples of RT-PCR results, see Figure 8.

### DISCUSSION

In the present study we comprehensively investigated the pattern of expression of the transcription factor AP-2γ, and established AP-2γ as a novel marker for neoplastic germ cells, including testicular carcinoma *in situ,* the common precursor of TGCTs. Furthermore, we discovered that AP-2γ was developmentally regulated in human gonads, with marked differences between the testes and ovaries.
Our investigations of the pattern of expression of AP-2γ in extra-gonadal normal and neoplastic tissues were largely in concert with previous studies, thus confirming the specificity of the antibody. The transcription factor was preferentially expressed in proliferating immature cells, which retained tissue-specific stem cell characteristics, suggesting a putative oncogenic function of AP-2γ.
In the testis, we found that the AP-2γ protein was expressed in early gonocytes but not in any cell type in the pre- or post-pubertal testis. The expression rapidly declined from approximately 22 weeks of gestation, and was gradually lost while gonocytes differentiated into infantile spermatogonia up to about 4 months of postnatal age, when the final stage of this differentiation takes place, probably stimulated by the hormonal surge at the "mini-puberty" (Andersson, AM, et al. J Clin Endocrinol Metab 1998, 83:675-81;Hadziselimovic, F, et al. J Urol 1986, 136:274-6). Interestingly, in a few pre-pubertal intersex cases, AP-2γ was observed in a subset of gonocytes outside the normal window of expression. This prolonged expression may reflect a delay in differentiation but may perhaps be a marker of the initiation of neoplastic transformation of these immature germ cells into "pre-CIS" cells, which then may further transform to a typical CIS pattern at the onset of puberty. In the ovary, AP-2γ protein was confined to oogonia and was not detectable after meiotic entry in oocytes. This pattern of expression suggests that the protein plays a role exclusively in undifferentiated germ cells. Analyses in cell lines confirmed that AP-2γ was most abundant in undifferentiated cells, as expression of the transcript was down-regulated after a transient induction in AP-2γ-positive NT-2 cells treated with RA for up to 15 days. In cells that do not differentiate (2102Ep) expression was constantly high. Oulad-Abdelghani and colleagues (Oulad-Abdelghani, M, et al. Exp Cell Res 1996, 225:338-47) reported a transient rise in AP-2γ levels with a peak after 12 hours of RA treatment in embryonal carcinoma derived P19 cells, but did not analyze levels beyond 24 hours. An association of AP-2γ with an immature state of germ cells was also observed in overt TGCTs, where classical seminoma and embryonal carcinoma were strongly positive, while highly differentiated somatic elements of teratomas were negative. Spermatocytic seminoma, which is not derived from CIS but from mature spermatogonia, was also AP-2y-negative. Overall, the pattern of expression of AP-2γ in TGCTs resembles that of KIT and OCT-4, which are also associated with an undifferentiated state and pluripotency, typical for cells retaining some stem-cell-like features. The high expression of AP-2γ in CIS supports our hypothesis that this pre-malignant lesion is derived from an early fetal germ cell. In adult testes AP-2γ was only seen either in CIS or in overt TGCTs, or in rare specimens harboring dysgenetic features, and in the latter only in gonadoblastoma and CIS cells. Thus, AP-2γ can be added to a list of markers for germ cell neoplasms that are also present in gonocytes but not detectable in the normal adult testis, such as PLAP, KIT or OCT-4 (Rajpert-De Meyts, E, et al. APMIS 2003, 111:267-78; discussion 278-9).

Besides an inverse association with cell differentiation, which was also noted by others in other cell systems (Jager, R, et al. Mol Cancer Res 2003, 1:921-9), very little is known concerning the biological function and the mechanism of action of AP-2γ, especially in germ cells. The most interesting question is what are the gene targets for AP-2γ in germ cells, and with which other proteins does this transcription factor interact. In other cell types AP-2y has been previously shown to interact with a number of genes that are also highly expressed in CIS cells, e.g. IGF-binding protein 5 (Jager, R, et al. Mol Cancer Res 2003, 1:921-9) or KIT (Rajpert-De Meyts, E, et al. Int J Androl 1994, 17:85-92). KIT appears to be of particular interest, as it was reported to be down-regulated in several types of tumors derived from tissues that express AP-2γ, e.g. breast cancer, colorectal cancer or melanoma (Ropponen, KM, et al. J Clin Pathol 2001, 54:533-8).

Estrogen signaling is another possible candidate pathway which could be regulated by AP-2y, as is suggested by its expression in the epithelium of milk ducts and the association with some forms of breast cancer. While the classic estrogen receptor (ERα) is not expressed in the testis (neither in the fetal life or adulthood), the ERβ is expressed in human fetal testis in the second trimester, and gonocytes are potential targets for estrogen action. This is of interest because it has been suggested that inappropriate exposure to estrogens during fetal life may have an impact on the development of CIS and TGCTs (Skakkebaek, NE, et al. Hum Reprod 2001, 16:972-8). Furthermore, the progression from CIS into an overt TGCT occurs after the endocrinological surge of puberty, when the intra-testicular levels of steroid hormones rise markedly. In the current study, we have analyzed the expression of AP-2γ in a breast tumor-derived cell line, MCF7, which is estrogen dependent. We have not observed any changes in expression, neither after treatment with estradiol, nor an anti-estrogen. This is in disagreement with a recent study (Orso, F, et al. Biochem J 2004, 377:429-38), which showed induction of AP-2γ by estrogens in several breast tumor cell lines. Whether AP-2γ is indeed involved in estrogen signaling remains an unresolved issue, since existing reports are partially contradictory and will require further investigations. We need also to keep in mind that the function of AP-2γ may be cell-type specific, as was demonstrated recently for the transcriptional activation of the *ERBB2* gene (Vernimmen, D, et al. Br J Cancer 2003, 89:899-906) and thus may be different in germ cells compared to somatic cell types.

In summary, our investigations suggest a role for AP-2γ in pathways regulating cell differentiation, including in fetal gonocytes, which was not previously known. Frequent expression of AP-2γ in various malignancies suggests that the pathways may be involved in oncogenesis. Finally, developmental expression of this transcription factor in the testis makes AP-2γ a very promising new marker for diagnosis of early stages of germ cell neoplasia. Example 10
Early diagnosis of pre-invasive testicular germ cell neoplasia by immunocytological detection of AP-2γ-positive cells in semen samples

In the above genome-wide gene expression profiling study of CIS cells, we have identified a number of genes expressed also in embryonic stem cells and foetal gonocytes but not in adult germ cells, thus providing many possible new markers for CIS.

One of such genes was TFAP2C (mapped to chromosome 20q13.2), which encodes the transcription factor activator protein-2 (AP-2γ). We established AP-2γ as a novel marker for foetal gonocytes and neoplastic germ cells, including testicular CIS, with a role in pathways regulating cell differentiation and a possible involvement in oncogenesis. The fact that AP-2y protein was not expressed in normal adult reproductive tract but was abundant in nuclei of CIS and tumour cells, which are better protected than the cytoplasm from degradation in semen due to a structural strength, prompted us to analyse the value of AP-2γ for detection of CIS and/or tumour cells in ejaculates in a series of patients and controls. The local ethics committee approved the study and participants gave written informed consent.

The present example presents a new non-invasive method capable of detecting testicular cancer at the pre-invasive carcinoma in situ (CIS) in a semen sample. The assay is based on immunocytological staining of semen samples for transcription factor AP-2γ, previously identified as a marker for neoplastic germ cells.

In an investigation of 104 andrological patients, cells positive for AP-2γ were found only in semen samples from patients diagnosed a priori with testicular neoplasms and in a 23-year-old control subject with oligozoospermia and no symptoms of a germ cell tumour (fig. 7). Testicular biopsies performed during the follow-up of this patient revealed widespread CIS in one testis, thus proving a diagnostic value of the new assay. Optimisation of the method is in progress, but a preliminary assessment gives promise that the method may have a place in screening of germ cell neoplasia in at-risk patients.

The number of patients that we have screened for the presence of immunoreactive AP-2γ is continuously increasing as we are testing patients as they appear in our clinic. So far we have tested 347 andrological patients (including the 104 mentioned above) and found CIS-like cells positive for AP-2γ in semen samples of 3 patients, including the first case described above (a 23-year-old subfertile control subject with widespread CIS confirmed by testicular biopsies). Two other patients are currently under clinical follow-up and being considered for testicular biopses.

### Materials and methods

A semen sample from each patient was diluted if necessary, divided in portions of 100 µl, spun down on a microscope slide with 400 µl PBS buffer, dried, fixed 10 min in formalin, washed and dried again. The immunocytochemical staining was performed with the monoclonal anti-AP-2γ antibody (6E4/4:sc-12762, Santa Cruz Biotechnology Inc., Santa Cruz, CA, USA) using a standard indirect peroxidase method, as previously described (Almstrup, K, et al. Cancer Res 2004, 64:4736-43), except for small modifications tested using control semen samples spiked with AP-2γ-positive seminoma cells (Fig. 7A). For negative control, another cytospin was incubated with a dilution buffer, and for positive control AP-2γ-positive seminoma cells were used. Staining was scored using an arbitrary 0-5 scale based on the intensity and morphological resemblance of stained elements to CIS or tumour cells: Score 0 - no staining, 1 - negative with some unspecific particles stained, 2 - trace reaction in small fragments of nucleus-resembling structures, 3 - clear staining in a part of a nucleus, with correct morphology, 4 - clear staining in a whole nucleus, with correct morphology, 5 - two or more cells with score 4. Scores 0-2 were classified as negative, while 3-5 were classified as positive.

### Results

At the evaluation of one of the first series of semen samples, we detected six AP-2y-positive cells in a sample from one of the control participants (Fig. 7B). He was a 23-year-old man that presented for routine semen analysis due to the infertility problem in the couple. The presence of one small testis and a very poor semen quality in combination with the finding of AP-2γ stained cells constituted a well-justified indication for bilateral open testicular biopsies, which were performed in agreement with the patient. The right-sided biopsy demonstrated normal testicular tissue, whereas the left-sided biopsy revealed numerous tubules containing CIS, which was confirmed with PLAP and AP-2γ staining (Fig.7D, E). The patient was advised to undergo unilateral orchidectomy, prior to which semen was cryopreserved.

A preliminary assessemnet of the value of the AP-2γ immunoassay was subsequently done after analysis of semen samples from 104 patients: 12 patients with CIS or testicular cancer prior to orchidectomy (with a subsequently verified germ cell neoplasm), 7 testicular cancer patients after operation (none had contralateral CIS), 12 patients with other diseases (e.g. referred for semen cryopreservation before treatment for non-testicular cancers and diseases) and 73 healthy or subfertile control patients.

The results are summarised in Table II and examples are shown in Fig. 7. We detected AP-2γ-positive cells in 5 of 12 patients harbouring a priori diagnosed testicular neoplasia, giving a sensitivity of our assay of 42%. However, after addition of the newly diagnosed control patient with CIS, the sensitivity is 46% (95% CI, 23 to 71%). It is especially noteworthy that we did not detect false positive staining among the healthy controls and patients with other cancers, suggesting a high specificity of the assay.

The large proportion of negative results in the testicular cancer patients could be due to a small volume of the semen sample used for the test (50-300 µl out of total normal volume 3-5 ml, depending on concentration and number of samples), thus stressing the need for either repeated analysis or larger volume analysed. Negative results could also be due to compression from the tumour causing obstruction of seminiferous tubules resulting in few or no CIS cells shed into semen. However, considering the variability of the number of CIS cells present in the tissue adjacent to a tumour, the sensitivity of our assay is reasonably good, as nearly half of the patients harbouring testicular germ cell neoplasm have been detected in this preliminary study.

The relatively low sensitivity nevertheless leaves room for improvement and further optimisation of the method is currently in progress. The results of planned studies in a wider panel of patients will confirm whether this assay should be offered widely to patients at risk for TGCT as a method of non-invasive screening for CIS. Such screening could be performed, for example, at andrology and fertility clinics in young men with atrophic testes, history of cryptorchidism or in need for an assisted reproduction technique. If diagnosis of testicular cancer is made at the pre-invasive CIS stage, the patient can be offered the most gentle and optimal treatment.

### References

Almstrup, K, Hoei-Hansen, CE, Wirkner, U, Blake, J, Schwager, C, Ansorge, W, Nielsen, JE, Skakkebaek, NE, Rajpert-De Meyts, E and Leffers, H, 2004, Embryonic stem cell-like features of testicular carcinoma in situ revealed by genome-wide gene expression profiling Cancer Res 64:4736-43

Andersson, AM, Toppari, J, Haavisto, AM, Petersen, JH, Simell, T, Simell, O and Skakkebaek, NE, 1998, Longitudinal reproductive hormone profiles in infants: peak of inhibin B levels in infant boys exceeds levels in adult men J Clin Endocrinol Metab 83:675-81

Andrews, PW, 1984, Retinoic acid induces neuronal differentiation of a cloned human embryonal carcinoma cell line in vitro Dev Biol 103:285-93

Draper, JS, Smith, K, Gokhale, P, Moore, HD, Maltby, E, Johnson, J, Meisner, L, Zwaka, TP, Thomson, JA and Andrews, PW, 2004, Recurrent gain of chromosomes 17q and 12 in cultured human embryonic stem cells Nat Biotechnol 22:53-4

Giwercman, A, Cantell, L and Marks, A, 1991, Placental-like alkaline phosphatase as a marker of carcinoma-in-situ of the testis. Comparison with monoclonal antibodies M2A and 43-9F APMIS 99:586-94

Hadziselimovic, F, Thommen, L, Girard, J and Herzog, B, 1986, The significance of postnatal gonadotropin surge for testicular development in normal and cryptorchid testes J Urol 136:274-6

Hoei-Hansen, CE, Nielsen, JE, Almstrup, K, Hansen, MA, Skakkebaek, NE, Rajpert-DeMeyts, E and Leffers, H, 2004, Identification of genes differentially expressed in testes containing carcinoma in situ Mol Hum Reprod 10:423-31

Jager, R, Werling, U, Rimpf, S, Jacob, A and Schorle, H, 2003, Transcription factor AP-2gamma stimulates proliferation and apoptosis and impairs differentiation in a transgenic model Mol Cancer Res 1:921-9

Jahnukainen, K, JorgensenN, Pollanen, P, Giwercman, A and Skakkebaek, NE, 1995, Incidence of testicular mononuclear cell infiltrates in normal human males and in patients with germ cell neoplasia Int J Androl 18:313-20

Jorgensen, M, Bevort, M, Kledal, TS, Hansen, BV, Dalgaard, M and Leffers, H, 1999, Differential display competitive polymerase chain reaction: an optimal tool for assaying gene expression Electrophoresis 20:230-40

Manivel, JC, Jessurun, J, Wick, MR and Dehner, LP, 1987, Placental alkaline phosphatase immunoreactivity in testicular germ-cell neoplasms Am J Surg Pathol 11:21-9

Nielsen, JE, Hansen, MA, Jorgensen, M, Tanaka, M, Almstrup, K, Skakkebaek, NE and Leffers, H, 2003, Germ cell differentiation-dependent and stage-specific expression of LANCL1 in rodent testis Eur J Histochem 47:215-22

Orso, F, Cottone, E, Hasleton, MD, Ibbitt, JC, Sismondi, P, Hurst, HC and De Bortoli, M, 2004, Activator protein-2gamma (AP-2gamma) expression is specifically induced by oestrogens through binding of the oestrogen receptor to a canonical element within the 5'-untranslated region Biochem J 377:429-38

Oulad-Abdelghani, M, Bouillet, P, Chazaud, C, Dolle, P and Chambon, P, 1996, AP-2.2: a novel AP-2-related transcription factor induced by retinoic acid during differentiation of P19 embryonal carcinoma cells Exp Cell Res 225:338-47

Pera, MF, 2004, Unnatural selection of cultured human ES cells? Nat Biotechnol 22:42-3

Rajpert-De Meyts, E, Bartkova, J, Samson, M, Hoei-Hansen, CE, Frydelund-Larsen, L, Bartek, J and Skakkebaek, NE, 2003, The emerging phenotype of the testicular carcinoma in situ germ cell APMIS 111:267-78; discussion 278-9

Rajpert-De Meyts, E, Hanstein, R, Jorgensen, N, Graem, N, Vogt, PH and Skakkebaek, NE, 2004, Developmental expression of POU5F1 (OCT-3/4) in normal and dysgenetic human gonads Hum Reprod

Rajpert-De Meyts, E and Skakkebaek, NE, 1994, Expression of the c-kit protein product in carcinoma-in-situ and invasive testicular germ cell tumours Int J Androl 17:85-92

Ramalho-Santos, M, Yoon, S, Matsuzaki, Y, Mulligan, RC and Melton, DA, 2002, "Stemness": transcriptional profiling of embryonic and adult stem cells Science 298:597-600

Ropponen, KM, Kellokoski, JK, Pirinen, RT, Moisio, KI, Eskelinen, MJ, Alhava, EM and Kosma, VM, 2001, Expression of transcription factor AP-2 in colorectal adenomas and adenocarcinomas; comparison of immunohistochemistry and in situ hybridisation J Clin Pathol 54:533-8

Sato, N, Sanjuan, IM, Heke, M, Uchida, M, Naef, F and Brivanlou, AH, 2003, Molecular signature of human embryonic stem cells and its comparison with the mouse Dev Biol 260:404-13

Skakkebaek, NE, Rajpert-De Meyts, E and Main, KM, 2001, Testicular dysgenesis syndrome: an increasingly common developmental disorder with environmental aspects Hum Reprod 16:972-8

Sperger, JM, Chen, X, Draper, JS, Antosiewicz, JE, Chon, CH, Jones, SB, Brooks, JD, Andrews, PW, Brown, PO and Thomson, JA, 2003, Gene expression patterns in human embryonic stem cells and human pluripotent germ cell tumors Proc Natl Acad Sci U S A 100:13350-5

Vernimmen, D, Gueders, M, Pisvin, S, Delvenne, P and Winkler, R, 2003, Different mechanisms are implicated in ERBB2 gene overexpression in breast and in other cancers Br J Cancer 89:899-906

### Items

1. A method for determining the presence of precursors of germ cell tumours in a sample, said method comprising:
   a) determining the expression profile of a group of markers in said sample, wherein the group of markers consists of at least one marker selected independently from the markers listed in table 1;
   b) comparing said expression profile with a reference expression profile;
   c) identifying whether the expression profile is different from said reference expression profile and
   evaluating whether the sample contains precursors of germ cell tumours if the expression profile is different from the reference expression profile.
2. A method for determining the presence of precursors of germ cell tumours in a sample, said method comprising:
   a) determining the intensity signal of the proteins encoded by each of the members of the group of markers in said sample, wherein the group of markers consists of at least one marker selected independently from the markers listed in table 1
   b) comparing each individual intensity signal with a corresponding reference intensity signal;
   c) identifying whether each individual intensity signal is different from said corresponding reference intensity signal and
   evaluating whether the sample contains precursors of germ cell tumours if the intensity signal is different from the corresponding reference intensity signal.
3. A method according to any of items 1-2, wherein the group of markers is selected from SEQ ID NO 1-157.
4. A method according to any of items 1-3, wherein the group of markers consists of at least one marker which in a sample with CIS in 100% of the seminiferous tubules is up-regulated more than 7 fold when compared to a reference sample.
5. A method according to any of items 1-4, wherein the expression profile distinguishes between classical seminoma and non-seminoma, in gonads or in extra-gonadal localizations.
6. A method for monitoring progression from precursors of germ cell tumours to overt cancer cells in an individual, comprising
   a) determining the expression profile of a group of markers in said sample, wherein the group of markers consists of at least one marker selected independently from the markers listed in table 1;
   b) comparing said expression profile with a reference expression profile;
   c) identifying whether the expression profile is different from said reference expression profile and
      evaluating the development stage of precursors of germ cell tumours to overt cancer cells if the expression profile is different from the reference expression profile.
7. A method for monitoring progression from precursors of germ cell tumours to overt cancer including in an individual, comprising
   a) determining the intensity signal of the proteins encoded by each of the members of the group of markers in said sample, wherein the group of markers consists of at least one marker selected independently from the markers listed in table 1
   b) comparing each individual intensity signal with a corresponding reference intensity signal;
   c) identifying whether each individual intensity signal is different from said corresponding reference intensity signal and
      evaluating the development stage of precursors of germ cell tumours to overt cancer cells if the intensity signal is different from the corresponding reference intensity signal.
8. A method according to any of items 6 or 7, wherein said method distinguish seminoma from non-seminoma.
9. A method according to any of items 6 or 7, wherein said method distinguish seminoma from CIS.
10. A method according to any of items 6 or 7, wherein said method distinguish non-seminoma from CIS.
11. A method of identifying precursors of germ cell tumours in a sample from a mammal, the method comprising
   assaying said sample by a quantitative detection assay and determining the expression profile and/or the intensity signal(s) of at least one marker selected from the group consisting of
      SEQ ID NO: 1 to SEQ ID NO: 255
   comparing said expression profile and/or the intensity signal(s) with reference value(s)
   identifying whether the expression profile and/or the intensity signal(s) of at least one marker from the sample is significantly different from the reference value, and
   evaluating whether the sample contains precursors of germ cell tumours if the expression profile and/or the intensity signal(s) is different from the reference value.
12. A method for determining the presence of precursors of germ cell tumours in an individual comprising detecting the presence or absence of at least one expression product,
   wherein said at least one expression product comprise a nucleotide sequence selected from the group consisting of SEQ ID 1-255 in a sample isolated from the individual.
13. A method of assessing the efficacy of a therapy for inhibiting precursors of germ cell tumours in a subject, the method comprising
   comparing the expression profile of a group of markers in a first sample obtained from the subject prior to providing at least a portion of the therapy to the subject and the expression profile of a group of markers in a second sample obtained from the subject following provision of the portion of the therapy, wherein a significantly altered expression profile of the marker(s) in the second sample, relative to the first sample, is an indication that the therapy is efficacious for inhibiting precursors of germ cell tumours in the subject
   wherein the group of markers consists of markers selected independently from the markers listed in table 1 and whereby the number of markers in the group is between 1 and 255.
14. A method of assessing the efficacy of a therapy for inhibiting precursors of germ cell tumours in a subject, the method comprising
   comparing the intensity signal of the proteins encoded by a group of markers in a first sample obtained from the subject prior to providing at least a portion of the therapy to the subject and the intensity signal of the proteins encoded by a group of markers in a second sample obtained from the subject following provision of the portion of the therapy, wherein a significantly altered intensity signal of the proteins encoded by the marker(s) in the second sample, relative to the first sample, is an indication that the therapy is efficacious for inhibiting precursors of germ cell tumours in the subject
      wherein the group of markers consists of markers selected independently from the markers listed in table 1 and whereby the number of markers in the group is between 1 and 255.
15. A method for analysing the presence of undifferentiated stem cells highly likely to progress to precursors of germ cell tumours and/or cancer in an individual, said method comprising
   a) determining the expression profile of a group of markers in said sample, wherein the group of markers consists of at least one marker selected independently from the markers listed in table 1;
   b) comparing said expression profile with a reference expression profile;
   c) identifying whether the expression profile is different from said reference expression profile and
   evaluating whether the sample contains undifferentiated stem cells highly likely to progress to precursors of germ cell tumours and/or cancer in the individual if the expression profile is different from the reference expression profile.
16. A method for analysing the presence of undifferentiated stem cells highly likely to progress to precursors of germ cell tumours and/or cancer in an individual, said method comprising
   a) determining the intensity signal of the proteins encoded by each of the members of the group of markers in said sample, wherein the group of markers consists of at least one marker selected independently from the markers listed in table 1
   b) comparing each individual intensity signal with a corresponding reference intensity signal;
   c) identifying whether each individual intensity signal is different from said corresponding reference intensity signal and
   evaluating whether the sample contains undifferentiated stem cells highly likely to progress to precursors of germ cell tumours and/or cancer in the individual if the intensity signal is different from the reference expression profile.
17. A method according to any of the preceding items, wherein the marker is selected from the group consisting of AP-2γ, PIM-2, TCL1A, NANOG and E-cadherin.
18. A method according to any of the preceding items, wherein the marker is AP-2γ.
19. A method according to any of the preceding items, wherein the marker is TCL1A.
20. A method according to any of the preceding items, wherein the marker is NANOG.
21. A method according to any of the preceding items, wherein the marker is PIM-2.
22. A method according to any of the preceding items, wherein the marker is E-cadherin.
23. A method according to item 16, wherein the intensity signal is determined by immunocytological detection.
24. Use of a method according to any of items 1 or 2 for screening for the presence of precursors of germ cell tumours in individual of a population.
25. A kit for use in an assay as defined in any one of items 1-23.

## Claims

1. A method for determining the presence of precursors of germ cell tumours in a sample, said method comprising:
a) determining the level of transcripts of a marker in said sample, wherein the marker is NANOG;
b) comparing said level of transcripts with a reference level of transcripts;
c) identifying whether the level of transcripts is different from said reference level of transcripts and
evaluating whether the sample contains precursors of germ cell tumours if the level of transcripts is different from the reference level of transcripts.

2. A method for determining the presence of precursors of germ cell tumours in a sample, said method comprising:
a) determining the intensity signal of the protein encoded by a marker in said sample, wherein the marker is NANOG;
b) comparing the intensity signal with a corresponding reference intensity signal;
c) identifying whether the intensity signal is different from said corresponding reference intensity signal and
evaluating whether the sample contains precursors of germ cell tumours if the intensity signal is different from the corresponding reference intensity signal.

3. A method according to any of claims 1-2, wherein the marker is set forth in SEQ ID NO 1.

4. A method according to any of claims 1-3, wherein the level of transcripts and/or intensity signal distinguishes between classical seminoma and non-seminoma, in gonads or in extra-gonadal localizations.

5. A method for monitoring progression from precursors of germ cell tumours to overt cancer cells in an individual, comprising
a) determining the level of transcripts of a marker in a sample, wherein the marker is NANOG;
b) comparing said level of transcripts with a reference level of transcripts;
c) identifying whether the level of transcripts is different from said reference level of transcripts and
evaluating the development stage of precursors of germ cell tumours to overt cancer cells if the level of transcripts is different from the reference level of transcripts.

6. A method for monitoring progression from precursors of germ cell tumours to overt cancer in an individual, comprising
a) determining the intensity signal of the protein encoded by a marker in a sample, wherein the marker is NANOG;
b) comparing the intensity signal with a corresponding reference intensity signal;
c) identifying whether the intensity signal is different from said corresponding reference intensity signal and
evaluating the development stage of precursors of germ cell tumours to overt cancer cells if the intensity signal is different from the corresponding reference intensity signal.

7. A method according to any of claims 5 or 6, wherein said method distinguish seminoma from non-seminoma.

8. A method according to any of claims 5 or 6, wherein said method distinguish seminoma from CIS.

9. A method according to any of claims 5 or 6, wherein said method distinguish non-seminoma from CIS.

10. A method of identifying precursors of germ cell tumours in a sample from a mammal, the method comprising
assaying said sample by a quantitative detection assay and determining the level of transcripts and/or the intensity signal of a marker, wherein said marker is NANOG;
comparing said level of transcripts and/or the intensity signal with a reference value
identifying whether the level of transcripts and/or the intensity signal of the marker from the sample is significantly different from the reference value, and
evaluating whether the sample contains precursors of germ cell tumours if the level of transcripts and/or the intensity signal is different from the reference value.

11. A method for determining the presence of precursors of germ cell tumours in an individual comprising detecting the presence or absence of an expression product, wherein said expression product comprise a nucleotide sequence from NANOG in a sample isolated from the individual.

12. A method of assessing the efficacy of a therapy for inhibiting precursors of germ cell tumours in a subject, the method comprising
comparing the level of transcripts a marker in a first sample obtained from the subject prior to providing at least a portion of the therapy to the subject and the level of transcripts of a marker in a second sample obtained from the subject following provision of the portion of the therapy, wherein a significantly altered level of transcripts of the marker in the second sample, relative to the first sample, is an indication that the therapy is efficacious for inhibiting precursors of germ cell tumours in the subject
wherein the marker is NANOG.

13. A method of assessing the efficacy of a therapy for inhibiting precursors of germ cell tumours in a subject, the method comprising
comparing the intensity signal of the protein encoded by a marker in a first sample obtained from the subject prior to providing at least a portion of the therapy to the subject and the intensity signal of the proteins encoded by a marker in a second sample obtained from the subject following provision of the portion of the therapy, wherein a significantly altered intensity signal of the protein encoded by the marker in the second sample, relative to the first sample, is an indication that the therapy is efficacious for inhibiting precursors of germ cell tumours in the subject
wherein the marker is NANOG.

14. A method for analysing the presence of undifferentiated stem cells highly likely to progress to precursors of germ cell tumours and/or cancer in an individual, said method comprising
a) determining the level of transcripts of a marker in a sample, wherein the marker is NANOG;
b) comparing said level of transcripts with a reference level of transcripts;
c) identifying whether the level of transcripts is different from said reference level of transcripts and
evaluating whether the sample contains undifferentiated stem cells highly likely to progress to precursors of germ cell tumours and/or cancer in the individual if the level of transcripts is different from the reference level of transcripts.

15. A method for analysing the presence of undifferentiated stem cells highly likely to progress to precursors of germ cell tumours and/or cancer in an individual, said method comprising
a) determining the intensity signal of the protein encoded by a marker in said sample, wherein the marker is NANOG,
b) comparing the intensity signal with a corresponding reference intensity signal;
c) identifying whether the intensity signal is different from said corresponding reference intensity signal and
evaluating whether the sample contains undifferentiated stem cells highly likely to progress to precursors of germ cell tumours and/or cancer in the individual if the intensity signal is different from the reference level of transcripts.

16. A method according to claim 15, wherein the intensity signal is determined by immunocytological detection.

17. Use of a method according to any of claims 1 or 2 for screening for the presence of precursors of germ cell tumours in individual of a population.
